(19) Europäisches Patentamt
European Patent Office
Office européen des brevets

(11) EP 3 949 987 A1

(12) # EUROPEAN PATENT APPLICATION
published in accordance with Art. 153(4) EPC

(43) Date of publication:
**09.02.2022 Bulletin 2022/06**

(21) Application number: **20778080.0**

(22) Date of filing: **24.03.2020**

(51) International Patent Classification (IPC):
*A61K 39/395* (2006.01) *A61K 45/00* (2006.01)
*A61P 35/00* (2006.01) *C07K 1/22* (2006.01)
*C07K 7/06* (2006.01) *C07K 7/08* (2006.01)
*C07K 16/28* (2006.01) *C07K 16/46* (2006.01)
*C12N 5/10* (2006.01) *A61K 47/68* (2017.01)
*C12N 15/13* (2006.01) *C12N 15/63* (2006.01)
*A61K 31/4192* (2006.01) *A61K 31/551* (2006.01)
*C12P 21/08* (2006.01)

(52) Cooperative Patent Classification (CPC):
**A61K 31/4192; A61K 31/551; A61K 39/395; A61K 45/00; A61K 47/68; A61P 35/00; C07K 1/22; C07K 7/06; C07K 7/08; C07K 16/28; C07K 16/46; C12N 5/10; C12N 15/63**

(86) International application number:
**PCT/JP2020/012885**

(87) International publication number:
**WO 2020/196475 (01.10.2020 Gazette 2020/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.03.2019 JP 2019057128**

(71) Applicant: **Daiichi Sankyo Company, Limited**
**Tokyo 103-8426 (JP)**

(72) Inventors:
- **HARADA Naoya**
**Tokyo 103-8426 (JP)**
- **YONEDA Kozo**
**Tokyo 103-8426 (JP)**
- **HAYAKAWA Ichiro**
**Tokyo 103-8426 (JP)**

(74) Representative: **Wallace, Sheila Jane**
**Marks & Clerk LLP**
**15 Fetter Lane**
**London EC4A 1BW (GB)**

(54) **ANTI-HER2 ANTIBODY-PYRROLOBENZODIAZEPINE DERIVATIVE CONJUGATE**

(57) A novel anti-HER2 antibody-pyrrolobenzodiazepine (PBD) derivative conjugate, a medicine having therapeutic effect against tumor with the antibody-drug conjugate, and a method for treating a tumor by using the antibody-drug conjugate or medicine.

[Figure 1]

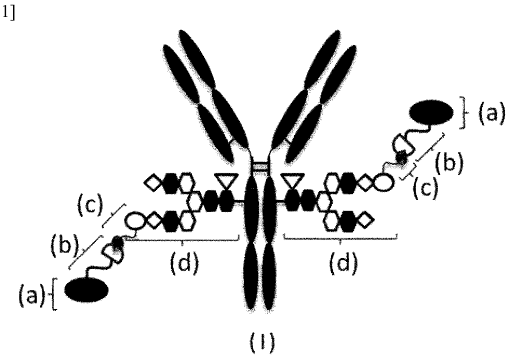


EP 3 949 987 A1

SEQUENCE LISTING

<110> DAIICHI SANKYO COMPANY, LIMITED

<120> Anti-HER2 antibody-Pyrrolobenzodiazepine Derivatives Conjugate

<130> SAP-858-PCT

<141> 2020-03-24

<150> JP 2019057128
<151> 2019-03-25

<160> 34

<170> PatentIn version 3.5

<210> 1
<211> 8
<212> PRT
<213> Mus musculus

<400> 1

Gly Phe Asn Ile Lys Asp Thr Tyr
1 5

<210> 2
<211> 8
<212> PRT
<213> Mus musculus

<400> 2

Ile Tyr Pro Thr Asn Gly Tyr Thr
1 5

<210> 3
<211> 13
<212> PRT
<213> Mus musculus

<400> 3

Ser Arg Trp Gly Gly Asp Gly Phe Tyr Ala Met Asp Tyr
1 5 10

<210> 4
<211> 13
<212> PRT
<213> Artificial Sequence

<220>
<223> CDRH3 of H01

<400> 4

Ser Arg Trp Gly Gly Asp Gly Phe Phe Ala Met Asp Tyr
1 5 10

<210> 5
<211> 6
<212> PRT
<213> Mus musculus

<400> 5

Gln Asp Val Asn Thr Ala
1 5

<210> 6
<211> 7
<212> PRT
<213> Mus musculus

<400> 6

Ser Ala Ser Phe Leu Tyr Ser
1 5

<210> 7
<211> 9
<212> PRT
<213> Mus musculus

<400> 7

Gln Gln His Tyr Thr Thr Pro Pro Thr
1 5

<210> 8
<211> 9
<212> PRT
<213> Artificial Sequence

<220>
<223> CDRL3 of L02/L05

<400> 8

Gln Gln His Ala Thr Thr Pro Pro Thr
1 5

<210> 9
<211> 449
<212> DNA
<213> Homo sapiens

<400> 9
gcctccggac tctagagcca ccatggtgct gcagacccag gtgttcatct ccctgctgct 60

gtggatctcc ggcgcgtacg gcgatatcgt gatgattaaa cgtacggtgg ccgccccctc 120

cgtgttcatc ttccccccct ccgacgagca gctgaagtcc ggcaccgcct ccgtggtgtg 180

cctgctgaat aacttctacc ccagagaggc caaggtgcag tggaaggtgg acaacgccct 240

gcagtccggg aactcccagg agagcgtgac cgagcaggac agcaaggaca gcacctacag 300

cctgagcagc accctgaccc tgagcaaagc cgactacgag aagcacaagg tgtacgcctg 360

cgaggtgacc caccagggcc tgagctcccc cgtcaccaag agcttcaaca gggggagtg 420

ttaggggccc gtttaaacgg gggaggcta 449

<210> 10
<211> 1137
<212> DNA
<213> Homo sapiens

<400> 10
ccagcctccg gactctagag ccaccatgaa acacctgtgg ttcttcctcc tgctggtggc 60

agctcccaga tgggtgctga gccaggtgca attgtgcagg cggttagctc agcctccacc 120

aagggcccaa gcgtcttccc cctggcaccc tcctccaaga gcacctctgg cggcacagcc 180

gccctgggct gcctggtcaa ggactacttc cccgaacccg tgaccgtgag ctggaactca 240

ggcgccctga ccagcggcgt gcacaccttc cccgctgtcc tgcagtcctc aggactctac 300

tccctcagca gcgtggtgac cgtgccctcc agcagcttgg gcacccagac ctacatctgc 360

aacgtgaatc acaagcccag caacaccaag gtggacaaga gagttgagcc caaatcttgt 420

gacaaaactc acacatgccc accctgccca gcacctgaag ccgcgggggg accctcagtc 480

ttcctcttcc ccccaaaacc caaggacacc ctcatgatct cccggacccc tgaggtcaca 540

tgcgtggtgg tggacgtgag ccacgaagac cctgaggtca agttcaactg gtacgtggac 600

ggcgtggagg tgcataatgc caagacaaag ccccgggagg agcagtacaa cagcacgtac 660

cgggtggtca gcgtcctcac cgtcctgcac caggactggc tgaatggcaa ggagtacaag 720

tgcaaggtct ccaacaaagc cctcccagcc cccatcgaga aaaccatctc caaagccaaa 780

ggccagcccc gggaaccaca ggtgtacacc ctgcccccat cccgggagga gatgaccaag 840

aaccaggtca gcctgacctg cctggtcaaa ggcttctatc ccagcgacat cgccgtggag 900

tgggagagca atggccagcc cgagaacaac tacaagacca cccctcccgt gctggactcc 960

gacggctcct tcttcctcta cagcaagctc accgtggaca agagcaggtg gcagcagggc 1020

aacgtcttct catgctccgt gatgcatgag gctctgcaca accactacac ccagaagagc 1080

ctctccctgt ctcccggcaa atgagatatc gggcccgttt aaacggggga ggctaac 1137

<210> 11
<211> 469
<212> PRT
<213> Artificial Sequence

<220>
<223> Trastuzumab A-Hwt aa

<400> 11

Met Lys His Leu Trp Phe Phe Leu Leu Leu Val Ala Ala Pro Arg Trp
1 5 10 15

Val Leu Ser Glu Val Gln Leu Val Glu Ser Gly Gly Gly Leu Val Gln
20 25 30

Pro Gly Gly Ser Leu Arg Leu Ser Cys Ala Ala Ser Gly Phe Asn Ile
35 40 45

Lys Asp Thr Tyr Ile His Trp Val Arg Gln Ala Pro Gly Lys Gly Leu
50 55 60

Glu Trp Val Ala Arg Ile Tyr Pro Thr Asn Gly Tyr Thr Arg Tyr Ala
65 70 75 80

Asp Ser Val Lys Gly Arg Phe Thr Ile Ser Ala Asp Thr Ser Lys Asn
85 90 95

Thr Ala Tyr Leu Gln Met Asn Ser Leu Arg Ala Glu Asp Thr Ala Val
100 105 110

Tyr Tyr Cys Ser Arg Trp Gly Gly Asp Gly Phe Tyr Ala Met Asp Tyr
115 120 125

Trp Gly Gln Gly Thr Leu Val Thr Val Ser Ser Ala Ser Thr Lys Gly
130 135 140

Pro Ser Val Phe Pro Leu Ala Pro Ser Ser Lys Ser Thr Ser Gly Gly
145 150 155 160

Thr Ala Ala Leu Gly Cys Leu Val Lys Asp Tyr Phe Pro Glu Pro Val
165 170 175

Thr Val Ser Trp Asn Ser Gly Ala Leu Thr Ser Gly Val His Thr Phe
180 185 190

Pro Ala Val Leu Gln Ser Ser Gly Leu Tyr Ser Leu Ser Ser Val Val
195 200 205

Thr Val Pro Ser Ser Ser Leu Gly Thr Gln Thr Tyr Ile Cys Asn Val
210 215 220

Asn His Lys Pro Ser Asn Thr Lys Val Asp Lys Arg Val Glu Pro Lys
225 230 235 240

Ser Cys Asp Lys Thr His Thr Cys Pro Pro Cys Pro Ala Pro Glu Ala
245 250 255

Ala Gly Gly Pro Ser Val Phe Leu Phe Pro Pro Lys Pro Lys Asp Thr
260 265 270

Leu Met Ile Ser Arg Thr Pro Glu Val Thr Cys Val Val Val Asp Val
275 280 285

Ser His Glu Asp Pro Glu Val Lys Phe Asn Trp Tyr Val Asp Gly Val
290 295 300

Glu Val His Asn Ala Lys Thr Lys Pro Arg Glu Glu Gln Tyr Asn Ser
305 310 315 320

Thr Tyr Arg Val Val Ser Val Leu Thr Val Leu His Gln Asp Trp Leu
325 330 335

Asn Gly Lys Glu Tyr Lys Cys Lys Val Ser Asn Lys Ala Leu Pro Ala
340 345 350

Pro Ile Glu Lys Thr Ile Ser Lys Ala Lys Gly Gln Pro Arg Glu Pro
355 360 365

Gln Val Tyr Thr Leu Pro Pro Ser Arg Glu Glu Met Thr Lys Asn Gln
370 375 380

Val Ser Leu Thr Cys Leu Val Lys Gly Phe Tyr Pro Ser Asp Ile Ala
385 390 395 400

Val Glu Trp Glu Ser Asn Gly Gln Pro Glu Asn Asn Tyr Lys Thr Thr

405 410 415

Pro Pro Val Leu Asp Ser Asp Gly Ser Phe Phe Leu Tyr Ser Lys Leu
420 425 430

Thr Val Asp Lys Ser Arg Trp Gln Gln Gly Asn Val Phe Ser Cys Ser
435 440 445

Val Met His Glu Ala Leu His Asn His Tyr Thr Gln Lys Ser Leu Ser
450 455 460

Leu Ser Pro Gly Lys
465

<210> 12
<211> 1407
<212> DNA
<213> Artificial Sequence

<220>
<223> Trastuzumab A-Hwt

<400> 12
atgaaacacc tgtggttctt cctcctgctg gtggcagctc ccagatgggt gctgagcgag 60

gtgcagctgg ttgaatctgg cggaggactg gttcagcctg gcggatctct gagactgtct 120

tgtgccgcca gcggcttcaa catcaaggac acctacatcc actgggtccg acaggcccct 180

ggcaaaggac ttgaatgggt cgccagaatc taccccacca acggctacac cagatacgcc 240

gactctgtga agggcagatt caccatcagc gccgacacca gcaagaacac cgcctacctg 300

cagatgaaca gcctgagagc cgaggacacc gccgtgtact actgttctag atggggaggc 360

gacggcttct acgccatgga ttattggggc cagggcaccc tggttaccgt tagctcagcc 420

tccaccaagg gcccaagcgt cttccccctg gcaccctcct ccaagagcac ctctggcggc 480

acagccgccc tgggctgcct ggtcaaggac tacttccccg aacccgtgac cgtgagctgg 540

aactcaggcg ccctgaccag cggcgtgcac accttccccg ctgtcctgca gtcctcagga 600

ctctactccc tcagcagcgt ggtgaccgtg ccctccagca gcttgggcac ccagacctac 660

atctgcaacg tgaatcacaa gcccagcaac accaaggtgg acaagagagt tgagcccaaa 720

tcttgtgaca aaactcacac atgcccaccc tgcccagcac ctgaagccgc gggggggaccc 780

tcagtcttcc tcttcccccc aaaacccaag gacaccctca tgatctcccg gacccctgag 840

gtcacatgcg tggtggtgga cgtgagccac gaagaccctg aggtcaagtt caactggtac 900

gtggacggcg tggaggtgca taatgccaag acaaagcccc gggaggagca gtacaacagc 960

acgtaccggg tggtcagcgt cctcaccgtc ctgcaccagg actggctgaa tggcaaggag 1020

tacaagtgca aggtctccaa caaagccctc ccagccccca tcgagaaaac catctccaaa 1080

gccaaaggcc agccccggga accacaggtg tacaccctgc ccccatcccg ggaggagatg 1140

accaagaacc aggtcagcct gacctgcctg gtcaaaggct tctatcccag cgacatcgcc 1200

gtggagtggg agagcaatgg ccagcccgag aacaactaca agaccacccc tcccgtgctg 1260

gactccgacg gctccttctt cctctacagc aagctcaccg tggacaagag caggtggcag 1320

cagggcaacg tcttctcatg ctccgtgatg catgaggctc tgcacaacca ctacacccag 1380

aagagcctct ccctgtctcc cggcaaa 1407

<210> 13
<211> 120
<212> PRT
<213> Artificial Sequence

<220>
<223> Variable region of Hwt aa

<400> 13

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser

115 120

<210> 14
<211> 360
<212> DNA
<213> Artificial Sequence

<220>
<223> Variable region of Hwt

<400> 14
gaggtgcagc tggttgaatc tggcggagga ctggttcagc ctggcggatc tctgagactg 60

tcttgtgccg ccagcggctt caacatcaag gacacctaca tccactgggt ccgacaggcc 120

cctggcaaag gacttgaatg ggtcgccaga atctacccca ccaacggcta caccagatac 180

gccgactctg tgaagggcag attcaccatc agcgccgaca ccagcaagaa caccgcctac 240

ctgcagatga acagcctgag agccgaggac accgccgtgt actactgttc tagatgggga 300

ggcgacggct tctacgccat ggattattgg ggccagggca ccctggttac cgttagctca 360

<210> 15
<211> 469
<212> PRT
<213> Artificial Sequence

<220>
<223> H01 aa

<400> 15

Met Lys His Leu Trp Phe Phe Leu Leu Leu Val Ala Ala Pro Arg Trp
1 5 10 15

Val Leu Ser Glu Val Gln Leu Val Glu Ser Gly Gly Gly Leu Val Gln
20 25 30

Pro Gly Gly Ser Leu Arg Leu Ser Cys Ala Ala Ser Gly Phe Asn Ile
35 40 45

Lys Asp Thr Tyr Ile His Trp Val Arg Gln Ala Pro Gly Lys Gly Leu
50 55 60

Glu Trp Val Ala Arg Ile Tyr Pro Thr Asn Gly Tyr Thr Arg Tyr Ala
65 70 75 80

Asp Ser Val Lys Gly Arg Phe Thr Ile Ser Ala Asp Thr Ser Lys Asn
85 90 95

Thr Ala Tyr Leu Gln Met Asn Ser Leu Arg Ala Glu Asp Thr Ala Val
100 105 110

Tyr Tyr Cys Ser Arg Trp Gly Gly Asp Gly Phe Phe Ala Met Asp Tyr
115 120 125

Trp Gly Gln Gly Thr Leu Val Thr Val Ser Ser Ala Ser Thr Lys Gly
130 135 140

Pro Ser Val Phe Pro Leu Ala Pro Ser Ser Lys Ser Thr Ser Gly Gly
145 150 155 160

Thr Ala Ala Leu Gly Cys Leu Val Lys Asp Tyr Phe Pro Glu Pro Val
165 170 175

Thr Val Ser Trp Asn Ser Gly Ala Leu Thr Ser Gly Val His Thr Phe
180 185 190

Pro Ala Val Leu Gln Ser Ser Gly Leu Tyr Ser Leu Ser Ser Val Val
195 200 205

Thr Val Pro Ser Ser Ser Leu Gly Thr Gln Thr Tyr Ile Cys Asn Val
210 215 220

Asn His Lys Pro Ser Asn Thr Lys Val Asp Lys Arg Val Glu Pro Lys
225 230 235 240

Ser Cys Asp Lys Thr His Thr Cys Pro Pro Cys Pro Ala Pro Glu Ala
245 250 255

Ala Gly Gly Pro Ser Val Phe Leu Phe Pro Pro Lys Pro Lys Asp Thr
260 265 270

Leu Met Ile Ser Arg Thr Pro Glu Val Thr Cys Val Val Val Asp Val
275 280 285

Ser His Glu Asp Pro Glu Val Lys Phe Asn Trp Tyr Val Asp Gly Val
290 295 300

Glu Val His Asn Ala Lys Thr Lys Pro Arg Glu Glu Gln Tyr Asn Ser
305 310 315 320

Thr Tyr Arg Val Val Ser Val Leu Thr Val Leu His Gln Asp Trp Leu

325 330 335

Asn Gly Lys Glu Tyr Lys Cys Lys Val Ser Asn Lys Ala Leu Pro Ala
340 345 350

Pro Ile Glu Lys Thr Ile Ser Lys Ala Lys Gly Gln Pro Arg Glu Pro
355 360 365

Gln Val Tyr Thr Leu Pro Pro Ser Arg Glu Glu Met Thr Lys Asn Gln
370 375 380

Val Ser Leu Thr Cys Leu Val Lys Gly Phe Tyr Pro Ser Asp Ile Ala
385 390 395 400

Val Glu Trp Glu Ser Asn Gly Gln Pro Glu Asn Asn Tyr Lys Thr Thr
405 410 415

Pro Pro Val Leu Asp Ser Asp Gly Ser Phe Phe Leu Tyr Ser Lys Leu
420 425 430

Thr Val Asp Lys Ser Arg Trp Gln Gln Gly Asn Val Phe Ser Cys Ser
435 440 445

Val Met His Glu Ala Leu His Asn His Tyr Thr Gln Lys Ser Leu Ser
450 455 460

Leu Ser Pro Gly Lys
465

<210> 16
<211> 1407
<212> DNA
<213> Artificial Sequence

<220>
<223> H01

<400> 16
atgaaacacc tgtggttctt cctcctgctg gtggcagctc ccagatgggt gctgagcgag 60

gtgcagctgg ttgaatctgg cggaggactg gttcagcctg gcggatctct gagactgtct 120

tgtgccgcca gcggcttcaa catcaaggac acctacatcc actgggtccg acaggcccct 180

ggcaaaggac ttgaatgggt cgccagaatc taccccacca acggctacac cagatacgcc 240

gactctgtga agggcagatt caccatcagc gccgacacca gcaagaacac cgcctacctg 300

cagatgaaca gcctgagagc cgaggacacc gccgtgtact actgttctag atggggaggc 360

gacggcttct tcgccatgga ttattggggc cagggcaccc tggttaccgt tagctcagcc 420

tccaccaagg gcccaagcgt cttcccctg gcaccctcct ccaagagcac ctctggcggc 480

acagccgccc tgggctgcct ggtcaaggac tacttccccg aacccgtgac cgtgagctgg 540

aactcaggcg ccctgaccag cggcgtgcac accttccccg ctgtcctgca gtcctcagga 600

ctctactccc tcagcagcgt ggtgaccgtg ccctccagca gcttgggcac ccagacctac 660

atctgcaacg tgaatcacaa gcccagcaac accaaggtgg acaagagagt tgagcccaaa 720

tcttgtgaca aaactcacac atgcccaccc tgcccagcac ctgaagccgc ggggggaccc 780

tcagtcttcc tcttcccccc aaaacccaag gacaccctca tgatctcccg gacccctgag 840

gtcacatgcg tggtggtgga cgtgagccac gaagaccctg aggtcaagtt caactggtac 900

gtggacggcg tggaggtgca taatgccaag acaaagcccc gggaggagca gtacaacagc 960

acgtaccggg tggtcagcgt cctcaccgtc ctgcaccagg actggctgaa tggcaaggag 1020

tacaagtgca aggtctccaa caaagccctc ccagccccca tcgagaaaac catctccaaa 1080

gccaaaggcc agccccggga accacaggtg tacaccctgc ccccatcccg ggaggagatg 1140

accaagaacc aggtcagcct gacctgcctg gtcaaaggct tctatcccag cgacatcgcc 1200

gtggagtggg agagcaatgg ccagcccgag aacaactaca agaccacccc tcccgtgctg 1260

gactccgacg gctccttctt cctctacagc aagctcaccg tggacaagag caggtggcag 1320

cagggcaacg tcttctcatg ctccgtgatg catgaggctc tgcacaacca ctacacccag 1380

aagagcctct ccctgtctcc cggcaaa 1407

<210> 17
<211> 120
<212> PRT
<213> Artificial Sequence

<220>
<223> Variable region of H01 aa

<400> 17

Glu Val Gln Leu Val Glu Ser Gly Gly Gly Leu Val Gln Pro Gly Gly
1 5 10 15

Ser Leu Arg Leu Ser Cys Ala Ala Ser Gly Phe Asn Ile Lys Asp Thr
20 25 30

Tyr Ile His Trp Val Arg Gln Ala Pro Gly Lys Gly Leu Glu Trp Val

35 40 45

Ala Arg Ile Tyr Pro Thr Asn Gly Tyr Thr Arg Tyr Ala Asp Ser Val
50 55 60

Lys Gly Arg Phe Thr Ile Ser Ala Asp Thr Ser Lys Asn Thr Ala Tyr
65 70 75 80

Leu Gln Met Asn Ser Leu Arg Ala Glu Asp Thr Ala Val Tyr Tyr Cys
85 90 95

Ser Arg Trp Gly Gly Asp Gly Phe Phe Ala Met Asp Tyr Trp Gly Gln
100 105 110

Gly Thr Leu Val Thr Val Ser Ser
115 120

<210> 18
<211> 360
<212> DNA
<213> Artificial Sequence

<220>
<223> Variable region of H01

<400> 18
gaggtgcagc tggttgaatc tggcggagga ctggttcagc ctggcggatc tctgagactg 60

tcttgtgccg ccagcggctt caacatcaag gacacctaca tccactgggt ccgacaggcc 120

cctggcaaag gacttgaatg ggtcgccaga atctacccca ccaacggcta caccagatac 180

gccgactctg tgaagggcag attcaccatc agcgccgaca ccagcaagaa caccgcctac 240

ctgcagatga acagcctgag agccgaggac accgccgtgt actactgttc tagatgggga 300

ggcgacggct tcttcgccat ggattattgg ggccagggca ccctggttac cgttagctca 360

<210> 19
<211> 234
<212> PRT
<213> Artificial Sequence

<220>
<223> Trastuzumab A-Lwt aa

<400> 19

Met Val Leu Gln Thr Gln Val Phe Ile Ser Leu Leu Leu Trp Ile Ser
1 5 10 15

```
Gly Ala Tyr Gly Asp Ile Gln Met Thr Gln Ser Pro Ser Ser Leu Ser
            20                  25                  30

Ala Ser Val Gly Asp Arg Val Thr Ile Thr Cys Arg Ala Ser Gln Asp
        35                  40                  45

Val Asn Thr Ala Val Ala Trp Tyr Gln Gln Lys Pro Gly Lys Ala Pro
    50                  55                  60

Lys Leu Leu Ile Tyr Ser Ala Ser Phe Leu Tyr Ser Gly Val Pro Ser
65                  70                  75                  80

Arg Phe Ser Gly Ser Arg Ser Gly Thr Asp Phe Thr Leu Thr Ile Ser
                85                  90                  95

Ser Leu Gln Pro Glu Asp Phe Ala Thr Tyr Tyr Cys Gln Gln His Tyr
            100                 105                 110

Thr Thr Pro Pro Thr Phe Gly Gln Gly Thr Lys Val Glu Ile Lys Arg
        115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230
```

<210> 20

<211> 702
<212> DNA
<213> Artificial Sequence

<220>
<223> Trastuzumab A-Lwt

<400> 20
atggtgctgc agacccaggt gttcatctcc ctgctgctgt ggatctccgg cgcgtacggc 60

gacatccaga tgacacagag ccctagcagc ctgtctgcca gcgtgggaga cagagtgacc 120

atcacctgta gagccagcca ggacgtgaac acagccgtgg cttggtatca gcagaagcct 180

ggcaaggccc ctaagctgct gatctacagc gccagctttc tgtacagcgg cgtgcccagc 240

agattcagcg gctctagaag cggcaccgac ttcaccctga ccataagcag tctgcagccc 300

gaggacttcg ccacctacta ctgtcagcag cactacacca cacctccaac ctttggccag 360

ggcaccaagg tggaaatcaa gcgtacggtg gccgcccct ccgtgttcat cttcccccc 420

tccgacgagc agctgaagtc cggcaccgcc tccgtggtgt gcctgctgaa taacttctac 480

cccagagagg ccaaggtgca gtggaaggtg gacaacgccc tgcagtccgg gaactcccag 540

gagagcgtga ccgagcagga cagcaaggac agcacctaca gcctgagcag caccctgacc 600

ctgagcaaag ccgactacga gaagcacaag gtgtacgcct gcgaggtgac ccaccagggc 660

ctgagctccc ccgtcaccaa gagcttcaac aggggggagt gt 702

<210> 21
<211> 107
<212> PRT
<213> Artificial Sequence

<220>
<223> Variable region of Lwt aa

<400> 21

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Val Asn Thr Ala
20 25 30

Val Ala Trp Tyr Gln Gln Lys Pro Gly Lys Ala Pro Lys Leu Leu Ile
35 40 45

Tyr Ser Ala Ser Phe Leu Tyr Ser Gly Val Pro Ser Arg Phe Ser Gly
50 55 60

Ser Arg Ser Gly Thr Asp Phe Thr Leu Thr Ile Ser Ser Leu Gln Pro
65 70 75 80

Glu Asp Phe Ala Thr Tyr Tyr Cys Gln Gln His Tyr Thr Thr Pro Pro
85 90 95

Thr Phe Gly Gln Gly Thr Lys Val Glu Ile Lys
100 105

<210> 22
<211> 321
<212> DNA
<213> Artificial Sequence

<220>
<223> Variable region of Lwt

<400> 22
gacatccaga tgacacagag ccctagcagc ctgtctgcca gcgtgggaga cagagtgacc 60

atcacctgta gagccagcca ggacgtgaac acagccgtgg cttggtatca gcagaagcct 120

ggcaaggccc ctaagctgct gatctacagc gccagctttc tgtacagcgg cgtgcccagc 180

agattcagcg gctctagaag cggcaccgac ttcaccctga ccataagcag tctgcagccc 240

gaggacttcg ccacctacta ctgtcagcag cactacacca cacctccaac ctttggccag 300

ggcaccaagg tggaaatcaa g 321

<210> 23
<211> 234
<212> PRT
<213> Artificial Sequence

<220>
<223> L02 aa

<400> 23

Met Val Leu Gln Thr Gln Val Phe Ile Ser Leu Leu Leu Trp Ile Ser
1 5 10 15

Gly Ala Tyr Gly Asp Ile Gln Met Thr Gln Ser Pro Ser Ser Leu Ser
20 25 30

Ala Ser Val Gly Asp Arg Val Thr Ile Thr Cys Arg Ala Ser Gln Asp
35 40 45

Val Asn Thr Ala Val Ala Trp Tyr Gln Gln Lys Pro Gly Lys Ala Pro

50 55 60

Lys Leu Leu Ile Tyr Ser Ala Ser Phe Leu Tyr Ser Gly Val Pro Ser
65 70 75 80

Arg Phe Ser Gly Ser Arg Ser Gly Thr Asp Phe Thr Leu Thr Ile Ser
85 90 95

Ser Leu Gln Pro Glu Asp Phe Ala Thr Tyr Tyr Cys Gln Gln His Ala
100 105 110

Thr Thr Pro Pro Thr Phe Gly Gln Gly Thr Lys Val Glu Ile Lys Arg
115 120 125

Thr Val Ala Ala Pro Ser Val Phe Ile Phe Pro Pro Ser Asp Glu Gln
130 135 140

Leu Lys Ser Gly Thr Ala Ser Val Val Cys Leu Leu Asn Asn Phe Tyr
145 150 155 160

Pro Arg Glu Ala Lys Val Gln Trp Lys Val Asp Asn Ala Leu Gln Ser
165 170 175

Gly Asn Ser Gln Glu Ser Val Thr Glu Gln Asp Ser Lys Asp Ser Thr
180 185 190

Tyr Ser Leu Ser Ser Thr Leu Thr Leu Ser Lys Ala Asp Tyr Glu Lys
195 200 205

His Lys Val Tyr Ala Cys Glu Val Thr His Gln Gly Leu Ser Ser Pro
210 215 220

Val Thr Lys Ser Phe Asn Arg Gly Glu Cys
225 230

<210> 24
<211> 702
<212> DNA
<213> Artificial Sequence

<220>
<223> L02

<400> 24
atggtgctgc agacccaggt gttcatctcc ctgctgctgt ggatctccgg cgcgtacggc 60

```
gacatccaga tgacacagag ccctagcagc ctgtctgcca gcgtgggaga cagagtgacc      120

atcacctgta gagccagcca ggacgtgaac acagccgtgg cttggtatca gcagaagcct      180

ggcaaggccc ctaagctgct gatctacagc gccagctttc tgtacagcgg cgtgcccagc      240

agattcagcg gctctagaag cggcaccgac ttcaccctga ccataagcag tctgcagccc      300

gaggacttcg ccacctacta ctgtcagcag cacgccacca cacctccaac atttggccag      360

ggcaccaagg tggaaatcaa gcgtacggtg gccgcccct ccgtgttcat cttcccccc        420

tccgacgagc agctgaagtc cggcaccgcc tccgtggtgt gcctgctgaa taacttctac      480

cccagagagg ccaaggtgca gtggaaggtg gacaacgccc tgcagtccgg gaactcccag      540

gagagcgtga ccgagcagga cagcaaggac agcacctaca gcctgagcag caccctgacc      600

ctgagcaaag ccgactacga gaagcacaag gtgtacgcct gcgaggtgac ccaccagggc      660

ctgagctccc ccgtcaccaa gagcttcaac aggggggagt gt                          702


<210>  25
<211>  107
<212>  PRT
<213>  Artificial Sequence

<220>
<223>  Variable region of L02 aa

<400>  25

Asp Ile Gln Met Thr Gln Ser Pro Ser Ser Leu Ser Ala Ser Val Gly
1               5                   10                  15


Asp Arg Val Thr Ile Thr Cys Arg Ala Ser Gln Asp Val Asn Thr Ala
            20                  25                  30


Val Ala Trp Tyr Gln Gln Lys Pro Gly Lys Ala Pro Lys Leu Leu Ile
        35                  40                  45


Tyr Ser Ala Ser Phe Leu Tyr Ser Gly Val Pro Ser Arg Phe Ser Gly
    50                  55                  60


Ser Arg Ser Gly Thr Asp Phe Thr Leu Thr Ile Ser Ser Leu Gln Pro
65                  70                  75                  80


Glu Asp Phe Ala Thr Tyr Tyr Cys Gln Gln His Ala Thr Thr Pro Pro
                85                  90                  95


Thr Phe Gly Gln Gly Thr Lys Val Glu Ile Lys
```

100 105

<210> 26
<211> 321
<212> DNA
<213> Artificial Sequence

<220>
<223> Variable region of L02

<400> 26
gacatccaga tgacacagag ccctagcagc ctgtctgcca gcgtgggaga cagagtgacc 60

atcacctgta gagccagcca ggacgtgaac acagccgtgg cttggtatca gcagaagcct 120

ggcaaggccc ctaagctgct gatctacagc gccagctttc tgtacagcgg cgtgcccagc 180

agattcagcg gctctagaag cggcaccgac ttcaccctga ccataagcag tctgcagccc 240

gaggacttcg ccacctacta ctgtcagcag cacgccacca cacctccaac atttggccag 300

ggcaccaagg tggaaatcaa g 321

<210> 27
<211> 234
<212> PRT
<213> Artificial Sequence

<220>
<223> L05 aa

<400> 27

Met Val Leu Gln Thr Gln Val Phe Ile Ser Leu Leu Leu Trp Ile Ser
1 5 10 15

Gly Ala Tyr Gly Asp Ile Gln Met Thr Gln Ser Pro Ser Ser Leu Ser
20 25 30

Ala Ser Val Gly Asp Arg Val Thr Ile Thr Cys Arg Ala Ser Gln Asp
35 40 45

Val Asn Thr Ala Val Ala Trp Tyr Gln Gln Lys Pro Gly Lys Ala Pro
50 55 60

Lys Ala Leu Ile Tyr Ser Ala Ser Phe Leu Tyr Ser Gly Val Pro Ser
65 70 75 80

Arg Phe Ser Gly Ser Arg Ser Gly Thr Asp Phe Thr Leu Thr Ile Ser
85 90 95

Ser Leu Gln Pro Glu Asp Phe Ala Thr Tyr Tyr Cys Gln Gln His Ala
100 105 110

Thr Thr Pro Pro Thr Phe Gly Gln Gly Thr Lys Val Glu Ile Lys Arg
115 120 125

Thr Val Ala Ala Pro Ser Val Phe Ile Phe Pro Pro Ser Asp Glu Gln
130 135 140

Leu Lys Ser Gly Thr Ala Ser Val Val Cys Leu Leu Asn Asn Phe Tyr
145 150 155 160

Pro Arg Glu Ala Lys Val Gln Trp Lys Val Asp Asn Ala Leu Gln Ser
165 170 175

Gly Asn Ser Gln Glu Ser Val Thr Glu Gln Asp Ser Lys Asp Ser Thr
180 185 190

Tyr Ser Leu Ser Ser Thr Leu Thr Leu Ser Lys Ala Asp Tyr Glu Lys
195 200 205

His Lys Val Tyr Ala Cys Glu Val Thr His Gln Gly Leu Ser Ser Pro
210 215 220

Val Thr Lys Ser Phe Asn Arg Gly Glu Cys
225 230

<210> 28
<211> 702
<212> DNA
<213> Artificial Sequence

<220>
<223> L05

<400> 28
atggtgctgc agacccaggt gttcatctcc ctgctgctgt ggatctccgg cgcgtacggc 60

gacatccaga tgacacagag ccctagcagc ctgtctgcca gcgtgggaga cagagtgacc 120

atcacctgta gagccagcca ggacgtgaac acagccgtgg cttggtatca gcagaagcct 180

ggcaaagccc ctaaggctct gatctacagc gccagctttc tgtacagcgg cgtgcccagc 240

agattcagcg gctctagaag cggcaccgac ttcaccctga ccataagcag tctgcagccc 300

gaggacttcg ccacctacta ctgtcagcag cacgccacca cacctccaac atttggccag 360

```
ggcaccaagg tggaaatcaa gcgtacggtg gccgcccct ccgtgttcat cttcccccc      420

tccgacgagc agctgaagtc cggcaccgcc tccgtggtgt gcctgctgaa taacttctac      480

cccagagagg ccaaggtgca gtggaaggtg gacaacgccc tgcagtccgg gaactcccag      540

gagagcgtga ccgagcagga cagcaaggac agcacctaca gcctgagcag caccctgacc      600

ctgagcaaag ccgactacga gaagcacaag gtgtacgcct gcgaggtgac ccaccagggc      660

ctgagctccc ccgtcaccaa gagcttcaac aggggggagt gt                        702
```

<210> 29
<211> 107
<212> PRT
<213> Artificial Sequence

<220>
<223> Variable region of L05 aa

<400> 29

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Ala Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Ala Thr Thr Pro Pro
                85                  90                  95

Thr Phe Gly Gln Gly Thr Lys Val Glu Ile Lys
            100                 105
```

<210> 30
<211> 321
<212> DNA
<213> Artificial Sequence

<220>
<223> Variable region of L05

<400> 30
gacatccaga tgacacagag ccctagcagc ctgtctgcca gcgtgggaga cagagtgacc 60

atcacctgta gagccagcca ggacgtgaac acagccgtgg cttggtatca gcagaagcct 120

ggcaaagccc ctaaggctct gatctacagc gccagctttc tgtacagcgg cgtgcccagc 180

agattcagcg gctctagaag cggcaccgac ttcaccctga ccataagcag tctgcagccc 240

gaggacttcg ccacctacta ctgtcagcag cacgccacca cacctccaac atttggccag 300

ggcaccaagg tggaaatcaa g 321

<210> 31
<211> 469
<212> PRT
<213> Artificial Sequence

<220>
<223> Heavy chain of Trastuzumab A2

<400> 31

Met Lys His Leu Trp Phe Phe Leu Leu Leu Val Ala Ala Pro Arg Trp
1 5 10 15

Val Leu Ser Glu Val Gln Leu Val Glu Ser Gly Gly Gly Leu Val Gln
20 25 30

Pro Gly Gly Ser Leu Arg Leu Ser Cys Ala Ala Ser Gly Phe Asn Ile
35 40 45

Lys Asp Thr Tyr Ile His Trp Val Arg Gln Ala Pro Gly Lys Gly Leu
50 55 60

Glu Trp Val Ala Arg Ile Tyr Pro Thr Asn Gly Tyr Thr Arg Tyr Ala
65 70 75 80

Asp Ser Val Lys Gly Arg Phe Thr Ile Ser Ala Asp Thr Ser Lys Asn
85 90 95

Thr Ala Tyr Leu Gln Met Asn Ser Leu Arg Ala Glu Asp Thr Ala Val
100 105 110

Tyr Tyr Cys Ser Arg Trp Gly Gly Asp Gly Phe Tyr Ala Met Asp Tyr
115 120 125

Trp Gly Gln Gly Thr Leu Val Thr Val Ser Ser Ala Ser Thr Lys Gly

130 135 140

Pro Ser Val Phe Pro Leu Ala Pro Ser Ser Lys Ser Thr Ser Gly Gly
145 150 155 160

Thr Ala Ala Leu Gly Cys Leu Val Lys Asp Tyr Phe Pro Glu Pro Val
165 170 175

Thr Val Ser Trp Asn Ser Gly Ala Leu Thr Ser Gly Val His Thr Phe
180 185 190

Pro Ala Val Leu Gln Ser Ser Gly Leu Tyr Ser Leu Ser Ser Val Val
195 200 205

Thr Val Pro Ser Ser Ser Leu Gly Thr Gln Thr Tyr Ile Cys Asn Val
210 215 220

Asn His Lys Pro Ser Asn Thr Lys Val Asp Lys Lys Val Glu Pro Lys
225 230 235 240

Ser Cys Asp Lys Thr His Thr Cys Pro Pro Cys Pro Ala Pro Glu Ala
245 250 255

Ala Gly Gly Pro Ser Val Phe Leu Phe Pro Pro Lys Pro Lys Asp Thr
260 265 270

Leu Met Ile Ser Arg Thr Pro Glu Val Thr Cys Val Val Val Asp Val
275 280 285

Ser His Glu Asp Pro Glu Val Lys Phe Asn Trp Tyr Val Asp Gly Val
290 295 300

Glu Val His Asn Ala Lys Thr Lys Pro Arg Glu Glu Gln Tyr Asn Ser
305 310 315 320

Thr Tyr Arg Val Val Ser Val Leu Thr Val Leu His Gln Asp Trp Leu
325 330 335

Asn Gly Lys Glu Tyr Lys Cys Lys Val Ser Asn Lys Ala Leu Pro Ala
340 345 350

Pro Ile Glu Lys Thr Ile Ser Lys Ala Lys Gly Gln Pro Arg Glu Pro
355 360 365

Gln Val Tyr Thr Leu Pro Pro Ser Arg Glu Glu Met Thr Lys Asn Gln
    370                 375                 380

Val Ser Leu Thr Cys Leu Val Lys Gly Phe Tyr Pro Ser Asp Ile Ala
385                 390                 395                 400

Val Glu Trp Glu Ser Asn Gly Gln Pro Glu Asn Asn Tyr Lys Thr Thr
                405                 410                 415

Pro Pro Val Leu Asp Ser Asp Gly Ser Phe Phe Leu Tyr Ser Lys Leu
            420                 425                 430

Thr Val Asp Lys Ser Arg Trp Gln Gln Gly Asn Val Phe Ser Cys Ser
        435                 440                 445

Val Met His Glu Ala Leu His Asn His Tyr Thr Gln Lys Ser Leu Ser
    450                 455                 460

Leu Ser Pro Gly Lys
465

<210>  32
<211>  234
<212>  PRT
<213>  Artificial Sequence

<220>
<223>  Light chain of Trastuzumab A2

<400>  32

Met Val Leu Gln Thr Gln Val Phe Ile Ser Leu Leu Leu Trp Ile Ser
1               5                   10                  15

Gly Ala Tyr Gly Asp Ile Gln Met Thr Gln Ser Pro Ser Ser Leu Ser
            20                  25                  30

Ala Ser Val Gly Asp Arg Val Thr Ile Thr Cys Arg Ala Ser Gln Asp
        35                  40                  45

Val Asn Thr Ala Val Ala Trp Tyr Gln Gln Lys Pro Gly Lys Ala Pro
    50                  55                  60

Lys Leu Leu Ile Tyr Ser Ala Ser Phe Leu Tyr Ser Gly Val Pro Ser
65                  70                  75                  80

Arg Phe Ser Gly Ser Arg Ser Gly Thr Asp Phe Thr Leu Thr Ile Ser
85 90 95

Ser Leu Gln Pro Glu Asp Phe Ala Thr Tyr Tyr Cys Gln Gln His Tyr
100 105 110

Thr Thr Pro Pro Thr Phe Gly Gln Gly Thr Lys Val Glu Ile Lys Arg
115 120 125

Thr Val Ala Ala Pro Ser Val Phe Ile Phe Pro Pro Ser Asp Glu Gln
130 135 140

Leu Lys Ser Gly Thr Ala Ser Val Val Cys Leu Leu Asn Asn Phe Tyr
145 150 155 160

Pro Arg Glu Ala Lys Val Gln Trp Lys Val Asp Asn Ala Leu Gln Ser
165 170 175

Gly Asn Ser Gln Glu Ser Val Thr Glu Gln Asp Ser Lys Asp Ser Thr
180 185 190

Tyr Ser Leu Ser Ser Thr Leu Thr Leu Ser Lys Ala Asp Tyr Glu Lys
195 200 205

His Lys Val Tyr Ala Cys Glu Val Thr His Gln Gly Leu Ser Ser Pro
210 215 220

Val Thr Lys Ser Phe Asn Arg Gly Glu Cys
225 230

<210> 33
<211> 450
<212> PRT
<213> Artificial Sequence

<220>
<223> Heavy chain of Trastuzumab

<400> 33

Glu Val Gln Leu Val Glu Ser Gly Gly Gly Leu Val Gln Pro Gly Gly
1 5 10 15

Ser Leu Arg Leu Ser Cys Ala Ala Ser Gly Phe Asn Ile Lys Asp Thr
20 25 30

```
Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
```

```
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450


<210>  34
<211>  214
<212>  PRT
<213>  Artificial Sequence

<220>
<223>  Light chain of Trastuzumab
```

<400> 34

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Val Asn Thr Ala
20 25 30

Val Ala Trp Tyr Gln Gln Lys Pro Gly Lys Ala Pro Lys Leu Leu Ile
35 40 45

Tyr Ser Ala Ser Phe Leu Tyr Ser Gly Val Pro Ser Arg Phe Ser Gly
50 55 60

Ser Arg Ser Gly Thr Asp Phe Thr Leu Thr Ile Ser Ser Leu Gln Pro
65 70 75 80

Glu Asp Phe Ala Thr Tyr Tyr Cys Gln Gln His Tyr Thr Thr Pro Pro
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

## Description

Technical Field

The present invention relates to a novel anti-HER2 antibody and an antibody-drug conjugate comprising the antibody.

Background Art

Antibody-drug conjugates (ADCs), which are used for treatment of cancer and so on, have a drug with cytotoxic activity conjugated to an antibody, for example, that binds to an antigen expressed on the surface of cancer cells and is capable of cellular internalization of the antigen through the binding. ADCs can effectively deliver the drug to cancer cells, and are thus expected to cause accumulation of the drug within the cancer cells and to kill the cancer cells.

A useful example of drugs to be used for ADCs is pyrrolobenzodiazepine (PBD). PBD exhibits cytotoxicity by binding to, for example, the PuGPu sequence in the DNA minor groove. Anthramycin, a naturally-occurring PBD, was first discovered in 1965, and since this discovery various naturally-occurring PBDs and analog PBDs thereof have been discovered (Non Patent Literatures 1 to 4).

The general structural formula of PBDs is represented by the following formula:

[Formula 1]

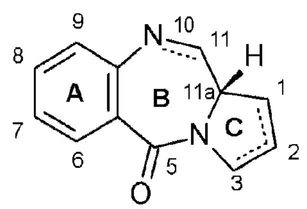

Known are PBDs different in the number of, types of, and sites of substituents in the A and C ring parts, and those different in degree of unsaturation in the B and C ring parts.

PBDs are known to come to have dramatically enhanced cytotoxicity through formation of a dimer structure (Non Patent Literatures 5, 6), and various ADCs with a dimer PBD have been reported (Patent Literatures 1 to 15). However, a PBD having a spiro ring at its C2-position and an ADC form thereof have not been known.

Human epidermal growth factor receptor 2 (HER2), a receptor protein tyrosine kinase, is a transmembrane receptor belonging to the epidermal growth factor receptor subfamily (Non Patent Literatures 7 to 12).

HER2 has been reported to be overexpressed in various types of cancer such as breast cancer and gastric cancer (Non Patent Literatures 13 to 18), and to be a negative prognostic factor in breast cancer (Non Patent Literatures 19, 20). Trastuzumab, Kadcyla, pertuzumab, lapatinib, and so on are known as anti-HER2 drugs effective for HER2-overexpressing cancers.

However, the responsiveness and intensity of activity, and the applicability are still insufficient, and there exist unmet needs for use of HER2 as a target.

Citation List

Patent Literature

Patent Literature 1: WO 2013/173496
Patent Literature 2: WO 2014/130879
Patent Literature 3: WO 2017/004330
Patent Literature 4: WO 2017/004025
Patent Literature 5: WO 2017/020972
Patent Literature 6: WO 2016/036804
Patent Literature 7: WO 2015/095124
Patent Literature 8: WO 2015/052322
Patent Literature 9: WO 2015/052534

Patent Literature 10: WO 2016/115191
Patent Literature 11: WO 2015/052321
Patent Literature 12: WO 2015/031693
Patent Literature 13: WO 2011/130613
Patent Literature 14: WO 2005/040170
Patent Literature 15: WO 2017/137556

Non Patent Literature

Non Patent Literature 1: Angewandte Chemie Internationl Edition 2016, 55, 2-29
Non Patent Literature 2: Chemical Reviews 2010, 111, 2815-2864
Non Patent Literature 3: In Antibiotics III. Springer Verlag, New York, pp.3-11
Non Patent Literature 4: Accounts of Chemical Research 1986, 19, 230
Non Patent Literature 5: Journal of the American Chemical Society 1992,114, 4939
Non Patent Literature 6: Journal of Organic Chemistry 1996, 61, 8141
Non Patent Literature 7: Science. 1985; 230(4730): 1132-1139.
Non Patent Literature 8: EMBO J. 1997; 16: 1647-1655.
Non Patent Literature 9: EMBO J. 1996; 15: 254-264.
Non Patent Literature 10: J Biom Chem. 1994; 269: 14661-14665.
Non Patent Literature 11: Science. 1987; 237: 178-182.
Non Patent Literature 12: Proc Natl Acad Sci U S A. 1987; 84: 7159-7163.
Non Patent Literature 13: Eur. J Surg Oncol. 1997 (23): 30-35.
Non Patent Literature 14: Oncogene. 2008; 27(47): 6120-6130.
Non Patent Literature 15: Oncol Rep. 2006; 15(1): 65-71.
Non Patent Literature 16: Science. 1987; 235: 177-182.
Non Patent Literature 17: Ann Oncol 19: 1523-1529, 2008.
Non Patent Literature 18: Mol Cell Biol 6: 955-958, 1986.
Non Patent Literature 19: Science. 1989; 244: 707-712.
Non Patent Literature 20: Diagn Mol Pathol 10: 139-152, 2001.

SUMMARY OF INVENTION

Problems to be resolved by the Invention

The present invention provides a novel anti-HER2 antibody, a novel anti-HER2 antibody-pyrrolobenzodiazepine (PBD) derivative conjugate, and a novel PBD derivative.

In addition, the present invention provides a pharmaceutical composition containing any of an anti-HER2 antibody, anti-HER2 antibody-PBD derivative conjugate, and a novel PBD derivative with antitumor activity.

Further, the present invention provides a method for treating cancer by using any of an anti-HER2 antibody, an anti-HER2 antibody-PBD derivative conjugate, and a novel PBD derivative.

Means of solving the Problems

The present inventors diligently examined to find that a novel anti-HER2 antibody-pyrrolobenzodiazepine (PBD) derivative conjugate has strong antitumor activity, thereby completing the present invention.

Specifically, the present invention relates to the following.

[1] An antibody-drug conjugate represented by the following formula:

[Formula 2]

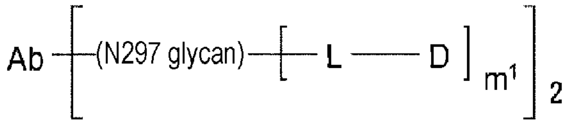

wherein

m$^1$ represents an integer of 1 or 2;
D is any one selected from the following group:

[Formula 3]

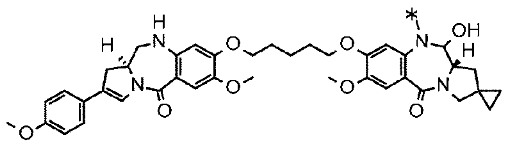

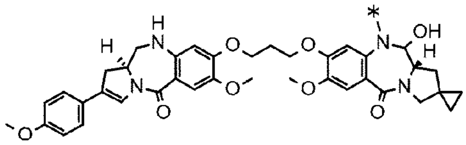

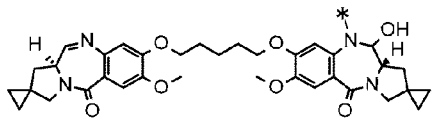

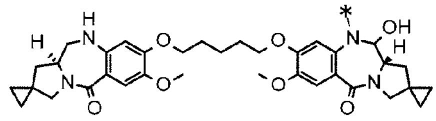

wherein

each asterisk (*) represents bonding to L;
L is a linker linking the glycan bonding to Asn297 of Ab (N297 glycan) and D;
the N297 glycan is optionally remodeled; and
Ab represents an antibody or a functional fragment of the antibody, wherein the antibody specifically binds to HER2 and comprises a heavy chain comprising CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 3 or an amino acid sequence having one to several amino acid substitutions in the amino acid sequence represented by SEQ ID NO: 3, and a light chain comprising CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 7 or an amino acid sequence having one to several amino acid substitutions in the amino acid sequence represented by SEQ ID NO: 7.

[2] The antibody-drug conjugate according to [1], wherein

L is represented by -Lb-La-Lp-NH-B-$CH_2$-O(C=O)-*, the asterisk (*) representing bonding to D;
B is a 1,4-phenyl group, a 2,5-pyridyl group, a 3,6-pyridyl group, a 2,5-pyrimidyl group, or a 2,5-thienyl group;
Lp represents any one selected from the following group:

-GGVA-, -GG-(D-)VA-, -VA-, -GGFG-, -GGPI-, -GGVCit-, -GGVK-, and - GGPL-;
La represents any one selected from the following group:

-C(=O)-$CH_2CH_2$-C(=O)-, -C(=O)-$(CH_2CH_2)_2$-C(=O)-,
-C(=O)-$CH_2CH_2$-C(=O)-NH-$(CH_2CH_2)_2$-C(=O)-,
-C(=O)-$CH_2CH_2$-C(=O)-NH-$(CH_2CH_2O)_2$-$CH_2$-C(=O)-,
-C(=O)-$CH_2CH_2$-NH-C(=O)-$(CH_2CH_2O)_4$-$CH_2CH_2$-C(=O)-, -$CH_2$-OC(=O)-, and -OC(=O)-; and
Lb is represented by the following formula:

[Formula 4]

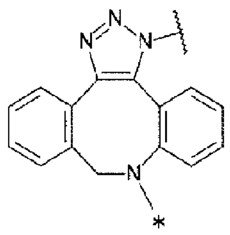

or

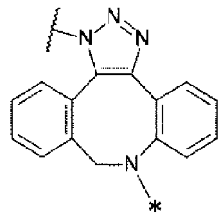

[Formula 5]

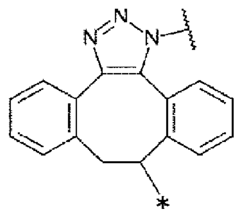

or

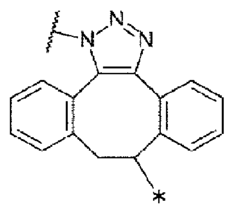

, or

[Formula 6]

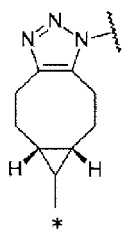

or

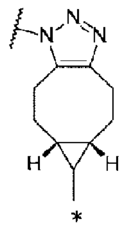

wherein, in each structural formula for Lb shown above,
each asterisk (*) represents bonding to La, and each wavy line represents bonding to N297 glycan or remodeled N297 glycan.

[3] The antibody-drug conjugate according to [1] or [2], wherein L represents any one selected from the following group:

$-Z^1-C(=O)-CH_2CH_2-C(=O)-GGVA-NH-B-CH_2-OC(=O)-$,
$-Z^1-C(=O)-CH_2CH_2-C(=O)-GG-(D-)VA-NH-B-CH_2-OC(=O)-$,
$-Z^1-C(=O)-CH_2CH_2-C(=O)-VA-NH-B-CH_2-OC(=O)-$,
$-Z^1-C(=O)-(CH_2CH_2)_2-C(=O)-VA-NH-B-CH_2-OC(=O)-$,
$-Z^1-C(=O)-CH_2CH_2-C(=O)-GGPI-NH-B-CH_2-OC(=O)-$,
$-Z^1-C(=O)-CH_2CH_2-C(=O)-GGFG-NH-B-CH_2-OC(=O)-$,
$-Z^1-C(=O)-CH_2CH_2-C(=O)-GGVCit-NH-B-CH_2-OC(=O)-$,
$-Z^1-C(=O)-CH_2CH_2-C(=O)-GGVK-NH-B-CH_2-OC(=O)-$,
$-Z^1-C(=O)-CH_2CH_2-C(=O)-GGPL-NH-B-CH_2-OC(=O)-$,
$-Z^1-C(=O)-CH_2CH_2-C(=O)-NH-(CH_2CH_2)_2-C(=O)-VA-NH-B-CH_2-OC(=O)-$,
$-Z^1-C(=O)-CH_2CH_2-C(=O)-NH-(CH_2CH_2O)_2-CH_2-C(=O)-VA-NH-B-CH_2-OC(=O)-$,
$-Z^1-C(=O)-CH_2CH_2-NH-C(=O)-(CH_2CH_2O)_4-CH_2CH_2-C(=O)-VA-NH-B-CH_2-OC(=O)-$,
$-Z^2-OC(=O)-GGVA-NH-B-CH_2-OC(=O)-$, and
$-Z^3-CH_2-OC(=O)-GGVA-NH-B-CH_2-OC(=O)-$, wherein
B represents a 1,4-phenyl group,

$Z^1$ represents the following structural formula:

[Formula 7]

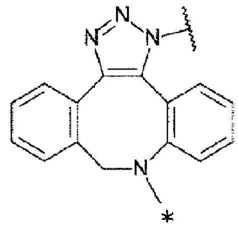

or

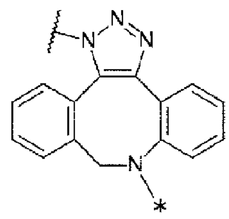

$Z^2$ represents the following structural formula:

[Formula 8]

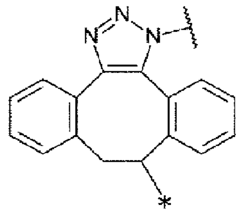

or

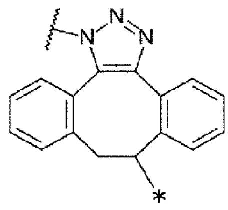

$Z^3$ represents the following structural formula:

[Formula 9]

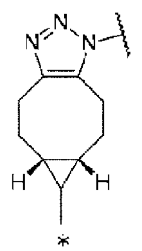

or

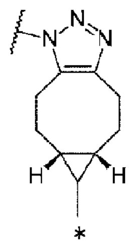

wherein, in each structural formula for $Z^1$, $Z^2$, and $Z^3$,
each asterisk (*) represents bonding to neighboring C(=O), OC(=O), or $CH_2$, and each wavy line represents bonding to N297 glycan or remodeled N297 glycan.

[4] The antibody-drug conjugate according to [3], wherein
L represents any one selected from the following group:

$-Z^1-C(=O)-CH_2CH_2-C(=O)-GGVA-NH-B-CH_2-OC(=O)-$,
$-Z^1-C(=O)-CH_2CH_2-C(=O)-VA-NH-B-CH_2-OC(=O)-$,
$-Z^1-C(=O)-(CH_2CH_2)_2-C(=O)-VA-NH-B-CH_2-OC(=O)-$,
$-Z^1-C(=O)-CH_2CH_2-C(=O)-GGVCit-NH-B-CH_2-OC(=O)-$,
$-Z^1-C(=O)-CH_2CH_2-C(=O)-NH-(CH_2CH_2)_2-C(=O)-VA-NH-B-CH_2-OC(=O)-$,
$-Z^1-C(=O)-CH_2CH_2-C(=O)-NH-(CH_2CH_2O)_2-CH_2-C(=O)-VA-NH-B-CH_2-OC(=O)-$, and
$-Z^1-C(=O)-CH_2CH_2-NH-C(=O)-(CH_2CH_2O)_4-CH_2CH_2-C(=O)-VA-NH-B-CH_2-OC(=O)-$, wherein
B is a 1,4-phenyl group,
$Z^1$ represents the following structural formula:

[Formula 10]

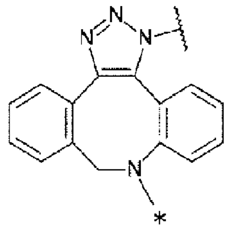

or

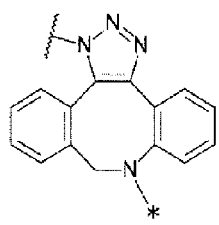

wherein, in the structural formula for $Z^1$, each asterisk (*) represents bonding to C(=O) neighboring to $Z^1$, and each wavy line represents bonding to N297 glycan or remodeled N297 glycan.

[5] The antibody-drug conjugate according to any one of [1] to [4], wherein D is any selected from the following group:

[Formula 11]

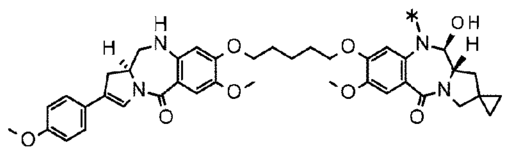

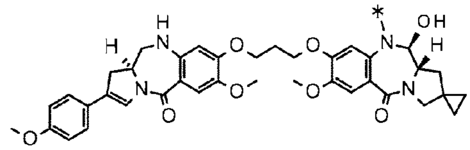

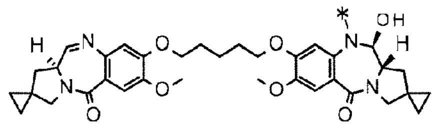

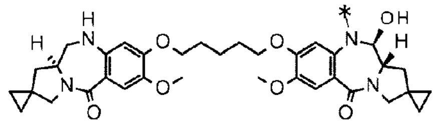

wherein
each asterisk (*) represents bonding to L.
[6] The antibody-drug conjugate according to any one of [1] to [5], wherein the antibody comprises a heavy chain comprising CDRH1, CDRH2, and CDRH3 and a light chain comprising CDRL1, CDRL2, and CDRL3 as described in any of the following (a) to (c):

(a) CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 4, and CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 8;
(b) CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 3, and CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 8; and
(c) CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 3, and CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 7.

[7] The antibody-drug conjugate according to any one of [1] to [6], wherein the antibody comprises a heavy chain variable region consisting of an amino acid sequence selected from the group consisting of the following (a) to (d) and a light chain variable region consisting of an amino acid sequence selected from the group consisting of the following (e) to (i):

(a) an amino acid sequence represented by SEQ ID NO: 13;

(b) an amino acid sequence represented by SEQ ID NO: 17;
(c) an amino acid sequence with a homology of at least 95% or higher to a sequence of a framework region excluding CDR sequences in any one of the sequences (a) and (b);
(d) an amino acid sequence having one to several amino acid deletions, substitutions, or additions in a sequence of a framework region excluding CDR sequences in any one of the sequences (a) and (b);
(e) an amino acid sequence represented by SEQ ID NO: 21;
(f) an amino acid sequence represented by SEQ ID NO: 25;
(g) an amino acid sequence represented by SEQ ID NO: 29;
(h) an amino acid sequence with a homology of at least 95% or higher to a sequence of a framework region excluding CDR sequences in any of the sequences (e) to (g); and
(i) an amino acid sequence having one to several amino acid deletions, substitutions, or additions in a sequence of a framework region excluding CDR sequences in any of the sequences (e) to (g).

[8] The antibody-drug conjugate according to [7], wherein the antibody comprises a heavy chain variable region and a light chain variable region as described in any of the following (a) to (c):

(a) a heavy chain variable region consisting of an amino acid sequence represented by SEQ ID NO: 17 and a light chain variable region consisting of an amino acid sequence represented by SEQ ID NO: 25;
(b) a heavy chain variable region consisting of an amino acid sequence represented by SEQ ID NO: 13 and a light chain variable region consisting of an amino acid sequence represented by SEQ ID NO: 29; and
(c) a heavy chain variable region consisting of an amino acid sequence represented by SEQ ID NO: 13 and a light chain variable region consisting of an amino acid sequence represented by SEQ ID NO: 21.

[9] The antibody-drug conjugate according to any one of [1] to [8], wherein the antibody is a chimeric antibody.
[10] The antibody-drug conjugate according to any one of [1] to [8], wherein the antibody is a humanized antibody.
[11] The antibody-drug conjugate according to [9] or [10], wherein the antibody comprises a heavy chain constant region of human IgG1, human IgG2, or human IgG4.
[12] The antibody-drug conjugate according to [11], wherein the heavy chain constant region of the antibody is a heavy chain constant region of human IgG1, and leucine at the 234- and 235-positions specified by EU Index numbering in the heavy chain constant region is substituted with alanine.
[13] The antibody-drug conjugate according to any one of [10] to [12], wherein the antibody comprises a heavy chain and a light chain as described in any one of the following (a) and (b):

(a) a heavy chain consisting of an amino acid sequence consisting of amino acid residues 20 to 469 of SEQ ID NO: 15 and a light chain consisting of an amino acid sequence consisting of amino acid residues 21 to 234 of SEQ ID NO: 23 (H01L02); and
(b) a heavy chain consisting of an amino acid sequence consisting of amino acid residues 20 to 469 of SEQ ID NO: 11 and a light chain consisting of an amino acid sequence consisting of amino acid residues 21 to 234 of SEQ ID NO: 27 (HwtL05).

[14] The antibody-drug conjugate according to any one of [10] to [12], wherein the antibody comprises a heavy chain and a light chain as described in any one of the following (a) and (b):

(a) a heavy chain consisting of an amino acid sequence consisting of amino acid residues 20 to 469 of SEQ ID NO: 11 and a light chain consisting of an amino acid sequence consisting of amino acid residues 21 to 234 of SEQ ID NO: 19; and
(b) a heavy chain consisting of an amino acid sequence consisting of amino acid residues 20 to 469 of SEQ ID NO: 31 and a light chain consisting of an amino acid sequence consisting of amino acid residues 21 to 234 of SEQ ID NO: 32.

[15] The antibody-drug conjugate according to any one of [1] to [14], wherein the antibody comprises one or two or more modifications selected from the group consisting of N-linked glycosylation, O-linked glycosylation, N-terminal processing, C-terminal processing, deamidation, isomerization of aspartic acid, oxidation of methionine, addition of a methionine residue at an N terminus, amidation of a proline residue, and deletion of one or two amino acid residues at the carboxyl terminus of a heavy chain.
[16] The antibody-drug conjugate according to [15], wherein one or several amino acid residues are deleted at the carboxyl terminus of a heavy chain of the antibody.
[17] The antibody-drug conjugate according to [16], wherein one amino acid residue is deleted at the carboxyl

terminus of each of the two heavy chains of the antibody.

[18] The antibody-drug conjugate according to any one of [1] to [5], wherein the antibody competes with the antibody according to any one of [6] to [17] for binding to HER2, or binds to a site of HER2 recognizable to the antibody according to any one of [6] to [17].

[19] The antibody-drug conjugate according to any one of [1] to [18], wherein the N297 glycan is a remodeled glycan.

[20] The antibody-drug conjugate according to any one of [1] to [19], wherein the N297 glycan is any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 12]

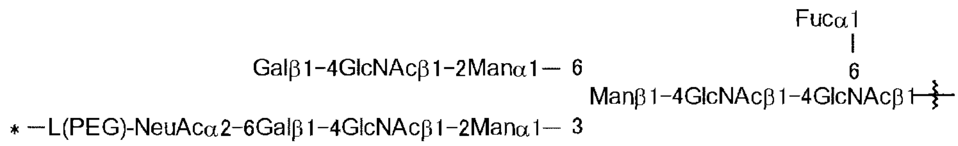

[N297-(Fuc)MSG1]

[Formula 13]

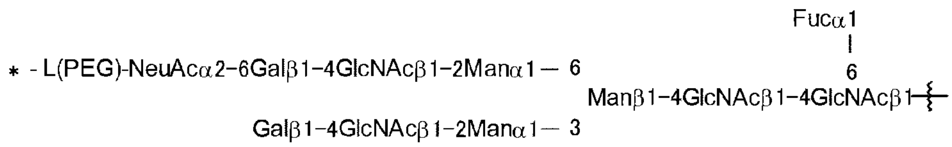

[N297-(Fuc)MSG2]

[Formula 14]

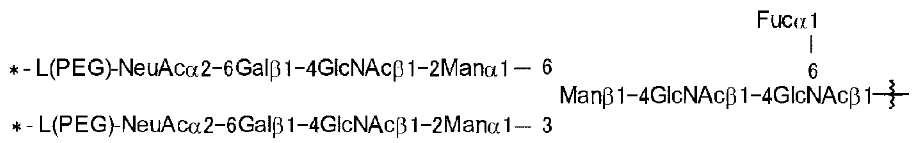

[N297-(Fuc)SG]

wherein

each wavy line represents bonding to Asn297 of the antibody,
L(PEG) in the N297 glycan represents $-NH-CH_2CH_2-(O-CH_2CH_2)n^5-*$, wherein $n^5$ represents an integer of 2 to 5, the amino group at the left end is bound via an amide bond to carboxylic acid at the 2-position of a sialic acid at the non-reducing terminal in each or either one of the 1-3 and 1-6 branched chains of β-Man in the N297 glycan, and the asterisk (*) at the right end represents bonding to a nitrogen atom at the 1- or 3-position of the triazole ring of $Z^1$ in L.

[21] The antibody-drug conjugate according to [20], wherein $n^5$ is 3.

[22] An antibody-drug conjugate represented by the following formula:

[Formula 15]

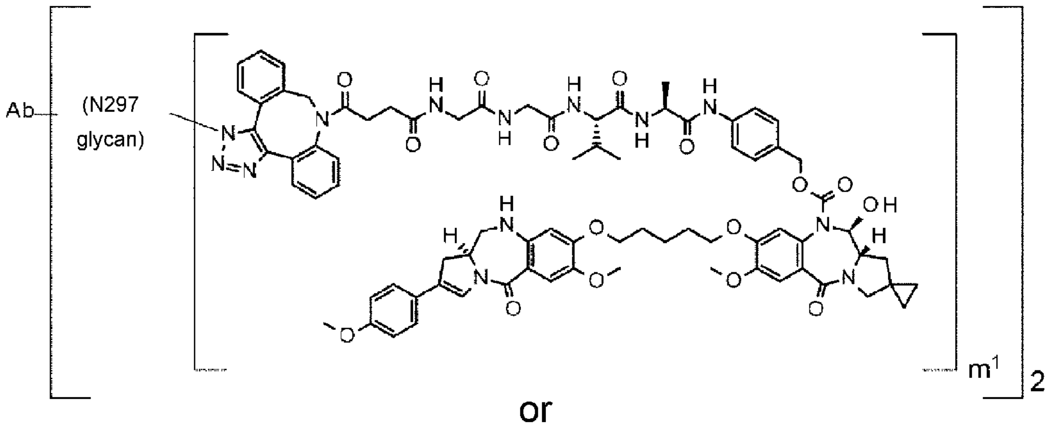

or

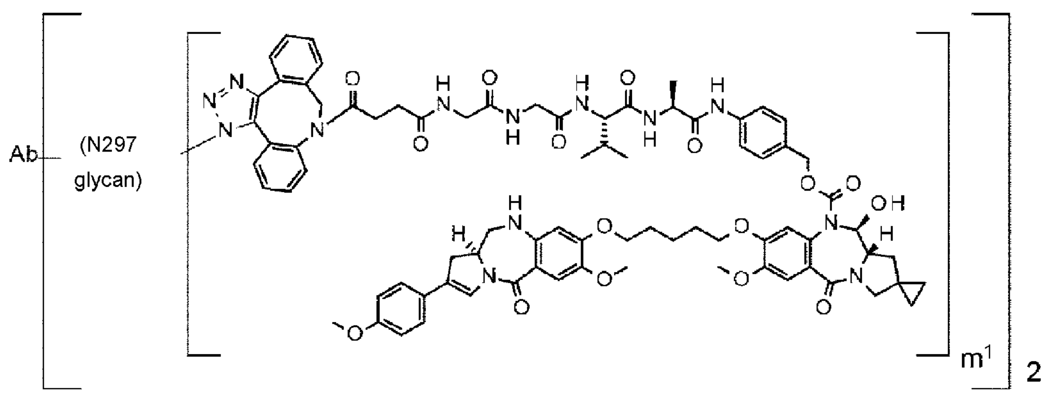

wherein, in each structural formula shown above,

$m^1$ represents an integer of 1 or 2;

Ab represents an antibody or a functional fragment of the antibody, wherein the antibody specifically binds to HER2 and comprises a heavy chain comprising CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 3 or an amino acid sequence having one to several amino acid substitutions in the amino acid sequence represented by SEQ ID NO: 3, and a light chain comprising CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 7 or an amino acid sequence having one to several amino acid substitutions in the amino acid sequence represented by SEQ ID NO: 7,

the N297 glycan is any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 16]

Fucα1
|
6
Galβ1-4GlcNAcβ1-2Manα1— 6
Manβ1-4GlcNAcβ1-4GlcNAcβ1—
* —L(PEG)-NeuAcα2-6Galβ1-4GlcNAcβ1-2Manα1— 3

[N297-(Fuc)MSG1]

[Formula 17]

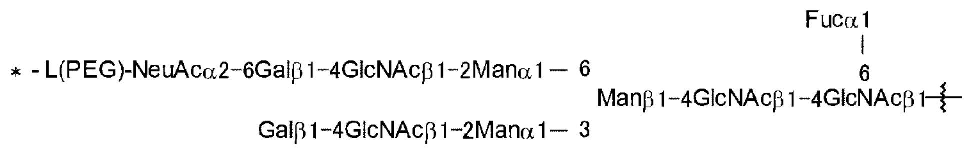

[N297-(Fuc)MSG2]

[Formula 18]

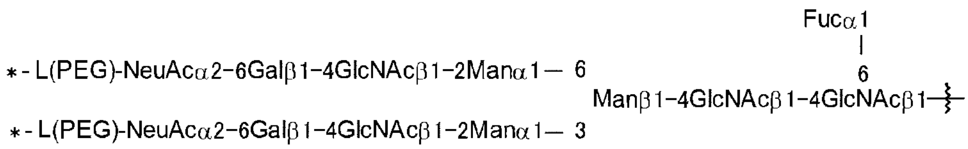

[N297-(Fuc)SG]

wherein

each wavy line represents bonding to Asn297 of the antibody,
L(PEG) in the N297 glycan represents $-NH-CH_2CH_2-(O-CH_2CH_2)_3-*$,
wherein the amino group at the left end is bound via an amide bond to carboxylic acid at the 2-position of a sialic acid at the non-reducing terminal of each or either one of the 1-3 or/and 1-6 branched chains of β-Man in the N297 glycan, and the asterisk (*) at the right end represents bonding to a nitrogen atom at the 1- or 3-position of the triazole ring in the corresponding structural formula.

[23] An antibody-drug conjugate represented by the following formula:

[Formula 19]

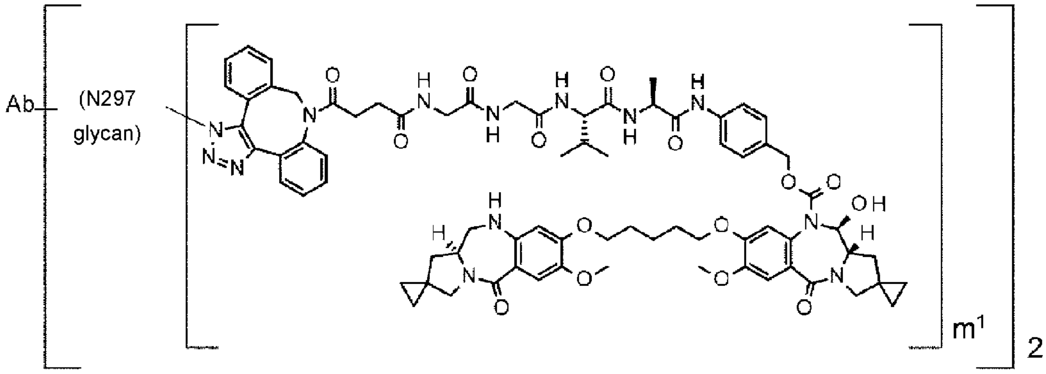

or

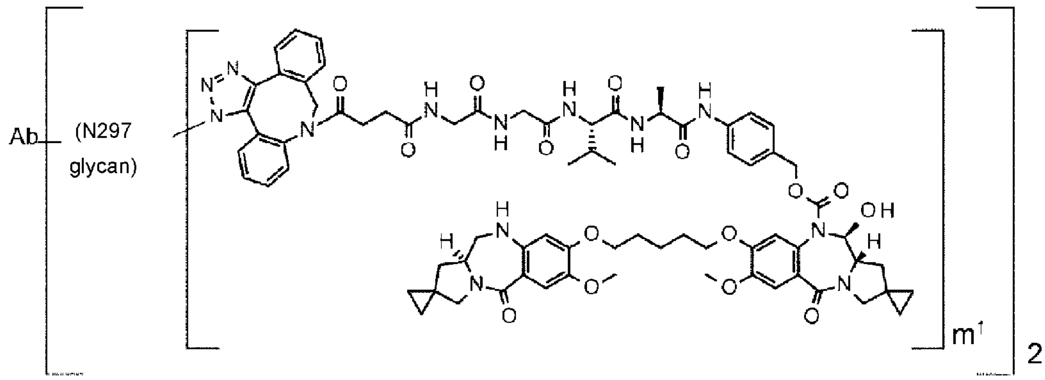

wherein, in each structural formula shown above,

$m^1$ represents an integer of 1 or 2;
Ab represents an antibody or a functional fragment of the antibody, wherein the antibody specifically binds to HER2 and comprises a heavy chain comprising CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 3 or an amino acid sequence having one to several amino acid substitutions in the amino acid sequence represented by SEQ ID NO: 3, and a light chain comprising CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 7 or an amino acid sequence having one to several amino acid substitutions in the amino acid sequence represented by SEQ ID NO: 7,
the N297 glycan is any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 20]

Fucα1
|
6
Galβ1−4GlcNAcβ1−2Manα1− 6
Manβ1−4GlcNAcβ1−4GlcNAcβ1−
∗ −L(PEG)-NeuAcα2−6Galβ1−4GlcNAcβ1−2Manα1− 3

[N297-(Fuc)MSG1]

[Formula 21]

Fucα1
|
6
∗ - L(PEG)-NeuAcα2-6Galβ1-4GlcNAcβ1-2Manα1— 6
Manβ1-4GlcNAcβ1-4GlcNAcβ1—
Galβ1-4GlcNAcβ1-2Manα1— 3

[N297-(Fuc)MSG2]

[Formula 22]

Fucα1
|
6
∗ - L(PEG)-NeuAcα2-6Galβ1-4GlcNAcβ1-2Manα1— 6
Manβ1-4GlcNAcβ1-4GlcNAcβ1—
∗ - L(PEG)-NeuAcα2-6Galβ1-4GlcNAcβ1-2Manα1— 3

[N297-(Fuc)SG]

wherein

each wavy line represents bonding to Asn297 of the antibody,
L(PEG) in the N297 glycan represents $-NH-CH_2CH_2-(O-CH_2CH_2)_3-*$,
wherein the amino group at the left end is bound via an amide bond to carboxylic acid at the 2-position of a sialic acid at the non-reducing terminal of each or either one of the 1-3 or/and 1-6 branched chains of β-Man in the N297 glycan, and the asterisk (*) at the right end represents bonding to a nitrogen atom at the 1- or 3-position of the triazole ring in the corresponding structural formula.

[24] An antibody-drug conjugate represented by the following formula:

[Formula 23]

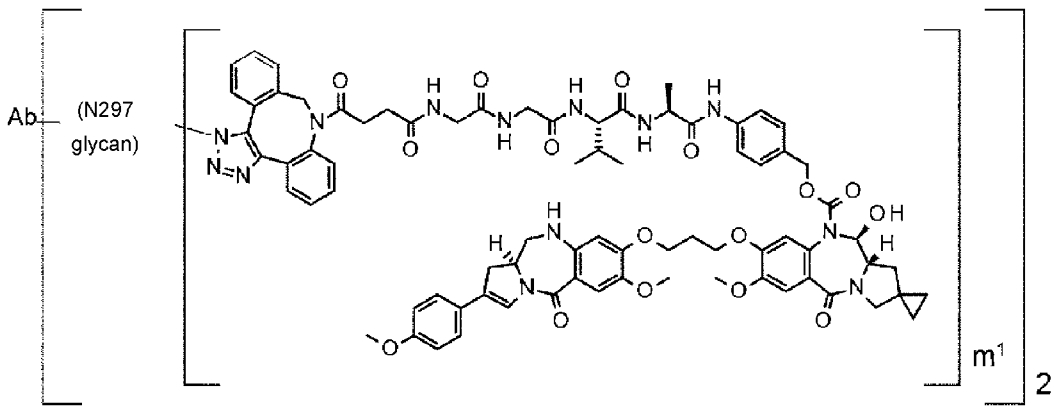

or

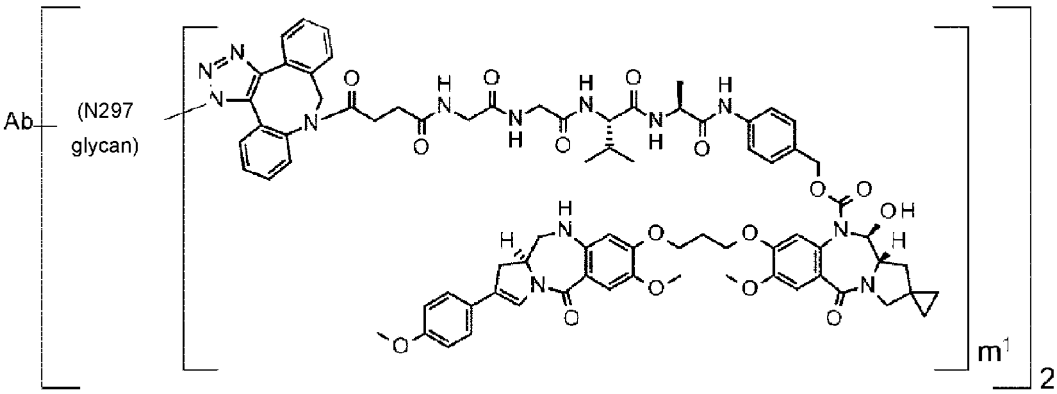

wherein, in each structural formula shown above,

$m^1$ represents an integer of 1 or 2;

Ab represents an antibody or a functional fragment of the antibody, wherein the antibody specifically binds to HER2 and comprises a heavy chain comprising CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 3 or an amino acid sequence having one to several amino acid substitutions in the amino acid sequence represented by SEQ ID NO: 3, and a light chain comprising CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 7 or an amino acid sequence having one to several amino acid substitutions in the amino acid sequence represented by SEQ ID NO: 7,

the N297 glycan is any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 24]

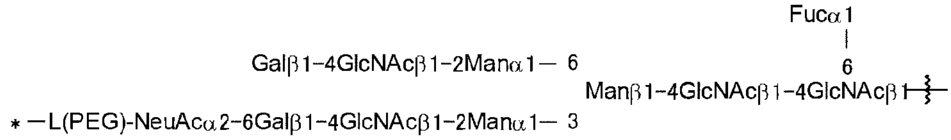

[N297-(Fuc)MSG1]

[Formula 25]

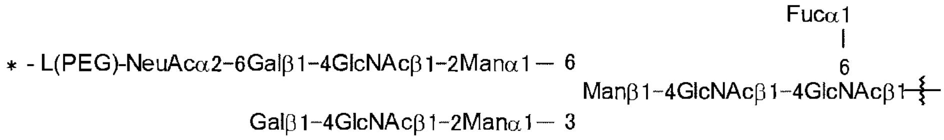

[N297-(Fuc)MSG2]

[Formula 26]

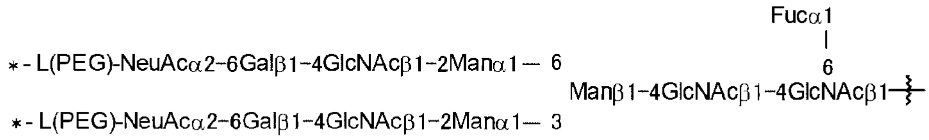

[N297-(Fuc)SG]

wherein

each wavy line represents bonding to Asn297 of the antibody,
L(PEG) in the N297 glycan represents $-NH-CH_2CH_2-(O-CH_2CH_2)_3-*$,
wherein the amino group at the left end is bound via an amide bond to carboxylic acid at the 2-position of a sialic acid at the non-reducing terminal of each or either one of the 1-3 or/and 1-6 branched chains of β-Man in the N297 glycan, and the asterisk (*) at the right end represents bonding to a nitrogen atom at the 1- or 3-position of the triazole ring in the corresponding structural formula.

[25] An antibody-drug conjugate represented by the following formula:

[Formula 27]

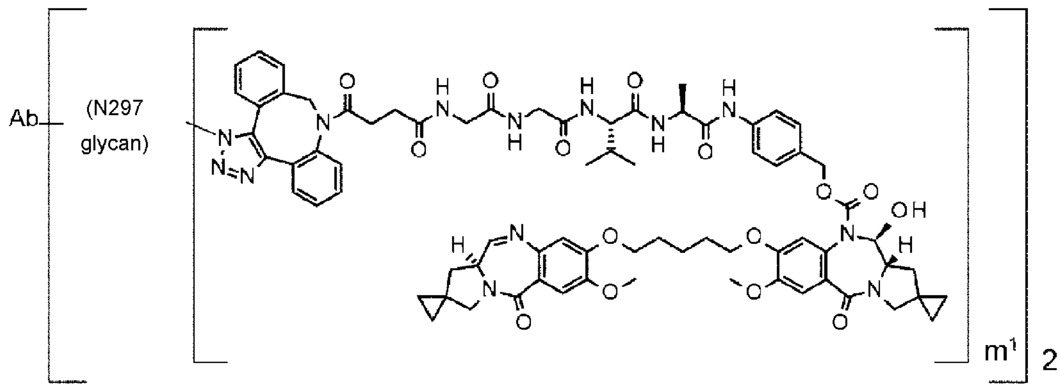

or

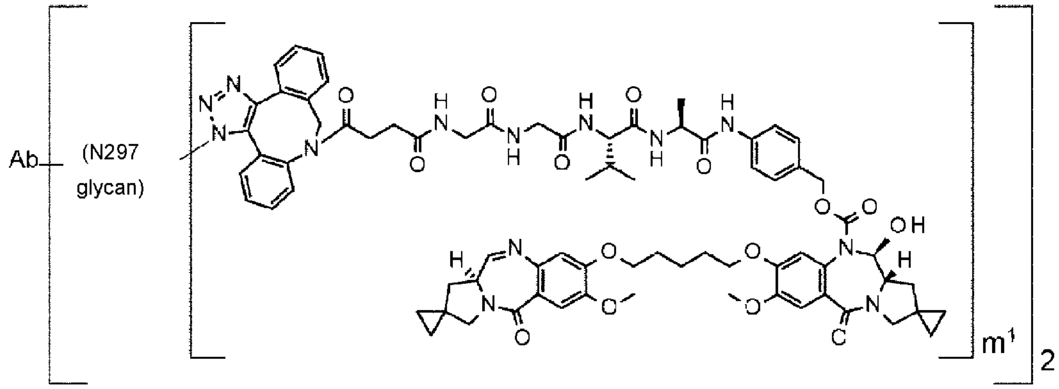

wherein, in each structural formula shown above,

$m^1$ represents an integer of 1 or 2;

Ab represents an antibody or a functional fragment of the antibody, wherein the antibody specifically binds to HER2 and comprises a heavy chain comprising CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 3 or an amino acid sequence having one to several amino acid substitutions in the amino acid sequence represented by SEQ ID NO: 3, and a light chain comprising CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 7 or an amino acid sequence having one to several amino acid substitutions in the amino acid sequence represented by SEQ ID NO: 7,

the N297 glycan is any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 28]

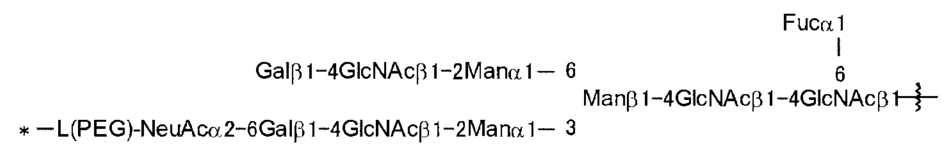

[N297-(Fuc)MSG1]

[Formula 29]

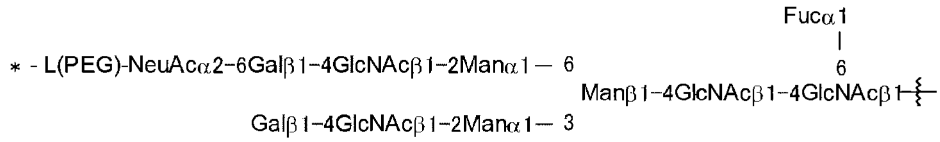

[N297-(Fuc)MSG2]

[Formula 30]

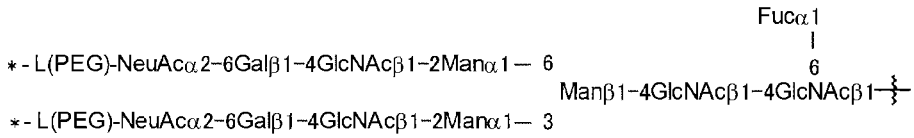

[N297-(Fuc)SG]

wherein

each wavy line represents bonding to Asn297 of the antibody,
L(PEG) in the N297 glycan represents $-NH-CH_2CH_2-(O-CH_2CH_2)_3-*$,
wherein the amino group at the left end is bound via an amide bond to carboxylic acid at the 2-position of a sialic acid at the non-reducing terminal of each or either one of the 1-3 or/and 1-6 branched chains of β-Man in the N297 glycan, and the asterisk (*) at the right end represents bonding to a nitrogen atom at the 1- or 3-position of the triazole ring in the corresponding structural formula.

[26] The antibody-drug conjugate according to any one of [22] to [25], wherein the antibody comprises a heavy chain comprising CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 4, and a light chain comprising CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 8.
[27] The antibody-drug conjugate according to any one of [22] to [25], wherein the antibody comprises a heavy chain comprising CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 3, and a light chain comprising CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 8.
[28] The antibody-drug conjugate according to any one of [22] to [25], wherein the antibody comprises a heavy chain comprising CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 3, and a light chain comprising CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 7.
[29] The antibody-drug conjugate according to any one of [22] to [26], wherein the antibody comprises a heavy chain variable region consisting of an amino acid sequence represented by SEQ ID NO: 17 and a light chain variable region consisting of an amino acid sequence represented by SEQ ID NO: 25.
[30] The antibody-drug conjugate according to any one of [22] to [25] and [27], wherein the antibody comprises a heavy chain variable region consisting of an amino acid sequence represented by SEQ ID NO: 13 and a light chain variable region consisting of an amino acid sequence represented by SEQ ID NO: 29.
[31] The antibody-drug conjugate according to any one of [22] to [25] and [28], wherein the antibody comprises a heavy chain variable region consisting of an amino acid sequence represented by SEQ ID NO: 13 and a light chain variable region consisting of an amino acid sequence represented by SEQ ID NO: 21.
[32] The antibody-drug conjugate according to any one of [22] to [26] and [29], wherein the antibody comprises a heavy chain consisting of an amino acid sequence consisting of amino acid residues 20 to 469 of SEQ ID NO: 15

and a light chain consisting of an amino acid sequence consisting of amino acid residues 21 to 234 of SEQ ID NO: 23.
[33] The antibody-drug conjugate according to any one of [22] to [25], [27], and [30], wherein the antibody comprises a heavy chain consisting of an amino acid sequence consisting of amino acid residues 20 to 469 of SEQ ID NO: 11 and a light chain consisting of an amino acid sequence consisting of amino acid residues 21 to 234 of SEQ ID NO: 27.
[34] The antibody-drug conjugate according to any one of [22] to [25], [28], and [31], wherein the antibody comprises a heavy chain consisting of an amino acid sequence consisting of amino acid residues 20 to 469 of SEQ ID NO: 11 and a light chain consisting of an amino acid sequence consisting of amino acid residues 21 to 234 of SEQ ID NO: 32.
[35] The antibody-drug conjugate according to any one of [22] to [25], [28], and [31], wherein the antibody comprises a heavy chain consisting of an amino acid sequence consisting of amino acid residues 20 to 469 of SEQ ID NO: 31 and a light chain consisting of an amino acid sequence consisting of amino acid residues 21 to 234 of SEQ ID NO: 32.
[36] The antibody-drug conjugate according to any one of [22] to [35], wherein the antibody comprises one or two or more modifications selected from the group consisting of N-linked glycosylation, O-linked glycosylation, N-terminal processing, C-terminal processing, deamidation, isomerization of aspartic acid, oxidation of methionine, addition of a methionine residue at an N terminus, amidation of a proline residue, and deletion of one or two amino acid residues at the carboxyl terminus of a heavy chain.
[37] An antibody or a functional fragment of the antibody, wherein the antibody specifically binds to HER2 and comprises a heavy chain comprising CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 3 or an amino acid sequence having one to several amino acid substitutions in the amino acid sequence represented by SEQ ID NO: 3, and a light chain comprising CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 7 or an amino acid sequence having one to several amino acid substitutions in the amino acid sequence represented by SEQ ID NO: 7.
[38] The antibody according to [37] or a functional fragment of the antibody, the antibody comprising a heavy chain comprising CDRH1, CDRH2, and CDRH3 and a light chain comprising CDRL1, CDRL2, and CDRL3 as described in any one of the following (a) and (b):

(a) CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 4, and CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 8; and
(b) CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 3, and CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 8.

[39] The antibody according to [37] or [38] or a functional fragment of the antibody, the antibody comprising a heavy chain variable region consisting of an amino acid sequence selected from the group consisting of the following (a) to (e) and a light chain variable region consisting of an amino acid sequence selected from the group consisting of the following (f) to (k):

(a) an amino acid sequence represented by SEQ ID NO: 13;
(b) an amino acid sequence represented by SEQ ID NO: 17;
(c) an amino acid sequence with a homology of at least 95% or higher to a sequence of a framework region excluding CDR sequences in any one of the sequences (a) and (b);
(d) an amino acid sequence having one to several amino acid deletions, substitutions, or additions in a sequence of a framework region excluding CDR sequences in any one of the sequences (a) and (b);
(e) an amino acid sequence represented by SEQ ID NO: 25;
(f) an amino acid sequence represented by SEQ ID NO: 29;
(g) an amino acid sequence with a homology of at least 95% or higher to a sequence of a framework region excluding CDR sequences in any of the sequences (e) and (f); and
(h) an amino acid sequence having one to several amino acid deletions, substitutions, or additions in a sequence of a framework region excluding CDR sequences in any of the sequences (e) and (f).

[40] The antibody according to [39] or a functional fragment of the antibody, the antibody comprising a heavy chain

variable region and a light chain variable region as described in any one of the following (a) and (b):

(a) a heavy chain variable region consisting of an amino acid sequence represented by SEQ ID NO: 17 and a light chain variable region consisting of an amino acid sequence represented by SEQ ID NO: 25; and
(b) a heavy chain variable region consisting of an amino acid sequence represented by SEQ ID NO: 13 and a light chain variable region consisting of an amino acid sequence represented by SEQ ID NO: 29.

[41] The antibody according to any one of [37] to [40] or a functional fragment of the antibody, the antibody being a chimeric antibody or a human antibody.
[42] The antibody according to any one of [37] to [40] or a functional fragment of the antibody, the antibody being a humanized antibody.
[43] The antibody according to [41] or [42] or a functional fragment of the antibody, the antibody comprising a heavy chain constant region of human IgG1, human IgG2, or human IgG4.
[44] The antibody according to [43] or a functional fragment of the antibody, wherein the heavy chain constant region is a heavy chain constant region of human IgG1, and leucine at the 234- and 235-positions specified by EU Index numbering in the heavy chain constant region is substituted with alanine.
[45] The antibody according to any one of [42] to [44] or a functional fragment of the antibody, the antibody comprising a heavy chain and a light chain as described in the following (a) or (b):

(a) a heavy chain consisting of an amino acid sequence consisting of amino acid residues 20 to 469 of SEQ ID NO: 15 and a light chain consisting of an amino acid sequence consisting of amino acid residues 21 to 234 of SEQ ID NO: 23 (H01L02); and
(b) a heavy chain consisting of an amino acid sequence consisting of amino acid residues 20 to 469 of SEQ ID NO: 11 and a light chain consisting of an amino acid sequence consisting of amino acid residues 21 to 234 of SEQ ID NO: 27 (HwtL05).

[46] An antibody or a functional fragment of the antibody, wherein the antibody competes with the antibody according to any one of [37] to [45] for binding to HER2, or binds to a site of HER2 recognizable to the antibody according to any one of [37] to [45].
[47] A polynucleotide encoding the antibody according to any one of [37] to [46] or a functional fragment of the antibody.
[48] An expression vector comprising the polynucleotide according to [47].
[49] A host cell transformed with the expression vector according to [48].
[50] The host cell according to [49], wherein the host cell is a eukaryotic cell.
[51] The host cell according to [49] or [50], wherein the host cell is an animal cell.
[52] A method for producing the antibody according to any one of [37] to [46] or a functional fragment of the antibody, the method comprising the steps of: culturing the host cell according to any one of [49] to [51]; and collecting a targeted antibody from the culture obtained in the step of culturing.
[53] An antibody obtained by using the method according to [52], or a functional fragment of the antibody.
[54] The antibody according to any one of [37] to [46] and [53] or a functional fragment of the antibody, the antibody comprising one or two or more modifications selected from the group consisting of N-linked glycosylation, O-linked glycosylation, N-terminal processing, C-terminal processing, deamidation, isomerization of aspartic acid, oxidation of methionine, addition of a methionine residue at an N terminus, amidation of a proline residue, and deletion of one or two amino acid residues at the carboxyl terminus of a heavy chain.
[55] The antibody according to [54] or a functional fragment of the antibody, wherein one or several amino acid residues are deleted at the carboxyl terminus of a heavy chain.
[56] The antibody according to [55] or a functional fragment of the antibody, wherein one amino acid residue is deleted at the carboxyl terminus of each of the two heavy chains.
[57] The antibody according to any one of [53] to [56] or a functional fragment of the antibody, wherein a proline residue at the carboxyl terminus of a heavy chain is further amidated.
[58] A method for producing a glycan-remodeled antibody, the method comprising the steps of:

i) culturing the host cell according to any one of [49] to [51] and collecting a targeted antibody from the culture obtained;
ii) treating the antibody obtained in step i) with hydrolase to produce a (Fucα1,6)GlcNAc-antibody; and
iii) reacting the (Fucα1,6)GlcNAc-antibody with a glycan donor molecule in the presence of transglycosidase, the glycan donor molecule obtained by introducing a PEG linker having an azide group to the carbonyl group of carboxylic acid at the 2-position of a sialic acid in MSG (9) or SG (10) and oxazolinating the reducing terminal.

[59] The method according to [58], further comprising the step of purifying the (Fuc$\alpha$1,6)GlcNAc-antibody through purification of a reaction solution in step ii) with a hydroxyapatite column.
[60] A glycan-remodeled antibody obtained by using the method according to [58] or [59].
[61] A method for producing the antibody-drug conjugate according to any one of [1] to [36], the method comprising a step of reacting the glycan-remodeled antibody according to [60] and a drug-linker.
[62] An antibody-drug conjugate obtained by using the method according to [60].
[63] The antibody-drug conjugate according to any one of [1] to [36], wherein the antibody is the antibody according to any one of [53] to [57] and [60].
[64] The antibody-drug conjugate according to any one of [1] to [36], [62], and [63], wherein the N297 glycan is N297-(Fuc)MSG1.
[65] The antibody-drug conjugate according to any one of [1] to [36], and [62] to [64], wherein $m^1$ is an integer of 1.
[66] The antibody-drug conjugate according to any one of [1] to [36] and [62] to [65], wherein the average number of conjugated drug molecules per antibody molecule in the antibody-drug conjugate is 1 to 3 or 3 to 5.
[67] A pharmaceutical composition comprising the antibody-drug conjugate according to any one of [1] to [36] and [62] to [66], or the antibody according to any one of [37] to [47], [53] to [57], and [60] or a functional fragment of the antibody.
[68] The pharmaceutical composition according to [67], being an antitumor drug.
[69] The pharmaceutical composition according to [68], wherein the tumor is expressing HER2.
[70] A method for treating a tumor, wherein the antibody-drug conjugate according to any one of [1] to [36] and [62] to [66], or the antibody according to any one of [37] to [47], [53] to [57], and [60] or a functional fragment of the antibody is administered to an individual.
[71] The method for treating a tumor according to [70], wherein the tumor is expressing HER2.
[72] A method for treating a tumor, wherein a pharmaceutical composition comprising the antibody-drug conjugate according to any one of [1] to [36] and [62] to [66], or the antibody according to any one of [37] to [47], [53] to [57], and [60] or a functional fragment of the antibody, and at least one antitumor drug are administered to an individual simultaneously, separately, or consecutively.
[73] The antibody according to any one of [37] to [47], [53] to [57], and [60] or a functional fragment of the antibody, wherein the antibody is conjugated to an additional compound.

## ADVANTAGEOUS EFFECTS OF INVENTION

The novel anti-HER2 antibody-pyrrolobenzodiazepine (PBD) derivative conjugate provided by the present invention is superior in antitumor activity and safety, and hence useful as an antitumor agent. In addition, the novel anti-HER2 antibody of the present invention recognizes an antigen expressed on tumor cells or binds to the antigen, and hence is useful as an antibody for the conjugate.

## BRIEF DESCRIPTION OF DRAWINGS

[Figure 1] Figure 1 is a schematic diagram of the antibody-drug conjugate of the present invention (the molecule of (I)). (a) indicates drug D, (b) indicates linker L, (c) indicates $N_3$-L(PEG)-, and (d) indicates N297 glycan (open ellipse: NeuAc(Sia), open hexagon: Man, filled hexagon: GlcNAc, open diamond: Gal, open inverted triangle: Fuc). (b) and (c) are combined together to form a triazole ring by reaction between the azide group (filled teardrop shape) of (c) and the spacer (open semicircle) of (b). The Y-shaped diagram represents antibody Ab. For convenience, in this schematic diagram, N297 glycan is indicated as N297-(Fuc)MSG and the diagram shows an embodiment wherein any one of two branches in each of N297 glycans has a sialic acid to which a PEG linker having an azide group ($N_3$-L(PEG)-) bonds while the other branch has no sialic acid at the non-reducing terminal (i.e. N297-(Fuc)MSG); however, another embodiment wherein each of two branches of N297 glycan has a sialic acid to which a PEG linker having an azide group bonds at the non-reducing terminal (i.e. N297-(Fuc)SG) is also acceptable. Unless otherwise stated, such a manner of illustration is applied throughout the present specification.
[Figure 2] Figure 2 is schematic diagrams illustrating the structures of a (Fuc$\alpha$1,6)GlcNAc-antibody (the molecule of A in (II) of Figure 2), which is a production intermediate of the antibody-drug conjugate of the present invention, and an MSG-type glycan-remodeled antibody (the molecule of (III) in B of Figure 2). In each of the diagrams, the Y-shaped diagram represents antibody Ab as in Figure 1. In A in Figure 2, (e) indicates N297 glycan consisting only of GlcNAc at the 6-position connected to 1-positions of Fuc via an $\alpha$ glycosidic bond. In B in Figure 2, (d) indicates the same N297 glycan as in Figure 1, and (f) indicates a structure of a PEG linker portion having an azide group, specifically, an azide group to be bonded to liker L at the end. The bonding mode of the PEG linker having an azide

group is as described for Figure 1.

[Figure 3] Figure 3 is a schematic diagram for the step of producing an MSG-type glycan-remodeled antibody from an antibody produced in an animal cell. As in Figure 2, molecules (II) and (III) in this Figure represent an (Fucα1,6)GlcNAc-antibody and an MSG-type glycan-remodeled antibody, respectively. Molecule (IV) is an antibody produced in an animal cell, and is a mixture of molecules with heterogeneous N297 glycan moieties. Figure 3A illustrates the step of producing homogeneous (Fucα1,6)GlcNAc-antibody (II) by treating heterogeneous N297 glycan moieties of (IV) with hydrolase such as EndoS. Figure 3B illustrates the step of producing the MSG-type glycan-remodeled antibody of (III) by subjecting GlcNAc of N297 glycan in antibody (II) to transglycosidase such as an EndoS D233Q/Q303L variant to transglycosylate the glycan of an MSG-type glycan donor molecule. The MSG-type glycan donor molecule used here has a sialic acid at the non-reducing terminal of MSG modified with a PEG linker having an azide group. Thus, resulting MSG-type N297 glycan-remodeled antibody also has a sialic acid at the non-reducing terminal modified in the same manner as described for Figure 2B. For convenience, Figure 3B shows MSG as a donor molecule. However, a glycan-remodeled antibody in which a linker molecule having an azide group bonds to each non-reducing terminal of N297 glycan also can be synthesized as the remodeled antibody of (III) by using SG (10) as a glycan donor.

[Figure 4] Figure 4 shows the amino acid sequences of CDRH1 to 3 of Trastuzumab A1 and A2, and an HwtL05 antibody heavy chains (Hwt) (SEQ ID NOs: 1 to 3).

[Figure 5] Figure 5 shows the amino acid sequences of CDRH1 to 3 of an H01L02 antibody heavy chain (H01) (SEQ ID NOs: 1, 2, 4).

[Figure 6] Figure 6 shows the amino acid sequences of CDRL1 to 3 of Trastuzumab A1 and A2 light chains (SEQ ID NOs: 5, 6 (amino acid sequence consisting of amino acid residues 1 to 3), 7).

[Figure 7] Figure 7 shows the amino acid sequences of CDRL1 to 3 of H01L02 antibody and HwtL05 antibody light chains (L02, L05) (SEQ ID NOs: 5, 6, 8).

[Figure 8] Figure 8 shows the amino acid sequence of Trastuzumab A1 heavy chain (Hwt) (SEQ ID NO: 11).

[Figure 9] Figure 9 shows the amino acid sequence of the variable region of A1, A2 and HwtL05 heavy chains (SEQ ID NO: 13). Each underline in the amino acid sequence indicates a CDR sequence.

[Figure 10] Figure 10 shows the amino acid sequence of an H01L02 antibody heavy chain (H01) (SEQ ID NO: 15).

[Figure 11] Figure 11 shows the amino acid sequence of the variable region of an H01L02 antibody heavy chain (SEQ ID NO: 17). Each underline in the amino acid sequence indicates a CDR sequence.

[Figure 12] Figure 12 shows the amino acid sequence of Trastuzumab A1 light chain (Lwt) (SEQ ID NO: 19).

[Figure 13] Figure 13 shows the amino acid sequence of the variable region of Trastuzumab A1 and A2 light chain (SEQ ID NO: 21). Each underline in the amino acid sequence indicates a CDR sequence.

[Figure 14] Figure 14 shows the amino acid sequence of an H01L02 antibody light chain (L02) (SEQ ID NO: 23).

[Figure 15] Figure 15 shows the amino acid sequence of the variable region of an H01L02 antibody light chain (SEQ ID NO:25). Each underline in the amino acid sequence indicates a CDR sequence.

[Figure 16] Figure 16 shows the amino acid sequence of an HwtL05 antibody light chain (L05) (SEQ ID NO: 27).

[Figure 17] Figure 17 shows the amino acid sequence of the variable region of an HwtL05 antibody light chain (SEQ ID NO: 29). Each underline in the amino acid sequence indicates a CDR sequence.

[Figure 18] Figure 18 shows the effects of the anti-HER2 antibody-drug conjugates ADC1 and ADC2 on subcutaneously transplanted KPL-4 cells, a human breast cancer cell line.

[Figure 19] Figure 19 shows the effects of the anti-HER2 antibody-drug conjugates ADC1 and ADC2 on subcutaneously transplanted JIMT-1 cells, a human breast cancer cell line.

[Figure 20] Figure 20 shows the effects of the anti-HER2 antibody-drug conjugates ADC1 and ADC2 on subcutaneously transplanted CFPAC-1 cells, a human pancreatic cancer cell line.

[Figure 21] Figure 21 shows the amino acid sequence of Trastuzumab A2 heavy chain (SEQ ID NO:31).

[Figure 22] Figure 22 shows the amino acid sequence of Trastuzumab A2 light chain (SEQ ID NO: 32).

[Figure 23] Figure 23 shows the effect of the anti-HER2 antibody-drug conjugate ADC5 on subcutaneously transplanted KPL-4 cells, a human breast cancer cell line.

## DESCRIPTION OF EMBODIMENTS

[Antibody-drug conjugate]

The anti-HER2 antibody-drug conjugate of the present invention is an antitumor drug having an antitumor compound conjugated via a linker structure moiety to an antibody capable of recognizing an antigen expressed on tumor cells or binding to the antigen.

The antibody-drug conjugate of the present invention is represented by the following formula:

[Formula 31]

$$\mathrm{Ab}\left[\text{(N297 glycan)}\left[\mathrm{L}-\mathrm{D}\right]_{m^1}\right]_2$$

wherein

$m^1$ is an integer of 1 or 2 (preferably, 1), D represents a drug, L represents a linker linking the N297 glycan and D, Ab represents an antibody or a functional fragment of the antibody, and the N297 glycan represents a glycan bonding to the side chain of Asn297 of the antibody. The N297 glycan may be a remodeled glycan.

<Drug>

Drug D of the present invention is preferably an antitumor compound. The antitumor compound develops antitumor effect, when a part or the entire of the linker of the antibody-drug conjugate of the present invention is cleaved in a tumor cell and the antitumor compound moiety is released.

The drug in the antibody-drug conjugate of the present invention, namely, the PBD derivative is any one selected from the following group:

[Formula 32]

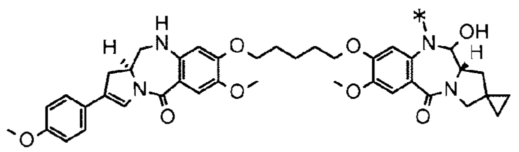

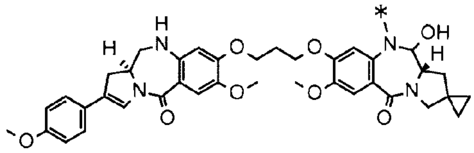

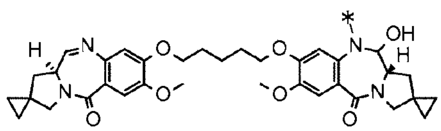

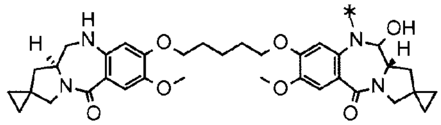

wherein each asterisk (*) represents bonding to L.

As shown in partial structures I(a) or I(b) below, the PBD derivative of the present invention has an asymmetric carbon at the 11'-position, and thus there exist optical isomers.

[Formula 33]

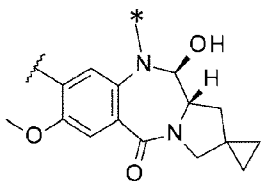

I (a)

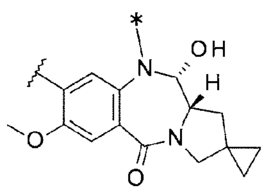

I (b)

Accordingly, the PBD derivative of the present invention in each case includes the optical isomers and mixtures of the optical isomers at any ratio. The absolute steric configuration at the 11'-position of the PBD derivative can be determined through X-ray crystal structure analysis or NMR such as a Mosher method for its crystalline product or intermediate, or a derivative thereof. Then, the absolute steric configuration may be determined by using a crystalline product or intermediate derivatized with a reagent having an asymmetric center whose steric configuration is known. As desired, stereoisomers of the synthesized compound according to the present invention may be obtained by isolating with a common optical resolution method or separation method.

There may exist stereoisomers, optical isomers due to an asymmetric carbon atom, geometric isomers, tautomers, or optical isomers such as d-forms, 1-forms and atropisomers for the antibody-drug conjugate of the present invention, and a free drug or production intermediate of the antibody-drug conjugate, and these isomers, optical isomers, and mixtures of them are all included in the present invention.

I(a) is preferred as the partial structure of the PBD derivative of the present invention. For example, the partial structure of the PBD derivative of the present invention is any one selected from the following group:

[Formula 34]

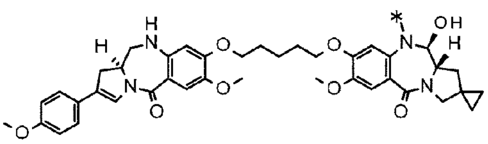

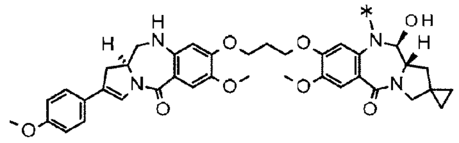

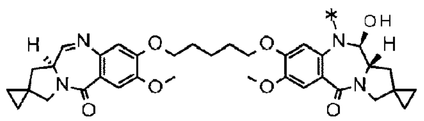

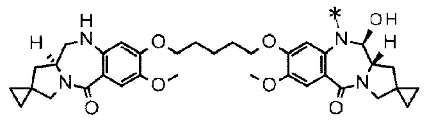

wherein each asterisk (*) represents bonding to L.

<Linker structure>

Linker L of the present invention is a linker linking the N297 glycan and D.

Linker L is represented by the following formula:

-Lb-La-Lp-NH-B-$CH_2$-O(C=O)-*

The asterisk (*) represents bonding to the nitrogen atom at the N10'-position of drug D, Lb represents a spacer which connects La to a N297 glycan or remodeled N297 glycan.

B represents a phenyl group or a heteroaryl group, and is preferably a 1,4-phenyl group, a 2,5-pyridyl group, a 3,6-pyridyl group, a 2,5-pyrimidyl group, or a 2,5-thienyl group, and more preferably a 1,4-phenyl group.

Lp represents a linker consisting of an amino acid sequence cleavable in vivo or in a target cell. Lp is, for example, cleaved by the action of an enzyme such as esterase and peptidase.

Lp is a peptide residue composed of two to seven (preferably, two to four) amino acids. That is, Lp is composed of an oligopeptide residue in which two to seven amino acids are connected via peptide bonding.

Lp is bound at the N terminal to a carbonyl group of La in Lb-La-, and forms at the C terminal an amide bond with the amino group (-NH-) of the part -NH-B-$CH_2$-O(C=O)- of the linker. The bond between the C terminal of Lp and -NH- is cleaved by the enzyme such as esterase.

The amino acids constituting Lp are not limited to particular amino acids, and, for example, are L- or D-amino acids, and preferably L-amino acids. The amino acids may be not only $\alpha$-amino acids, but may include an amino acid with structure, for example, of $\beta$-alanine, $\varepsilon$-aminocaproic acid, or $\gamma$-aminobutyric acid, and may further include a non-natural amino acid such as an N-methylated amino acid.

The amino acid sequence of Lp is not limited to a particular amino acid sequence, and examples of amino acids that constitute Lp may include, but are not limited to, glycine (Gly; G), valine (Val; V), alanine (Ala; A), phenylalanine (Phe; F), glutamic acid (Glu; E), isoleucine (Ile; I), proline (Pro; P), citrulline (Cit), leucine (Leu; L), serine (Ser; S), lysine (Lys; K), and aspartic acid (Asp; D). Preferred among them are glycine (Gly; G), valine (Val; V), alanine (Ala; A), and citrulline (Cit).

Any of these amino acids may appear multiple times, and Lp has an amino acid sequence including arbitrarily selected amino acids. Drug release pattern may be controlled via amino acid type.

Specific examples of linker Lp may include, but are not limited to, -GGVA-, - GG-(D-)VA-, -VA-, -GGFG-, -GGPI-, -GGVCit-, -GGVK-, -GG(D-)PI-, -GGPL-, - EGGVA, -PI-, -GGF-, DGGF-, (D-)D-GGF-, -EGGF-, -SGGF-, -KGGF-, -DGGFG-, - GGFGG-, -DDGGFG-, -KDGGFG-, and -GGFGGGF-.

Here, "(D-)V" indicates D-valine, "(D)-P" indicates D-proline, and "(D-)D" indicates D-aspartic acid.

Linker Lp is preferably any of the following:

-GGVA-, -GG-(D-)VA-, -VA-, -GGFG-, -GGPI-, -GGVCit-, -GGVK-, -GG(D-)PI-, and -GGPL-.

Linker Lp is more preferably any of the following:
-GGVA-, -GGVCit-, and -VA-.

La represents any one selected from the following group: $-C(=O)-(CH_2CH_2)n^2-C(=O)-$, $-C(=O)-(CH_2CH_2)n^2-C(=O)-NH-(CH_2CH_2)n^3-C(=O)-$, $-C(=O)-(CH_2CH_2)n^2-C(=O)-NH-(CH_2CH_2O)n^3-CH_2-C(=O)-$, $-C(=O)-(CH_2CH_2)n^2-NH-C(=O)-(CH_2CH_2O)n^3-CH_2CH_2-C(=O)-$, and $-(CH_2)n^4-O-$ $C(=O)-$
wherein,
$n^2$ represents an integer of 1 to 3 (preferably, 1 or 2), $n^3$ represents an integer of 1 to 5 (preferably, an integer of 2 to 4, more preferably, 2 or 4), and $n^4$ represents an integer of 0 to 2 (preferably, 0 or 1).

La preferably represents any one selected from the following group: $-C(=O)-CH_2CH_2-C(=O)-$, $-C(=O)-(CH_2CH_2)_2-C(=O)-$, $-C(=O)-CH_2CH_2-C(=O)-NH-(CH_2CH_2)_2-C(=O)-$ $-C(=O)-CH_2CH_2-C(=O)-NH-(CH_2CH_2O)_2-CH_2-C(=O)-$, $-C(=O)-CH_2CH_2-NH-C(=O)-(CH_2CH_2O)_4-CH_2CH_2-C(=O)-$, $-CH_2-OC(=O)-$, and $-OC(=O)-$, and
La is more preferably $-C(=O)-CH_2CH_2-C(=O)-$ or $-C(=O)-(CH_2CH_2)_2-C(=O)-$.

Spacer Lb is not limited to a particular spacer, and examples thereof may include, but are not limited to, a spacer represented by the following formulas.

[Formula 35]

(Lb-1) 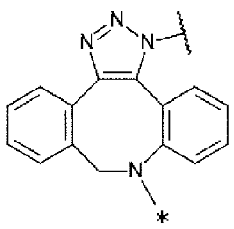 or 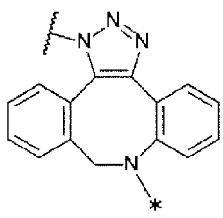

[Formula 36]

(Lb-2) 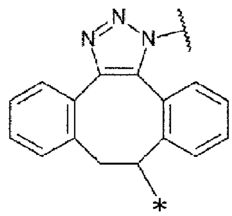 or 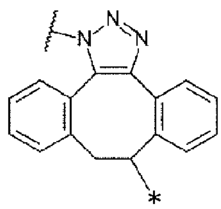

[Formula 37]

(Lb-3) 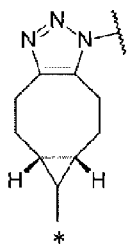 or 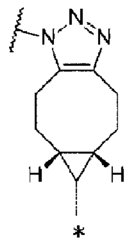

In the structural formulas for Lb shown above, each asterisk (*) represents bonding to -(C=O) or $-(CH_2)n^4$ at the left end of La, and each wavy line represents bonding to a glycan or remodeled glycan of Ab.

In each structural formula for Lb (Lb-1, Lb-2, or Lb-3) shown above, the triazole ring site formed through click reaction of an azide group and DBCO (Dibenzocyclooctyne) provides structures of geometric isomers, and one Lb exists as any one of the two structures or as a mixture of both of them. That is, there exist two or four ($m^1$ is 1 or 2) "-L-D" moieties per molecule of the antibody-drug conjugate of the present invention, and either one of the two structures exists or both of them coexist as Lb (Lb-1, Lb-2, or Lb-3) in L of each of the two or four "-L-D" moieties.

L is preferably represented by $-Lb-La-Lp-NH-B-CH_2-O(C=O)-*$, wherein

B is a 1,4-phenyl group,
Lp represents any one selected from the following group:
-GGVA-, -GG-(D-)VA-, -VA-, -GGFG-, -GGPI-, -GGVCit-, -GGVK-, and -GGPL-,
La represents any one selected from the following group: $-C(=O)-CH_2CH_2-C(=O)-$, $-C(=O)-(CH_2CH_2)_2-C(=O)-$, $-C(=O)-CH_2CH_2-C(=O)-NH-(CH_2CH_2)_2-C(=O)-$, $-C(=O)-CH_2CH_2-C(=O)-NH-(CH_2CH_2O)_2-CH_2-C(=O)-$, $-C(=O)-CH_2CH_2-NH-C(=O)-(CH_2CH_2O)_4-CH_2CH_2-C(=O)-$, $-CH_2-OC(=O)-$, and - OC(=O)-, and
Lb represents any of the structural formulas above for Lb.

L is more preferably any one selected from the following group: $-Z^1-C(=O)-CH_2CH_2-C(=O)-$GGVA-NH-B-$CH_2-OC(=O)-$, $-Z^1-C(=O)-CH_2CH_2-C(=O)-$GG-(D-)VA-NH-B-$CH_2-OC(=O)-$, $-Z^1-C(=O)-CH_2CH_2-C(=O)-$VA-NH-B-$CH_2-OC(=O)-$, $-Z^1-C(=O)-(CH_2CH_2)_2-C(=O)-$VA-NH-B-$CH_2-OC(=O)-$, $-Z^1-C(=O)-CH_2CH_2-C(=O)-$GGPI-NH-B-$CH_2-OC(=O)-$, $-Z^1-C(=O)-CH_2CH_2-C(=O)-$GGFG-NH-B-$CH_2-OC(=O)-$, $-Z^1-C(=O)-CH_2CH_2-C(=O)-$GGVCit-NH-B-$CH_2-OC(=O)-$, $-Z^1-C(=O)-CH_2CH_2-C(=O)-$GGVK-NH-B-$CH_2-OC(=O)-$, $-Z^1-C(=O)-CH_2CH_2-C(=O)-$GGPL-NH-B-$CH_2-OC(=O)-$, $-Z^1-C(=O)-CH_2CH_2-C(=O)-NH-(CH_2CH_2)_2-C(=O)-$VA-NH-B-$CH_2-OC(=O)-$, $-Z^1-C(=O)-CH_2CH_2-C(=O)-NH-(CH_2CH_2O)_2-CH_2-C(=O)-$VA-NH-B-$CH_2-OC(=O)-$, $-Z^1-C(=O)-CH_2CH_2-NH-C(=O)-(CH_2CH_2O)_4-CH_2CH_2-C(=O)-$VA-NH-B-$CH_2-OC(=O)-$, $-Z^2-OC(=O)-$GGVA-NH-B-$CH_2-OC(=O)-$, $-Z^3-CH_2-OC(=O)-$GGVA-NH-B-$CH_2-OC(=O)-$wherein

$Z^1$ represents the following structural formula as described for Lb;

[Formula 38]

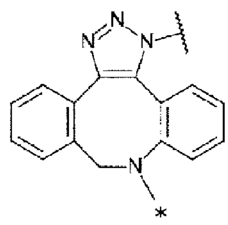

or

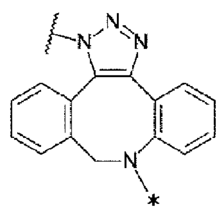

$Z^2$ represents the following structural formula as described for Lb:

[Formula 39]

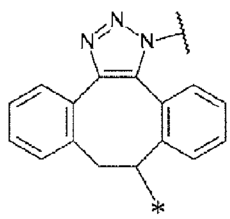

or

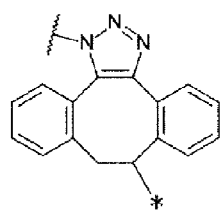

$Z^3$ represents the following structural formula as described for Lb:

[Formula 40]

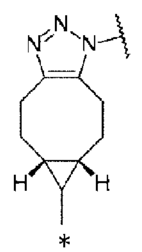

or

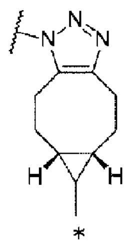

B is a 1,4-phenyl group.

L is most preferably any of the following: $-Z^1-C(=O)-CH_2CH_2-C(=O)-GGVA-NH-B-CH_2-OC(=O)-$, $-Z^1-C(=O)-CH_2CH_2-C(=O)-VA-NH-B-CH_2-OC(=O)-$, $-Z^1-C(=O)-(CH_2CH_2)_2-C(=O)-VA-NH-B-CH_2-OC(=O)-$, $-Z^1-C(=O)-CH_2CH_2-C(=O)-GGVCit-NH-B-CH_2-OC(=O)-$, $-Z^1-C(=O)-CH_2CH_2-C(=O)-NH-(CH_2CH_2)_2-C(=O)-VA-NH-B-CH_2-OC(=O)-$, $-Z^1-C(=O)-CH_2CH_2-C(=O)-NH-(CH_2CH_2O)_2-CH_2-C(=O)-VA-NH-B-CH_2-OC(=O)-$, and $-Z^1-C(=O)-CH_2CH_2-NH-C(=O)-(CH_2CH_2O)_4-CH_2CH_2-C(=O)-VA-NH-B-CH_2-OC(=O)-$, wherein
B is a 1,4-phenyl group, and $Z^1$ represents the following structural formula as described for Lb:

[Formula 41]

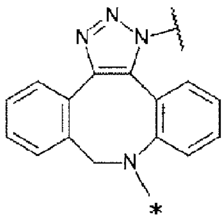

or

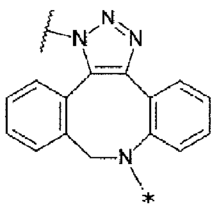

<Free drug>

The free drug of the antibody-drug conjugate of the present invention is one selected from the following group:

[Formula 42]

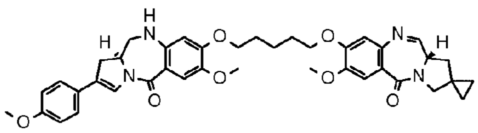

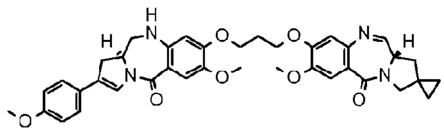

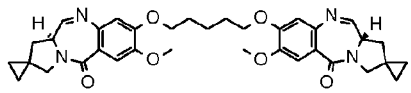

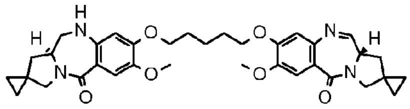

The free drug of the present invention is generated through a process in which the antibody-drug conjugate of the present invention migrates into tumor cells and the portion of linker L in the antibody-drug conjugate is then cleaved. This free drug was found to have anti-tumor cell effect.

<Antibody>

In the present invention, "cancer" and "tumor" are used for the same meaning.

In the present invention, a "gene" refers to nucleotides or a nucleotide sequence including a nucleotide sequence encoding amino acids of protein or a complementary strand thereof. The meaning of a "gene" encompasses, for example, a polynucleotide, an oligonucleotide, DNA, mRNA, cDNA, and RNA as a nucleotide sequence including a nucleotide sequence encoding amino acids of protein or a complementary strand thereof. Examples of the "HER2 gene" may include, but are not limited to, DNA, mRNA, cDNA, and cRNA including a nucleotide sequence encoding the amino acid sequence of HER2 protein.

In the present invention, "nucleotides", "polynucleotide", and "nucleotide sequence" have the same meaning as that of "nucleic acids", and the meaning of "nucleotides" and "nucleotide sequence" encompasses, for example, DNA, RNA, a probe, an oligonucleotide, a polynucleotide, and a primer.

In the present invention, "polypeptide", "peptide", and "protein" are used interchangeably.

In the present invention, "HER2" is used for the same meaning as HER2 protein.

In the present invention, "cells" include cells in an animal individual and cultured cells.

In the present invention, "cellular cytotoxic activity" refers to causing pathological change to cells in any way,

which includes causing, not only direct traumas, but also all types of damage in the structure and function of cells such as cleavage of DNA, formation of a nucleotide dimer, cleavage of a chromosome, damage of the mitotic apparatus, and lowered activity of various enzymes.

In the present invention, an "epitope" refers to a partial peptide or partial three-dimensional structure of an antigen to which a particular antibody (e.g., an anti-HER2 antibody) binds (a partial peptide or partial three-dimensional structure of HER2). An epitope as such a partial peptide (e.g., a partial peptide of HER2) can be determined by using any method well known to those skilled in the art, such as immunoassay.

A "CDR" in the present invention refers to a complementarity determining region. It is known that each of heavy chains and light chains of an antibody molecule have three CDRs. CDRs, which are also called a hypervariable region, are located in variable regions of heavy chains and light chains of an antibody and is a site with particularly high variation of the primary structure. Three CDRs are separately located in the primary structure of the polypeptide chain of each of heavy chains and light chains. Regarding CDRs of antibodies, herein, CDRs of a heavy chain refer to CDRH1, CDRH2, and CDRH3 from the amino terminus of the heavy chain amino acid sequence, and CDRs of a light chain refer to CDRL1, CDRL2, and CDRL3 from the amino terminus of the light chain amino acid sequence. These sites are located in the proximity of each other in the three-dimensional structure, determining specificity to an antibody to bind.

In the present invention, "hybridize under stringent conditions" refers to hybridization in the commercially available hybridization solution ExpressHyb Hybridization Solution (Clontech) at 68°C, or hybridization using a filter with DNA fixed thereto in the presence of 0.7 to 1.0 M NaCl at 68°C and washing at 68°C with 0.1 to 2 × SSC solution (1 × SSC solution contains 150 mM NaCl and 15 mM sodium citrate), or hybridization under conditions equivalent thereto.

In the present invention, "one to several" refers to 1 to 10, one to nine, one to eight, one to seven, one to six, one to five, one to four, one to three, or one or two.

In the present invention, an antibody capable of recognizing or binding to HER2 is occasionally called as an "anti-HER2 antibody". Such antibodies include chimeric antibodies, humanized antibodies, and human antibodies.

The binding activity of the antibody against tumor cells can be confirmed using flow cytometry. The incorporation of the antibody into tumor cells can be confirmed using (1) an assay of visualizing an antibody incorporated in cells under a fluorescence microscope using a secondary antibody (fluorescently labeled) binding to the therapeutic antibody (Cell Death and Differentiation (2008) 15, 751-761), (2) an assay of measuring a fluorescence intensity incorporated in cells using a secondary antibody (fluorescently labeled) binding to the therapeutic antibody (Molecular Biology of the Cell, Vol. 15, 5268-5282, December 2004), or (3) a Mab-ZAP assay using an immunotoxin binding to the therapeutic antibody wherein the toxin is released upon incorporation into cells to inhibit cell growth (Bio Techniques 28: 162-165, January 2000). As the immunotoxin, a recombinant complex protein of a diphtheria toxin catalytic domain and protein G may also be used.

In the present invention, "high internalization ability" refers to the situation that the survival rate (which is a relative rate to the cell survival rate without addition of the antibody as 100%) of targeted antigen-expressing cells (e.g., HER2-expressing cells) with addition of the antibody and a saporin-labeled anti-mouse or rat IgG antibody is preferably 70% or less, and more preferably 60% or less.

Now, the anti-HER2 antibody used in the present invention will be described. An embodiment described below is an example of representative embodiments of the present invention, and the scope of the present invention is not interpreted as being narrower by the embodiment.

1. HER2

Human HER2 protein is composed of a signal sequence consisting of 22 N-terminal amino acid residues, an extracellular domain consisting of 630 amino acid residues, a transmembrane domain consisting of 23 amino acid residues, and an intracellular domain consisting of 580 amino acid residues.

The amino acid sequence of and DNA sequence for human HER2 are published in public databases, and can be referred to, for example, from accession numbers of M11730 (Genbank) and NP_004439.2 (NCBI).

2. Anti-HER2 antibody

The anti-HER2 antibody of the present invention is an antibody capable of targeting tumor cells, and specifically has a property of recognizing a tumor cell, a property of binding to a tumor cell, a property of being incorporated and internalizing in a tumor cell, and so on. Accordingly, the anti-HER2 antibody according to the present invention can be used for an antibody-drug conjugate by conjugating via a linker with a compound having antitumor activity.

The anti-HER2 antibody of the present invention may have antitumor activity.

The anti-HER2 antibody of the present invention may be obtained using a method usually carried out in the art, which involves immunizing animals with an antigenic polypeptide and collecting and purifying antibodies produced in vivo. Alternatively, antibody-producing cells which produce antibodies against the antigen are fused with myeloma

cells according to the method known in the art to establish hybridomas, from which monoclonal antibodies can in turn be obtained.

The anti-HER2 antibody of the present invention is desired to have the following properties.

(1) An antibody having the following properties (a) and (b).

(a) Recognizing or binding to HER2.

The antibody of the present invention recognizes the HER2. In other words, the antibody of the present invention binds to the HER2. The antibody of the present invention preferably binds to HER2, and more preferably specifically binds to HER2.

In the present invention, "specific recognition", that is, "specific binding" refers to binding being not nonspecific adsorption. Examples of determination criteria on whether binding is specific or not may include, but not limited to, dissociation constants (hereinafter, referred to as "KD"). A preferred KD value of the antibody of the present invention to HER2 is $1 \times 10^{-5}$ M or less, $5 \times 10^{-6}$ M or less, $2 \times 10^{-6}$ M or less, or $1 \times 10^{-6}$ M or less, and more preferably $5 \times 10^{-7}$ M or less, $2 \times 10^{-7}$ M or less, or $1 \times 10^{-7}$ M or less.

Binding between an antigen and an antibody in the present invention may be measured or determined by an analysis method such as an ELISA method, an RIA method, and surface plasmon resonance (hereinafter, referred to as "SPR"). Binding between an antigen expressed on a cell surface and an antibody may be measured, for example, by a flow cytometry method.

(b) Having activity to internalize in HER2-expressing cell through binding to HER2.

(2) The antibody according to (1), wherein HER2 is human HER2.

The anti-HER2 antibody of the present invention is not limited to a particular anti-HER2 antibody and may be any anti-HER2 antibody that recognizes or binds to HER2, and is preferably an antibody specified with an amino acid sequence shown in Sequence Listing of the present application.

The method of the present invention for obtaining the anti-HER2 monoclonal antibody typically involves the following steps, but is not limited to the following.

(Method using hybridoma)

(a) Purification of a biopolymer for use as the antigen or preparation of antigen-expressing cells, and administration of the biopolymer or antigen-expressing cells to an animal;
(b) collection of tissue (e.g., a lymph node) including antibody-producing cells from the animal for which immunoreaction has been induced;
(c) preparation of myeloma cells (e.g., mouse myeloma SP2/0-ag14 cells);
(d) cell fusion of antibody-producing cells and myeloma cells;
(e) selection of a hybridoma group producing the targeted antibody;
(f) division into single cell clones (cloning);
(g) an optional step of culture of the hybridoma for mass production of a monoclonal antibody or rearing of an animal to which the hybridoma was transplanted; and
(h) examination of the physiological activity (internalization activity) and the binding specificity of the thus-produced monoclonal antibody, or testing of properties as a labeling reagent.

Examples of methods to be used here for measuring antibody titers may include, but not limited to, flow cytometry and a Cell-ELISA method.

Further, even if a monoclonal antibody was independently obtained by steps (a) to (h) in "Production of anti-HER2 antibody" again, or a monoclonal antibody was separately obtained by using another method, an antibody having internalization activity equivalent to that of the anti-HER2 antibody obtained by the method can be obtained. An example of such antibodies is an antibody that binds to an epitope for the anti-HER2 antibody obtained by the method. If a monoclonal antibody newly produced binds to a partial peptide or partial three-dimensional structure to which the anti-HER2 antibody binds, it can be determined that the monoclonal antibody binds to the same epitope. By confirming that the monoclonal antibody competes with the anti-HER2 antibody for binding to HER2 (i.e., the monoclonal antibody interferes with binding between the anti-HER2 antibody and HER2), it can be determined, even when the specific sequence or structure of an epitope has not been determined, that the monoclonal antibody binds to an epitope for the anti-HER2 antibody. If epitope identity has been confirmed, the monoclonal antibody is strongly expected to have antigen-binding ability, biological activity, and/or internalization activity equivalent to that of the anti-HER2 antibody.

The antibody of the present invention includes, in addition to the monoclonal antibody against HER2, a gene recombinant antibody obtained by artificial modification for the purpose of decreasing heterologous antigenicity to humans such as a chimeric antibody, a humanized antibody, and a human antibody. These antibodies can be produced using a known method.

(1) Chimeric antibody

Examples of the chimeric antibody may include, but not limited to, an antibody in which antibody variable and constant regions are derived from different species, for example, a chimeric antibody in which a mouse- or rat-derived antibody variable region is connected to a human-derived antibody constant region (Proc. Natl. Acad. Sci. U.S.A., 81, 6851-6855, (1984), etc.).

(2) Humanized antibody

Examples of the humanized antibody may include, but not limited to, an antibody obtained by incorporating only the complementarity determining regions (CDRs) into a human-derived antibody (e.g., Nature (1986) 321, p. 522-525), an antibody obtained by grafting a part of the amino acid residues of a framework as well as the CDR sequences to a human antibody by a CDR-grafting method (WO90/07861), an antibody in which a part of the CDR amino acid sequences has been modified (WO2012/075581, WO2011/084496, US2018/0501692), and an antibody humanized using a gene conversion mutagenesis strategy (US5821337). The amino acid sequences of CDRs can be determined according to a known method such as the Kabat definition, the Chothia definition, the Abm definition, and IMGT; however, CDRs in the present invention may be those defined according to any method.

Examples of the humanized anti-HER2 antibody of the present invention may include, but not limited to, an H01L02 antibody, an HwtL05 antibody, Trastuzumab A1 (HwtLwt), and Trastuzumab A2. The anti-HER2 antibody of the present invention may be any humanized antibody, without limited to a particular humanized antibody, that retains all the six CDR sequences of the H01L02 antibody, HwtL05 antibody, Trastuzumab A1 (HwtLwt), or Trastuzumab A2 and has HER2-binding activity, and in addition the anti-HER2 antibody may be any humanized antibody, without limited to a particular humanized antibody, such that its humanized antibody variant in which one to several (preferably, one or two, more preferably, one) CDR amino acid sequences have been modified also recognizes HER2 protein, or has the HER2 protein-binding activity of the original antibody.

Examples of the anti-HER2 antibody of the present invention or a functional fragment thereof may include, but not limited to, an antibody comprising a heavy chain having a variable region comprising:

CDRH1 consisting of an amino acid sequence represented by SEQ ID NO: 1 (Figure 4) in Sequence Listing, or an amino acid sequence obtained by substituting one to several (preferably, one or two) amino acids in the aforementioned amino acid sequence;
CDRH2 consisting of an amino acid sequence represented by SEQ ID NO: 2 (Figure 4) in Sequence Listing, or an amino acid sequence obtained by substituting one to several (preferably, one or two) amino acids in the aforementioned amino acid sequence; and
CDRH3 consisting of an amino acid sequence represented by SEQ ID NO: 3 (Figure 4) in Sequence Listing, or an amino acid sequence obtained by substituting one to several (preferably, one or two) amino acids in the aforementioned amino acid sequence; and

a light chain having a variable region comprising:

CDRL1 consisting of an amino acid sequence represented by SEQ ID NO: 5 (Figure 6) in Sequence Listing, or an amino acid sequence obtained by substituting one to several (preferably, one or two) amino acids in the aforementioned amino acid sequence;
CDRL2 consisting of an amino acid sequence consisting of amino acid residues 1 to 3 of SEQ ID NO: 6 (Figure 6) in Sequence Listing, or an amino acid sequence obtained by substituting one to several (preferably, one or two) amino acids in the aforementioned amino acid sequence; and
CDRL3 consisting of an amino acid sequence represented by SEQ ID NO: 7 (Figure 6) in Sequence Listing, or an amino acid sequence obtained by substituting one to several (preferably, one or two) amino acids in the aforementioned amino acid, and
recognizing the HER2 protein of the present invention or having the HER2 protein-binding activity of the antibody,

or a functional fragment of the antibody.

Preferred examples of CDR amino acid substitution in the humanized anti-HER2 antibody or functional fragment

thereof may include, but not limited to, substitution of one to several (preferably, one or two, or one) amino acids in CDLH3 or CDRL3 as described above, and an example thereof is CDRH3 represented by SEQ ID NO: 4 (Figure 5) in Sequence Listing, which is obtained by substituting amino acid residue 9 of SEQ ID NO: 3 in Sequence Listing, or CDRL3 represented by SEQ ID NO: 8 (Figure 7) in Sequence Listing, which is obtained by substituting amino acid residue 4 of SEQ ID NO: 7 in Sequence Listing.

Examples of the antibody comprising a heavy chain comprising CDRH1, CDRH2, and CDRH3 and a light chain comprising CDRL1, CDRL2, and CDRL3 in the present invention may include, but not limited to, any one of the following (a) and (b):

(a) CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 4, and CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 8 (H01L02);

(b) CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 3, and CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 8 (HwtL05); and

(c) CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 3, and CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 7 (Trastuzumab A1 or A2).

Examples of the heavy chain variable region of the humanized antibody comprising the above-described CDRH1 to 3 may include, but not limited to, an amino acid sequence represented by SEQ ID NO: 13 (Figure 9) in Sequence Listing and an amino acid sequence represented by SEQ ID NO: 17 (Figure 11) in Sequence Listing, and examples of the light chain variable region of the humanized antibody comprising the above-described CDRL1 to 3 may include, but not limited to, an amino acid sequence represented by SEQ ID NO: 21 (Figure 13) in Sequence Listing, an amino acid sequence represented by SEQ ID NO: 25 (Figure 15) in Sequence Listing and an amino acid sequence represented by SEQ ID NO: 29 (Figure 17) in Sequence Listing.

Preferred examples of humanized antibodies including a combination of the above heavy chain variable region and light chain variable region may include, but not limited to:

a humanized antibody comprising a heavy chain variable region consisting of an amino acid sequence represented by SEQ ID NO: 17 (Figure 11) in Sequence Listing and a light chain variable region consisting of an amino acid sequence represented by SEQ ID NO: 25 (Figure 15) in Sequence Listing (H01L02);

a humanized antibody comprising a heavy chain variable region consisting of an amino acid sequence represented by SEQ ID NO: 13 (Figure 9) in Sequence Listing and a light chain variable region consisting of an amino acid sequence represented by SEQ ID NO: 29 (Figure 17) in Sequence Listing (HwtL05); and

a humanized antibody comprising a heavy chain variable region consisting of an amino acid sequence represented by SEQ ID NO: 13 (Figure 9) in Sequence Listing and a light chain variable region consisting of an amino acid sequence represented by SEQ ID NO: 21 (Figure 13) in Sequence Listing (Trastuzumab A1 (HwtLwt), Trastuzumab A2).

Examples of humanized antibodies including a combination of a heavy chain comprising the above heavy chain variable region and a light chain comprising the above light chain variable region may include, but not limited to:

a humanized antibody comprising a heavy chain consisting of an amino acid sequence consisting of amino acid residues 20 to 469 of SEQ ID NO: 15 (Figure 10) in Sequence Listing and a light chain consisting of an amino acid sequence consisting of amino acid residues 21 to 234 of SEQ ID NO: 23 (Figure 14) in Sequence Listing (H01L02);

a humanized antibody comprising a heavy chain consisting of an amino acid sequence consisting of amino acid residues 20 to 469 of SEQ ID NO: 11 (Figure 8) in Sequence Listing and a light chain consisting of an amino acid sequence consisting of amino acid residues 21 to 234 of SEQ ID NO: 27 (Figure 16) in Sequence Listing (HwtL05);

a humanized antibody comprising a heavy chain consisting of an amino acid sequence consisting of amino acid residues 20 to 469 of SEQ ID NO: 11 (Figure 8) in Sequence Listing and a light chain consisting of an amino acid sequence consisting of amino acid residues 21 to 234 of SEQ ID NO: 19 (Figure 12) in Sequence Listing (Trastuzumab

A1 (HwtLwt)); and

a humanized antibody comprising a heavy chain consisting of an amino acid sequence consisting of amino acid residues 20 to 469 of SEQ ID NO: 31 (Figure 21) in Sequence Listing and a light chain consisting of an amino acid sequence consisting of amino acid residues 21 to 234 of SEQ ID NO: 32 (Figure 22) in Sequence Listing (Trastuzumab A2).

As described later, one or two amino acids may be deleted at the carboxyl terminus of each of the humanized antibodies H01L02, HwtL05, Trastuzumab A1, and Trastuzumab A2, and such deletion variants are also included in the present invention.

Examples of the heavy chain of deletion variants may include, but not limited to, a heavy chain including an amino acid sequence consisting of amino acid residues 20 to 468 of SEQ ID NO: 11, 15, or 31 in Sequence Listing.

Examples of such deletion variants may include, but not limited to:

a humanized antibody comprising a heavy chain consisting of an amino acid sequence consisting of amino acid residues 20 to 468 of SEQ ID NO: 15 (Figure 10) in Sequence Listing and a light chain consisting of an amino acid sequence consisting of amino acid residues 21 to 234 of SEQ ID NO: 23 (Figure 14) in Sequence Listing (H01L02);

a humanized antibody comprising a heavy chain consisting of an amino acid sequence consisting of amino acid residues 20 to 468 of SEQ ID NO: 11 (Figure 8) in Sequence Listing and a light chain consisting of an amino acid sequence consisting of amino acid residues 21 to 234 of SEQ ID NO: 27 (Figure 16) in Sequence Listing (HwtL05);

a humanized antibody comprising a heavy chain consisting of an amino acid sequence consisting of amino acid residues 20 to 468 of SEQ ID NO: 11 (Figure 8) in Sequence Listing and a light chain consisting of an amino acid sequence consisting of amino acid residues 21 to 234 of SEQ ID NO: 19 (Figure 12) in Sequence Listing (Trastuzumab A1 (HwtLwt)); and

a humanized antibody comprising a heavy chain consisting of an amino acid sequence consisting of amino acid residues 20 to 468 of SEQ ID NO: 31 (Figure 21) in Sequence Listing and a light chain consisting of an amino acid sequence consisting of amino acid residues 21 to 234 of SEQ ID NO: 32 (Figure 22) in Sequence Listing (Trastuzumab A2).

In the heavy chain amino acid sequence represented by SEQ ID NO: 11 (Figure 8), 15 (Figure 10), or 31 (Figure 21) in Sequence Listing, an amino acid sequence consisting of amino acid residues 1 to 19 is the signal sequence, an amino acid sequence consisting of amino acid residues 20 to 139 is the heavy chain variable region, and an amino acid sequence consisting of amino acid residues 140 to 469 is the heavy chain constant region.

In the light chain amino acid sequence represented by SEQ ID NO: 19 (Figure 12), 23 (Figure 14), 27 (Figure 16), or 32 (Figure 22) in Sequence Listing, the amino acid sequence consisting of amino acid residues 1 to 20 is the signal sequence, the amino acid sequence consisting of amino acid residues 21 to 127 is the light chain variable region, and the amino acid sequence consisting of amino acid residues 128 to 234 is the light chain constant region.

The nucleotide sequence encoding the heavy chain amino acid sequence of the humanized antibody H01L02 and that encoding the light chain amino sequence of the humanized antibody H01L02 are a polynucleotide represented by SEQ ID NO: 16 and a polynucleotide represented by SEQ ID NO: 24, respectively;

the nucleotide sequence encoding the heavy chain amino acid sequence of the humanized antibody HwtL05 and that encoding the light chain amino sequence of the humanized antibody HwtL05 are a polynucleotide represented by SEQ ID NO: 12 and a polynucleotide represented by SEQ ID NO: 28, respectively; and

the nucleotide sequence encoding the heavy chain amino acid sequence of the humanized antibody Trastuzumab A1 and that encoding the light chain amino sequence of the humanized antibody Trastuzumab A1 are a polynucleotide represented by SEQ ID NO: 12 and a polynucleotide represented by SEQ ID NO: 20, respectively.

The nucleotide sequence encoding the amino acid sequence of the heavy chain variable region of the humanized antibody H01L02 and that encoding the light chain variable region of the humanized antibody H01L02 are a polynucleotide represented by SEQ ID NO: 18 and a polynucleotide represented by SEQ ID NO: 26, respectively;

the nucleotide sequence encoding the amino acid sequence of the heavy chain variable region of the humanized antibody HwtL05 and that encoding the light chain variable region of the humanized antibody HwtL05 are a polynucleotide represented by SEQ ID NO: 14 and a polynucleotide represented by SEQ ID NO: 30, respectively; and

the nucleotide sequence encoding the amino acid sequence of the heavy chain variable region of the humanized antibody Trastuzumab A1 and that encoding the light chain variable region of the humanized antibody Trastuzumab A1 are a polynucleotide represented by SEQ ID NO: 14 and a polynucleotide represented by SEQ ID NO: 22, respectively.

In the nucleotide sequence represented by SEQ ID NO: 12, or 16 in Sequence Listing, a nucleotide sequence consisting of nucleotide residues 1 to 57 is encoding the signal sequence of the humanized antibody heavy chain, a nucleotide sequence consisting of nucleotide residues 58 to 417 is encoding the amino acid sequence of the variable region of the humanized antibody heavy chain, and a nucleotide sequence consisting of nucleotide residues 418 to 1407 is encoding the constant region of the antibody heavy chain.

In the nucleotide sequence represented by SEQ ID NO: 20, 24, or 28 in Sequence Listing, a nucleotide sequence consisting of nucleotide residues 1 to 60 is encoding the signal sequence of the humanized antibody light chain, a nucleotide sequence consisting of nucleotide residues 61 to 381 is encoding the amino acid sequence of the variable region of the humanized antibody light chain, and a nucleotide sequence consisting of nucleotide residues 382 to 702 is encoding the constant region of the antibody light chain.

As long as having binding activity to HER2, any antibody that has an identity or homology of 80% or higher, preferably of 90% or higher, more preferably of 95% or higher, even more preferably of 97% or higher, most preferably of 99% or higher, to the amino acid sequence of any of the antibodies including the above combinations of a heavy chain variable region and a light chain variable region and the antibodies including the above combinations of a heavy chain and a light chain is also included in the antibody of the present invention.

As long as having binding activity to HER2, any antibody that includes CDRs consisting of the amino acid sequences of the CDRs of any of the antibodies including the above combinations of a heavy chain variable region and a light chain variable region and the antibodies including the above combinations of a heavy chain and a light chain, wherein the amino acid sequence of the antibody excluding the amino acid sequences of the CDRs has an amino acid identity or homology of 80% or higher, preferably of 90% or higher, more preferably of 95% or higher, even more preferably of 97% or higher, the most preferably of 99% or higher, is also included in the antibody of the present invention.

Further, an antibody having biological activity equivalent to each of the above antibodies may be selected through combining amino acid sequences obtained by substituting, deleting, or adding one or several amino acid residues in the amino acid sequence of the heavy chain or light chain. The substitution of an amino acid herein is preferably conservative amino acid substitution (WO 2013154206, etc.).

The conservative amino acid substitution is substitution that occurs in an amino acid group with related amino acid side chains. Such amino acid substitution is preferably carried out to such a degree that the properties of the substance having the original amino acid sequence are not decreased.

Homology between two amino acid sequences may be determined by using default parameters of Blast algorithm version 2.2.2 (Altschul, Stephen F., Thomas L.Madden, Alejandro A. Schaaffer, Jinghui Zhang, Zheng Zhang, Webb Miller, and David J.Lipman (1997), "Gapped BLAST and PSI-BLAST: a new generation of protein database search programs", Nucleic Acids Res. 25: 3389-3402). Blast algorithm may be also used by accessing to the Internet (www.ncbi.nlm.nih.gov/blast).

(3) Human antibody

Further examples of the antibody of the present invention may include, but not limited to, human antibodies capable of binding to HER2. The human anti-HER2 antibody includes a human antibody having only an antibody gene sequence derived from a human chromosome. Human anti-HER2 antibodies that can be obtained by using known methods (Nature Genetics (1997) 16, p. 133-143, Nucl. Acids Res. (1998) 26, p.3447-3448, Animal Cell Technology: Basic and Applied Aspects, vol.10, p.69-73, Kluwer Academic Publishers, 1999., Proc. Natl. Acad. Sci. USA (2000) 97, p.722-727, Investigative Ophthalmology & Visual Science. (2002) 43(7), p.2301-2308, Briefings in Functional Genomics and Proteomics (2002), 1(2), p.189-203, Ophthalmology (2002) 109(3), p.427-431, Nature Biotechnology (2005), 23, (9), p.1105-1116, WO92/01047, WO92/20791, WO93/06213, WO93/11236, WO93/19172, WO95/01438, WO95/15388, Annu. Rev. Immunol (1994) 12, p.433-455, etc.) are also known.

Chimeric antibodies, humanized antibodies, human antibodies, and so on obtained by using the above method may be evaluated for binding activity to an antigen, for example, by using a known method to screen for a preferred antibody.

Another example of indicators in comparing characteristics among antibodies is stability of antibodies. Differential scanning calorimetry (DSC) is an apparatus capable of quickly and accurately measuring thermal denaturation midpoints (Tm), a good indicator for relative structural stability of protein. Difference in thermal stability can be compared through comparison of Tm values measured with DSC. Storage stability of antibodies is known to be correlated with thermal stability of antibodies to some degree (Pharmaceutical Development and Technology (2007) 12, p. 265-273), and hence thermal stability may be used as an indicator to screen for a preferred antibody. Examples of other indicators for screening for an antibody may include, but not limited to, a high yield in appropriate host cells and a low agglutinating property in aqueous solution. It is needed to screen for the most suitable antibody for administration to humans through comprehensive determination based on the above-described indicators, for example, because an antibody with the highest yield does not necessarily exhibit the highest thermal stability.

The antibody of the present invention includes "antibodies that bind to a site to which the anti-HER2 antibody provided by the present invention binds". That is, the present invention also includes antibodies that bind to a site on HER2 protein that Trastuzumab A1 (HwtLwt), Trastuzumab A2, the H10L02 antibody, or the H02L05 antibody of the present invention recognizes.

The antibody of the present invention includes modified variants of the antibody. The modified variant refers to a variant obtained by subjecting the antibody of the present invention to chemical or biological modification. Examples of the chemically modified variant may include, but not limited to, variants including a linkage of a chemical moiety to an amino acid skeleton, and variants with chemical modification of an N-linked or O-linked carbohydrate chain. Examples of the biologically modified variant may include, but not limited to, variants obtained by post-translational modification (e.g., N-linked or O-linked glycosylation, N- or C-terminal processing, deamidation, isomerization of aspartic acid, oxidation of methionine), and variants in which a methionine residue has been added to the N terminus by being expressed in a prokaryotic host cell. Further, an antibody labeled so as to enable the detection or isolation of the antibody of the present invention or an antigen, for example, an enzymelabeled antibody, a fluorescence-labeled antibody, and an affinity-labeled antibody are also included in the meaning of the modified variant. Such a modified variant of the antibody of the present invention is useful for improving the stability and blood retention of the antibody, reducing the antigenicity thereof, detecting or isolating an antibody or an antigen, and so on.

Further, by regulating the modification of a glycan which is linked to the antibody of the present invention (glycosylation, defucosylation, etc.), the antibodydependent cellular cytotoxic activity can be enhanced. As the technique for regulating the modification of a glycan of antibodies, WO 1999/54342, WO 2000/61739, WO 2002/31140, WO 2007/133855, WO 2013/120066 etc., are known. However, the technique is not limited thereto. In the antibody of the present invention, antibodies in which the modification of a glycan is regulated are also included.

Such modification may be applied at any position or a desired position in an antibody or a functional fragment of the antibody, and the same type or two or more different types of modification may be applied at one or two or more positions.

In the present invention, the meaning of a "modified variant of an antibody fragment" also includes a "fragment of a modified variant of an antibody".

If an antibody gene is temporarily isolated and then introduced into an appropriate host to produce an antibody, an appropriate combination of a host and an expression vector can be used. Specific examples of the antibody gene may include, but not limited to, combination of a gene encoding the heavy chain sequence or the like of an antibody described herein and a gene encoding the light chain sequence or the like of an antibody described herein. To transform host cells, a heavy chain sequence gene or the like and a light chain sequence gene or the like may be inserted into the same expression vector, or inserted into separate expression vectors.

If eukaryotic cells are used as a host, animal cells, plant cells, and eukaryotic microorganisms may be used. Particularly, examples of animal cells may include, but not limited to, mammalian cells, such as COS cells (Cell (1981) 23, p. 175-182, ATCC CRL-1650), as monkey cells, the mouse fibroblast NIH3T3 (ATCC No. CRL-1658), a dihydrofolate reductase-deficient strain (Proc. Natl. Acad. Sci. U.S.A. (1980) 77, p. 4126-4220) of Chinese hamster ovary cells (CHO cells, ATCC CCL-61), and FreeStyle 293F cells (Invitrogen).

If prokaryotic cells are used, for example, Escherichia coli or Bacillus subtilis may be used.

A targeted antibody gene is introduced into these cells by transformation, and the transformed cells are cultured in vitro to afford an antibody. Sequence difference among antibodies may result in different yields in the culture, and hence antibodies that allow easy production of a medicine may be selected out of antibodies having equivalent binding activity by using yields as an indicator. Accordingly, the antibody of the present invention includes antibodies obtained by using a method for producing the antibody, the method including the steps of: culturing the transformed host cell; and collecting a targeted antibody or a functional fragment of the antibody from a culture obtained in the step of culturing.

The antibody gene is preferably a polynucleotide including a polynucleotide described in any one of (a) to (e):

(a) a combination of a polynucleotide encoding the heavy chain amino acid sequence and a polynucleotide encoding the light chain amino acid sequence of an antibody of any one of the H1L02 antibody, the HwtL05 antibody, Trastuzumab A1 (HwtLwt), and Trastuzumab A2;

(b) a combination of a polynucleotide encoding a heavy chain amino acid sequence including the sequences of CDRH1 to CDRH3 and a polynucleotide encoding a light chain amino acid sequence including the sequences of CDRL1 to CDRL3 of an antibody of any one of the H1L02 antibody, the HwtL05 antibody, Trastuzumab A1 (HwtLwt), and Trastuzumab A2;

(c) a combination of a polynucleotide encoding a heavy chain amino acid sequence comprising the amino acid sequence of the heavy chain variable region and a polynucleotide encoding a light chain amino acid sequence comprising the amino acid sequence of the light chain variable region of an antibody of any one of the H1L02 antibody, the HwtL05 antibody, Trastuzumab A1 (HwtLwt), and Trastuzumab A2;

(d) a polynucleotide that is hybridizable with nucleotides consisting of a polynucleotide complementary to the polynucleotide according to any one of (a) to (c) under stringent conditions and is encoding the amino acid sequence of an antibody capable of binding to HER2; and
(e) a polynucleotide encoding the amino acid sequence of a polypeptide obtained by substituting, deleting, adding, or inserting 1 to 50, 1 to 45, 1 to 40, 1 to 35, 1 to 30, 1 to 25, 1 to 20, 1 to 15, 1 to 10, one to eight, one to six, one to five, one to four, one to three, one or two, or one amino acid(s) in the polynucleotide according to any one of (a) to (c), and is encoding the amino acid sequence of an antibody capable of binding to HER2.

The present invention includes a nucleotide encoding the antibody of the present invention or a functional fragment of the antibody, or a modified variant of the antibody or functional fragment; a recombinant vector including the gene inserted therein; and a cell including the gene or the vector introduced therein.
The present invention includes a method for producing an antibody or a functional fragment of the antibody, or a modified variant of the antibody or functional fragment, the method including the steps of: culturing the cell; and collecting from the culture an antibody or a functional fragment of the antibody, or a modified variant of the antibody or functional fragment.
It is known that a lysine residue at the carboxyl terminus of the heavy chain of an antibody produced in a cultured mammalian cell is deleted (Journal of Chromatography A, 705: 129-134 (1995)), and it is also known that two amino acid residues, glycine and lysine, at the carboxyl terminus of the heavy chain of an antibody produced in a cultured mammalian cell are deleted and a proline residue newly located at the carboxyl terminus is amidated (Analytical Biochemistry, 360: 75-83 (2007)). However, such deletion and modification of the heavy chain sequence do not affect the antigen-binding ability and the effector function (the activation of complement, antibody-dependent cellular cytotoxicity, etc.) of the antibody. Therefore, in the antibody according to the present invention, antibodies subjected to such modification and functional fragments of the antibody are also included, and deletion variants in which one or two amino acids have been deleted at the carboxyl terminus of the heavy chain, variants obtained by amidation of deletion variants (for example, a heavy chain in which the carboxyl terminal proline residue has been amidated), and the like are also included. The type of deletion variants having a deletion at the carboxyl terminus of the heavy chain of the antibody according to the present invention is not limited to the above variants as long as the antigen-binding ability and the effector function are conserved. The two heavy chains constituting the antibody according to the present invention may be of one type selected from the group consisting of a full-length heavy chain and the above-described deletion variant, or may be of two types in combination selected therefrom. The ratio of the amount of each deletion variant can be affected by the type of cultured mammalian cells which produce the antibody according to the present invention and the culture conditions; however, an antibody in which one amino acid residue at the carboxyl terminus has been deleted in both of the two heavy chains in the antibody according to the present invention can be preferably exemplified as a main component of molecules of the antibody.
Examples of isotypes of the anti-HER2 antibody of the present invention may include, but not limited to, IgG (IgG1, IgG2, IgG3, IgG4), and preferred examples thereof include IgG1, IgG2, and IgG4.
If IgG1 is used as the isotype of the antibody of the present invention, the effector function may be adjusted by substituting some amino acid residues in the constant region. Examples of variants of IgG1 with the effector function lowered or attenuated may include, but not limited to, IgG1 LALA (IgG1-L234A,L235A) and IgG1 LAGA (IgG1-L235A,G237A), and a preferred variant of IgGl is IgG1 LALA. The L234A, L235A indicates substitution of leucine with alanine at the 234- and 235-positions specified by EU-index numbering (Proc. Natl. Acad. Sci. U.S.A., Vol. 63, No. 1 (May 15, 1969), p. 78-85), and the G237A indicates substitution of glycine with alanine at the 237-position specified by EU-index numbering.
Typical examples of bioactivity of antibodies may include, but not limited to, antigen-binding activity, activity to internalize in cells expressing an antigen by binding to the antigen, activity to neutralize antigen activity, and activity to enhance antigen activity, and the function of the antibody according to the present invention is binding activity to HER2, and preferably activity to internalize in HER2-expression cells by binding to HER2.
The antibody obtained may be purified to a homogeneous state. For separation/purification of the antibody, separation/purification methods commonly used for protein can be used. For example, the antibody may be separated/purified by appropriately selecting and combining column chromatography, filter filtration, ultrafiltration, salting-out, dialysis, preparative polyacrylamide gel electrophoresis, isoelectric focusing, and so on (Strategies for Protein Purification and Characterization: A Laboratory Course Manual, Daniel R. Marshak et al. eds., Cold Spring Harbor Laboratory Press (1996); Antibodies: A Laboratory Manual. Ed Harlow and David Lane, Cold Spring Harbor Laboratory (1988)), but separation/purification methods are not limited thereto.
Examples of chromatography may include, but not limited to, affinity chromatography, ion-exchange chromatography, hydrophobic chromatography, gel filtration chromatography, reversed-phase chromatography, and adsorption chromatography.
These chromatographies may be carried out using liquid chromatography such as HPLC and FPLC.
Examples of columns for affinity chromatography may include, but not limited to, a Protein A column and a

Protein G column.

Alternatively, the antibody may be purified by utilizing binding activity to an antigen with a carrier to which the antigen has been immobilized.

<N297 Glycan>

Recently has been reported a method for remodeling heterogeneous glycoprotein of an antibody by enzymatic reaction or the like to homogeneously introduce a glycan having a functional group (ACS Chemical Biology 2012, 7, 110, ACS Medicinal Chemistry Letters 2016, 7, 1005, Bioconjugate Chemistry 2015, 26, 2233, Angew. Chem. Int. Ed. 2016, 55, 2361-2367, US 2016361436).

In the glycan remodeling of the present invention, using hydrolase, heterogeneous glycans added to a protein (e.g., an antibody) are cleaved off to leave only GlcNAc at each terminus thereby producing a homogenous protein moiety with GlcNAc (hereinafter, referred to as an "acceptor"). Subsequently, an arbitrary glycan separately prepared (hereinafter, referred to as a "donor") is provided, and the acceptor and the donor are linked together by using transglycosidase. Thereby, a homogeneous glycoprotein with arbitrary glycan structure can be synthesized.

In the present invention, a "glycan" refers to a structural unit of two or more monosaccharides bonded together via glycosidic bonds. Specific monosaccharides and glycans are occasionally abbreviated, for example, as "GlcNAc-", "MSG-", and so on. When any of these abbreviations is used in a structural formula, the abbreviation is shown with an intention that an oxygen atom or nitrogen atom involved in a glycosidic bond at the reducing terminal to another structural unit is not included in the abbreviation indicating the glycan, unless specifically defined.

In the present invention, a monosaccharide as a basic unit of a glycan is indicated for convenience so that in the ring structure, the position of a carbon atom bonding to an oxygen atom constituting the ring and directly bonding to a hydroxy group (or an oxygen atom involved in a glycosidic bond) is defined as the 1-position (the 2-position only for sialic acids), unless otherwise specified. The names of compounds in Examples are each provided in view of the chemical structure as a whole, and that rule is not necessarily applied.

When a glycan is indicated as a sign (e.g., GLY, SG, MSG, GlcNAc) in the present invention, the sign is intended, unless otherwise defined, to include carbon atoms ranging to the reducing terminal and not to include N or O involved in an N- or O-glycosidic bond.

In the present invention, unless specifically stated, a partial structure when a glycan is linking to a side chain of an amino acid is indicated in such a manner that the side chain portion is indicated in parentheses, for example, "(SG-)Asn".

The antibody-drug conjugate of the present invention is represented by the following formula:

[Formula 43]

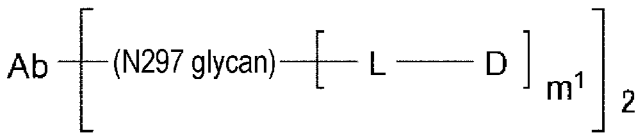

wherein an antibody Ab or a functional fragment of the antibody bonds via a N297 glycan or remodeled N297 glycan to L, and preferably bonds via a remodeled N297 glycan of Ab to L.

Glycans in Ab of the present invention are N-linked glycans or O-linked glycans, and preferably N-linked glycans.

N-linked glycans and O-linked glycans bond to an amino acid side chain of an antibody via an N-glycosidic bond and an O-glycosidic bond, respectively.

IgG has a well conserved N-linked glycan on an asparagine residue at the 297-position of the Fc region of the heavy chain (hereinafter, referred to as "Asn297 or N297"), and the N-linked glycan is known to contribute to the activity and kinetics of the antibody molecule (Biotechnol. Prog., 2012, 28, 608-622, Anal. Chem., 2013, 85, 715-736).

The amino acid sequence in the constant region of IgG is well conserved, and each amino acid is specified by Eu index numbering in Edelman et al. (Proc. Natl. Acad. Sci. U.S.A., Vol. 63, No. 1 (May 15, 1969), p. 78-85). For example, Asn297, to which an N-linked glycan is added in the Fc region, corresponds to the 297-position in Eu index numbering, and each amino acid is uniquely specified by Eu index numbering, even if the actual position of the amino acid has varied through fragmentation of the molecule or deletion of a region.

In the antibody-drug conjugate of the present invention, the antibody or functional fragment of the antibody more preferably bonds to L via a glycan bonding to a side chain of Asn297 thereof (hereinafter, referred to as "N297 glycan"), and the antibody or functional fragment of the antibody even more preferably bonds via the N297 glycan to L, wherein the N297 glycan is a remodeled N297 glycan.

SGP, an abbreviation for sialyl glycopeptide, is a representative N-linked complex glycan. SGP can be separated/purified from the yolk of a hen egg, for example, by using a method described in WO 2011/0278681. Purified products of SGP are commercially available (Tokyo Chemical Industry Co., Ltd., FUSHIMI Pharmaceutical Co., Ltd.), and may be purchased. For example, disialooctasaccharide (Tokyo Chemical Industry Co., Ltd.), a glycan formed by deleting one GlcNAc at the reducing terminal in the glycan moiety of SG (hereinafter, referred to as "SG (10)", is commercially available.

In the present invention, a glycan structure formed by deleting a sialic acid at a non-reducing terminal only in either one of the branched chains of β-Man in SG (10) refers to MSG (9), and a structure having a sialic acid only in the 1-3 branched chain is called as MSG1, and a structure having a sialic acid only in the 1-6 branched chain is called as MSG2.

The remodeled glycan of the present invention is N297-(Fuc)MSG1, N297-(Fuc)MSG2, or a mixture of N297-(Fuc)MSG1 and N297-(Fuc)MSG2, or N297-(Fuc)SG, and is preferably N297-(Fuc)MSG1, N297-(Fuc)MSG2, or N297-(Fuc)SG, and is more preferably N297-(Fuc)MSG1 or N297-(Fuc)MSG2.

N297-(Fuc)MSG1 is represented by the following structural formula or sequence formula:

[Formula 44]

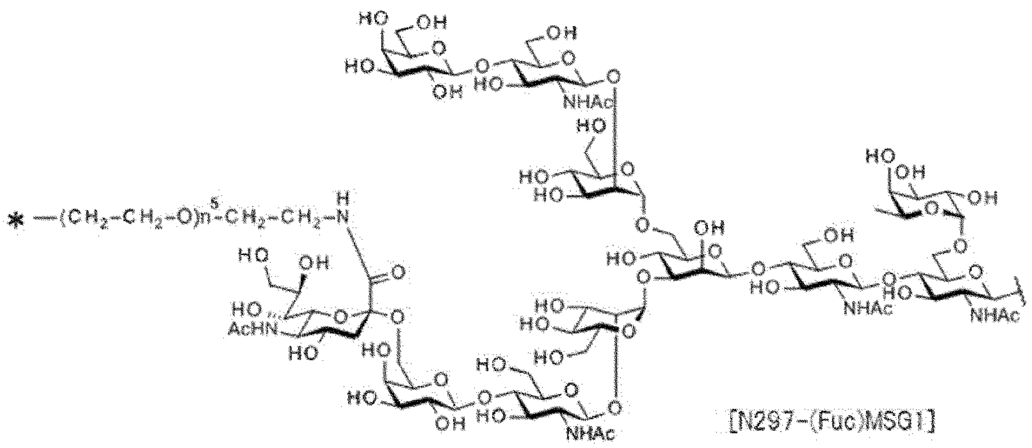

[N297-(Fuc)MSG1]

[Formula 45]

Fucα1
|
6
Galβ1-4GlcNAcβ1-2Manα1— 6
Manβ1-4GlcNAcβ1-4GlcNAcβ1
∗ —L(PEG)-NeuAcα2-6Galβ1-4GlcNAcβ1-2Manα1— 3

[N297-(Fuc)MSG1]

In the formulas, each wavy line represents bonding to Asn297 of the antibody,

L(PEG) represents $-NH-CH_2CH_2-(O-CH_2CH_2)n^5-*$, wherein the amino group at the left end represents bonding via an amide bond to carboxylic acid at the 2-position of a sialic acid at the non-reducing terminal in the 1-3 branched chain of β-Man in the N297 glycan, the asterisk (*) at the right end represents bonding to a nitrogen atom at the 1- or 3-position of the 1,2,3-triazole ring of Lb in linker L, and $n^5$ is an integer of 2 to 10, and preferably an integer of 2 to 5.

N297-(Fuc)MSG2 is represented by the following structural formula or sequence formula:

[Formula 46]

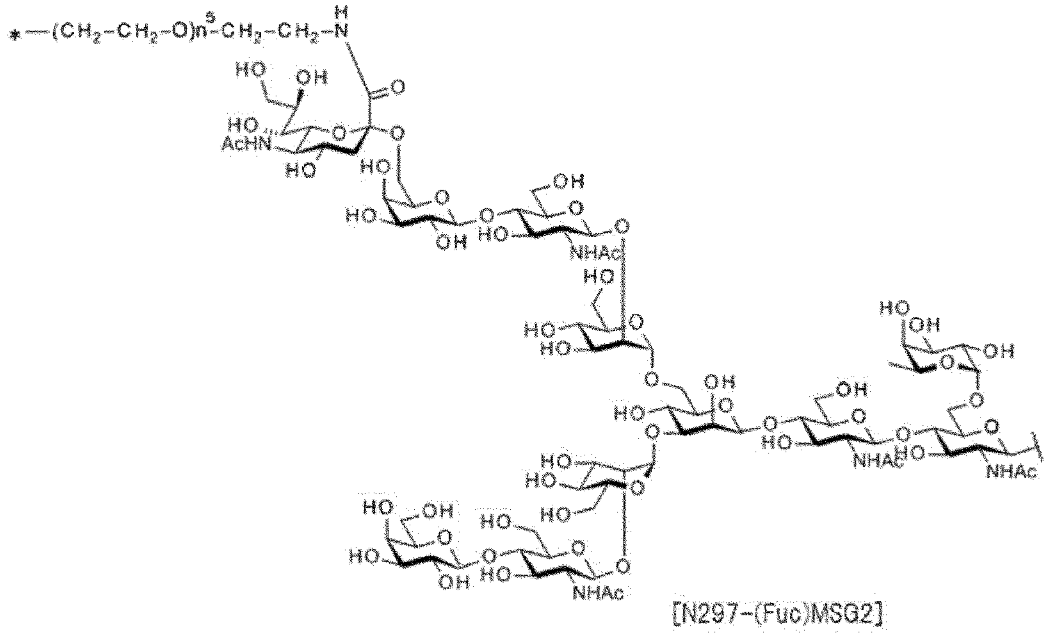

[N297-(Fuc)MSG2]

[Formula 47]

Fucα1
|
6

* - L(PEG)-NeuAcα2-6Galβ1-4GlcNAcβ1-2Manα1— 6

Manβ1-4GlcNAcβ1-4GlcNAcβ1-

Galβ1-4GlcNAcβ1-2Manα1— 3

[N297-(Fuc)MSG2]

In the formulas, each wavy line represents bonding to Asn297 of the antibody,

L(PEG) represents $-NH-CH_2CH_2-(O-CH_2CH_2)n^5-*$, wherein the amino group at the left end represents bonding via an amide bond to carboxylic acid at the 2-position of a sialic acid at the non-reducing terminal in the 1-6 branched chain of β-Man in the N297 glycan, the asterisk (*) at the right end represents bonding to a nitrogen atom at the 1- or 3-position of the 1,2,3-triazole ring of Lb in linker L, and $n^5$ is an integer of 2 to 10, and preferably an integer of 2 to 5.

N297-(Fuc)SG is represented by the following structural formula or sequence formula:

[Formula 48]

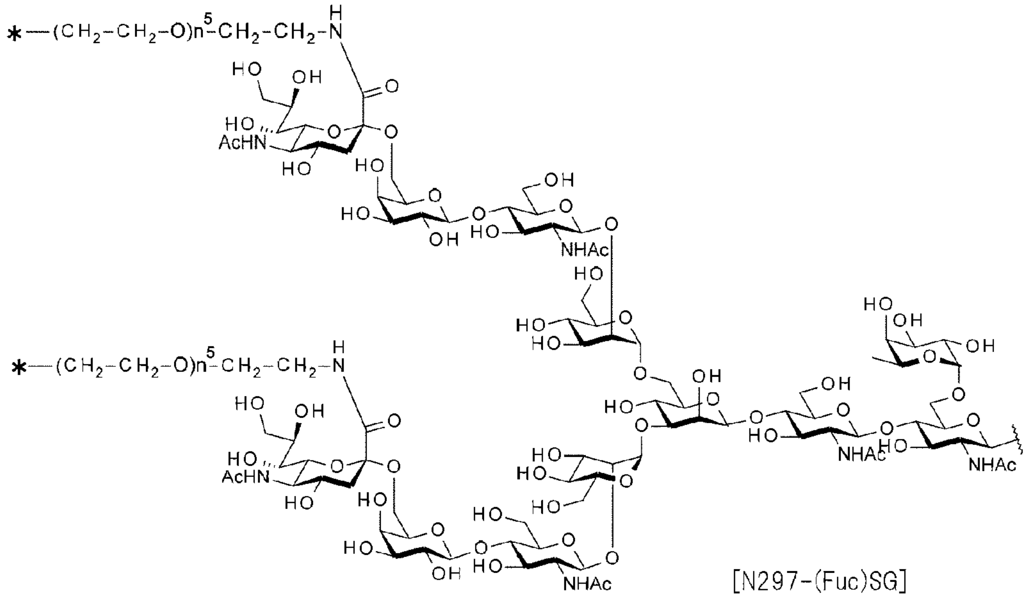

[N297-(Fuc)SG]

[Formula 49]

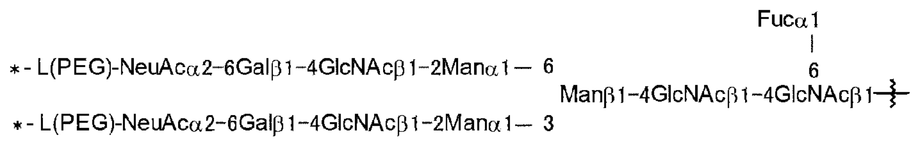

[N297-(Fuc)SG]

In the formulas, each wavy line represents bonding to Asn297 of the antibody,

L(PEG) represents $-NH-CH_2CH_2-(O-CH_2CH_2)n^5-*$, wherein the amino group at the left end represents bonding via an amide bond to carboxylic acid at the 2-position of a sialic acid at the non-reducing terminal in each of the 1-3 branched chain and 1-6 branched chain of β-Man in the N297 glycan, the asterisk (*) at the right end represents bonding to a nitrogen atom at the 1- or 3-position of the 1,2,3-triazole ring of Lb in linker L, and $n^5$ is an integer of 2 to 10, and preferably an integer of 2 to 5.

If N297 glycan of the antibody in the antibody-drug conjugate of the present invention is N297-(Fuc)MSG1, N297-(Fuc)MSG2, or a mixture of them, the antibody-drug conjugate is a molecule to which two molecules of drug-linker (-L-D) have been conjugated ($m^1 = 1$) since the antibody is a dimer (see Figure 1).

For example, Example 9: ADC1 is in the case that N297 glycan is N297-(Fuc)MSG1.

If N297 glycan of the antibody in the antibody-drug conjugate of the present invention is N297-(Fuc)SG, the antibody-drug conjugate is a molecule to which four molecules of drug linker (-L-D) have been conjugated ($m^1 = 2$) since the antibody is a dimer.

N297 glycan is preferably N297-(Fuc)MSG1, N297-(Fuc)MSG2, or N297-(Fuc)SG, more preferably N297-(Fuc)MSG1 or N297-(Fuc)MSG2, and most preferably N297-(Fuc)MSG1.

If N297 glycan is N297-(Fuc)MSG1, N297-(Fuc)MSG2, or N297-(Fuc)SG, an ADC of homogenous quality can be obtained.

The present invention provides a method for producing a glycan-remodeled antibody or a functional fragment of the antibody, the method including the following steps of:

i) culturing the above-described host cell (e.g., an animal cell (such as a CHO cell)) and collecting a targeted antibody from a culture obtained;

ii) treating the antibody obtained in step i) with hydrolase to produce an antibody with N297 glycan being (Fucα1,6)GlcNAc ((Fucαl,6)GlcNAc-antibody) (Figure 3A);

preferably further purifying the (Fucαl,6)GlcNAc-antibody through a step including purification of the reaction solution with a hydroxyapatite column; and

iii) reacting the (Fucαl,6)GlcNAc-antibody with a glycan donor molecule in the presence of transglycosidase to

synthesize a glycan-remodeled antibody with an azide group introduced to a sialic acid, the glycan donor molecule obtained by introducing a PEG linker having an azide group ($N_3$-L(PEG)) to the carbonyl group of carboxylic acid at the 2-position of a sialic acid in MSG (9) or SG (10) and oxazolinating the reducing terminal (Figure 3B).

The present invention includes glycan-remodeled antibodies and functional fragments of the antibodies, and modified variants of the antibodies and functional fragments obtained by using the production method.

The production intermediate of the present antibody-drug conjugate has an alkyne structure reactive with an azide group, such as DBCO (dibenzocyclooctyne) (see Example 2-3). Therefore, the antibody-drug conjugate of the present invention can be produced by reacting the production intermediate with an MSG1-type, MSG2-type, or SG-type glycan-remodeled antibody or a functional fragment of the antibody, where the antibody, in which a PEG linker having an azide group has been introduced to a sialic acid of a glycan, is obtained through steps i) to iii).

With regard to N297 glycan in the present invention, fucosylated GlcNAc-(Fucα1,6)GlcNAc) at the reducing terminal is preferably derived from an antibody produced in an animal cell, and a portion of the glycan located to the non-reducing terminal side of (Fucα1,6)GlcNAc preferably has been remodeled into the above-described glycan structure as MSG (MSG1, MSG2) or SG. In each case, carboxylic acid bonding to the 2-position of a sialic acid at the non-reducing terminal is used for bonding to L(PEG).

Such a glycan-remodeled antibody having MSG- (MSG1-, MSG2-) or SG-type N297 glycan may be produced by using a method as illustrated in Figure 3, for example, in accordance with a method described in WO 2013/120066. If an antibody is produced as a gene-recombinant protein by using an animal cell as a host in accordance with a known method (step i), the N297 glycan has, as a base structure, a fucosylated N-linked glycan structure, whereas a mixture of antibody molecules having glycans of various structures with various modifications for the structure of the non-reducing terminal or constituent saccharides or fragments of such antibody molecules is provided (IV in Figure 3A). Treatment of such an antibody produced with an animal cell with hydrolase such as EndoS causes hydrolysis of the glycosidic bond at GlcNAcβ1-4GlcNAc in the chitobiose structure at the reducing terminal, providing antibody molecules of single glycan structure having only (Fucα1,6)GlcNAc as N297 glycan (referred to as "(Fucα1,6)GlcNAc-antibody", see A in Figure 2) (Figure 3A) (step ii)).

For the enzyme for the hydrolysis reaction of N297 glycan, for example, Endo S or a variant enzyme retaining the hydrolysis activity may be used.

By reacting the (Fucα1,6)GlcNAc-antibody obtained in the above hydrolysis reaction, as a glycan acceptor molecule, and an MSG- (MSG1-, MSG2-) or SG-type glycan donor molecule with use of transglycosidase (e.g., WO 2017010559) such as EndoS D233Q and EndoS D233Q/Q303L variants, an antibody of the above-described structure including MSG- (MSG1-, MSG2-) or SG type N297 glycan (see B in Figure 2) can be obtained (Figure 3B) (step iii)-1, iii)-2).

If the number of conjugated drug molecules per antibody molecule, $m^1$, in the antibody-drug conjugate is 1, a glycan donor molecule having MSG (MSG1, MSG2) as glycan is employed. For such glycan, commercially available monosialo-Asn free (1S2G/1G2S-10NC-Asn, GlyTech, Inc., hereinafter, referred to as "(MSG-)Asn") as a raw material may be separated in accordance with a method described in Example 3 to obtain (MSG-)Asn1 or (MSG2-)Asn, which may be employed, or a mixture of them may be employed without separation.

If the number of conjugated drug molecules per antibody molecule, $m^1$, in the antibody-drug conjugate is 2, a glycan donor molecule including SG (10) as glycan is used for the transglycosylation reaction. For such SG (10) glycan, for example, that obtained from SGP through hydrolysis or the like may be used, or SG (10) glycan such as commercially available disialooctasaccharide (Tokyo Chemical Industry Co., Ltd.) may be used.

MSG- (MSG1-, MSG2-) or SG-type glycan included in the donor molecule has a PEG linker having an azide group ($N_3$-L(PEG)) at the 2-position of a sialic acid therein.

It is preferred to use an activated form such as an oxazolinated form formed by treatment with 2-chloro-1,3-dimethyl-1H-benzimidazol-3-ium-chloride for GlcNAc at the reducing terminal of MSG (MSG1, MSG2) or SG-type glycan included in the donor molecule (J. Org. Chem., 2009, 74(5), 2210-2212).

Various enzymes for use in transglycosylation reaction (transglycosidase) may be employed that have activity of transferring complex glycan to N297 glycan; however, EndoS D233Q, a modified product for which hydrolysis reaction is suppressed by substituting Asp at the 233-position of EndoS with Gln, is a preferred transglycosidase. Transglycosylation reaction using EndoS D233Q is described, for example, in WO 2013/120066. Alternatively, a modified enzyme such as EndoS D233Q/Q303L (WO 2017/010559), which is obtained by further adding a mutation to EndoS D233Q, may be used.

The purification operation for the antibody after the glycan remodeling for the antibody (glycohydrolysis and transglycosylation reaction) is intended to separate low-molecular-weight compounds and enzymes used for the reaction, and gel filtration chromatography, ion-exchange chromatography, affinity chromatography, and so on are typically used for such purification, and additional purification with a hydroxyapatite column may be further carried out. That is, the present invention provides a method for producing an antibody-drug conjugate, the method including, subsequent to the step of purifying an intermediate from reaction solution after glycohydrolysis of an antibody, the additional step of

purifying with a hydroxyapatite column. According to an example of reports on glycan remodeling (JACS. 2012, 134, 12308-12318., Angew. Chem. Int. Ed. 2016, 55, 2361-2367), reaction solution after treatment of an antibody with hydrolase is purified only with a Protein A column (affinity chromatography column); however, this purification method has been proved to be incapable of completely removing hydrolase (e.g., EndoS), and affect the subsequent transglycosylation reaction because of the residual enzyme. In view of such a result, examination was made on purification methods to find that when purification of reaction solution after treatment of an antibody with hydrolase was carried out using a Protein A column and a hydroxyapatite column (CHT column, Bio-Rad Laboratories, Inc.) in the order presented, the reaction efficiency of the subsequent glycosylation reaction was enhanced, without the influence of a residual enzyme.

The antibody-drug conjugate of the present invention is the most preferably one antibody-drug conjugate selected from the following group:

[Formula 50]

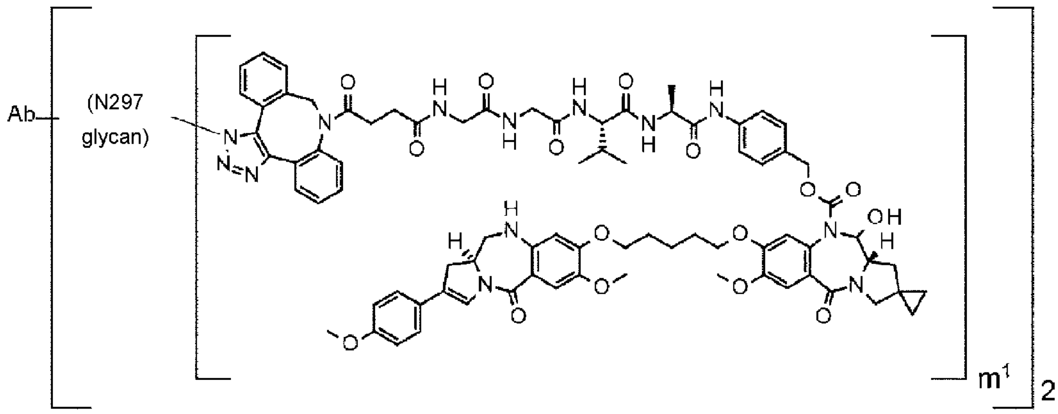

or

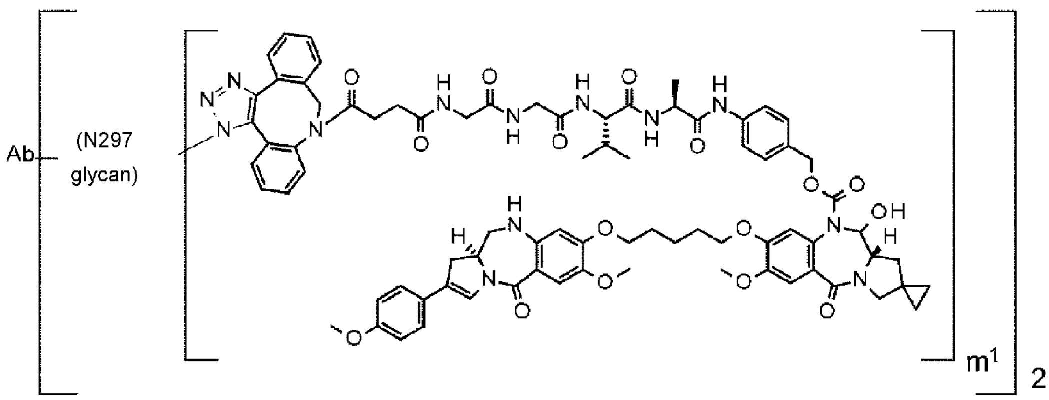

[Formula 51]

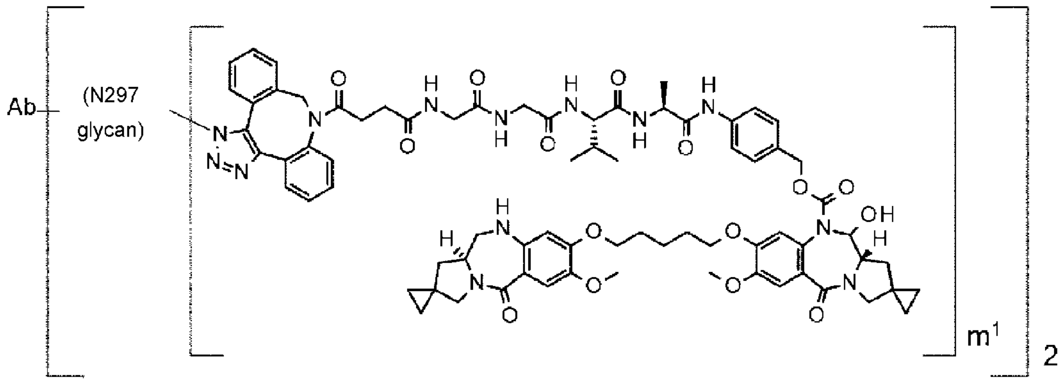

or

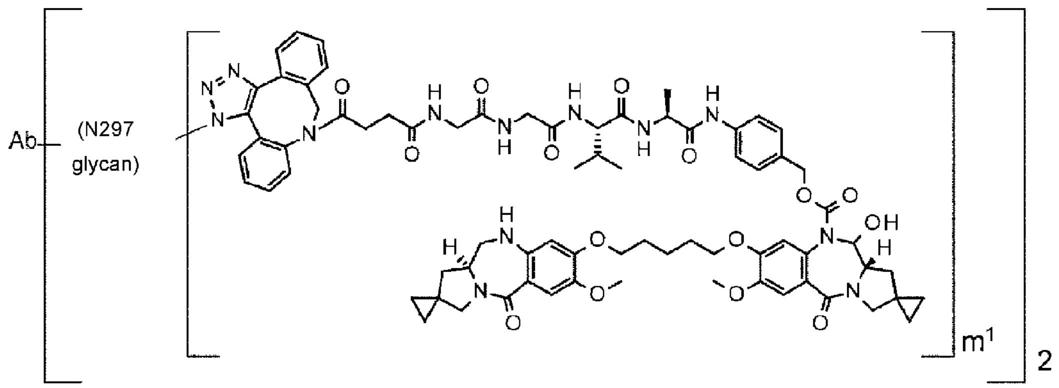

[Formula 52]

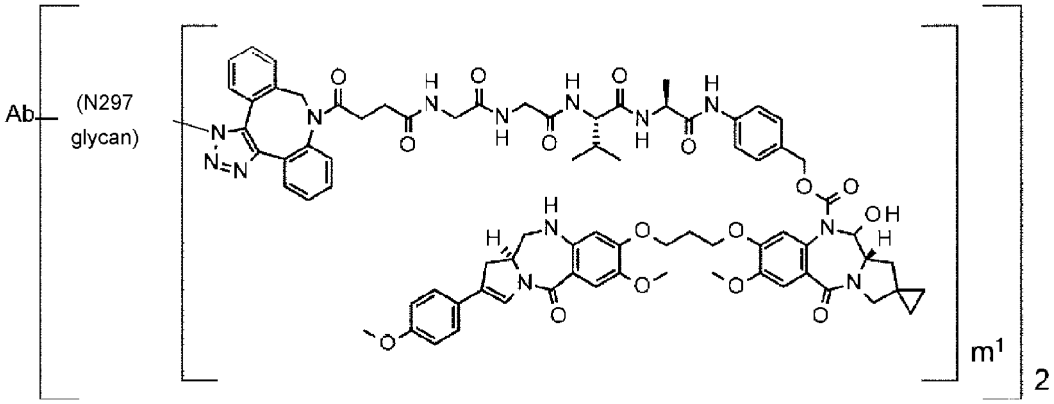

or

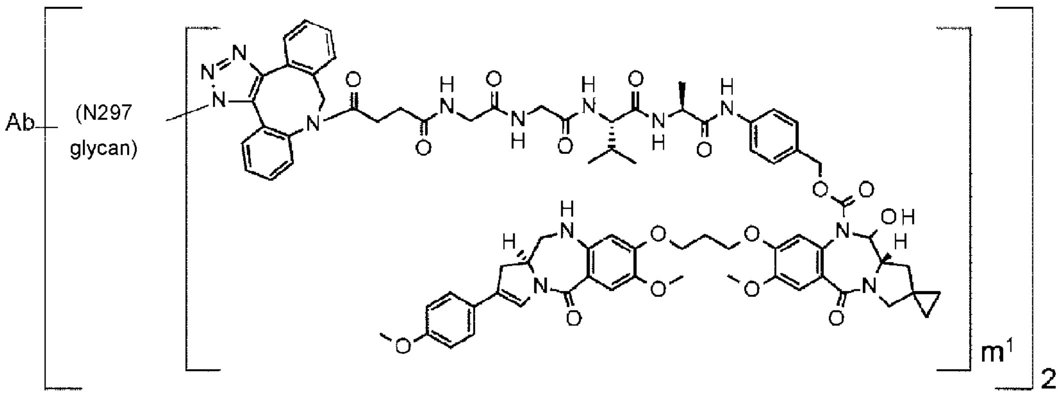

[Formula 53]

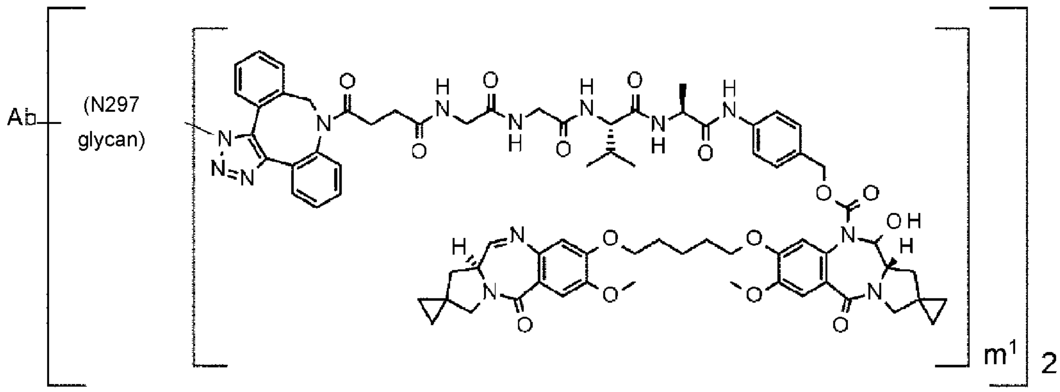

or

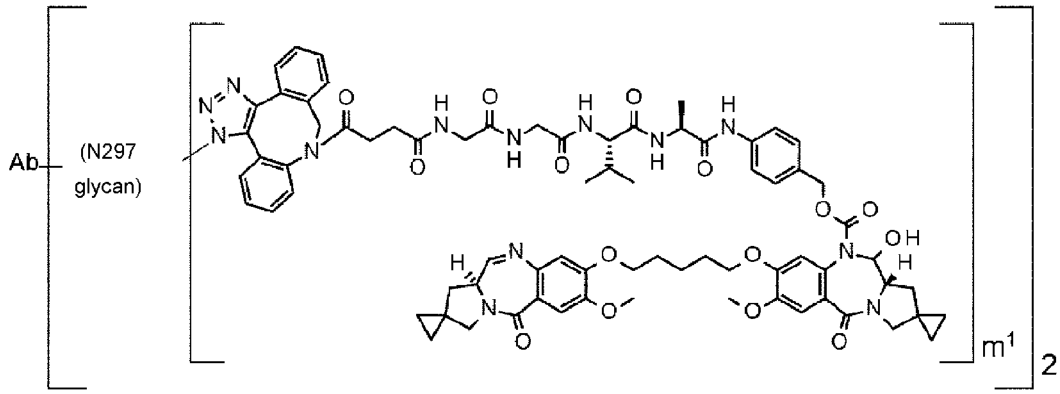

[Formula 54]

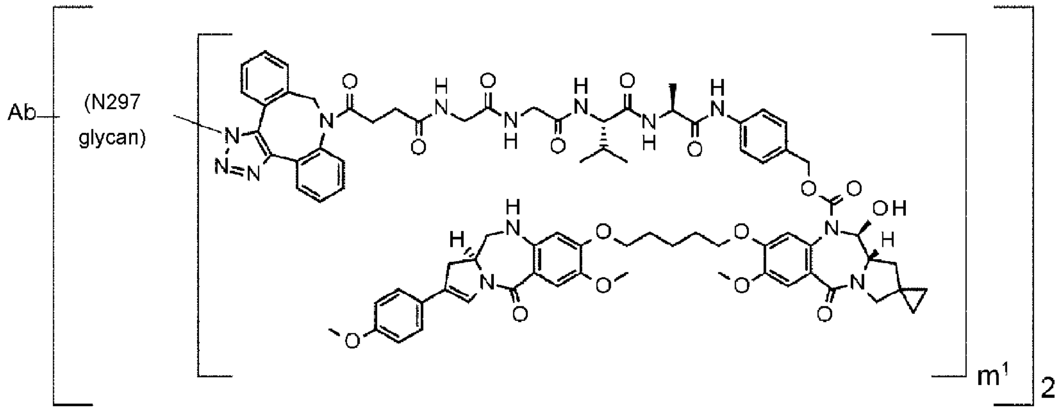

or

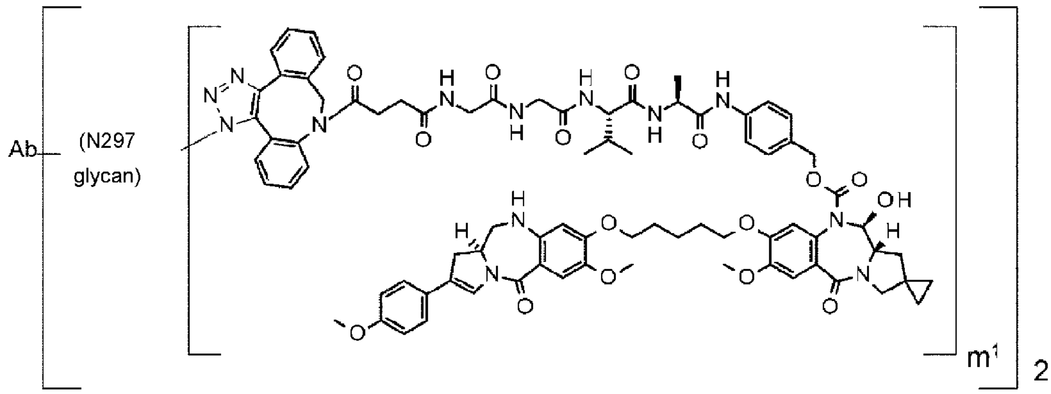

[Formula 55]

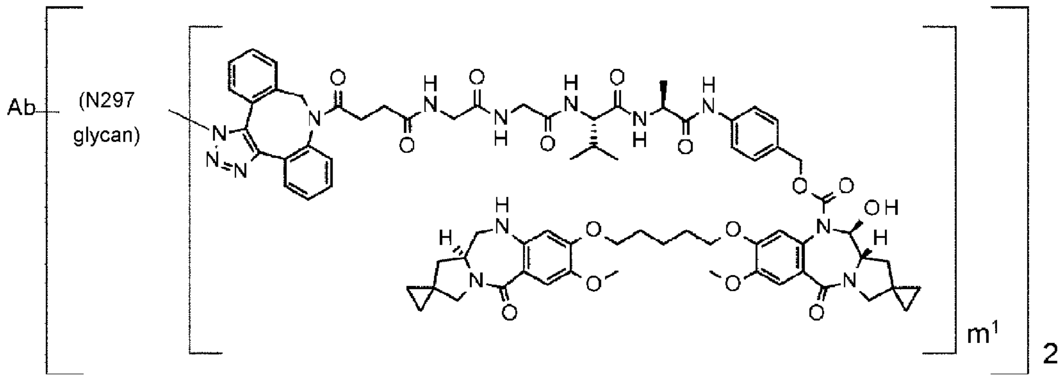

or

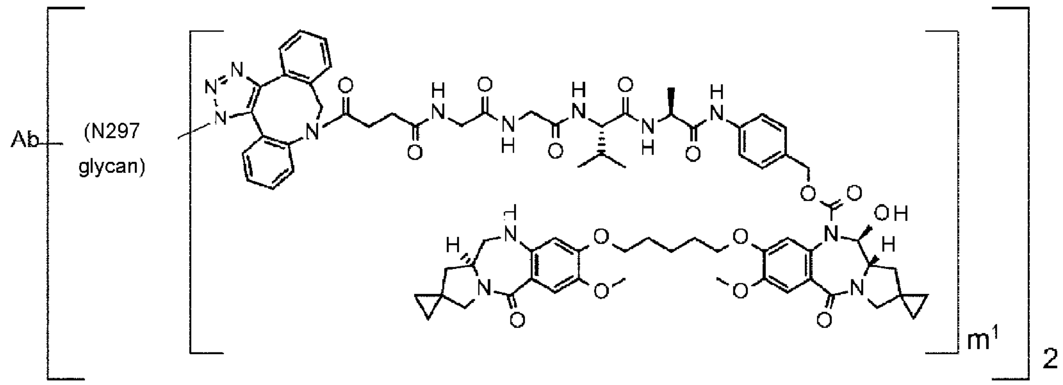

[Formula 56]

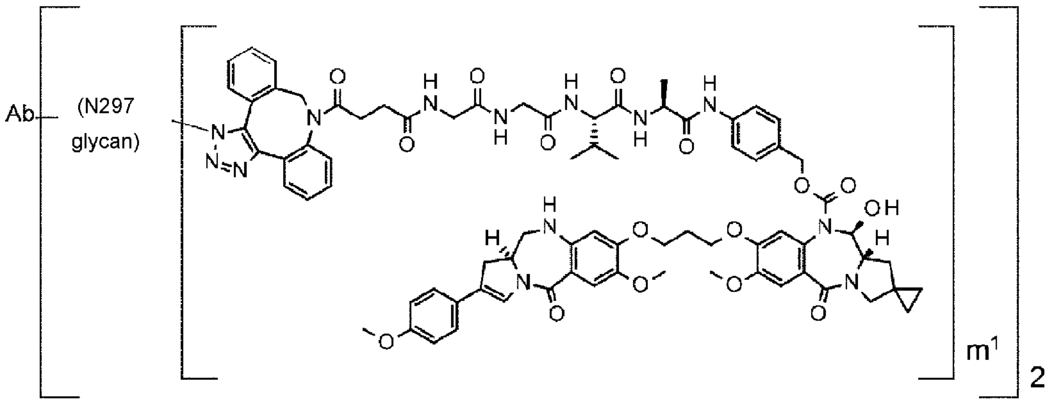

or

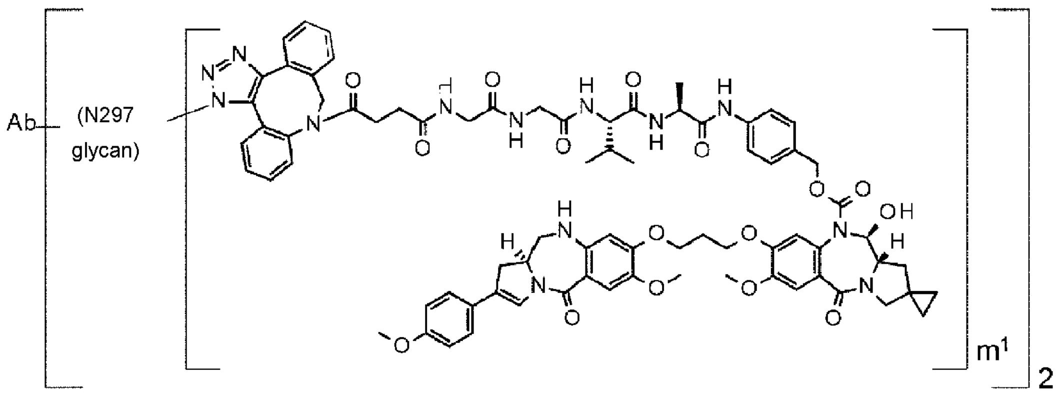

[Formula 57]

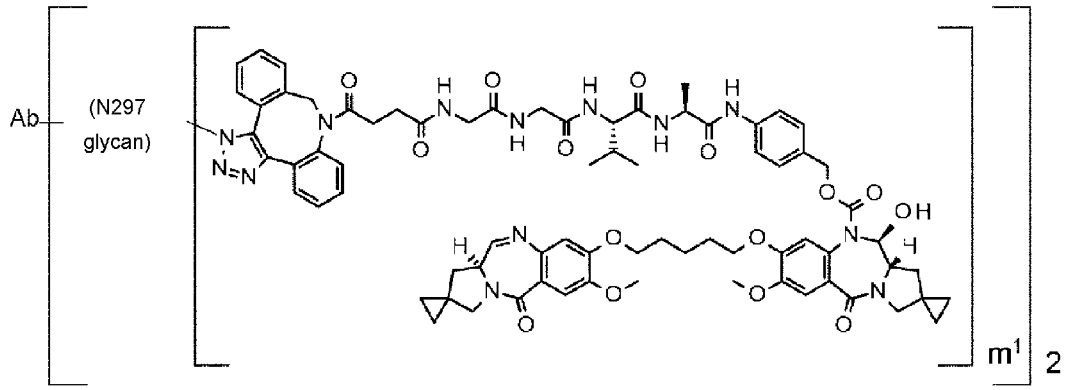

or

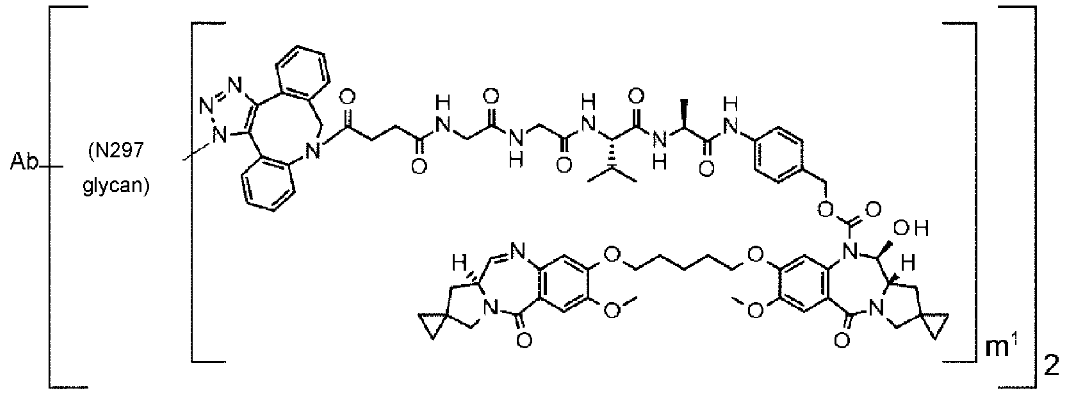

In each of the structural formulas above,

$m^1$ represents an integer of 1 or 2 (preferably, $m^1$ is an integer of 1),
antibody Ab is the anti-HER2 antibody or a functional fragment of the antibody,
N297 glycan represents any one of N297-(Fuc)MSG1, N297-(Fuc)MSG2, and a mixture of them, and N297-(Fuc)SG (preferably, N297-(Fuc)MSG1),
L(PEG) represents $-NH-CH_2CH_2-(O-CH_2CH_2)_3-*$, wherein the amino group at the left end represents bonding via an amide bond to carboxylic acid at the 2-position of a sialic acid at the non-reducing terminal of each or either one of the 1-3 and 1-6 branched chains (preferably, the 1-3 branched chain) of β-Man in N297 glycan, and the asterisk (*) at the right end represents bonding to a nitrogen atom at the 1- or 3-position of the triazole ring of Lb in linker L.

Although structures with two or four units ($m^1$ = 1 or 2) of "-(N297 glycan)-L-D" in each of which N297 glycan bonds to the nitrogen atom at the 1-position of the triazole ring of Lb in L in one conjugate molecule ("(N297 glycan)-(N1Lb)L-D") or structures with two or four units ($m^1$ = 1 or 2) of "-(N297 glycan)-L-D" in each of which N297 glycan bonds to the nitrogen atom at the 3-position of the triazole ring of Lb in L in one conjugate molecule ("(N297 glycan)-(N3Lb)L-D") are illustrated as the most preferred antibody-drug conjugate for convenience, antibody-drug conjugates having both "(N297 glycan)-(N1Lb)L-D" (if $m^1$ = 1, then one unit, if $m^1$ = 2, then one, two, or three units) and "(N297 glycan)-(N3Lb)L-D" (if $m^1$ = 1, then one unit, if $m^1$ = 2, then three, two, or one unit) in one conjugate molecule are also included. In other words, either one of "(N297 glycan)-(N1Lb)L-D" and "(N297 glycan)-(N3Lb)L-D" exists or both of them coexist in one conjugate molecule.

The anti-HER2 antibody or anti-HER2 antibody-drug conjugate of the present invention exhibits strong tumor activity (in vivo antitumor activity, in vitro anticellular activity) and satisfactory in vivo kinetics and physical properties, and have high safety, and hence is useful as a pharmaceutical.

The number of conjugated drug molecules per antibody molecule is an important factor having influence on efficacy and safety for the antibody-drug conjugate of the present invention. Antibody-drug conjugates are produced with reaction conditions, such as the amounts of raw materials and reagents to be reacted, specified so as to give a constant number of conjugated drug molecules, but, in contrast to chemical reaction of low-molecular-weight compounds, a mixture with different numbers of conjugated drug molecules is typically obtained. Numbers of conjugated drug molecules per antibody molecule are specified as the average value, namely, the average number of conjugated drug

molecules (DAR: Drug to Antibody Ratio). The number of pyrrolobenzodiazepine derivative molecules conjugated to an antibody molecule is controllable, and 1 to 10 pyrrolobenzodiazepine derivative molecules can be conjugated as the average number of conjugated drug molecules per antibody molecule (DAR), but preferably the number is one to eight, and more preferably one to five.

If the antibody bonds via a remodeled glycan of the antibody to L in the antibody-drug conjugate of the present invention, the number of conjugated drug molecules per antibody molecule in the antibody-drug conjugate, $m^2$, is an integer of 1 or 2. If the glycan is N297 glycan and the glycan is N297-(Fuc)MSG1, N297-(Fuc)MSG2, or a mixture of N297-(Fuc)MSG1 and N297-(Fuc)MSG2, $m^2$ is 1, and DAR is in the range of 1 to 3 (preferably, in the range of 1.0 to 2.5, more preferably, in the range of 1.2 to 2.2, or 1.6 to 2.2). If the N297 glycan is N297-(Fuc)SG, $m^2$ is 2, and DAR is in the range of 3 to 5 (preferably, in the range of 3.2 to 4.8, more preferably, in the range of 3.5 to 4.2).

Those skilled in the art could engineer the reaction method to conjugate a required number of drug molecules to each antibody molecule on the basis of the description in Examples herein, and obtain an antibody with a controlled number of conjugated pyrrolobenzodiazepine derivative molecules.

The antibody-drug conjugate, free drug, or production intermediate of the present invention may absorb moisture, allow adhesion of adsorbed water, or become a hydrate when being left to stand in the atmosphere or recrystallized, and such compounds and salts containing water are also included in the present invention.

The antibody-drug conjugate, free drug, or production intermediate of the present invention may be converted into a pharmaceutically acceptable salt, as desired, if having a basic group such as an amino group. Examples of such salts may include, but not limited to, hydrogen halide salts such as hydrochlorides and hydroiodides; inorganic acid salts such as nitrates, perchlorates, sulfates, and phosphates; lower alkanesulfonates such as methanesulfonates, trifluoromethanesulfonates, and ethanesulfonates; arylsufonates such as benzenesulfonates and p-toluenesulfonates; organic acid salts such as formates, acetates, malates, fumarates, succinates, citrates, tartrates, oxalates, and maleates; and amino acid salts such as ornithinates, glutamates, and aspartates.

If the antibody-drug conjugate, free drug, or production intermediate of the present invention has an acidic group such as a carboxy group, a base addition salt can be generally formed. Examples of pharmaceutical acceptable salts may include, but not limited to, alkali metal salts such as sodium salts, potassium salts, and lithium salts; alkali earth metal salts such as calcium salts and magnesium salts; inorganic salts such as ammonium salts; and organic amine salts such as dibenzylamine salts, morpholine salts, phenylglycine alkyl ester salts, ethylenediamine salts, N-methylglucamates, diethylamine salts, triethylamine salts, cyclohexylamine salts, dicyclohexylamine salts, N,N'-dibenzylethylenediamine salts, diethanolamine salts, N-benzyl-N-(2-phenylethoxy)amine salts, piperazine salts, tetramethylammonium salts, and tris(hydroxymethyl)aminomethane salts.

The antibody-drug conjugate, free drug, or production intermediate of the present invention may exist as a hydrate, for example, by absorbing moisture in the air. The solvate of the present invention is not limited to a particular solvate and may be any pharmaceutically acceptable solvate, and specifically hydrates, ethanol solvates, 2-propanol solvates, and so on are preferred. The antibody-drug conjugate, free drug, or production intermediate of the present invention may be its N-oxide form if a nitrogen atom is present therein, and these solvates and N-oxide forms are included in the scope of the present invention.

The present invention includes compounds labeled with various radioactive or nonradioactive isotopes. The antibody-drug conjugate, free drug, or production intermediate of the present invention may contain one or more constituent atoms with non-natural ratios of atomic isotopes. Examples of atomic isotopes may include, but not limited to, deuterium ($^2H$), tritium ($^3H$), iodine-125 ($^{125}I$), and carbon-14 ($^{14}C$). The compound of the present invention may be radiolabeled with a radioactive isotope such as tritium ($^3H$), iodine-125 ($^{125}I$), and carbon-14 ($^{14}C$). The radiolabeled compound is useful as a therapeutic or prophylactic agent, a reagent for research such as an assay reagent, and a diagnostic agent such as a diagnostic agent for in vivo imaging. Isotopic variants of the antibody-drug conjugate of the present invention are all included in the scope of the present invention, regardless of whether they are radioactive or not.

[Production methods]

Scheme R: Preparation of ant-HER2 antibody

A glycan-remodeled antibody may be produced by using a method as illustrated in Figure 3, for example, according to a method described in WO 2013/120066.

[Formula 58]

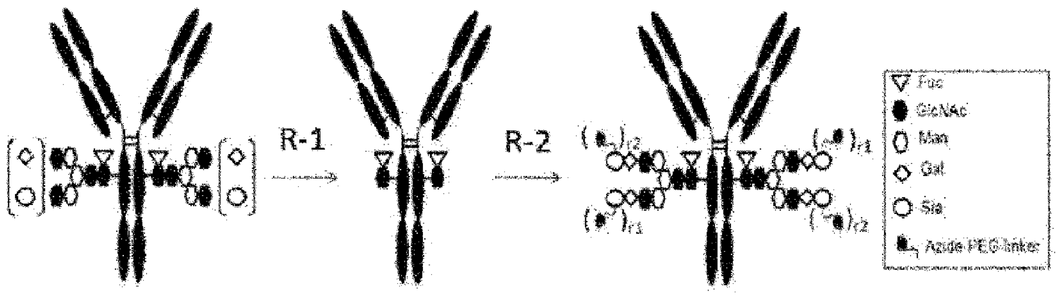


(r1, r2) = any of (1,0), (0,1), and (1,1)

Step R-1: Hydrolysis of glycosidic bond at GlcNAcβ1-4GlcNAc of chitobiose structure at reducing terminal

The step is a step of preparing a glycan-truncated antibody by cleaving N-linked glycan bonding to asparagine at the 297-position of the amino acid sequence of a targeted antibody (N297-linked glycan) with use of a known enzymatic reaction.

A targeted antibody (20 mg/mL) in buffer solution (e.g., 50 mM phosphate buffer solution) is subjected to hydrolysis reaction of the glycosidic bond between GlcNAcβ1 and 4GlcNAc in the chitobiose structure at the reducing terminal with use of hydrolase such as the enzyme EndoS at 0°C to 40°C. The reaction time is 10 minutes to 72 hours, and preferably 1 hour to 6 hours. The amount of the wild-type enzyme EndoS to be used is 0.1 to 10 mg, preferably 0.1 to 3 mg, to 100 mg of the antibody. After the completion of the reaction, purification with affinity chromatography and/or purification with a hydroxyapatite column, each described later, are/is carried out to produce a (Fucα1,6)GlcNAc antibody with the glycan hydrolyzed between GlcNAcβ1 and 4GlcNAc.

Step R-2: Transglycosylation reaction

The step is a step of producing a glycan-remodeled antibody by bonding the (Fucα1,6)GlcNAc antibody to MSG- (MSG1-, MSG2-) or SG-type glycan oxazoline form (hereinafter, referred to as "azide glycan oxazoline form") having a PEG linker including an azide group with use of enzymatic reaction.

The glycan-truncated antibody in buffer solution (e.g., phosphate buffer solution) is subjected to transglycosylation reaction by reacting with an azide glycan oxazoline form in the presence of a catalytic amount of transglycosidase such as EndoS (D233Q/Q303L) at 0°C to 40°C. The reaction time is 10 minutes to 72 hours, and preferably 1 hour to 6 hours. The amount of the enzyme EndoS (D233Q/Q303L) to be used is 1 to 10 mg, preferably 1 to 3 mg, to 100 mg of the antibody, and the amount of the azide glycan oxazoline form to be used is 2 equivalents to an excessive equivalent, preferably 2 equivalents to 20 equivalents.

After the completion of the reaction, purification with affinity chromatography and purification with a hydroxyapatite column are carried out to afford a purified glycan-remodeled antibody.

The azide glycan oxazoline form may be prepared according to methods described in Examples 3 to 5. By using a reaction known in the field of synthetic organic chemistry (e.g., condensation reaction), $N_3$-$(CH_2CH_2$-$O)n_5$-$CH_2CH_2$-$NH_2$, a PEG linker including an azide group ($N_3$-L(PEG)), may be introduced to MSG (MSG(9), MSG1, MSG2) or disialooctasaccharide (SG(10), Tokyo Chemical Industry Co., Ltd.). Specifically, carboxylic acid at the 2-position of a sialic acid and the amino group at the right end of $N_3$-$(CH_2CH_2$-$O)n_5$-$CH_2CH_2$-$NH_2$ undergo a known condensation reaction to form an amide bond.

MSG, MSG1, or MSG2 may be obtained by hydrolysis of the (MSG-)Asn or separated/purified (MSG1-)Asn or (MSG2-)Asn with hydrolase such as EndoM.

In preparing the glycan-remodeled antibody, concentration of an aqueous solution of an antibody, measurement of concentration, and buffer exchange may be carried out according to common operations A to C in the following.

(Common operation A: Concentration of aqueous solution of antibody)

A solution of an antibody or antibody-drug conjugate was placed in a container of an Amicon Ultra (30,000 to 50,000, MWCO, Millipore Corporation), and the solution of an antibody or antibody-drug conjugate, which is described later, was concentrated through a centrifugation operation (centrifugation at 2000 G to 4000 G for 5 to 20 minutes) using a centrifuge (Allegra X-15R, Beckman Coulter, Inc.).

(Common operation B: Measurement of antibody concentration)

Measurement of antibody concentration was carried out by using a UV measurement apparatus (Nanodrop 1000, Thermo Fisher Scientific Inc.) according to a method specified by the manufacturer. Then, 280 nm absorption coefficients, being different among antibodies (1.3 mL $mg^{-1}$ $cm^{-1}$ to 1.8 mL $mg^{-1}$ $cm^{-1}$), were used.

(Common operation C: Buffer exchange for antibody)

A buffer solution (e.g., phosphate buffered saline (pH 6.0), phosphate buffer (pH 6.0)) was added to an aqueous solution of an antibody, which was concentrated according to common operation A. This operation was carried out several times, and the antibody concentration was then measured by using common operation B, and adjusted to 10 mg/mL with a buffer solution (e.g., phosphate buffered saline (pH 6.0), phosphate buffer (pH 6.0)).

Scheme S: Conjugation

The production method is a method for producing an antibody-drug conjugate by conjugating the above-described glycan-remodeled antibody to production intermediate (2) through SPAAC reaction (strain-promoted alkyne azide cycloaddition: JACS. 2004, 126, 15046-15047).

[Formula 59]

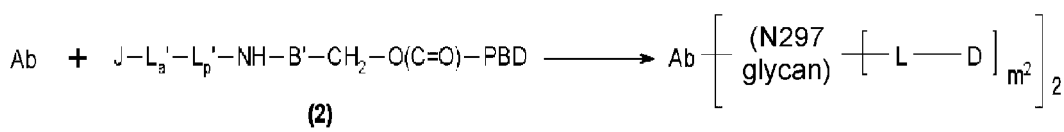

(2)

In the formula, Ab represents the glycan-remodeled antibody,

La', Lp', and B' are synonymous with La, Lp, and B, respectively,
J represents any one of the following structures,
wherein each asterisk (*) represents bonding to La'.

[Formula 60]

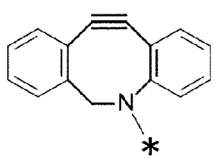
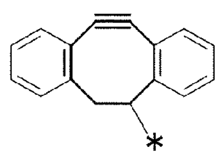
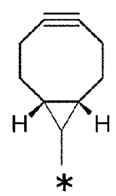

J-La'-Lp'-NH-B'-$CH_2$-O(C=O)-PBD can be synthesized, for example, by using any of methods described in Examples 2-1 to 2-6.

SPAAC reaction proceeds by mixing a buffer solution (sodium acetate solution, sodium phosphate, sodium borate solution, or the like, or a mixture thereof) of antibody Ab and a solution obtained by dissolving compound (2) in an appropriate solvent (dimethyl sulfoxide (DMSO), dimethylformamide (DMF), dimethylacetamide (DMA), N-methyl-2-pyridone (NMP), propylene glycol (PG), or the like, or a mixture thereof).

The amount of moles of compound (2) to be used is 2 mol to an excessive amount of moles, preferably 1 mol to 30 mol, per mole of the antibody, and the ratio of the organic solvent is preferably 1 to 200% v/v to the buffer of the antibody. The reaction temperature is 0°C to 37°C, and preferably 10°C to 25°C, and the reaction time is 1 to 150 hours, and preferably 6 hours to 100 hours. The pH in the reaction is preferably 5 to 9.

Antibody-drug conjugate can be identified from each other through buffer exchange, purification, and measurement of antibody concentration and average number of conjugated drug molecules per antibody molecule according to common operations A to C described above and common operations D to F described later.

(Common operation D: Purification of antibody-drug conjugate)

An NAP -25 column was equilibrated with acetic acid buffer solution (10 mM, pH 5.5; herein, referred to as ABS) containing commercially available sorbitol (5%). To this NAP-25 column, an aqueous reaction solution of an antibody-drug conjugate (about 1.5 to 2.5 mL) was applied, and eluted with a buffer in an amount specified by the manufacturer to separate and collect an antibody fraction. The fraction separated and collected was again applied to the NAP-25 column, and a gel filtration purification operation to elute with a buffer was repeated twice or three times in total to afford the antibody-drug conjugate with an unbound drug-linker, dimethyl sulfoxide, and propylene glycol removed. As necessary, the concentration of the solution of the antibody-drug conjugate was adjusted through common operations A to C.

(Common operation E: Measurement of antibody concentration of antibody-drug conjugate)

The concentration of the conjugated drug in an antibody-drug conjugate can be calculated by using the Lambert-Beer's law shown below.

Expression (I) using the Lambert-Beer's law is as follows.

[Expression 1]

$$\underline{A_{280}} = \underline{\varepsilon_{280}(L\cdot mol^{-1}\cdot cm^{-1})}\cdot\underline{C(mol\cdot L^{-1})}\cdot\underline{l(cm)} \qquad \text{Expression(I)}$$

$$\text{Absorbance} = \text{Molar absorption coefficient} \times \text{Molarity} \times \text{Optical path length}$$

Here, A280 denotes absorbance of an aqueous solution of an antibody-drug conjugate at 280 nm, ε280 denotes the molar absorption coefficient of an antibody-drug conjugate at 280 nm, and C ($mol\cdot L^{-1}$) denotes the molarity of an antibody-drug conjugate. From expression (I), the molarity of an antibody-drug conjugate, C ($mol\cdot L^{-1}$), can be determined by using expression (II) below.

[Expression 2]

$$C(mol\cdot L^{-1}) = \frac{A_{280}}{\varepsilon_{280}(L\cdot mol^{-1}\cdot cm^{-1})\cdot l(cm)} \qquad \text{Expression (II)}$$

Further, the both sides are multiplied by the molar mass of the antibody-drug conjugate, MW ($g\cdot mol^{-1}$), to determine the weight concentration of the antibody-drug conjugate, C' ($mg\cdot mL^{-1}$) (expression (III)).

[Expression 3]

$$C'(mg\cdot mL^{-1}) = MW(g\cdot mol^{-1})\cdot C(mol\cdot L^{-1}) = \frac{A_{280}\cdot MW(g\cdot mol^{-1})}{\varepsilon_{280}(L\cdot mol^{-1}\cdot cm^{-1})\cdot l(cm)} \qquad \text{Expression(III)}$$

Values used for the expression and applied to Examples will be described.

The absorbance A280 used was a measured value of UV absorbance of an aqueous solution of an antibody-drug conjugate at 280 nm. For molar mass, MW ($g\cdot mol^{-1}$), an estimated value of the molecular weight of an antibody was calculated from the amino acid sequence of the antibody, and used as an approximate value of the molar mass of an antibody-drug conjugate. The optical path length, 1 (cm), used in measurement was 1 cm.

The molar absorption coefficient, ε280, of the antibody-drug conjugate can be determined by using expression (IV) below.

[Expression 4]

$$\varepsilon_{280} = \text{Molar absorption Coefficient of antibody } \varepsilon_{Ab,\,280} + \text{Molar absorption Coefficient of drug } \varepsilon_{DL,\,280} \times \text{Number of conjugated drug molecules} \qquad \text{Expression (IV)}$$

Here, $\varepsilon_{Ab,\,280}$ denotes the molar absorption coefficient of an antibody at 280 nm, and $\varepsilon_{DL,\,280}$ denotes the molar absorption coefficient of a drug at 280 nm.

By using a known calculation method (Protein Science, 1995, vol. 4, 2411-2423), $\varepsilon_{Ab,\,280}$ can be estimated from the amino acid sequence of an antibody. In Examples, the molar absorption coefficient of Trastuzumab A1 antibody used was $\varepsilon_{Ab,\,280}$ = 215057 (calculated estimated value); and the molar absorption coefficient of Trastuzumab A2 used was $\varepsilon_{Ab,\,280}$ = 215380 (calculated estimated value). The molar absorption coefficient of the H01L02 antibody used was $\varepsilon_{Ab,\,280}$ = 210014 (calculated estimated value), the molar absorption coefficient of the HwtL05 antibody used was $\varepsilon_{Ab,\,280}$ = 212834 (calculated estimated value), the molar absorption coefficient of the LPS antibody used was $\varepsilon_{Ab,\,280}$ = 230300 (calculated estimated value).

$\varepsilon_{DL,\,280}$ was calculated for use from a measured value obtained in each UV measurement. Specifically, the absorbance of a solution obtained by dissolving a conjugate precursor (drug) with a certain molarity was measured, and expression (I), the Lambert-Beer's law, was applied thereto, and the resulting value was used.

(Common operation F: Measurement of average number of conjugated drug molecules per antibody molecule in antibody-drug conjugate)

The average number of conjugated drug molecules per antibody molecule in an antibody-drug conjugate can be determined through high-performance liquid chromatography (HPLC) with the following method.

[F-1. Preparation of sample for HPLC analysis (reduction of antibody-drug conjugate)]

A solution of an antibody-drug conjugate (about 1 mg/mL, 60 μL) is mixed with an aqueous solution of dithiothreitol (DTT) (100 mM, 15 μL). The mixture is incubated at 37°C for 30 minutes to prepare a sample in which the disulfide bond between the L chain and H chain of the antibody-drug conjugate cleaved, and this sample is used for HPLC analysis.

[F-2. HLPC analysis]

HPLC analysis is carried out under the following conditions.

HPLC system: Agilent 1290 HPLC system (Agilent Technologies)
Detector: Ultraviolet absorption spectrometer (measurement wavelength: 280 nm, 329 nm)
Column: BEH Phenyl (2.1 × 50 mm, 1.7 μm, Waters Acquity)
Column temperature: 75°C
Mobile phase A: 0.1% trifluoroacetic acid (TFA)-15% isopropyl alcohol aqueous solution
Mobile phase B: 0.075% TFA-15% isopropyl alcohol acetonitrile solution
Gradient program: 14%-36% (0 min to 15 min), 36%-80% (15 min to 17 min), 80%-14% (17 min to 17.1 min), 14%-14% (17.1 min to 23 min)
Sample injection volume: 5μL

[F-3. Data analysis]

[F-3-1] An H chain with a conjugated drug molecule(s) (H chain with one conjugated drug molecule: $H_1$, H chain with two conjugated drug molecules: $H_2$) have hydrophobicity increased in proportion to the number of conjugated drug molecules and have longer retention time as compared to H chain (Ho) of an antibody without any conjugated drug molecule, and hence $L_0$, $H_0$, $H_1$, and $H_2$, are eluted in the presented order. Through comparison of retention

time, each peak detected can be assigned to $L_0$, $H_0$, $H_1$, or $H_2$. In addition, conjugation of the drug can be confirmed via absorption at a wavelength of 329 nm, which is characteristic to the drug.
[F-3-2] Since each drug-linker absorbs UV, peak area values are corrected by using the following expression with the molar absorption coefficients of an L chain, H chain, and drug-linker according to the number of conjugated drug-linker molecules.

[Expression 5]

$$\text{Corrected H chain peak area (Hi)} = \text{Peak area} \times \frac{\text{Molar absorption coefficient of H chain}}{\text{Molar absorption coefficient of H chain} + \text{Number of conjugated drug molecules} \times \text{Molar absorption coefficient of drug-linker}}$$

Here, for the molar absorption coefficients (280 nm) of the L chain and H chain of each antibody, values estimated from the amino acid sequences of the L chain and H chain of the antibody by using a known calculation method (Protein Science, 1995, vol. 4, 2411-2423) may be used.
In the case of Trastuzumab A1, 81488 was used as the molar absorption coefficient of the H chain estimated from the amino acid sequence. In the case of Trastuzumab A2, 81478 was used as the molar absorption coefficient of the H chain estimated from the amino acid sequence. In the case of the H01L02 antibody, similarly, 79989 was used as the molar absorption coefficient of the H chain; in the case of the HwtL05 antibody, 81488 was used as the molar absorption coefficient of the H chain; in the case of the LPS antibody, 77470 was used as the molar absorption coefficient of the H chain; and the molar absorption coefficient (280 nm) measured for drug-linker 1 (Example 2-3), as a conjugate precursor, was used as the molar absorption coefficient (280 nm) of each drug-linker.
[F-3-3] The peak area ratio (%) of each chain to the total of corrected peak areas is calculated by using the following expression.

[Expression 6]

$$\text{H chain peak area ratio} = \frac{A_{Hi}}{A_{H0} + A_{H1} + A_{H2}} \times 100$$

$A_{Hi}$: Hi corrected peak area

[F-3-4] The average number of conjugated drug molecules per antibody molecule in an antibody-drug conjugate is calculated by using the following expression.

[Expression 7]

Average number of conjugated drug molecules = ($L_0$ peak area ratio x0 + $L_0$ peak area ratio x1 + $H_0$ peak area ratio x0 + $H_1$ peak area ratio x1)/100x2

<Medicine>

The antibody-drug conjugate of the present invention exhibits cellular cytotoxic activity to cancer cells, and hence may be used as a medicine, in particular, a therapeutic agent and/or prophylactic agent for cancer.
Examples of cancers to which the antibody-drug conjugate of the present invention is applied may include lung cancer, urothelial cancer, colorectal cancer, prostate cancer, ovarian cancer, pancreatic cancer, breast cancer, bladder cancer, gastric cancer, gastric and intestinal stromal tumor, uterine cervix cancer, esophageal cancer, squamous cell carcinoma, peritoneal cancer, liver cancer, hepatocellular cancer, colon cancer, rectal cancer, colon and rectal cancer, endometrial cancer, uterine cancer, salivary gland cancer, kidney cancer, vulvar cancer, thyroid cancer, penis cancer, and metastatic forms of them, however, there is no limitation thereto as long as cancer cells to be treated are expressing protein recognizable for the antibody in the antibody-drug conjugate.
The antibody-drug conjugate of the present invention can be preferably administered to mammals, and are more preferably administered to humans.
Substances used in a pharmaceutical composition containing the antibody-drug conjugate of the present invention may be suitably selected and applied from formulation additives or the like that are generally used in the field in view of the dose or concentration for administration.

The antibody-drug conjugate of the present invention may be administered as a pharmaceutical composition containing one or more pharmaceutically applicable components. For example, the pharmaceutical composition typically contains one or more pharmaceutical carriers (e.g., sterilized liquid (including water and oil (petroleum oil and oil of animal origin, plant origin, or synthetic origin (such as peanut oil, soybean oil, mineral oil, and sesame oil)))). Water is a more typical carrier when the pharmaceutical composition above is intravenously administered. Saline solution, an aqueous dextrose solution, and an aqueous glycerol solution can also be used as a liquid carrier, in particular, for an injection solution. Suitable pharmaceutical vehicles are known in the art. If desired, the composition above may also contain a trace amount of a moisturizing agent, an emulsifying agent, or a pH buffering agent. Examples of suitable pharmaceutical carriers are disclosed in "Remington's Pharmaceutical Sciences" by E. W. Martin. The formulations correspond to the administration mode.

Various delivery systems are known and they may be used to administer the antibody-drug conjugate of the present invention. Examples of the administration route may include, but are not limited to, intradermal, intramuscular, intraperitoneal, intravenous, and subcutaneous routes. The administration may be made by injection or bolus injection, for example. According to a specific preferred embodiment, the administration of the above ligand-drug conjugate form is done by injection. Parenteral administration is a preferred administration route.

According to a representative embodiment, the pharmaceutical composition is prescribed, as a pharmaceutical composition suitable for intravenous administration to humans, according to conventional procedures. The composition for intravenous administration is typically a solution in a sterile and isotonic aqueous buffer. If necessary, the medicine may contain a solubilizing agent and a local anesthetic to alleviate pain at an injection site (e.g., lignocaine). Generally, the ingredients above are provided either individually as a dried lyophilized powder or an anhydrous concentrate contained in each container which is obtained by sealing in an ampoule or a sachet with indication of the amount of the active agent, or as a mixture in a unit dosage form. When the pharmaceutical composition is to be administered by injection, it may be administered from an injection bottle containing water or saline of sterile pharmaceutical grade. When the medicine is administered by injection, an ampoule of sterile water or saline for injection may be provided so that the aforementioned ingredients are admixed with each other before administration.

The pharmaceutical composition of the present invention may be a pharmaceutical composition containing only the antibody-drug conjugate of the present invention, or a pharmaceutical composition containing the antibody-drug conjugate and at least one cancer treating agent other than the antibody-drug conjugate. The antibody-drug conjugate of the present invention may be administered in combination with other cancer treating agents, and thereby the anti-cancer effect may be enhanced. Other anti-cancer agents used for such purpose may be administered to an individual simultaneously with, separately from, or subsequently to the antibody-drug conjugate, and may be administered while varying the administration interval for each. Examples of such cancer treating agents may include abraxane, carboplatin, cisplatin, gemcitabine, irinotecan (CPT-11), paclitaxel, pemetrexed, sorafenib, vinblastin, agents described in International Publication No. WO 2003/038043, LH-RH analogues (e.g., leuprorelin, goserelin), estramustine phosphate, estrogen antagonists (e.g., tamoxifen, raloxifene), and aromatase inhibitors (e.g., anastrozole, letrozole, exemestane), but are not limited thereto as long as they are agents having an antitumor activity.

The pharmaceutical composition can be formulated into a lyophilization formulation or a liquid formulation as a formulation having the selected composition and required purity. When formulated as a lyophilization formulation, it may be a formulation containing suitable formulation additives that are used in the art. Also for a liquid formulation, it may be formulated as a liquid formulation containing various formulation additives that are used in the art.

The composition and concentration of the pharmaceutical composition may vary depending on the administration method. However, the antibody-drug conjugate contained in the pharmaceutical composition of the present invention can exhibit a pharmaceutical effect even at a small dosage when the antibody-drug conjugate has a higher affinity for an antigen, that is, a higher affinity (lower Kd value) in terms of the dissociation constant (Kd value) for the antigen. Thus, for determining the dosage of the antibody-drug conjugate, the dosage may be set in view of the situation relating to the affinity of the antibody-drug conjugate with the antigen. When the antibody-drug conjugate of the present invention is administered to a human, for example, about 0.001 to 100 mg/kg can be administered once or administered in several portions with intervals of 1 to 180 days.

The antibody of the present invention or a functional fragment of the antibody may be used as a medicine. In this case, the above description of "antibody-drug conjugate" in the above chapter <Medicine> may be appropriately read as a description of the "antibody or functional fragment of the antibody."

Further, the free drug of the present invention (novel PBD derivative compound), a salt of the free drug, and hydrates of them may be used as a medicine. In this case, the above description of "antibody-drug conjugate" in the above chapter <Medicine> may be appropriately read as a description of the "free drug (novel PBD derivative compound), a salt of the free drug, and hydrates of them."

Examples

Example 1: Trastuzumab A2 antibody and Trastuzumab variants Production of Trastuzumab A2 antibody

Herein, "Trastuzumab" is also called HERCEPTIN (R), huMAb4D5-8, or rhuMAb4D5-8, and is a humanized IgG1 antibody comprising a heavy chain consisting of an amino acid sequence consisting of amino acid residues 1 to 450 of SEQ ID NO: 33 and a light chain consisting of an amino acid sequence consisting of amino acid residues 1 to 214 of SEQ ID NO: 34 (US5821337).

Trastuzumab A2 antibody used herein is a constant region-modified IgG1 antibody of Trastuzumab obtained by mutating leucine (L) at the 237- and 238-positions with alanine (A) in the heavy chain amino acid sequence of Trastuzumab. The heavy chain amino acid sequence and light chain amino acid sequence of Trastuzumab are represented by SEQ ID NO: 33 and SEQ ID NO: 34, respectively. The heavy chain amino acid sequence and light chain amino acid sequence of Trastuzumab A2 antibody are represented by SEQ ID NO: 31 and SEQ ID NO: 32, respectively.

Design of Trastuzumab variants (Trastuzumab A1 (HwtLwt), H01L02 antibody, and HwtL05 antibody)

1-1: Design of Trastuzumab variants

1-1-1: Design of heavy chain of Trastuzumab A1

Designed was a heavy chain having the heavy chain variable region of Trastuzumab and being an isotype of IgG1 in which leucine at the 234- and 235-positions specified by EU Index numbering had been substituted with alanine (herein, referred to as "heavy chain of Trastuzumab A1" or "Hwt"). A nucleotide sequence encoding an amino acid sequence of SEQ ID NO: 11 is represented by SEQ ID NO: 12.

1-1-2: Preparation of variable region-modified variant of Trastuzumab

A heavy chain in which tyrosine at the 105-position specified by EU Index numbering in the Hwt amino acid sequence had been substituted with phenylalanine was named H01. The amino acid sequence of H01 is represented by SEQ ID NO: 15. A nucleotide sequence encoding the amino acid sequence of SEQ ID NO: 15 is represented by SEQ ID NO: 16.

A light chain in which tyrosine at the 92-position specified by EU Index numbering in the light chain amino acid sequence of Trastuzumab had been substituted with alanine was named L02. The amino acid sequence of L02 is represented by SEQ ID NO: 23. A nucleotide sequence encoding the amino acid sequence of SEQ ID NO: 23 is represented by SEQ ID NO: 24.

A light chain in which leucine at the 46-position and tyrosine at the 92-position specified by EU Index numbering in the light chain amino acid sequence of Trastuzumab had been substituted with alanine was named L05. The amino acid sequence of L05 is represented by SEQ ID NO: 27. A nucleotide sequence encoding the amino acid sequence of SEQ ID NO: 27 is represented by SEQ ID NO: 28.

1-1-3: Design of Trastuzumab variants with combination of heavy chain and light chain

An antibody consisting of Hwt and Lwt is referred to as "Trastuzumab A1", "HwtLwt antibody", or "HwtLwt". An antibody consisting of H01 and L02 is referred to as "H01L02 antibody" or "H01L02". An antibody consisting of Hwt and L05 is referred to as "HwtL05 antibody" or "HwtL05".

1-2: Production of Trastuzumab A1 (HwtLwt), H01L02 antibody, and HwtL05 antibody

1-2-1: Construction of light chain expression plasmid pCMA-LK

About 5.4 kb of a fragment obtained by digesting the plasmid pcDNA3.3-TOPO/LacZ (Invitrogen) with the restriction enzymes XbaI and PmeI was linked to a DNA fragment including a DNA sequence encoding the human light chain signal sequence and human κ chain constant region, as represented by SEQ ID NO: 9, by using an In-Fusion HD PCR Cloning Kit (Clontech) to prepare pcDNA3.3/LK. A neomycin expression unit was removed from the pcDNA3.3/LK to construct pCMA-LK.

1-2-2: Construction of IgG1LALA-type heavy chain expression plasmid pCMA-G1LALA

A DNA fragment obtained by digesting the pCMA-LK with XbaI and PmeI to remove the light chain signal sequence and human κ chain constant region was linked to a DNA fragment including a DNA sequence encoding the human heavy chain signal sequence and human IgG1LALA constant region, as represented by SEQ ID NO: 10, by using an In-Fusion HD PCR Cloning Kit (Clontech) to construct pCMA-G1LALA.

1-2-3: Construction of Trastuzumab A1 heavy chain expression plasmid

A DNA fragment consisting of nucleotide residues 36 to 434 of the nucleotide sequence encoding the heavy chain of Trastuzumab A1 (Hwt), as represented by SEQ ID NO: 12, was synthesized (GeneArt). The pCMA-G1LALA was cleaved with the restriction enzyme BlpI, and the synthesized DNA fragment was inserted into the cleaved portion by using an In-Fusion HD PCR Cloning Kit (Clontech) to construct an expression plasmid.

1-2-4: Construction of H01 expression plasmid

A DNA fragment consisting of nucleotide residues 36 to 434 of the nucleotide sequence encoding H01, as represented by SEQ ID NO: 16, was synthesized (GeneArt). An expression plasmid was constructed by using the same method as in Example 1-2-3.

1-2-5: Construction of Trastuzumab A1 light chain expression plasmid

A DNA fragment consisting of nucleotide residues 37 to 402 of the nucleotide sequence encoding the light chain of Trastuzumab A1 (Lwt), as represented by SEQ ID NO: 20, was synthesized (GeneArt). The pCMA-LK was cleaved with the restriction enzyme BsiWI, and the synthesized DNA fragment was inserted into the cleaved portion by using an In-Fusion HD PCR Cloning Kit (Clontech) to construct an expression plasmid.

1-2-6: Construction of L02 expression plasmid

A DNA fragment consisting of nucleotide residues 37 to 402 of the nucleotide sequence for L02, as represented by SEQ ID NO: 24, was synthesized (GeneArt). An expression plasmid was constructed by using the same method as in Example 1-2-5.

1-2-7: Construction of L05 expression plasmid

A DNA fragment consisting of nucleotide residues 37 to 402 of the nucleotide sequence for L05, as represented by SEQ ID NO: 28, was synthesized (GeneArt). An expression plasmid was constructed by using the same method as in Example 1-2-5.

1-3: Preparation of Trastuzumab A1, H01L02 antibody, and HwtL05 antibody

1-3-1: Production of Trastuzumab A1, H01L02 antibody, and HwtL05 antibody

FreeStyle 293F cells (Invitrogen) were passaged and cultured in accordance with the instruction manual. Into a 3 L Fernbach Erlenmeyer Flask (Corning Incorporated), $1.2 \times 10^9$ FreeStyle 293F cells (Invitrogen) in the logarithmic growth phase were seeded, and diluted with FreeStyle 293 expression medium (Invitrogen) to adjust to $2.0 \times 10^6$ cells/mL. To 40 mL of Opti-Pro SFM medium (Invitrogen), 0.24 mg of the heavy chain expression plasmid, 0.36 mg of the light chain expression plasmid, and 1.8 mg of polyethyleneimine (Polyscience, Inc., #24765) were added and gently stirred, and further left to stand for 5 minutes, and then added to the FreeStyle 293F cells. After shaking culture at 90 rpm in an incubator at 37°C and 8% $CO_2$ for 4 hours, 600 mL of EX-CELL VPRO medium (SAFC Biosciences, Inc.), 18 mL of GlutaMAX I (Gibco), and 30 mL of Yeastolate Ultrafiltrate (Gibco) were added, and the resultant was subjected to shaking culture at 90 rpm in an incubator at 37°C and 8% $CO_2$ for 7 days, and the resulting culture supernatant was filtered through a Disposable Capsule Filter (ADVANTEC, #CCS-045-E1H).

Trastuzumab A1, H01L02 antibody, and HwtL05 antibody were produced with the combinations of a heavy chain expression plasmid and a light chain expression plasmid corresponding to the combinations of a heavy chain and a light chain shown in Example 1-1-3.

1-3-2: Purification of Trastuzumab A1, H01L02 antibody, and HwtL05 antibody

The culture supernatant obtained in Example 1-3-1 was purified through rProtein A affinity chromatography in one step. The culture supernatant was applied to a column packed with MabSelectSuRe (produced by GE Healthcare Bioscience) equilibrated with PBS, and the column was then washed with PBS in an amount twice or more the column volume. Subsequently, elution was carried out with a 2 M solution of arginine hydrochloride (pH 4.0), and a fraction containing the antibody was collected. The fraction was subjected to buffer displacement to 50 mM phosphate buffer solution (pH 6.0) by dialysis (Thermo Scientific, Slide-A-Lyzer Dialysis Cassette). The antibody was concentrated with a Centrifugal UF Filter Device VIVASPIN20 (molecular weight cutoff: UF10K, Sartorius AG) to adjust the IgG concentration to 20 mg/mL or more. Finally, the fraction was filtered through a Minisart-Plus filter (Sartorius AG), and the resultant was used as a purified sample.

1-4: Measurement of activity of Trastuzumab variants

1-4-1: Evaluation of binding activity of Trastuzumab variants

A Biacore T200 (produced by GE Healthcare Bioscience) was used for measurement of the dissociation constant of Trastuzumab A1, the H01L02 antibody, or the HwtL05 antibody prepared in Example 1-3 and human HER2, wherein a capture method was performed in which an antibody was captured as a ligand to an AntiHuman IgG (Fc) antibody immobilized with a Human Antibody Capture Kit (produced by GE Healthcare Bioscience), and the antigen was measured as an analyte. HBS-EP+ (produced by GE Healthcare Bioscience) was used as a running buffer, and a CM5 (produced by GE Healthcare Bioscience) was used as a sensor chip. After adding 0.1 μg/mL or 0.2 μg/mL antibody onto the chip at 10 μL/min for 60 seconds, a dilution series of Recombinant human HER2/ErbB2 (ACRO Biosystems) solution (0.5 to 8 μg/mL) was added at a flow rate of 30 μL/min for 120 seconds, and the dissociated phase was subsequently monitored for 600 seconds for Trastuzumab A1, 300 seconds for H01L02, and 120 seconds for HwtL05. As a regeneration solution, 3 M magnesium chloride (produced by GE Healthcare Bioscience) was added at a flow rate of 20 μL/min for 30 seconds. A 1: 1 association model was used for data analysis, and association rate constants ka, dissociation rate constants kd, and dissociation constants (KD; KD = kd/ka) were calculated. Table 1 shows the results.

[Table 1]

| Dissociation constant of antibody and human HER2 | |
|---|---|
| name | KD (nM) |
| Trastuzumab A1 | 1.11 |
| H01L02 | 15. 4 |
| HwtL05 | 187 |

[Synthesis of production intermediate (Drug-linker)]

Example 2

[Example 2-1: Intermediate 1]

[Formula 61]

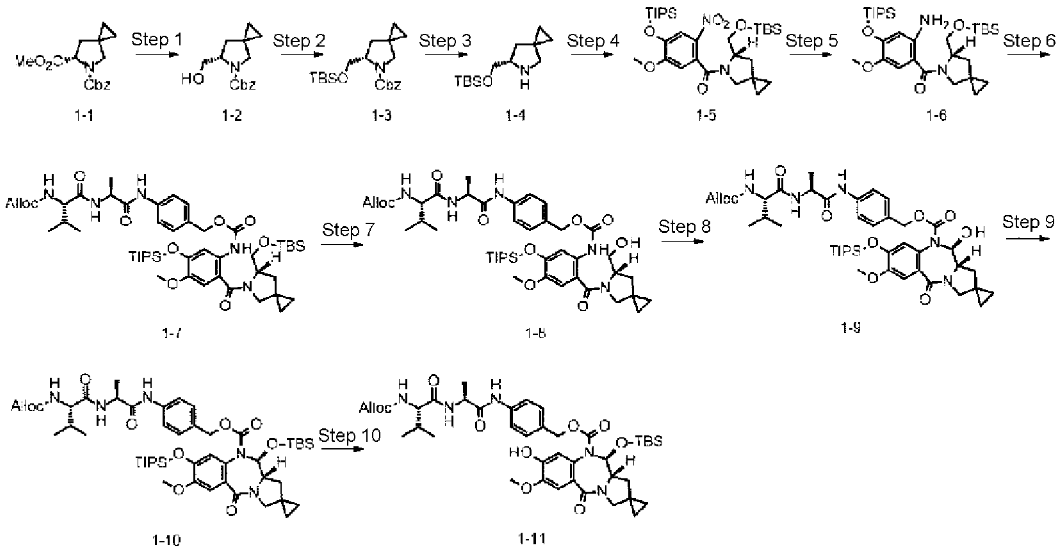


1-1 1-2 1-3 1-4 1-5 1-6

1-7 1-8 1-9

1-10 1-11

Step 1: Benzyl (6S)-6-(hydroxymethyl)-5-azaspiro[2.4]heptane-5-carboxylate (1-2)

To a solution of 5-benzyl 6-methyl (6S)-5-azaspiro[2.4]heptane-5,6-dicarboxylate (1-1) (104 mmol, WO 2012087596) in tetrahydrofuran (500 mL), lithium borohydride (4.30 g, 178 mmol) was added in small portions at 0°C. The resultant was stirred at 0°C for 30 minutes, and then stirred at room temperature for 2 hours. Water (180 mL) and 2 N hydrochloric acid (186 mL) were added at 0°C, and the resultant was distillated under reduced pressure. The resulting residue was extracted with ethyl acetate four times, and the organic layer was washed with brine and then dried over anhydrous sodium sulfate. The resultant was distillated under reduced pressure, and the resulting residue (1-2) (27.9 g, 90%) was directly used for the subsequent reaction.

Step 2: Benzyl (6S)-6-({[tert-butyl(dimethyl)silyl]oxy}methyl)-5-azaspiro[2.4]heptane-5-carboxylate (1-3)

To a solution of compound (1-2) obtained in Step 1 (27.9 g, 107 mmol) and imidazole (14.5 g, 214 mmol) in dichloromethane (300 mL), tert-butyldimethylsilyl chloride (24.2 g, 160 mmol) was added at room temperature, and the resultant was stirred at room temperature for 18 hours. The reaction solution was washed with a saturated aqueous citric acid, a saturated aqueous sodium hydrogen carbonate, and brine, dried over anhydrous sodium sulfate, and then distillated under reduced pressure. The resulting residue was purified by silica gel column chromatography [hexane:ethyl acetate = 100:0 (v/v) to 50:50 (v/v)] to afford the desired compound (1-3) (32.5 g, 81%).
MS(APCI)m/z:376(M+H)$^+$

Step 3: (6S)-6-({[tert-Butyl(dimethyl)silyl]oxy}methyl)-5-azaspiro[2.4]heptane (1-4)

To a solution of compound (1-3) obtained in Step 2 (32.5 g, 86.5 mmol) in ethanol (400 mL), 7.5% palladium carbon catalyst (moisture content: 54%, 5.00 g) was added at room temperature, and the resultant was stirred under the hydrogen atmosphere at room temperature for 6 hours. The reaction solution was filtered through a Celite, and the filtrate was distillated under reduced pressure to afford the desired compound (1-4) (21.3 g, quantitative).
MS(APCI, ESI)m/z:242(M+H)$^+$

Step 4: [(6S)-6-({[tert-Butyl(dimethyl)silyl]oxy}methyl)-5-azaspiro[2.4]hept-5-yl] (5-methoxy-2-nitro-4-{[tri(propan-2-yl)silyl]oxy}phenyl)methanone (1-5)

To a solution of 5-methoxy-2-nitro-4-{tri(propan-2-yl)silyl]oxy}benzoic acid (52.2 g, 141 mmol, US 20150283262) and 1-hydroxybenzotriazole monohydrate (23.8 g, 155 mmol) in dichloromethane (500 mL), N,N'-dicyclohexylcarbodiimide (35.0 g, 170 mmol) was added under ice-cooling. The reaction mixture was stirred at room temperature. After the carboxylic acid disappeared, a solution of compound (1-4) obtained in Step 3 (34.1 g, 141 mmol) and triethylamine (29.4 mL, 212 mmol) in dichloromethane (100 mL) was slowly added dropwise thereto at -60°C. After the reaction solution was stirred at room temperature overnight, saturated aqueous sodium hydrogen carbonate was added to the reaction mixture, and the reaction mixture was extracted with chloroform. The organic layer was washed with water and brine, and dried over anhydrous magnesium sulfate. The resultant was distillated under reduced pressure, and to the resulting

residue ethyl acetate and diethyl ether were added, and the solid contents were removed through filtration, and the filtrate was distillated under reduced pressure, and the resulting residue was purified by silica gel column chromatography [hexane:ethyl acetate = 100:0 (v/v) to 25:75 (v/v)] to afford the desired compound (1-5) (55.0 g, 66%).

MS(APCI, ESI)m/z:593$(M+H)^+$

Step 5: (2-Amino-5-methoxy-4-{[tri(propan-2-yl)silyl]oxy}phenyl)[(6S)-6-({[tert-butyl(dimethyl)silyl]oxy}methyl)-5-azaspiro[2.4]hept-5-yl]methanone (1-6)

To a solution of compound (1-5) obtained in Step 4 (55.0 g, 92.8 mmol) in ethanol (300 mL), 7.5% palladium carbon (10.0 g) was added under the nitrogen atmosphere. The nitrogen balloon was immediately replaced with a hydrogen balloon, and the reaction mixture was vigorously stirred under the hydrogen atmosphere at room temperature. After the raw materials disappeared, the reaction mixture was filtered, and the filtrate was distillated under reduced pressure to afford the desired compound (1-6) (52.2 g, 100%), which was directly used for the subsequent reaction.

MS(APCI, ESI)m/z:563$(M+H)^+$

Step 6: N-[(Prop-2-en-1-yloxy)carbonyl]-L-valyl-N-[4-({[(2-{[(6S)-6-({[tert-butyl(dimethyl)silyl]oxy}methyl)-5-azaspiro[2.4]hept-5-yl]carbonyl}-4-methoxy-5-{[tri(propan-2-yl)silyl]oxy}phenyl)carbamoyl]oxy}methyl)phenyl]-L-alaninamide (1-7)

To a solution of compound (1-6) obtained in Step 5 (18.6 g, 33.0 mmol) and triethylamine (6.26 mL, 45.2 mmol) in THF (300 mL), triphosgene (4.22 g, 14.2 mmol) was slowly added on an ethanol-ice bath. After the addition, a mixed solution of N-[(prop-2-en-1-yloxy)carbonyl]-L-valyl-N-[4-(hydroxymethyl)phenyl]-L-alaninamide (11.4 g, 30.2 mmol, WO 2011130598) and triethylamine (6.26 mL, 45.2 mmol) in tetrahydrofuran (100 mL) and N,N-dimethylformamide (30 mL) was slowly added dropwise to the ice-cooled reaction mixture. After the dropwise addition, the ice bath was removed, and the reaction mixture was stirred under the nitrogen atmosphere at 40°C. After the raw materials disappeared, water was added to the reaction mixture, and the reaction mixture was extracted with ethyl acetate. The organic layer was washed with brine, and dried over anhydrous sodium sulfate. After filtration followed by distillation under reduced pressure, the resulting residue was purified by silica gel column chromatography [hexane: ethyl acetate = 100:0 (v/v) to 40:60 (v/v)] to afford the desired compound (1-7) (23.5 g, 74%).

MS(APCI, ESI)m/z:966$(M+H)^+$

Step 7: N-[(Prop-2-en-1-yloxy)carbonyl]-L-valyl-N-[4-({[(2-{[(6S)-6-(hydroxymethyl)-5-azaspiro[2.4]hept-5-yl]carbonyl}-4-methoxy-5-{[tri(propan-2-yl)silyl]oxy}phenyl)carbamoyl]oxy}methyl)phenyl]-L-alaninamide (1-8)

To a solution of compound (1-7) obtained in Step 6 (23.5 g, 24.3 mmol in tetrahydrofuran (50 mL), methanol (50 mL) and water (44 mL), acetic acid (200 mL) was added at room temperature. The reaction mixture was stirred at room temperature. After the raw materials disappeared, the reaction mixture was extracted with ethyl acetate. The organic layer was washed with water and brine, and dried over anhydrous sodium sulfate. After filtration followed by distillation under reduced pressure, the resulting residue was purified by silica gel column chromatography [hexane:ethyl acetate = 100:0 (v/v) to 0:100 (v/v)] to afford the desired compound (1-8) (18.0 g, 87%).

MS(APCI, ESI)m/z:852$(M+H)^+$

Step 8: N-[(Prop-2-en-1-yloxy)carbonyl]-L-valyl-N-{4-[({[(11'S,11a'S)-11'-hydroxy-7'-methoxy-5'-oxo-8'-{[tri(propan-2-yl)silyl]oxy}-11',11a'-dihydro-1'H-spiro[cyclopropane-1,2'-pyrrolo[2,1-c][1,4]benzodiazepine]-10'(5'H)-yl]carbonyl}oxy)methyl]phenyl} -L-alaninamide (1-9)

To a solution of dimethyl sulfoxide (3.75 mL, 52.8 mmol) in dichloromethane (300 mL), oxalyl chloride (2.17 mL, 25.3 mmol) was slowly added dropwise under the nitrogen atmosphere at -78°C. After the dropwise addition, the reaction mixture was stirred at -78°C. A solution of compound (1-8) obtained in Step 7 (18.0 g, 21.1 mmol) in dichloromethane (50.0 mL) was slowly added to the reaction mixture. Triethylamine (14.6 mL, 105 mmol) was added to the reaction solution at -78°C. After the addition, the refrigerant bath was removed, and the temperature was slowly raised to room temperature. After the raw materials disappeared, water was added to the reaction mixture, and the reaction mixture was extracted with chloroform (200 mL). The organic layer was washed with water and brine, and dried over anhydrous magnesium sulfate. After filtration followed by distillation under reduced pressure, the resulting residue was purified by silica gel column chromatography [hexane:ethyl acetate = 100:0 (v/v) to 0:60 (v/v)] to afford the desired compound (1-9) (16.5 g, 92%).

MS(APCI, ESI)m/z:850$(M+H)^+$

Step 9: N-[(Prop-2-en-1-yloxy)carbonyl]-L-valyl-N-{4-[({[(11S,11a'S)-11'-{[tert-butyl(dimethyl)silyl]oxy}-7'-methoxy-5'-oxo-8'-{[tri(propan-2-yl)silyl]oxy}-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide(1-10)

To a solution of compound (1-9) obtained in Step 8 (12.0 g, 14.1 mmol) and 2,6-lutidine (6.58 mL, 56.5 mmol) in dichloromethane (200 mL), tert-butyldimethylsilyl trifluoromethylsulfonate (9.73 mL, 42.3 mmol) was slowly added dropwise under the nitrogen atmosphere at 0°C. After stirring under ice-cooling for 10 minutes, the ice bath was removed, and stirring was performed at room temperature. After the raw materials disappeared, water was added to the reaction mixture, and the reaction mixture was extracted with chloroform, washed with water and brine, and dried over anhydrous sodium sulfate. After filtration followed by distillation under reduced pressure, the resulting residue was purified by silica gel column chromatography [hexane:ethyl acetate = 100:0(v/v) to 25:75(v/v)] to afford the desired compound (1-10) (8.12 g, 60%).

MS(APCI, ESI)m/z:964$(M+H)^+$

Step 10: N-[(Prop-2-en-1-yloxy)carbonyl]-L-valyl-N-{4-[({[(11'S,11a'S)-11'-{[tert-butyl(dimethyl)silyl]oxy}-8'-hydroxy-7'-methoxy-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (1-11)

To a solution of compound (1-10) obtained in Step 9 (8.12 g, 8.42 mmol) in N,N-dimethylformamide (90 mL) and water (2 mL), lithium acetate (0.611 g, 9.26 mmol) was added, and the resultant was stirred at room temperature. After the raw materials disappeared, water was added to the reaction mixture, and the reaction mixture was extracted with ethyl acetate. The organic layer was washed with water and brine, and dried over anhydrous sodium sulfate. After filtration followed by distillation under reduced pressure, the resulting residue was purified by silica gel column chromatography [hexane:ethyl acetate = 100:0 (v/v) to 0:100 (v/v)] to afford the desired compound (1-11) (5.48 g, 81%).
$^1$H-NMR(400MHz, $CDCl_3$, 20.9°C)δ:8.76-8.60(1H,m),7.45-7.44(2H,m),7.21(1H,s),7.10-7.09(2H,m),6.81-6.74(1H,m),6.65(1H,s),6.23(1H,s),6.01-5.99(1H,m),5.95-5.84(1H,m),5.41-5.20(2H,m),5.16(1H,m),4.84(1H,m),4.67-4.54(4H,m),4.05-4.03(1H,m),3.87(3H,s),3.71(1H,m),3.55-3.51(1H,m),3.26(1H,m),2.35(1H,m),2.18-2.12(1H,m),1.55-1.42(4H,m),0.97-0.92(6H,m),0.81(9H,s),0.76-0.61(4H,m),0.20--0.06(6H,m).

MS(APCI, ESI)m/z:808$(M+H)^+$

$^1$H-NMR (500M Hz, $CDCl_3$, 27°C) δ: 8.76 (1H, s), 7.43 (2H, brd), 7.20 (1H, s), 7.08 (2H, d, J=8.3 Hz), 7.00 (1H, br), 6.66 (1H, s), 6.44 (1H, s), 6.00 (1H, H-11', d, J11', 11'a=9.2 Hz), 5.89 (1H, m), 5.53 (1H, brd), 5.30 (1H, d, J=17.2 Hz), 5.20 (1H, d, J=10.3 Hz), 5.15 (1H, d, JABq=12.5 Hz), 4.85 (1H, d, JABq=12.5 Hz), 4.66 (1H, m), 4.60-4.52 (2H, m), 4.07 (1H, m), 3.84 (3H, s), 3.71 (1H, H-3'β, d, Jgem=11.7 Hz), 3.53 (1H, H-11'a, m), 3.26 (1H, H-3'α, d, Jgem=11.7 Hz), 2.35 (1H, H-1'β, dd, Jl' β, 11'a=8.30 Hz, Jgem=13.1 Hz), 2.14 (1H, m), 1.54 (1H, H-1' α, d, Jgem=13.1 Hz), 1.41 (3H, d, J=6.90 Hz), 0.95 (3H, d, J=6.80 Hz), 0.92 (3H, d, J=6.80 Hz), 0.81 (9H, s), 0.80-0.70 (1H, m), 0.70-0.59 (3H, m), 0.2-0.06 (6H, m)

The absolute steric configuration at the 11'-position of compound (1-11) was analyzed by correlation obtained from its selective 1D ROESY spectrum (a figure below). Correlation was found between 1'α-H and 11'-H, between 3'α-H and 11'-H, and between 1'β-H and 3'β-H, and thus the absolute steric configuration at the 11'-position was revealed to be S-configuration.

[Formula 62]

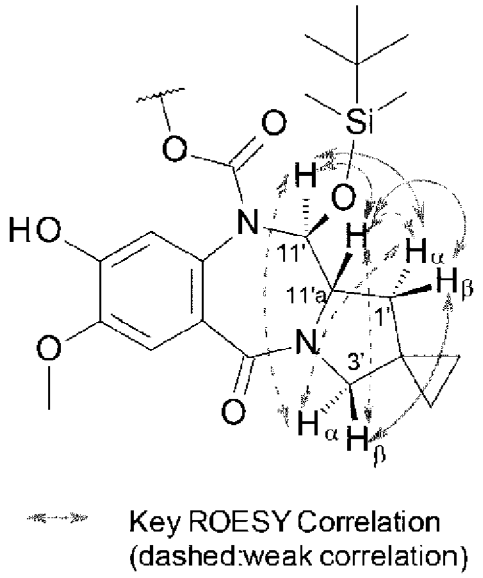


Significant correlation obtained from Selective 1D ROESY spectrum

Accordingly, the absolute steric configuration at the 11'-position of each of compound (1-11), compound (1-9) and compound (1-10), each of which had the same absolute steric configuration as compound (1-11), compound (3-11), which was synthesized with compound (1-11), compound (3-12), compound (3-13), and drug-linker 1 (compound (3-14)), compound (4-9), compound (4-10), compound (4-11), and drug-linker 2 (compound (4-12)), and compound (6-10), compound (6-11), compound (6-12), and drug-linker 4 (compound (6-13)) was revealed to be S-configuration. Further, the absolute steric configuration at the 11'-position of each of compound (5-9), compound (5-10), and drug-linker 3 (compound (5-11)), which were obtained by the same synthesis procedure, was determined to be S-configuration.

[Example 2-2: Intermediate 2]

[Formula 63]

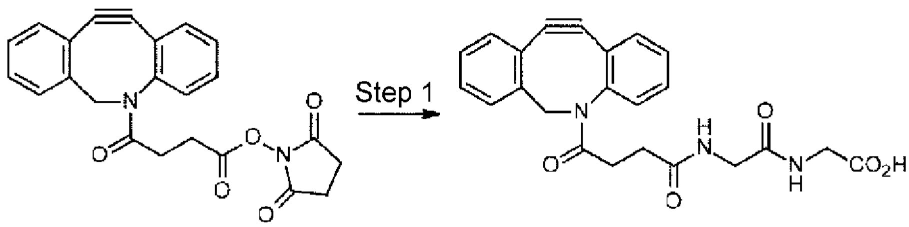


2-1 2-2

Step 1: N-[4-(11,12-Didehydrodibenzo[b,f]azocin-5(6H)-yl))-4-oxobutanoyl]glycylglycine (2-2)

To a solution of glycylglycine (0.328 g, 2.49 mmol) and N,N-diisopropylethylamine (0.433 mL, 2.49 mmol) in N,N-dimethylformamide (20 mL), 1-{[4-(11,12-didehydrodibenzo[b,f]azocin-5(6H)-yl))-4-oxobutanoyl]oxy}pyrrolidine-2,5-dione (2-1) (1.00 g, 2.49 mmol, Click Chemistry Tools) and water (10 mL) were added at room temperature, and the resultant was stirred at the same temperature overnight. The resultant was distillated under reduced pressure, and the resulting residue was purified by silica gel column chromatography [an organic layer for distribution with chloroform to chloroform:methanol:water = 7:3:1(v/v/v)] to afford the desired compound (0.930 g, 89%).

$^1$H-NMR(DMSO-$D_6$)δ:12.58(1H,s),8.14-8.12(1H,m),8.08-8.07(1H,m), 7.69-7.68(1H,m),7.62-7.61(1H,m),7.53-7.45(3H,m),7.40-7.29(3H,m),5.05-5.01(1H,m),3.73-3.72(2H,m),3.66-3.60(3H, m),2.66-2.60(1H,m),2.33-2.24(1H,m),2.08-2.04(1H,m),1.81-1.77(1H,m).

MS(APCI, ESI)m/z:420[(M+H)$^+$],

[Example 2-3: Drug-linker 1]

[Formula 64]

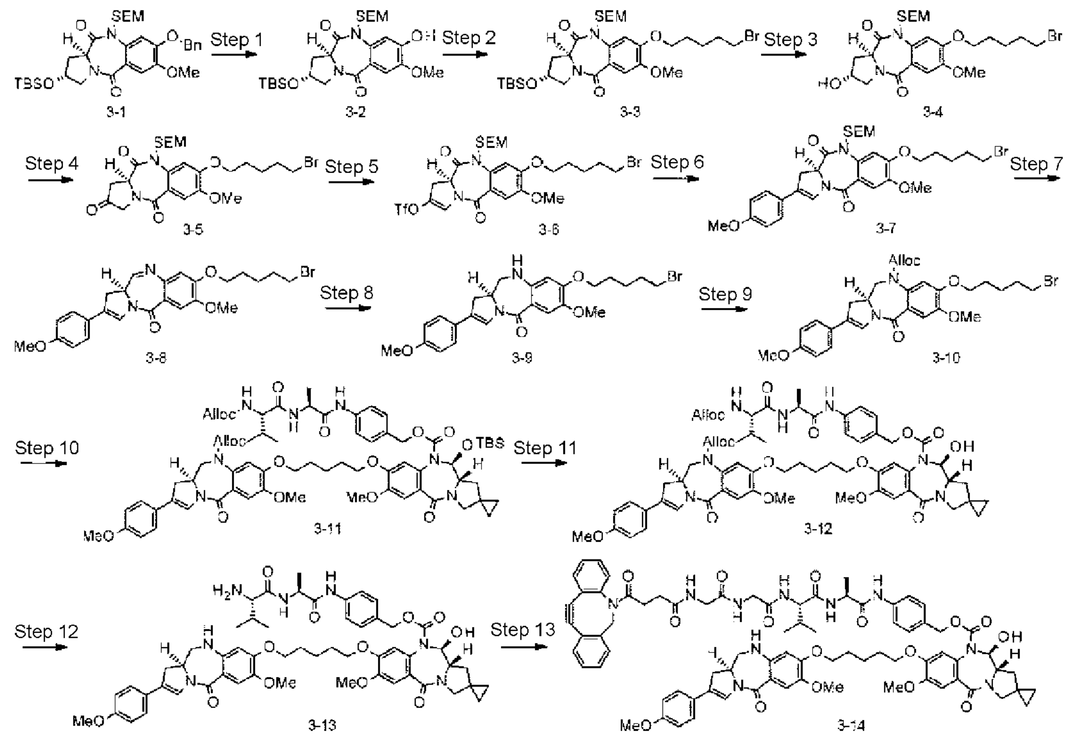


Step 1: (2R,11aS)-2-{[tert-Butyl(dimethyl)silyl]oxy}-8-hydroxy-7-methoxy-10-{[2-(trimethylsilyl)ethoxy]methyl}-2,3-dihydro-1H-pyrrolo[2,1-c][1,4]benzodiazepin-5,11(10H,11aH)-dione(3-2)

To a solution of (2R,11aS)-8-(benzyloxy)-2-{[tert-butyl(dimethyl)silyl]oxy}-7-methoxy-10-{[2-(trimethylsilyl)ethoxy]methyl}-2,3-dihydro-1H-pyrrolo[2,1-c][1,4]benzodiazepin-5,11(10H,11aH)-dione (3-1) (25.5 g, 41.6 mmol, WO 2016149546) in tetrahydrofuran (150 mL) and ethanol (150 mL), 5% palladium carbon (moisture content: 54%, 10.0 g) was added under the nitrogen atmosphere, and the reaction solution was then stirred under the hydrogen atmosphere at room temperature for 3 days. Chloroform was added to the reaction solution, which was filtered through a Celite, and the filtrate was then distillated under reduced pressure. The resulting residue was purified by silica gel column chromatography [hexane:ethyl acetate = 100:0 (v/v) to 50:50 (v/v)] to afford the desired compound (3-2) (19.4 g, 89%).

MS(APCI, ESI)m/z:523(M+H)$^+$

Step 2: (2R,11aS)-8-[(5-Bromopentyl)oxy]-2-{[tert-butyl(dimethyl)silyl]oxy}-7-methoxy-10-{[2-(trimethylsilyl)ethoxy]methyl}-2,3-dihydro-1H-pyrrolo[2,1-c][1,4]benzodiazepin-5,11(10H,11aH)-dione (3-3)

To a solution of compound (3-2) obtained in Step 1 (10.8 g, 20.7 mmol) in N,N-dimethylformamide (30 mL), 1,5-dibromopentane (23.8 g, 103 mmol) and potassium carbonate (3.43 g, 24.8 mmol) were added at room temperature. After stirring at room temperature for 3 hours, water was added to the reaction solution, which was extracted with ethyl acetate. The organic layer obtained was washed with brine and dried over sodium sulfate, and distillated under reduced pressure. The resulting residue was purified by silica gel column chromatography [hexane:ethyl acetate = 90:10 (v/v)

to 50:50 (v/v)] to afford the desired compound (3-3) (14.5 g, quantitative).

MS(APCI, ESI)m/z:673[81Br,$(M+H)^+$],671[79Br,$(M+H)^+$],

Step 3: (2R,11aS)-8-[(5-Bromopentyl)oxy]-2-hydroxy-7-methoxy-10-{[2-(trimethylsilyl)ethoxy]methyl}-2,3-dihydro-1H-pyrrolo[2,1-c][1,4]benzodiazepin-5,11(10H,11aH)-dione(3-4)

To a solution of compound (3-3) obtained in Step 2 (21.5 mmol) in tetrahydrofuran (40 mL), a 1 mol/L tetrahydrofuran solution of tetrabutylammonium fluoride (28.0 mL, 28.0 mmol) was added at 0°C. After stirring at room temperature for 30 minutes, water was added to the reaction solution, which was extracted with ethyl acetate, and the organic layer obtained was washed with brine. The resultant was dried over sodium sulfate, and then distillated under reduced pressure. The resulting residue was purified by silica gel column chromatography [chloroform:methanol = 97.5:2.5 (v/v) to 92.5:7.5 (v/v)] to afford the desired compound (3-4) (11.3 g, 94%).

MS(APCI, ESI)m/z:559[81Br,$(M+H)^+$],557[79Br,$(M+H)^+$],

Step 4: (11aS)-8-[(5-Bromopentyl)oxy]-7-methoxy-10-{[2-(trimethylsilyl)ethoxy]methyl}-1H-pyrrolo[2,1-c][1,4]benzodiazepin-2,5,11 (3H, 10H, 11aH)-trione (3-5)

Compound (3-4) obtained in Step 3 (11.3 g, 20.2 mmol), tetrabutylammonium bromide (0.325 g, 1.01 mmol), and potassium bromide (0.240 g, 2.02 mmol) were dissolved in a saturated aqueous sodium hydrogen carbonate (60 mL)/dichloromethane (60 mL), to which nor-AZADO (0.0279 g, 0.202 mmol) and sodium hypochlorite pentahydrate (2.03 g, 27.2 mmol) were added at 0°C, and the resultant was stirred at 0°C for 30 minutes. Because the raw materials remained, sodium hypochlorite pentahydrate (1.00 g, 13.4 mmol) was added thereto at 0°C, and the resultant was stirred at 0°C for 15 minutes. Sodium hypochlorite pentahydrate (0.300 g, 4.03 mmol) was further added thereto at 0°C, and the resultant was stirred at 0°C for 15 minutes, and the disappearance of the raw materials was confirmed by TLC. An aqueous solution of sodium thiosulfate was added to the reaction solution, which was extracted with chloroform, and the organic layer obtained was dried over sodium sulfate. The resultant was distillated under reduced pressure, and the resulting residue was purified by silica gel column chromatography [hexane:ethyl acetate = 75:25(v/v) to 40:60(v/v)] to afford the desired compound (3-5) (9.74 g, 87%).

MS(APCI, ESI)m/z:557[81Br,$(M+H)^+$],555[79Br,$(M+H)^+$].

Step 5: (11aS)-8-[(5-Bromopentyl)oxy]-7-methoxy-5,11-dioxo-10-{[2-(trimethylsilyl)ethoxy]methyl}-5,10,11,11a-tetrahydro-1H-pyrrolo[2,1-c][1,4]benzodiazepin-2-yl trifluoromethanesulfonate (3-6)

To a solution of compound (3-5) obtained in Step 4 (9.74 g, 17.5 mmol) in dichloromethane (160 mL), 2,6-lutidine (8.17 mL, 70.1 mmol) was added at -40°C, and the resultant was stirred at -40°C for 10 minutes. Anhydrous trifluoromethanesulfonic acid (8.85 mL, 52.6 mmol) was added to the reaction solution at -40°C, and the resultant was stirred at -40°C for 30 minutes. To the reaction solution, a 10% aqueous solution of citric acid was added, which was extracted with chloroform, and the organic layer obtained was dried over sodium sulfate. The resultant was distillated under reduced pressure, and the resulting residue was purified by silica gel column chromatography [hexane:ethyl acetate = 95:5 (v/v) to 70:35 (v/v)] and then purified by NH2 silica gel chromatography [hexane:ethyl acetate = 95:5 (v/v) to 65:35 (v/v)] to afford the desired compound (3-6) (7.10 g, 59%).

MS(APCI, ESI)m/z:689[81Br,$(M+H)^+$],687[79Br,$(M+H)^+$],

Step 6: (11aS)-8-[(5-Bromopentyl)oxy]-7-methoxy-2-(4-methoxyphenyl)-10-{[2-(trimethylsilyl)ethoxy]methyl}-1H-pyrrolo[2,1-c][1,4]benzodiazepin-5,11(10H,11aH)-dione (3-7)

To a mixture of compound (3-6) obtained in Step 5 (2.00 g, 2.91 mmol), 4-methoxyphenylboronic acid (0.884 g, 5.82 mmol), tetrakis(triphenylphosphine)palladium (0) (0.336 g, 0.291 mmol) and sodium carbonate (1.23 g, 11.6 mmol), toluene (20 mL), ethanol (10 mL) and water (10 mL) were added at room temperature. The reaction solution was stirred at room temperature for 30 minutes, and the reaction solution was then extracted with ethyl acetate, and the extract was washed with water and brine. The organic layer was dried over sodium sulfate, and then distillated under reduced pressure. The resulting residue was purified by silica gel column chromatography [hexane:ethyl acetate = 90:10 (v/v) to 50:50 (v/v)] to afford the desired compound (3-7) (1.71 g, 91%).

MS(APCI, ESI)m/z:647[81Br,$(M+H)^+$],645[79Br,$(M+H)^+$],

Step 7:(11aS)-8-[(5-Bromopentyl)oxy]-7-methoxy-2-(4-methoxyphenyl)-1,11a-dihydro-5H-pyrrolo[2,1-c][1,4]benzodiazepin-5-one (3-8)

Compound (3-7) obtained in Step 6 (0.789 g, 1.22 mmol) was dissolved in ethanol (10 mL) and tetrahydrofuran (10 mL), and 2.0 M tetrahydrofuran solution of lithium borohydride (6.11 mL, 12.2 mmol) was added thereto at 0°C, and the resultant was stirred at 0°C for 3 hours. Water was added to the reaction solution, which was extracted with chloroform, and the organic layer obtained was dried over sodium sulfate. The resultant was distillated under reduced pressure, and the resulting residue was dissolved in dichloromethane (10 mL), ethanol (20 mL) and water (10 mL), to which silica gel (4 g) was added at room temperature, and the resultant was stirred at room temperature for 4 days. The silica gel was removed through filtration, and water was added thereto, and the resultant was extracted with chloroform. The organic layer obtained was dried over sodium sulfate. The resultant was distillated under reduced pressure, and the resulting residue was purified by silica gel column chromatography [hexane:ethyl acetate = 60:40 (v/v) to 25:75 (v/v)] to afford the desired compound (3-8) (0.496 g, 81%).

MS(APCI, ESI)m/z:501[81Br,$(M+H)^+$],499[79Br,$(M+H)^+$].

Step 8: (11aS)-8-[(5-Bromopentyl)oxy]-7-methoxy-2-(4-methoxyphenyl)-1,10,11,11a-tetrahydro-5H-pyrrolo[2,1-c][1,4]benzodiazepin-5-one (3-9)

To a solution of compound (3-8) obtained in Step 7 (0.496 g, 0.992 mmol) in dichloromethane (20 mL), sodium triacetoxyborohydride (0.421 g, 1.99 mmol) was added at 0°C. After stirring at room temperature for 2 hours, a saturated aqueous sodium hydrogen carbonate was added thereto, and the resultant was extracted with chloroform. The organic layer was dried over sodium sulfate, and distillated under reduced pressure, and the resulting residue was then purified by silica gel column chromatography [hexane:ethyl acetate = 60:40 (v/v) to 25:75 (v/v)] to afford the desired compound (3-9) (0.426 g, 86%).

MS(APCI, ESI)m/z:503[81Br,$(M+H)^+$],501[79Br,$(M+H)^+$].

Step 9: Prop-2-en-1-y1 (11aS)-8-[(5-bromopentyl)oxy]-7-methoxy-2-(4-methoxyphenyl)-5-oxo-11,11a-dihydro-1H-pyrrolo[2,1-c][1,4]benzodiazepin-10(5H)-carboxylate (3-10)

To a solution of compound (3-9) obtained in Step 8 (0.426 g, 0.849 mmol) in dichloromethane (30 mL), pyridine (0.102 mL 1.27 mmol) and allyl chloroformate (0.374 mL, 3.54 mmol) were added at 0°C, and the resultant was stirred at 0°C for 15 minutes. To the reaction solution, a 10% aqueous solution of citric acid was added, which was extracted with chloroform, and the organic layer obtained was washed with a saturated aqueous sodium hydrogen carbonate, and then dried over sodium sulfate. The resultant was distillated under reduced pressure, and the resulting residue was purified by silica gel column chromatography [hexane:ethyl acetate = 90:10 (v/v) to 50:50 (v/v)] to afford the desired compound (3-10) (0.465 g, 94%).

MS(APCI, ESI)m/z:587[81Br,$(M+H)^+$],585[79Br,$(M+H)^+$].

Step 10: N-[(Prop-2-en-1-yloxy)carbonyl]-L-valyl-N-{4-[({[(111'S,11a'S)-11'-{[tert-butyl(dimethyl)silyl]oxy}-7'-methoxy-8'-{[5-({(11aS)-7-methoxy-2-(4-methoxyphenyl)-5-oxo-10-[(prop-2-en-1-yloxy)carbonyl]-5,10,11,11a-tetrahydro-1H-pyrrolo[2,1-c][1,4]benzodiazepin-8-yl}oxy)pentyl]oxy}-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (3-11)

To a solution of compound (1-11) obtained in Step 10 of Example 2-1 (0.130 g, 0.161 mmol) and compound (3-10) obtained in Step 9 (0.104 g, 0.177 mmol) in N,N-dimethylformamide (3 mL), potassium carbonate (0.0266 g, 0.193 mmol) was added at room temperature, and the resultant was stirred at room temperature overnight. The reaction solution was diluted with ethyl acetate, and washed with water and brine, and then dried over sodium sulfate. The resultant was distillated under reduced pressure, and the resulting residue was then purified by NH2-silica gel column chromatography [hexane:ethyl acetate = 70:30 (v/v) to 0:100 (v/v)] to afford the desired compound (3-11) (0.184 g, 87%).

MS(APCI, ESI)m/z: 1312$(M+H)^+$

Step 11: N-[(Prop-2-en-1-yloxy)carbonyl]-L-valyl-N-{4-[({[(11'S,11a'S)-11'-hydroxy-7'-methoxy-8'-{[5-({(11aS)-7-methoxy-2-(4-methoxyphenyl)-5-oxo-10-[(prop-2-en-1-yloxy)carbonyl]-5,10,11,11a-tetrahydro-1H-pyrrolo[2,1-c][1,4]benzodiazepin-8-yl}oxy)pentyl]oxy}-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide(3-12)

To a solution of compound (3-11) obtained in Step 10 (0.1837 g, 0.140 mmol) and acetic acid (0.048 mL, 0.840 mmol) in tetrahydrofuran (5.00 mL), a 1 mol/L tetrahydrofuran solution of tetrabutylammonium fluoride (0.700 mL, 0.700

mmol) was added at room temperature, and the resultant was stirred at room temperature for 3 hours. The reaction solution was diluted with ethyl acetate, and the organic layer was washed with a saturated aqueous sodium hydrogen carbonate and brine, and then dried over sodium sulfate. The resultant was distillated under reduced pressure, and the resulting residue was purified by silica gel chromatography [chloroform:methanol = 99.5:0.5(v/v) to 95:5(v/v)] to afford the desired compound (3-12) (0.178 g, quantitative).

MS(APCI, ESI)m/z: 1198$(M+H)^+$

Step 12: L-Valyl-N-{4-[({[(11'S,11a'S)-11'-hydroxy-7'methoxy-8'[(5-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}pentyl)oxy]-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (3-13)

To a solution of compound (3-12) obtained in Step 11 (0.140 mmol) in dichloromethane (2 mL), pyrrolidine (0.0579 mL, 0.700 mmol) and tetrakis(triphenylphosphine)palladium (0) (0.0162 g, 0.0140 mmol) were added at room temperature, and the resultant was stirred at room temperature for 15 minutes. After distillation under reduced pressure, the resulting residue was purified by silica gel chromatography [chloroform:methanol = 99.5:0.5(v/v) to 92.5:7.5(v/v)] to afford the desired compound (3-13) (0.143 g, 99%).

MS(APCI, ESI)m/z: 1030$(M+H)^+$

Step 13: N-[4-(11,12-Didehydrodibenzo[b,f]azocin-5 (6H)-yl)-4-oxobutanoyl]glycylglycyl-L-valyl-N-{4-[({[(11'S,11a'S)-11'-hydroxy-7'-methoxy-8'-[(5-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}pentyl)oxy]-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (3-14)

To a mixture of compound (2-2) obtained in Step 1 of Example 2-2 (0.0640 g, 0.153 mmol) and N-ethoxycarbonyl-2-ethoxy-1,2-dihydroquinoline (0.0446 g, 0.180 mmol), dichloromethane (2 mL) was added at room temperature, and the resultant was stirred at room temperature for 15 minutes. To the reaction solution, a solution of compound (3-13) obtained in Step 12 (0.143 g, 0.139 mmol) in dichloromethane (2 mL) was added, and the resultant was stirred at room temperature for 5 hours, and then distillated under reduced pressure. The resulting residue was purified by silica gel column chromatography chloroform:methanol = 99.5:0.5 (v/v) to 92.5:7.5 (v/v)] to afford the desired compound (3-14) (0.103 g, 52%).

1H-NMR (DMSO-D6) δ: 9.93 (1H, s), 8.21-8.16 (2H, m), 8.07-8.04 (1H, m), 7.83-7.64 (2H, m), 7.60-7.55 (3H, m), 7.51-7.28 (10H, m), 7.19-7.16 (2H, m), 7.10-7.04 (1H, m), 6.92-6.90 (2H, m), 6.76-6.70 (1H, m), 6.39 (1H, s), 5.77-5.75 (1H, m), 5.21-5.18 (1H, m), 5.03-4.99 (1H, m), 4.82-4.79 (1H, m), 4.37-4.35 (1H, m), 4.21-4.20 (2H, m), 4.02-3.24 (26H, m), 3.16-3.13 (1H, m), 2.79-2.59 (2H, m), 2.39-2.28 (2H, m), 2.05-1.97 (2H, m), 1.91-1.77 (4H, m), 1.57-1.54 (3H, m), 1.28-1.23 (3H, m), 0.85-0.80 (6H, m), 0.67-0.61 (4H, m).

MS(APCI, ESI)m/z:1431$(M+H)^+$

[Example 2-4: Drug-linker 2]

[Formula 65]

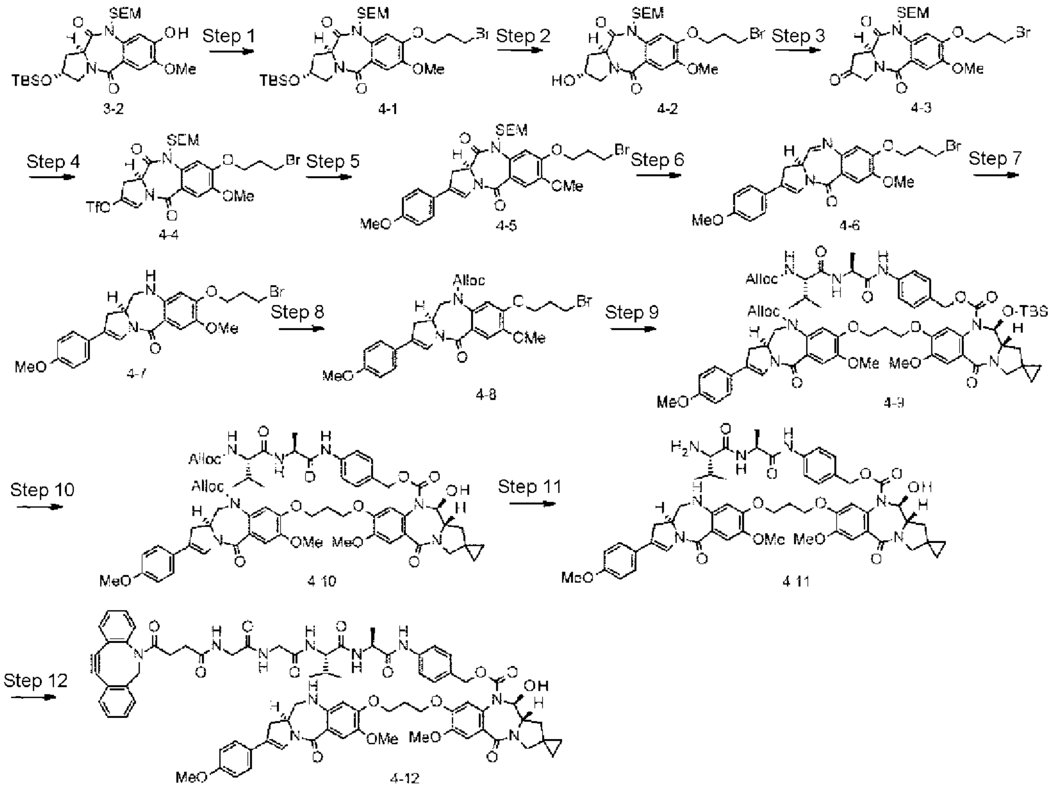


Step 1: (2R,11aS)-8-(3-Bromopropoxy)-2-{[tert-butyl(dimethyl)silyl]oxy}-7-methoxy-10-{[2-(trimethylsilyl)ethoxy]methyl}-2,3-dihydro-1H-pyrrolo[2,1-c][1,4]benzodiazepin-5,11(10H,11aH)-dione (4-1)

Compound (3-1) obtained in Step 1 of Example 2-3 (5.06 g, 9.67 mmol) and 1,3-dibromopropane (4.93 mL, 48.4 mmol) were reacted in the same manner as in Step 2 of Example 2-3 to afford the desired compound (4-1) (4.85 g, 78%).
MS(APCI, ESI)m/z:645[81Br,$(M+H)^+$],643[79Br,$(M+H)^+$].

Step 2: (2R,11aS)-8-(3-Bromopropoxy)-2-hydroxy-7-methoxy-10-{[2-(trimethylsilyl)ethoxy]methyl}-2,3-dihydro-1H-pyrrolo[2,1-c][1,4]benzodiazepin-5,11(10H,11aH)-dione (4-2)

Compound (4-1) obtained in Step 1 (4.85 g, 7.54 mmol) was reacted in the same manner as in Step 3 of Example 2-3 to afford the desired compound (4-2) (4.05 g, quantitative).
MS(APCI, ESI)m/z:531[81Br,$(M+H)^+$],529[79Br,$(M+H)^+$].

Step 3: (11aS)-8-(3-Bromopropoxy)-7-methoxy-10-{[2-(trimethylsilyl)ethoxy]methyl}-1H-pyrrolo[2,1-c][1,4]benzodiazepin-2,5,11 (3H,10H,11aH)-trione (4-3)

Compound (4-2) obtained in Step 2 (7.54 mmol) was reacted in the same manner as in Step 4 of Example 2-3 to afford the desired compound (4-3) (3.73 g, 93%). $^1$H-NMR($CDCl_3$)δ:7.34(1H,s),7.29(1H,s),5.56-5.53(1H,m),4.72-4.69(1H,m),4.67-4.61(1H,m),4.23-4.17(3H,m),3.97-3.88(4H,m),3.82-3.75(1H,m),3.74-3.56(4H,m),2.82-2.77(1H,m),2.43-2.38(2H,m),1.06-0.94(2H,m),0.08-0.00(9H,m).

Step 4: (11aS)-8-(3-Bromopropoxy)-7-methoxy-5,11-dioxo-10-{[2-(trimethylsilyl)ethoxy]methyl}-5,10,11,11a-tetrahydro-1H-pyrrolo[2,1-c][1,4]benzodiazepin-2-yl trifluoromethanesulfonate (4-4)

Compound (4-3) obtained in Step 3 (3.73 g, 7.08 mmol) was reacted in the same manner as in Step 5 of Example 2-3 to afford the desired compound (4-4) (3.27 g, 70%).
MS(APCI, ESI)m/z:661[81Br,$(M+H)^+$],659[79Br,$(M+H)^+$].

Step 5: (11aS)-8-(3-Bromopropoxy)-7-methoxy-2-(4-methoxyphenyl-10-{[2-(trimethylsilyl)ethoxy]methyl}-1H-pyrrolo[2,1-c][1,4]benzodiazepin-5,11(10H,11aH)-dione (4-5)

Compound (4-4) obtained in Step 4 (3.27 g, 4.96 mmol) was reacted in the same manner as in Step 6 of Example 2-3 to afford the desired compound (4-5) (2.49 g, 81%).

MS(APCI, ESI)m/z:619[81Br,$(M+H)^+$],617[79Br,$(M+H)^+$].

Step 6: (11aS)-8-(3-Bromopropoxy)-7-methoxy-2-(4-methoxyphenyl)-1,11a-dihydro-5H-pyrrolo[2,1-c][1,4]benzodiazepin-5-one (4-6)

Compound (4-5) obtained in Step 5 (2.49 g, 4.04 mmol) was reacted in the same manner as in Step 7 of Example 2-3 to afford the desired compound (4-6) (1.59 g, 84%).

MS(APCI, ESI)m/z:473[81Br,$(M+H)^+$],471[79Br,$(M+H)^+$].

Step 7: (11aS)-8-(3-Bromopropoxy)-7-methoxy-2-(4-methoxyphenyl)-1,10,11,11a-tetrahydro-5H-pyrrolo[2,1-c][1,4]benzodiazepin-5-one (4-7)

Compound (4-6) obtained in Step 6 (1.59 g, 3.38 mmol) was reacted in the same manner as in Step 8 of Example 2-3 to afford the desired compound (4-7) (1.39 g, 87%).

MS(APCI, ESI)m/z:475[81Br,$(M+H)^+$],473[79Br,$(M+H)^+$].

Step 8: Prop-2-en-1-yl (11aS)-8-(3-bromopropoxy)-7-methoxy-2-(4-methoxyphenyl)-5-oxo-11,11a-dihydro-1H-pyrrolo[2,1-c][1,4]benzodiazepin-10(5H)-carboxylate (4-8)

Compound (4-7) obtained in Step 7 (1.40 g, 2.95 mmol) was reacted in the same manner as in Step 9 of Example 2-3 to afford the desired compound (4-8) (0.885 g, 54%).

MS(APCI, ESI)m/z:559[81Br,$(M+H)^+$],557[79Br,$(M+H)^+$].

Step 9: N-{[(Prop-2-en-1-yl)oxy]carbonyl}-L-valyl-N-[4-({[(11'S,11'aS)-11'-{[tert-butyl(dimethyl)silyl]oxy}-7'-methoxy-8'-(3-{[(11aS)-7-methoxy-2-(4-methoxyphenyl)-5-oxo-10-{[(prop-2-en-1-yl)oxy]carbonyl}-5,10,11,11a-tetrahydro-1H-pyrrolo[2,1-c][1,4]benzodiazepin-8-yl]oxy}propoxy)-5'-oxo-11',11'a-dihydro-1'H,3'H-spiro[cyclopropane-1,2'-pyrrolo[2,1-c][1,4]benzodiazepine]-10'(5'H)-carbonyl]oxy}methyl)phenyl]-L-alaninamide (4-9)

Compound (4-8) obtained in Step 8 (0.0381 g, 0.0683 mmol) and compound (1-11) obtained in Step 10 of Example 2-1 (0.0552 g, 0.0683 mmol) were reacted in the same manner as in Step 10 of Example 2-3 to afford the desired compound (4-9) (0.0712 g, 81%).

MS(APCI, ESI)m/z: 1284$(M+H)^+$.

Step 10: N-{[(Prop-2-en-1-yl)oxy]carbonyl}-L-valyl-N-[4-({[(11'S,11'aS)-11'-hydroxy-7'-methoxy-8'-(3-{[(11aS)-7-methoxy-2-(4-methoxyphenyl)-5-oxo-10-{[(prop-2-en-1-yl)oxy]carbonyl}-5,10,11,11a-tetrahydro-1H-pyrrolo[2,1-c][1,4]benzodiazepin-8-yl]oxy}propoxy)-5'-oxo-11',11'a-dihydro-1'H,3'H-spiro[cyclopropane-1,2'-pyrrolo[2,1-c][1,4]benzodiazepine]-10'(5'H)-carbonyl]oxy}methyl)phenyl]-L-alaninamide (4-10)

Compound (4-9) obtained in Step 9 (0.0712 g, 0.0554 mmol) was reacted in the same manner as in Step 11 of Example 2-3 to afford the desired compound (4-10) (0.0671 g, quantitative).

MS(APCI, ESI)m/z: 1170$(M+H)^+$.

Step 11: L-Valyl-N-[4-({[(11'S,11'aS)-11'-hydroxy-7'-methoxy-8'-(3-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}propoxy)-5'-oxo-11',11'a-dihydro-1'H,3'H-spiro[cyclopropane-1,2'-pyrrolo[2,1-c][1,4]benzodiazepine]-10'(5'H)-carbonyl]oxy}methyl)phenyl]-L-alaninamide (4-11)

Compound (4-10) obtained in Step 10 (0.0571 mmol) was reacted in the same manner as in Step 12 of Example 2-3 to afford the desired compound (4-11) (0.0574 g, 99%).
$^1H$-NMR($CDCl_3$)$\delta$:9.16(1H,s),7.93-7.91(1H,m),7.55-7.52(1H,m),7.50-7.47(3H,m),7.35-7.32(2H,m),7.21(1H,s),7.13-7.11(2H,m),6.90-6.87(2H,m),6.40(1H,s),6.08(1H,s),5.90-5.87(1H,m),5.37-5.34(1H,m),4.73-4.53(3H,m),4.23-4.08(5H,m),3.89(3H,s),3.82(3H,s),3.78-3.72(5H,m),3.57-3.51(3H,m),3.38-3.30(3H,m),2.76-2.71(1H,m),2.36-2.24(4H,m),1.78-1.42(6H,m),1.00-0.98(3H,m),0.87-0.84(3H,m),0.74-0.62(4H,m).

MS(APCI, ESI)m/z:1002(M+H)+.

Step 12: N-[4-(11,12-Didehydrodibenzo[b,f]azocin-5(6H)-yl)-4-oxobutanoyl]glycylglycyl-L-valyl-N-[4-({[(11'aS)-11'-hydroxy-7'-methoxy-8'-(3-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}propoxy)-5'-oxo-11',11'a-dihydro-1'H,3'H-spiro[cyclopropane-1,2'-pyrrolo[2,1-c][1,4]benzodiazepine]-10'(5'H)-carbonyl]oxy}methyl)phenyl]-L-alaninamide (4-12)

Compound (4-11) obtained in Step 11 (0.189 g, 0.189 mmol) was reacted with compound (2-2) obtained in Step 1 of Example 2-2 (0.087 g, 0.207 mmol) in the same manner as in Step 13 of Example 2-3 to afford the desired compound (4-12) (0.169 g, 64%).

MS(APCI, ESI)m/z: 1402(M+H)+.

[Example 2-5: Drug-linker 3]

[Formula 66]

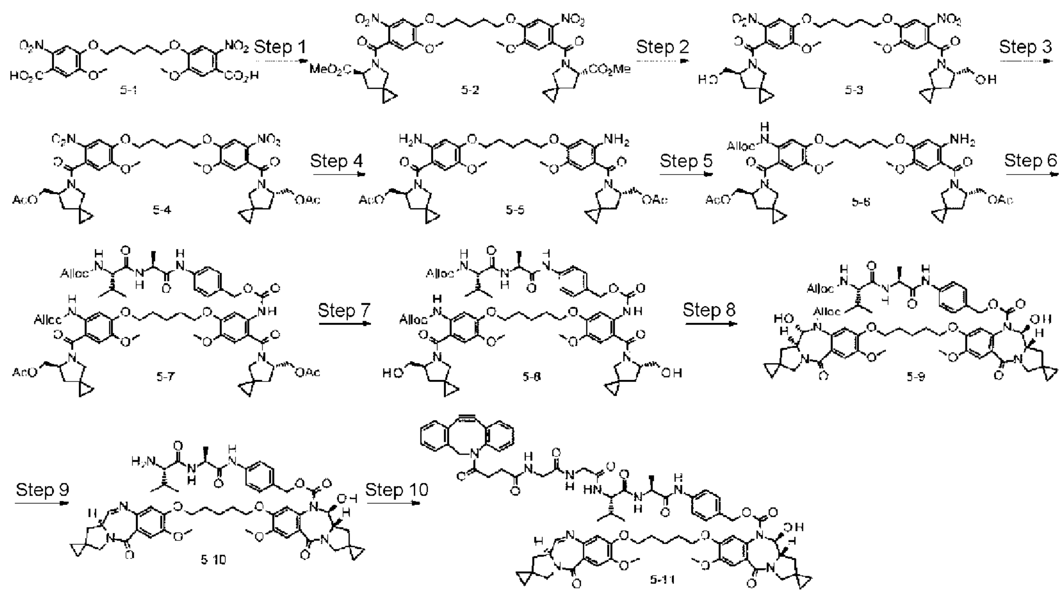


Step 1: Dimethyl(6S,6'S)-5,5'-{1,5-pentanediylbis[oxy(5-methoxy-2-nitrobenzene-4,1-diyl)carbonyl]}bis(5-azaspiro[2.4]heptane-6-carboxylate) (5-2)

To a solution of 4,4'-[1,5-pentanediylbis(oxy)]bis(5-methoxy-2-nitrobenzoic acid) (5-1) (5.41 g, 10.9 mmol, Journal of Medicinal Chemistry 2004, 47, 1161) in dichloromethane (50 mL), oxalyl chloride (5.63 mL, 65.7 mmol) was added at 0°C, and N,N-dimethylformamide (0.0844 mL, 1.09 mmol) was added dropwise. The temperature of the reaction solution was raised to room temperature, and the reaction solution was stirred for 2 hours. The resultant was distillated under reduced pressure, and the resulting residue was dissolved in dichloromethane (100 mL), which was added dropwise to dichloromethane solution (100 mL) of methyl (6S)-5-azaspiro[2.4]heptane-6-carboxylate hydrochloride (4.28 g, 24.1 mmol, Tetrahedron Letters 2012. 53. 3847) and triethylamine (6.07 mL, 43.8 mmol) under the nitrogen atmosphere at -40°C. The temperature of the reaction solution was raised to 0°C, and the reaction solution was stirred for 2 hours. To the reaction mixture, 1 N hydrochloric acid (100 mL) was added, and the organic layer was washed with water and brine, and dried over anhydrous sodium sulfate. The resultant was distillated under reduced pressure to afford the desired compound (5-2) (8.40 g, quantitative).

MS(APCI, ESI)m/z:769(M+H)+.

Step 2: {1,5-Pentanediylbis[oxy (5-methoxy-2-nitrobenzen-4,1-diyl)]}bis{[(6S)-6-(hydroxymethyl)-5-azaspiro[2.4]hept-5-yl]methanone} (5-3)

To a solution of compound (5-2) obtained in Step 1 (8.40 g, 10.9 mmol) in tetrahydrofuran (100 mL), lithium borohydride (714 mg, 32.8 mmol) was added, and the resultant was stirred at 0°C for 30 minutes, and the temperature was raised to room temperature, and stirring was performed for 1 hour. After 1 N hydrochloric acid was added at 0°C, the resultant was extracted with ethyl acetate, and washed with brine, and then dried over anhydrous sodium sulfate. The solvent was distilled off under reduced pressure to afford the desired compound (5-3) (7.70 g, 99%).
MS(APCI, ESI)m/z:713(M+H)$^+$.

Step 3: Pentan-1,5-diylbis[oxy (5-methoxy-2-nitrobenzen-4,1-diyl)carbonyl (6*S*)-5-azaspiro[2.4]heptan-5,6-diylmethanediyl] diazetate (5-4)

Compound (5-3) obtained in Step 2 (7.70 g, 10.8 mmol) was dissolved in pyridine (20 mL) and acetic anhydride (10 mL, 105.9 mmol), which was stirred at room temperature. The resultant was distillated under reduced pressure to afford the desired compound (5-4) (8.38 g, 97%).
MS(APCI, ESI)m/z:797(M+H)$^+$.

Step 4: 1,5-Pentanediylbis[oxy (2-amino-5-methoxybenzen-4,1-diyl)carbonyl (6S)-5-azaspiro[2.4]heptan-5,6-diylmethanediyl] diacetate (5-5)

To a solution of compound (5-4) obtained in Step 3 (8.28 g, 10.4 mmol) in N,N-dimethylformamide (100 mL), 5% palladium carbon (moisture content: 54%, 1.00 g) was added, and the reaction solution was then vigorously stirred under the hydrogen atmosphere at room temperature for 6 hours. The resultant was filtered through a Celite, and the filtrate was then distillated under reduced pressure, and the resulting residue was purified by silica gel column chromatography [chloroform:methanol = 100:0(v/v) to 90:10(v/v)] to afford the desired compound (5-5) (5.05 g, 66%).
MS(APCI, ESI)m/z:737(M+H)$^+$.

Step 5: {(6*S*)-5-[4-({5-[4-({(6*S*)-6-[(Acetyloxy)methyl]-5-azaspiro[2.4]hept-5-yl}carbonyl)-5-amino-2-methoxyphenoxy]pentyl}oxy)-5-methoxy-2-{[(prop-2-en-1-yloxy)carbonyl]amino}benzoyl]-5-azaspiro[2.4]hept-6-yl}methylacetate (monoallyloxycarbonyl form) (5-6)

To a solution of compound (5-5) obtained in Step 4 (5.05 g, 6.85 mmol) in dichloromethane (100 mL), pyridine (1.10 mL, 13.7 mmol) was added, and allyl chloroformate (0.725 mL, 6.85 mmol) was added thereto under the nitrogen atmosphere at -78°C, and the resultant was stirred for 2 hours. The resultant was distillated under reduced pressure, and the resulting residue was purified by silica gel column chromatography [hexane:ethyl acetate = 70:30 (v/v) to 100:0 (v/v), chloroform:methanol = 100:0 (v/v) to 90:10 (v/v)] to afford the monoallyloxycarbonyl form (5-6) (2.63 g, 47%) as the desired compound.
MS(APCI, ESI)m/z:821(M+H)$^+$.

Step 6: N-[(2-Propen-1-yloxy)carbonyl]-L-valyl-N-{4-[({[2-({(6S)-6-[(acetyloxy)methyl]-5-azaspiro[2.4]hept-5-yl}carbonyl)-5-({5-[4-({(6S)-6-[(acetyloxy)methyl]-5-azaspiro[2.4]hept-5-yl}carbonyl)-2-methoxy-5-{[(2-propen-1-yloxy)carbonyl]amino}phenoxy]pentyl}oxy)-4-methoxyphenyl]carbamoyl}oxy)methyl]phenyl}-L-alaninamide (5-7)

Monoallyloxycarbonyl form (5-6) obtained in Step 5 (2.00 g, 2.44 mmol) and N-[(prop-2-en-1-yloxy)carbonyl]-L-valyl-N-[4-(hydroxymethyl)phenyl]-L-alaninamide (1.10 g, 2.92 mmol, WO2011130598) were reacted in the same manner as in Step 6 of Example 2-1 to afford the desired compound (5-7) (2.64 g, 89%).
MS(APCI, ESI)m/z: 1224(M+H)+.

Step 7: N-[(2-Propen-1-yloxy)carbonyl]-L-valyl-N-[4-({[(2-{[(6S)-6-(hydroxymethyl)-5-azaspiro[2.4]hept-5-yl]carbonyl}-5-{[5-(4-{[(6S)-6-(hydroxymethyl)-5-azaspiro[2.4]hept-5-yl]carbonyl}-2-methoxy-5-{[(2-propen-1-yloxy)carbonyl]amino}phenoxy)pentyl]oxy}-4-methoxyphenyl)carbamoyl]oxy}methyl)phenyl]-L-alaninamide (5-8)

To a solution of compound (5-7) obtained in Step 6 (2.64 g, 2.16 mmol) in methanol (10 mL), potassium carbonate (1.49 g, 10.8 mmol) was added, and the resultant was stirred at room temperature for 3 hours. A saturated aqueous ammonium chloride (100 mL) was added to the reaction mixture, which was extracted with ethyl acetate. The organic layer was dried over anhydrous sodium sulfate. The resultant was distillated under reduced pressure to afford the desired compound (5-8) (2.21 g, 90%).

MS(APCI, ESI)m/z:1140$(M+H)^+$.

Step 8: N-[(2-Propen-1-yloxy)carbonyl]-L-valyl-N-{4-[({[(11'S,11a'S)-11'-hydroxy-8'-{[5-({(11'S,11a'S)-11'-hydroxy-7'-methoxy-5'-oxo-10'-[(2-propen-1-yloxy)carbonyl]-5',10',11',11a'-tetrahydro-1'H-spiro[cyclopropane-1,2'-pyrrolo[2,1-c][1,4]benzodiazepine]-8'-yl}oxy)pentyl]oxy}-7'-methoxy-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (5-9)

To a solution of compound (5-8) obtained in Step 7 (2.03 g, 1.78 mmol) in dichloromethane (50 mL), Dess-Martin periodinane (1.59 g, 3.74 mmol) was added, and the resultant was stirred at room temperature overnight. A saturated aqueous sodium hydrogen carbonate (100 mL) was added to the reaction mixture, which was extracted with chloroform. The organic layer was dried over anhydrous sodium sulfate. The resultant was distillated under reduced pressure, and the resulting residue was purified by silica gel column chromatography [chloroform:methanol = 100:0(v/v) to 90:10(v/v)] to afford the desired compound (5-9) (2.05 g, quantitative).

MS(APCI, ESI)m/z:1136$(M+H)^+$.

Step 9: L-Valyl-N-{4-[({[(11a'S)-11'-hydroxy-7'-methoxy-8'-[(5-{[(11'S,11a'S)-7'-methoxy-5'-oxo-5',11a'-dihydro-1'H-spiro[cyclopropane-1,2'-pyrrolo[2,1-c][1,4]benzodiazepine]-8'-yl]oxy}pentyl)oxy]-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (5-10)

Compound (5-9) obtained in Step 8 (2.05 g, 1.80 mmol) was reacted in the same manner as in Step 12 of Example 2-3 to afford the desired compound (5-10) (1.02 g, 60%).

MS(APCI, ESI)m/z:950$(M+H)^+$.

Step 10: N-[4-(11,12-Didehydrodibenzo[b,f]azocin-5 (6H)-yl)-4-oxobutanoyl]glycylglycyl-L-valyl-N-{4-[({[(11'S,11a'S)-11'-hydroxy-7'-methoxy-8'-[(5-{[(11a'S)-7'-methoxy-5'-oxo-5',11a'-dihydro-1'H-spiro[cyclopropane-1,2'-pyrrolo[2,1-c][1,4]benzodiazepine]-8'-yl]oxy}pentyl)oxy]-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (5-11)

Compound (5-10) obtained in Step 9 (0.710 g, 0.747 mmol) and compound (2-2) obtained in Step 1 of Example 2-2 (0.313 g, 0.747 mmol) were dissolved in mixed solvent of dichloromethane (1.5 mL) and methanol (0.1 mL). Thereto, 4-(4,6-dimethoxy-1,3,5-triazin-2-yl)-4-methylmorpholinium chloride (0.264 g, 0.897 mmol) was added, and the resultant was stirred at room temperature for 1 hour. The resultant was distillated under reduced pressure, and the resulting residue was purified by silica gel column chromatography [chloroform:methanol = 100:0 (v/v) to 80:20 (v/v)] to afford the desired compound (5-11) (0.671 g, 66%).

$^1$H-NMR(DMSO-$D_6$)$\delta$:9.91(1H,s),8.32(1H,s),8.23-7.91(3H,m),7.81-7.19(14H,m),7.04(1H,m),6.80-6.62(3H,m),5.77-5.75(1H,m),5.20(1H,m),5.01(1H,m),4.79(1H,m),4.46-4.35(1H,m),4.04(4H,m),3.86-3.38(18H,m),3.22-3.15(2H,m),2.67-2.63(1H,m),2.46-2.23(3H,m),2.09-1.91(2H,m),1.80-1.78(5H,m),1.57(3H,m),1.27(3H,s), 1.11-1.04(1H,m),0.87-0.79(6H,m),0.63-0.55(6H,m).

MS(APCI, ESI)m/z:1351$(M+H)^+$.

[Example 2-6: Drug-linker 4]

[Formula 67]

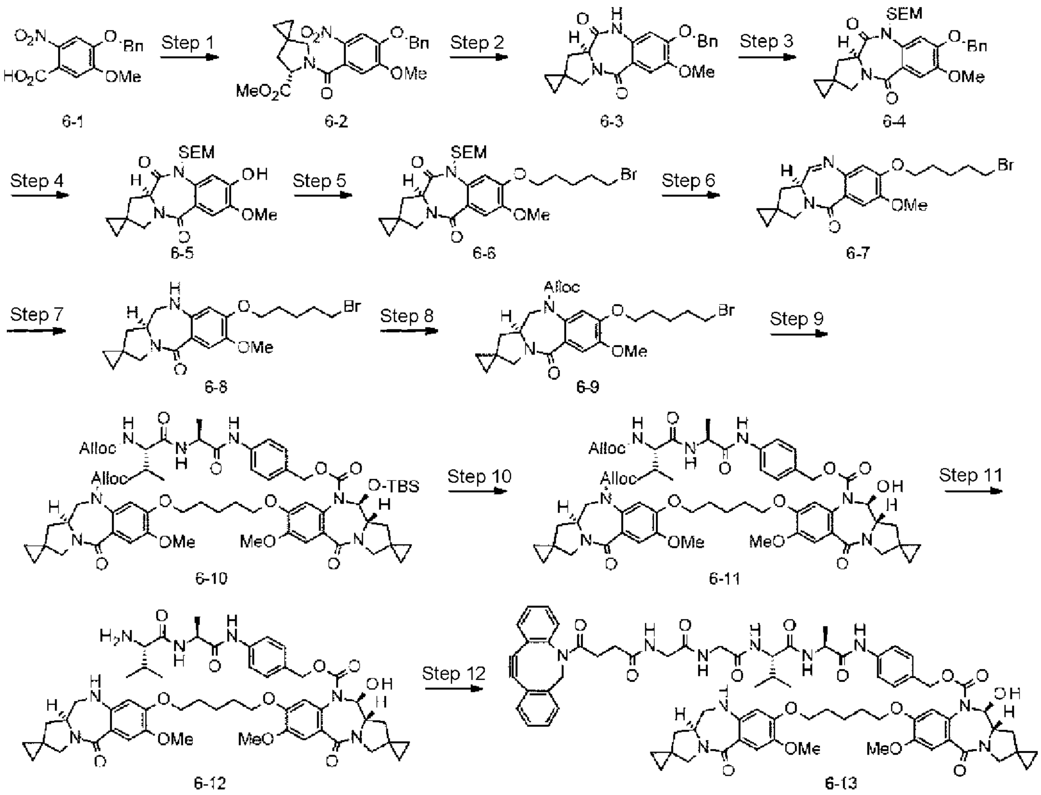


6-1 6-2 6-3 6-4 6-5 6-6 6-7 6-8 6-9 6-10 6-11 6-12 6-13

Step 1: Methyl (6S)-5-[4-(benzyloxy)-5-methoxy-2-nitrobenzoyl]-5-azaspiro[2.4]heptane-6-carboxylate (6-2)

To a solution of 4-(benzyloxy)-5-methoxy-2-nitrobenzoic acid (6-1) (6.07 g, 20.0 mmol, Tetrahedron 1995, 51, 5617) and N,N-dimethylformamide (1.08 mL, 13.9 mmol) in dichloromethane (100 mL), oxalyl chloride (3.43 mL, 40.0 mmol) was added dropwise under ice-cooling over 5 minutes. The reaction solution was stirred at room temperature for 5 hours, and then distillated under reduced pressure, and the resulting residue was dissolved in dichloromethane (20 mL), which was distillated under reduced pressure. After this operation was repeated three times, the residue was suspended in dichloromethane (5 mL), to which excessive amounts of diethyl ether and hexane were added, and the following filtration and drying under reduced pressure afforded the crude acyl chloride. The acyl chloride obtained was dissolved in dichloromethane and cooled to -40°C (dry ice-acetonitrile bath), to which methyl (6S)-5-azaspiro[2.4]heptane-6-carboxylate hydrochloride (4.22 g, 22.0 mmol, Tetrahedron Letters 2012. 53. 3847) and triethylamine (3.36 mL, 24.2 mmol) were gradually added. The temperature of the reaction mixture was raised to room temperature overnight. To the reaction mixture, 1 N hydrochloric acid was added, and the reaction mixture was extracted with dichloromethane. The organic layer was washed with water, a saturated aqueous sodium hydrogen carbonate, and brine, and dried over anhydrous sodium sulfate. The resultant was distillated under reduced pressure, and the resulting residue was purified by silica gel column chromatography [hexane:ethyl acetate = 100:0 to 50:50] to afford the desired compound (6-2) (6.55 g, 80%).

MS (APCI, ESI)m/z:441 $(M+H)^+$

Step 2: (11a'S)-8'-(Benzyloxy)-7'-methoxy-1'H-spiro[cyclopropane-1,2'-pyrrolo[2,1-c][1,4]benzodiazepine]-5',11'(10'H,11a'H)-dione (6-3)

To a solution of compound (6-2) obtained in Step 1 (6.55 g, 16.0 mmol) in ethanol (150 mL) and tetrahydrofuran (150 mL), Raney-nickel (7.00 g) was added under the nitrogen atmosphere. Hydrazine monohydrate (7 mL) was added to the reaction mixture, and the temperature was gradually raised to 50°C. After stirring at 50°C for 2 hours, Raney-nickel (3.00 g) and hydrazine monohydrate (3 mL) were added thereto, and the resultant was stirred for 1 hour. THF (100 mL) was added to the reaction mixture, which was filtered through a Celite. The resultant was distillated under reduced pressure, and the resulting residue was purified by silica gel column chromatography [hexane:ethyl acetate = 100:0 to 25:75] to afford the desired compound (6-3) (4.42 g, 73%).

MS(APCI, ESI)m/z:379(M+H)$^+$

Step 3: (11a'S)-8'-(Benzyloxy)-7'-methoxy-10'-{[2-(trimethylsilyl)ethoxy]methyl}-1'H-spiro[cyclopropane-1,2'-pyrrolo[2,1-c][1,4]benzodiazepine]-5',11'(10'H,11a'H)-dione (6-4)

To a solution of compound (6-3) obtained in Step 2 (10.0 g, 26.4 mmol) in tetrahydrofuran (150 mL), a 2.6 mol/L normal-hexane solution of normal-butyllithium (12.0 mL, 31.8 mmol) was added slowly dropwise at -40°C. The reaction solution was stirred at -40°C for 15 minutes, and 2-(chloromethoxy)ethyltrimethylsilane (5.57 mL, 31.7 mmol) was then added slowly dropwise thereto. After the reaction solution was stirred at room temperature for 3 hours, water was added thereto, and the resultant was extracted with ethyl acetate. The organic layer was washed with water and brine, and dried over anhydrous sodium sulfate. After distillation under reduced pressure, the resulting residue was purified by silica gel column chromatography [hexane:ethyl acetate = 100:0 to 30:70] to afford the desired compound (6-4) (11.8 g, 88%).

MS(APCI, ESI)m/z:509(M+H)$^+$

Step 4: (11a'S)-8'-Hydroxy-7'-methoxy-10'-{[2-(trimethylsilyl)ethoxy]methyl}-1'H-spiro[cyclopropane-1,2'-pyrrolo[2,1-c][1,4]benzodiazepine]-5',11'(10'H,11a'H)-dione (6-5)

To a solution of compound (6-4) obtained in Step 3 (18.7 g, 36.8 mmol) in tetrahydrofuran (50 mL) and ethanol (100 mL), a 5% palladium carbon catalyst (5.00 g) was added under the nitrogen atmosphere. The nitrogen balloon was immediately replaced with a hydrogen balloon, and the reaction mixture was stirred under the hydrogen atmosphere for 6 hours. The reaction mixture was diluted by addition of chloroform and filtered through a Celite, and the filtrate was then distillated under reduced pressure, and the resulting residue was purified by silica gel column chromatography [hexane:ethyl acetate = 100:0 to 25:75] to afford the desired compound (6-5) (15.1 g, 98%).
MS(APCI, ESI)m/z:419(M+H)$^+$

Step 5: (11a'S)-8'-[(5-Bromopentyl)oxy]-7'-methoxy-10'-{[2-(trimethylsilyl)ethoxy]methyl}-1'H-spiro[cyclopropane-1,2'-pyrrolo[2,1-c][1,4]benzodiazepine]-5',11'(10'H,11a'H)-dione (6-6)

Compound (6-5) obtained in Step 4 (2.77 g, 6.62 mmol) was reacted in the same manner as in Step 2 of Example 2-3 to afford the desired compound (6-6) (3.31 g, 88%). $^1$H-NMR($CDCl_3$)$\delta$:7.36(1H,s),7.25(1H,s),5.55(1H,m),4.65(1H,m),4.24-4.23(1H,m),4.11-4.03(2H,m),3.93(3H,s),3.85-3.78(1H,m),3.72-3.69(2H,m),3.46-3.39(3H,m),2.47-2.44(1H,m),2.25-2.22(1H,m),1.95-1.91(4H,m),1.67-1.59(1H,m),1.03-0.95(2H,m),0.90-0.85(1H,m),0.70-0.66(4H,m),0.05(9H,s).

Step 6: (11a'S)-8'-[(5-Bromopentyl)oxy]-7'-methoxy-1',11a'-dihydro-5'H-spiro[cyclopropane-1,2'-pyrrolo[2,1-c][1,4]benzodiazepine]-5'-one (6-7)

Compound (6-6) obtained in Step 5 (3.31 g, 5.83 mmol) was reacted in the same manner as in Step 7 of Example 2-3 to afford the desired compound (6-7) (1.11 g, 45%). $^1$H-NMR($CDCl_3$)$\delta$:7.81(1H,m),7.53(1H,s),6.82(1H,s),4.13-4.06(2H,m),3.97(3H,s),3.88-3.83(1H,m),3.69(1H,m),3.52-3.39(3H,m),2.55-2.52(1H,m),2.06-1.89(5H,m),1.67-1.63(2H,m),0.76-0.72(4H,m).

Step 7: (11a'S)-8'-[(5-Bromopentyl)oxy]-7'-methoxy-1',10',11',11a'-tetrahydro-5'H-spiro[cyclopropane-1,2'-pyrrolo[2,1-c][1,4]benzodiazepine]-5'-one (6-8)

Compound (6-7) obtained in Step 6 (2.56 g, 6.08 mmol) was reacted in the same manner as in Step 8 of Example 2-3 to afford the desired compound (6-8) (1.15 g, 45%). $^1$H-NMR($CDCl_3$)$\delta$:7.60(1H,s),6.07(1H,s),4.11-4.04(1H,m),3.99(2H,m),3.87-3.84(1H,m),3.85(3H,s),3.73(1H,m),3.58-3.53(2H,m),3.47-3.42(3H,m),2.03-1.78(6H,m),1.65-1.63(2H,m),0.77-0.56(4H,m).

Step 8: Prop-2-en-1-yl (11a'S)-8'-[(5-bromopentyl)oxy]-7'-methoxy-5'-oxo-11',11a'-dihydro-1'H-spiro[cyclopropane-1,2'-pyrrolo[2,1-c][1,4]benzodiazepine]-10'(5'H)-carboxylate (6-9)

Compound (6-8) obtained in Step 7 (1.15 g, 2.72 mmol) was reacted in the same manner as in Step 9 of Example 2-3 to afford the desired compound (6-9) (1.14 g, 82%). $^1$H-NMR($CDCl_3$)$\delta$:7.23(1H,s),6.69(1H,s),5.79(1H,s),5.13-5.10(2H,m),4.68-4.66(1H,m),4.48-4.45(2H,m),4.01(2H,m),3.92(3H,s),3.76(1H,m),3.54-3.37(3H,m),2.39(1H,m),1.95-1.90(4H,m),1.68-1.61(3H,m),1.44(1H,m),0.75-0.66(4H,m).

Step 9: N-[(Prop-2-en-1-yloxy)carbonyl]-L-valyl-N-{4-[({[(11'S,11a'S)-11'-{[tert-butyl(dimethyl)silyl]oxy}-7'-methoxy-8'-{[5-({(11a'S)-7'-methoxy-5'-oxo-10'-[(prop-2-en-1-yloxy)carbonyl]-5',10',11',11a'-tetrahydro-1'H-spiro[cyclopropane-1,2'-pyrrolo[2,1-c][1,4]benzodiazepine]-8'-yl}oxy)pentyl]oxy}-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (6-10)

Compound (6-9) obtained in Step 8 (0.374 g, 0.737 mmol) and compound (1-11) obtained in Step 10 of Example 2-1 (0.452 g, 0.56 mmol) were reacted in the same manner as in Step 10 of Example 2-3 to afford the desired compound (6-10) (0.589 g, 65%).
MS (APCI, ESI)m/z:1234 $(M+H)^+$

Step 10: N-[(Prop-2-en-1-yloxy)carbonyl]-L-valyl-N-{4-[({[(11'S,11a'S)-11'-hydroxy-7'-methoxy-8'-{[5-({(11a'S)-7'-methoxy-5'-oxo-10'-[(prop-2-en-1-yloxy)carbonyl]-5',10',11',11a'-tetrahydro-1'H-spiro[cyclopropane-1,2'-pyrrolo[2,1-c][1,4]benzodiazepine]-8'-yl}oxy)pentyl]oxy}-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (6-11)

Compound (6-10) obtained in Step 9 (0.589 g, 0.477 mmol) was reacted in the same manner as in Step 11 of Example 2-3 to afford the desired compound (6-11) (0.382 g, 71%).
$^1H$-NMR($CDCl_3$)$\delta$:8.90(1H,s),7.55(2H,m),7.25-7.21(2H,m),6.74(2H,m),6.38(1H,s),5.90-5.87(5H,m),5.33-5.09(8H,m),4.66-4.60(8H,m),3.98-3.91(10H,m),3.77-3.30(12H,m),2.42-2.36(2H,m),1.77-1.39(6H,m),0.91-0.70(14H,m).

Step 11: L-Valyl-N-{4-[({[(11'S,11a'S)-11'-hydroxy-7'-methoxy-8'-[(5-{[(11a'S)-7'-methoxy-5'-oxo-5',10',11',11a'-tetrahydro-1'H-spiro[cyclopropane-1,2'-pyrrolo[2,1-c][1,4]benzodiazepine]-8'-yl]oxy}pentyl)oxy]-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (6-12)

Compound (6-11) obtained in Step 10 (0.382 g, 0.341 mmol) was reacted in the same manner as in Step 12 of Example 2-3 to afford the desired compound (6-12) (0.200 g, 62%).
MS (APCI, ESI)m/z:952 $(M+H)^+$

Step 12: N-[4-(11,12-Didehydrodibenzo[b,f]azocin-5 (6H)-yl)-4-oxobutanoyl]glycylglycyl-L-valyl-N-{4-[({[(11'S,11a'S)-11'-hydroxy-7'-methoxy-8'-[(5-{[(11a'S)-7'-methoxy-5'-oxo-5',10',11',11a'-tetrahydro-1'H-spiro[cyclopropane-1,2'-pyrrolo[2,1-c][1,4]benzodiazepine]-8'-yl]oxy}pentyl)oxy]-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (6-13)

Compound (6-12) obtained in Step 11 (0.0560 g, 0.0588 mmol) and compound (2-2) obtained in Step 1 of Example 2-2 (0.022 g, 0.053 mmol) were reacted in the same manner as in Step 13 of Example 2-3 to afford the desired compound (6-13) (0.0500 g, 63%).
MS (APCI, ESI)m/z:1354 $(M+H)^+$

[Synthesis of glycan donor]

Example 3: [$N_3$-PEG(3)]-MSG1-Ox

[Formula 68]

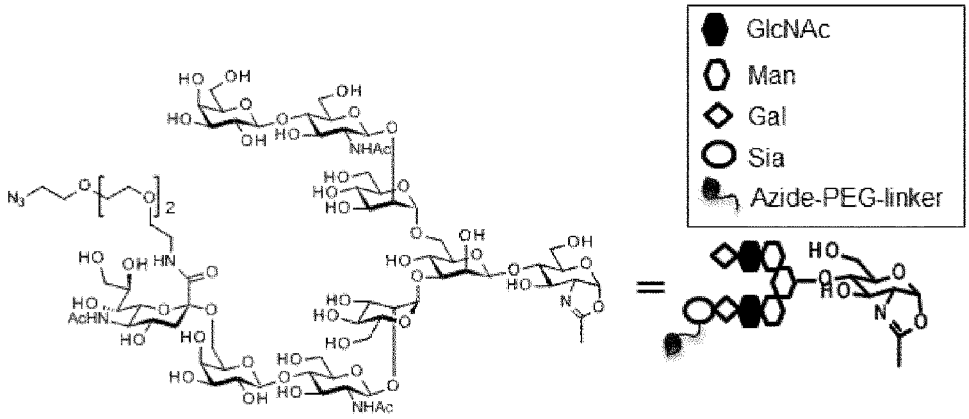


Step 1: (MSG1-)Asn

The commercially available product monosialo-Asn free (1S2G/1G2S-10NC-Asn, produced by GlyTech, Inc.) (referred to as "(MSG-)Asn") (500 mg) was subjected to separation/purification by reversed-phase HPLC under conditions below to separate into (MSG1-)Asn eluted as the 1st main peak (retention time: around 15 to 19 min) and (MSG2-)Asn eluted as the 2nd main peak (retention time: around 21 to 26 min). The eluent used was a 0.1% formic acid aqueous solution, the apparatus used was an ELS-PDA trigger preparative system (produced by JASCO Corporation), the column used was an Inertsil ODS-3 (10 um, 30ϕ × 250 mm, produced by GL Sciences, Inc.), and the flow rate was 30 mL/min. Fractions of the first peak UV-detected (210 nm) during the elution were separated, and freeze-dried to afford the desired compound (238 mg).

Step 2: MSG1

The compound obtained in Step 1 (229 mg) was dissolved in 200 mM phosphate buffer solution (pH 6.25) (1145 μL), to which an aqueous solution (100 μL) of EndoM (produced by Tokyo Chemical Industry Co., Ltd., 1 U/mL)) was added, and the resultant was incubated at 35°C for 6 days. After the completion of the reaction, the reaction solution was subjected to ultrafiltration with a VIVASPIN 15R (Hydrosart membrane, 30K, 6,000 ×G), and the filtered solution obtained was subjected to separation/purification by reversed-phase HPLC. The eluent used was a 0.1% trifluoroacetic acid aqueous solution, the apparatus used was an ELS-PDA trigger preparative system (produced by JASCO Corporation), and the column used was an Inertsil ODS-3 (produced by GL Sciences, Inc.). Fractions corresponding to the peak of the desired compound UV-detected (210 nm) during the elution were separated, and freeze-dried to afford the desired compound (117 mg).

Step 3: [$N_3$-PEG(3)]-MSG1

Into a 5 mL sampling tube (Ina-Optica Co., Ltd.), 11-azide-3,6,9-trioxaundecane-1-amine (0.108 mL, 0.541 mmol) and an aqueous solution (1.2 mL) of MSG1 obtained in Step 2 (117 mg, 0.068 mmol) were added, and the resultant was stirred for 1 hour and then freeze-dried. Into the 5 mL sampling tube after freeze-drying, an N,N-dimethylformamide solution (1.2 mL) of O-(7-azabenzotriazol-1-yl)-N,N,N',N'-tetramethyluronium hexafluorophosphate (103 mg, 0.27 mmol) and diisopropylethylamine (0.046 mL, 0.27 mmol) were added, followed by stirring at 37°C for 3 hours. After the completion of the reaction, the reaction solution was transferred into a centrifuge tube (50 mL) into which diethyl ether (20 mL) had been added in advance. The solid matter was precipitated by using a small centrifuge (Hitachi Koki Co., Ltd., CF16RX) and the supernatant was removed. Diethyl ether (10 mL) was further added, and the resultant was centrifuged and then decanted. Subsequently, acetonitrile (10 mL) was added and the resultant was subjected twice to an operation of centrifugation followed by decantation, and dried under reduced pressure to afford a crude product. The resulting solid matter was subjected to separation/purification by reversed-phase HPLC under the same conditions as in Step 2 to afford the desired compound (94.2 mg).

Step 4: [$N_3$-PEG(3)]-MSG1-Ox

Into a 5 mL sampling tube (produced by Ina-Optica Co., Ltd.), the compound synthesized in Step 3 (100 mg)

and an aqueous solution (520 μL) of 2-chloro-1,3-dimethyl-1H-benzimidazol-3-ium-chloride (produced by FUSHIMI Pharmaceutical Co., Ltd., 56 mg, 0.257 mmol) was added. To the reaction solution after being ice-cooled, an aqueous solution (520 μL) of tripotassium phosphate (165 mg, 0.78 mmol) was added, followed by stirring under ice-cooling for 3 hours. The resulting reaction solution was subjected to ultrafiltration with an Amicon Ultra (Ultracel 30K, produced by Merck Millipore) to remove the solid matter. The filtered solution was purified by gel filtration chromatography. The apparatus used was a Purif-Rp2 (produced by Shoko Scientific Co., Ltd.), the column used was a HiPrep 26/10 Desalting (produced by GE Healthcare), the mobile phase used was 0.03%-$NH_3$ aqueous solution, and the flow rate was 10 mL/min and the fraction volume was 10 mL. Fractions containing the desired compound UV-detected (220 nm) during the elution were collected together, to which a 1 N aqueous solution of sodium hydroxide (104 μL, 0.104 mmol) was added, and the resultant was freeze-dried to afford the desired compound (84 mg).

Example 4: [$N_3$-PEG(3)]-MSG-Ox

[Formula 69]

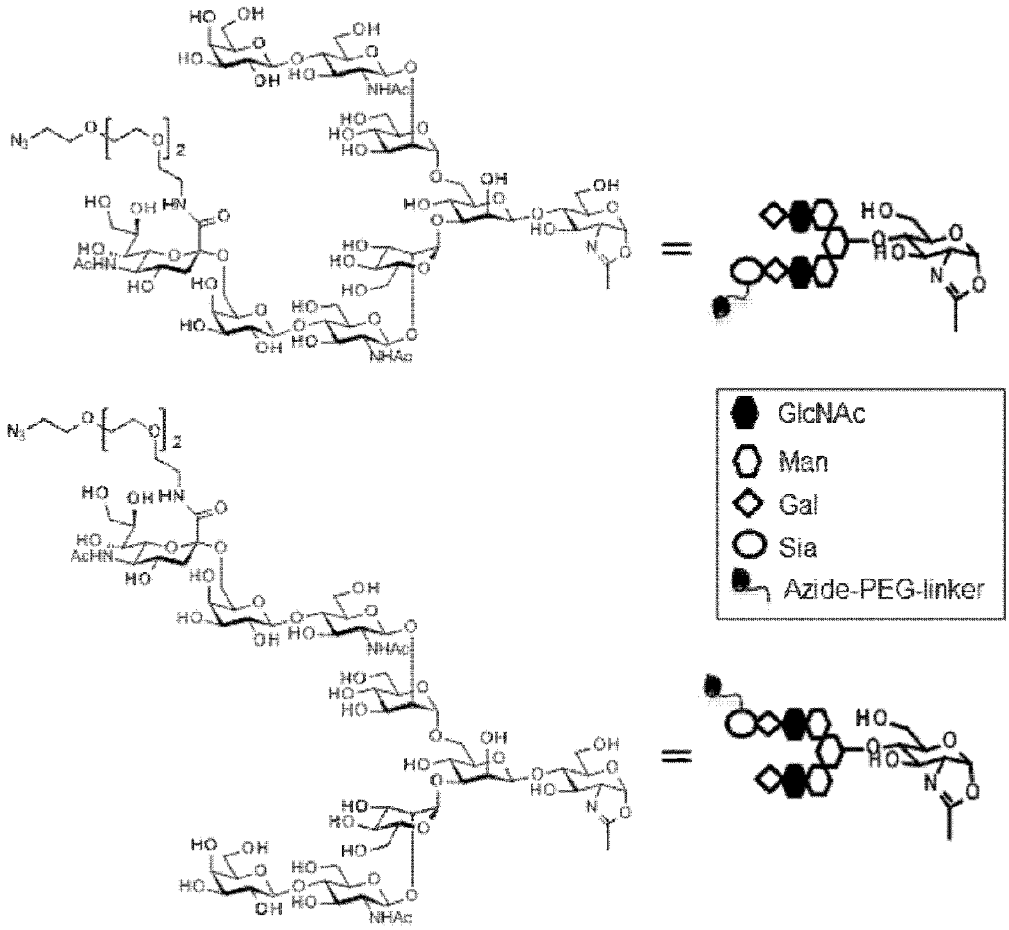


Step 1: Preparation of (MSG-)Asn

The commercially available product 1S2G/1G2S-10NC-Asn-Fmoc (produced by GlyTech, Inc.) (referred to as "Fmoc-(MSG-)Asn") (1000 mg) was dissolved in ethanol/water (1/1) (10 mL), to which a 1 N aqueous solution of sodium hydroxide (1.75 mL, 4 equivalents) was added, followed by stirring at room temperature for 3 hours. After the completion of the reaction, the reaction solution was subjected to ultrafiltration with an Amicon Ultra (30K, produced by Millipore Corporation) to remove the solid matter, and 1 N hydrochloric acid (832 μL, 1.9 equivalents) was added to the filtered solution obtained. The solvent was removed using the high-speed evaporator V-10 (produced by Biotage). Acetonitrile was added thereto, and the solvent was removed using the high-speed evaporator V-10 (produced by Biotage), and the resultant was then subjected to separation/purification by reversed-phase HPLC. The eluent was a 0.1% trifluoroacetic

acid aqueous solution and a 0.1% trifluoroacetic acid acetonitrile solution, the apparatus used was a Purif-Rp2 (produced by Shoko Scientific Co., Ltd.), and the column used was an Inertsil ODS-3 (produced by GL Sciences, Inc.). Fractions containing the desired compound UV-detected (220 nm) during the elution were collected together, and freeze-dried. This was dissolved again in pure water, and a pH test paper strip indicated that the solution was acidic. Hence, 18% aqueous ammonia (150 μL) was added thereto and it was confirmed with a pH test paper strip that the solution had become basic, and the solution was freeze-dried again. The desired compound obtained (840 mg) was directly used for the subsequent reaction.

Step 2: Synthesis of MSG

The compound obtained in Step 1 (840 mg) was dissolved in 200 mM phosphate buffer solution (pH 6.25) (6000 μL), to which an aqueous solution (200 μL) of EndoM (produced by Tokyo Chemical Industry Co., Ltd., 1 U/mL) was added, and the resultant was incubated at 28°C for 26 hours. Because the reaction had not completed, an aqueous solution (50 μL) of EndoM (produced by Tokyo Chemical Industry Co., Ltd., 1 U/mL)) was added, and the resultant was incubated at 28°C for 2 hours, and then left to stand at room temperature until the completion of the reaction. After the completion of the reaction, the reaction solution was subjected to ultrafiltration using an Amicon Ultra (30K, produced by Millipore Corporation). Trifluoroacetic acid (80 μL) was added to the filtered solution obtained, which was subjected to separation/purification by reversed-phase HPLC. The eluent was a 0.1% trifluoroacetic acid aqueous solution and a 0.1% trifluoroacetic acid acetonitrile solution, the apparatus used was a Purif-Rp2 (produced by Shoko Scientific Co., Ltd.), and the column used was an Inertsil ODS-3 (produced by GL Sciences, Inc.). Fractions containing the desired compound UV-detected (220 nm) during the elution were collected together, and freeze-dried. This was dissolved again in pure water in order to remove the residual trifluoroacetic acid, and thus the desired compound (618 mg) was obtained as a colorless solid.

ESI-MS: Calcd for $C_{66}H_{110}N_4O_{49}$ : $[M+H]^+$ 1743.62, Found 1743.63

Step 3: Synthesis of [$N_3$-PEG (3)]-MSG

In accordance with the procedure of Step 3 of Example 3 using the compound obtained in Step 2 (120 mg), the desired compound (88.6 mg) was obtained. ESI-MS: Calcd for $C_{73}H_{124}N_8O_{51}$ :$[M+2H]^{2+}$ 965.37, Found 965.37

Step 4 Synthesis of [$N_3$-PEG (3)]-MSG-Ox

In accordance with the procedure of Step 4 of Example 3 using the synthesized compound obtained in Step 3 (100 mg), the desired compound (88 mg) was obtained.

Example 5: [$N_3$-PEG (3)]$_2$-SG (10)-Ox

[Formula 70]

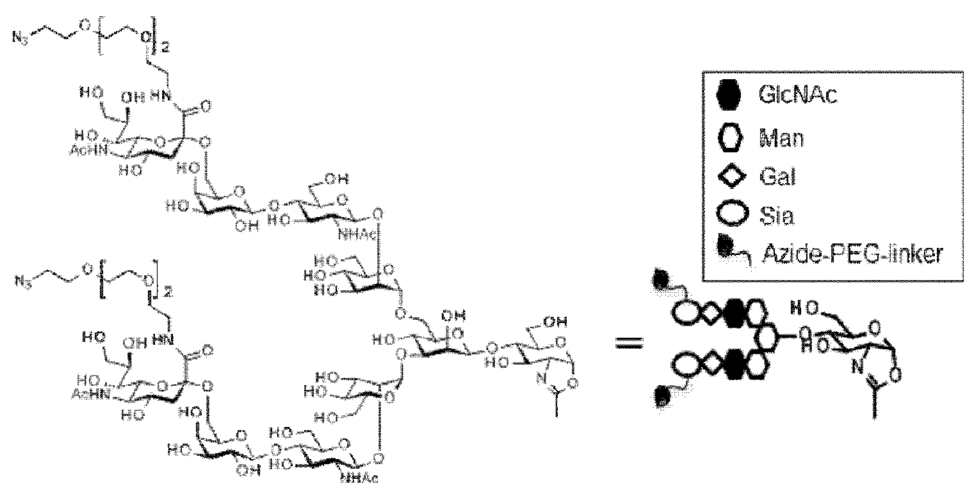

Step 1: $[N_3$-PEG $(3)]_2$-SG (10)

Into a 5 mL sampling tube (Ina-Optica Co., Ltd), an aqueous solution (0.5 mL) of 11-azide-3,6,9-trioxaundecane-1-amine (0.096 mL, 0.485 mmol) and disialooctasaccharide (50 mg, 0.24 mmol) were added, and the resultant was stirred for 1 hour and then freeze-dried. Into the 5 mL sampling tube after freeze-drying, an N,N-dimethylformamide solution (0.6 mL) of O-(7-azabenzotriazol-1-yl)-N,N,N',N'-tetramethyluronium hexafluorophosphate (92 mg, 0.24 mmol) and diisopropylethylamine (0.042 mL, 0.24 mmol) were added, followed by stirring at 37°C for 4 hours. After the completion of the reaction, the reaction solution was transferred into a centrifuge tube (50 mL) into which diethyl ether (20 mL) had been added in advance. The solid matter was precipitated by using a small centrifuge (Hitachi Koki Co., Ltd., CF16RX) and the supernatant was removed. Diethyl ether (20 mL) was added and the resultant was decanted. Subsequently, acetonitrile (20 mL) was added and the resultant was decanted, and then dried under reduced pressure to afford a crude product. The resulting solid matter was dissolved in an appropriate amount of a 0.2% trifluoroacetic acid aqueous solution, and subjected to separation/purification by reversed-phase HPLC. The eluent was a 0.1% trifluoroacetic acid aqueous solution and a 0.1% trifluoroacetic acid acetonitrile solution, the apparatus used was a Purif-Rp2 (produced by Shoko Scientific Co., Ltd.), and the column used was an Inertsil ODS-3 (produced by GL Sciences, Inc.). Fractions containing the desired compound UV-detected (220 nm) during the elution were collected together, and freeze-dried to afford the desired compound (42 mg).

Step 2: $[N_3$-PEG $(3)]_2$-SG (10)-Ox

Into a 5 mL sampling tube (produced by Ina-Optica Co., Ltd), the compound synthesized in Step 1 (40 mg) and an aqueous solution (200 μL) of 2-chloro-1,3-dimethyl-1H-benzimidazol-3-ium-chloride (produced by FUSHIMI Pharmaceutical Co., Ltd. 17.9 mg, 0.083 mmol) was added. To the reaction solution after being ice-cooled, an aqueous solution (200 μL) of tripotassium phosphate (52.6 mg, 0.25 mmol) was added, followed by stirring under ice-cooling for 2 hours. The resulting reaction solution was subjected to ultrafiltration using an Amicon Ultra (Ultracel 30K, produced by Merck Millipore) to remove the solid matter. The filtered solution was purified by gel filtration chromatography. The apparatus used was a Purif-Rp2 (produced by Shoko Scientific Co., Ltd.), the column used was a HiPrep 26/10 Desalting (produced by GE Healthcare), the mobile phase used was 0.03% $NH_3$ aqueous solution, and the flow rate was 10 mL/min and the fraction volume was 10 mL. Fractions containing the desired compound UV-detected (220 nm) during the elution were collected together, to which a 1 N aqueous solution of sodium hydroxide (33 μL, 0.033 mmol) was added, and the resultant was freeze-dried to afford the desired compound (34 mg).

[Preparation of glycan remodelling antibodies]

Example 6: Trastuzumab A1 or A2 antibody-$[MSG1\text{-}N_3]_2$

[Formula 71]

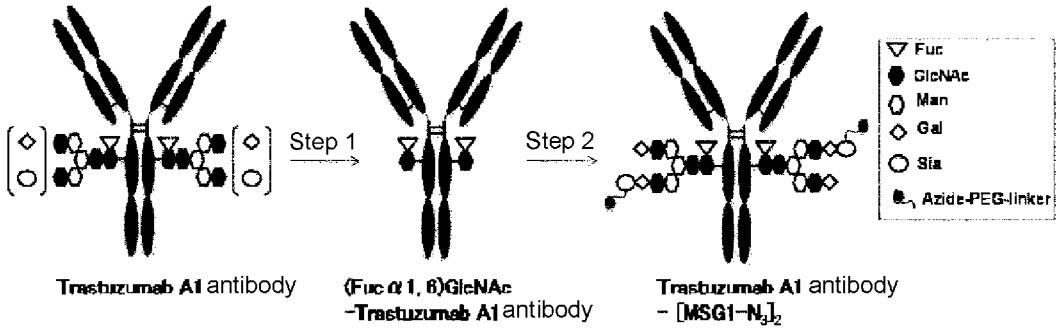


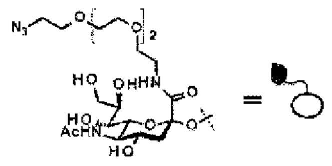

Step 1: Preparation of (Fucα1,6)GlcNAc-Trastuzumab A1 antibody

To the ca. 22.3 mg/mL Trastuzumab A1 antibody solution (50 mM phosphate buffer (pH 6.0)) prepared in Example 1-3 (2.69 mL), 0.156 mL of 7.7 mg/mL wild-type EndoS solution (PBS) was added, and the resultant was incubated at 37°C for 4 hours. The progress of the reaction was confirmed by using Experion electrophoresis station (produced by Bio-Rad Laboratories, Inc.). After the completion of the reaction, purification by affinity chromatography and purification with a hydroxyapatite column were carried out according to the following methods.

(1) Purification by affinity chromatography

Purification apparatus: AKTA avant (produced by GE Healthcare)
Column: HiTrap rProtein A FF (5 mL) (produced by GE Healthcare)
Flow rate: 5 mL/min (1.25 mL/min in charging)

Each reaction solution obtained above was purified in multiple separate operations. In connecting to the column, the reaction solution was added to the upper part of the column, and 4 CV (Column Volume) of binding buffer (20 mM phosphate buffer (pH 6.0)) was flowed at 1.25 mL/min and 5 CV thereof was further flowed at 5 mL/min. In intermediate washing, 15 CV of washing solution (20 mM phosphate buffer (pH 7.0), 0.5 M sodium chloride solution) was flowed. In elution, 6 CV of elution buffer (ImmunoPure IgG Eution buffer, produced by Pierce) was flowed. The eluate was immediately neutralized with 1 M Tris buffer (pH 9.0). Fractions containing the desired compound were subjected to buffer exchange to 5 mM phosphate buffer/50 mM-morpholinoethanesulfonic acid (MES) solution (pH 6.8) by using common operation C.

(2) Purification by hydroxyapatite chromatography

Purification apparatus: AKTA avant (produced by GE Healthcare)
Column: Bio-Scale Mini CHT Type I cartridge (5 mL) (produced by Bio-Rad Laboratories, Inc.)
Flow rate: 5 mL/min (1.25 mL/min in charging)

The solution obtained in (1) was added to the upper part of the column, and 4 CV of solution A (5 mM phosphate buffer/50 mM morpholinoethanesulfonic acid (MES) solution (pH 6.8)) was flowed at 1.25 mL/min and 3 CV thereof was further flowed at 5 mL/min. Thereafter, elution was carried out with solution A and solution B (5 mM phosphate buffer/50 mM morpholinoethanesulfonic acid (MES) solution (pH 6.8), 2 M sodium chloride solution). The elution conditions were solution A: solution B = 100:0 to 0:100 (15 CV). Further, 5 CV of washing solution (500 mM phosphate buffer (pH 6.5)) was flowed.
Fractions containing the desired compound were subjected to buffer exchange by using common operation C

to afford a 6.08 mg/mL (Fucα1,6)GlcNAc-Trastuzumab A1 antibody solution (50 mM phosphate buffer (pH 6.0)) (6.10 mL).

Step 2: Preparation of Trastuzumab A1 antibody-[MSG1-$N_3$]$_2$

To the 6.08 mg/mL (Fucα1,6)GlcNAc-Trastuzumab A1 antibody solution (50 mM phosphate buffer (pH 6.0)) obtained in Step 1 (6.10 mL), a solution (0.200 mL) of the glycan (9.78 mg) synthesized in Step 4 of Example 3 in 50 mM phosphate buffer (pH 6.0) and 5.80 mg/mL EndoS D233Q/Q303L solution (PBS) (0.128 mL) were added, and the resultant was incubated at 30°C for 3 hours. The progress of the reaction was confirmed by using an Experion electrophoresis station (produced by Bio-Rad Laboratories, Inc.). After the completion of the reaction, purification by affinity chromatography and purification by hydroxyapatite chromatography were carried out as in Step 1, and fractions containing the desired compound were then subjected to buffer exchange to phosphate buffered saline (pH 6.0) by using common operation C to afford a 10.2 mg/mL Trastuzumab A1 antibody [MSG-$N_3$]$_2$ solution (phosphate buffered saline (pH 6.0)) (3.65 mL).

The operations same as in Steps 1 and 2 of Example 6 were carried out using Trastuzumab A2 antibody (Example 1) to afford Trastuzumab A2 antibody-[MSG1-$N_3$]$_2$.

Example 7: H01L02-antibody-[MSG1-$N_3$]$_2$

[Formula 72]

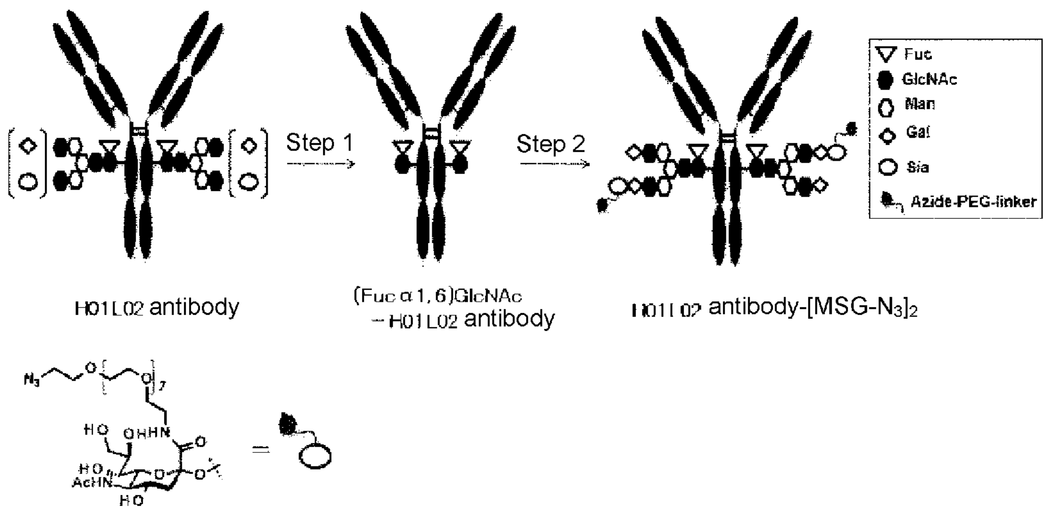


Step 1: Preparation of (Fucα1,6)GlcNAc-H01L02 antibody

The operations same as in Step 1 of Example 6 were carried out using the ca. 24.3 mg/mL H01L02 antibody solution (50 mM phosphate buffer (pH 6.0)) prepared in Example 1-3 (1.65 mL) to afford 20.0 mg/mL (Fucα1,6)GlcNAc-H01L02 antibody solution (50 mM phosphate buffer (pH 6.0)) (1.48 mL).

Step 2: Preparation of H01L02 antibody-[MSG1-$N_3$]$_2$

The operations same as in Step 2 of Example 6 were carried out using the 20.0 mg/mL (Fucα1,6)GlcNAc-H01L02 antibody solution (50 mM phosphate buffer (pH 6.0)) obtained in Step 1 (1.48 mL) to afford 10.0 mg/mL H01L02 antibody-[MSG1-$N_3$]$_2$ solution (phosphate buffered saline (pH 6.0)) (1.5 mL).

Example 8: HwtL05 antibody-[MSG1-$N_3$]$_2$

[Formula 73]

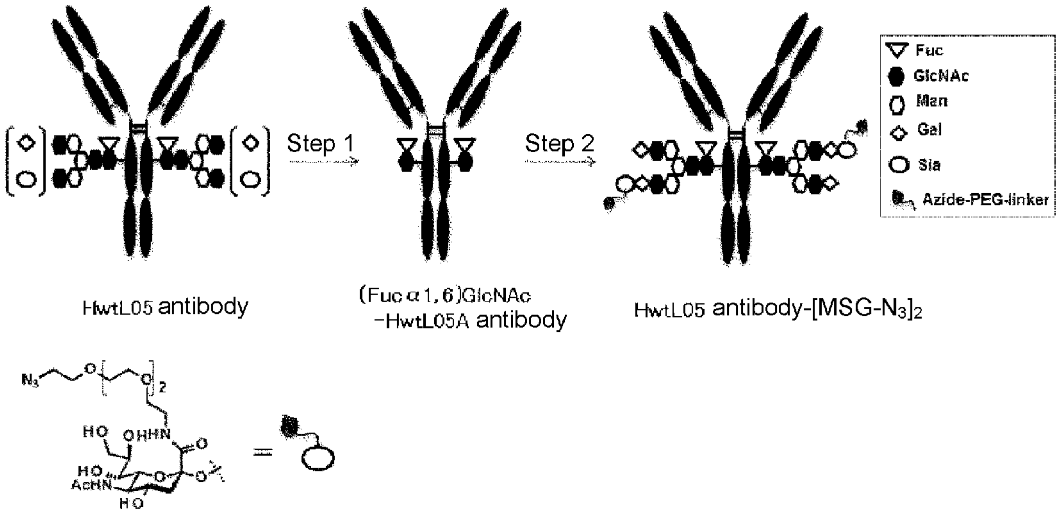


Step 1: Preparation of (Fucα1,6)GlcNAc-HwtL05 antibody

The operations same as in Step 1 of Example 6 were carried out using the ca. 24.5 mg/mL anti-HwtL05 antibody (50 mM phosphate buffer (pH 6.0)) prepared in Example 1-3 (3.00 mL) to afford 20.39 mg/mL (Fucα1,6)GlcNAc-HwtL05 antibody solution (50 mM phosphate buffer (pH 6.0)) (2.8 mL).

Step 2: Preparation of HwtL05 antibody-[MSG1-$N_3$]$_2$

The operations same as in Step 2 of Example 6 were carried out using the 20.39 mg/mL (Fucα1,6)GlcNAc-HwtL05 antibody solution (50 mM phosphate buffer (pH 6.0)) obtained in Step 1 (2.1 mL) to afford 10.04 mg/mL HwtL05 antibody-[MSG1-$N_3$]$_2$ solution (phosphate buffered saline (pH 6.0)) (4.0 mL).

[Synthesis of ADC]

Example 9: ADC1

As shown in the following reaction formula, ADC1 was synthesized by conjugating the antibody obtained in Step 2 of Example 6 with drug-linker 1 (3-14) obtained in Example 2-3. In the formula, R represents the drug-linker used in Examples.

[Formula 74]

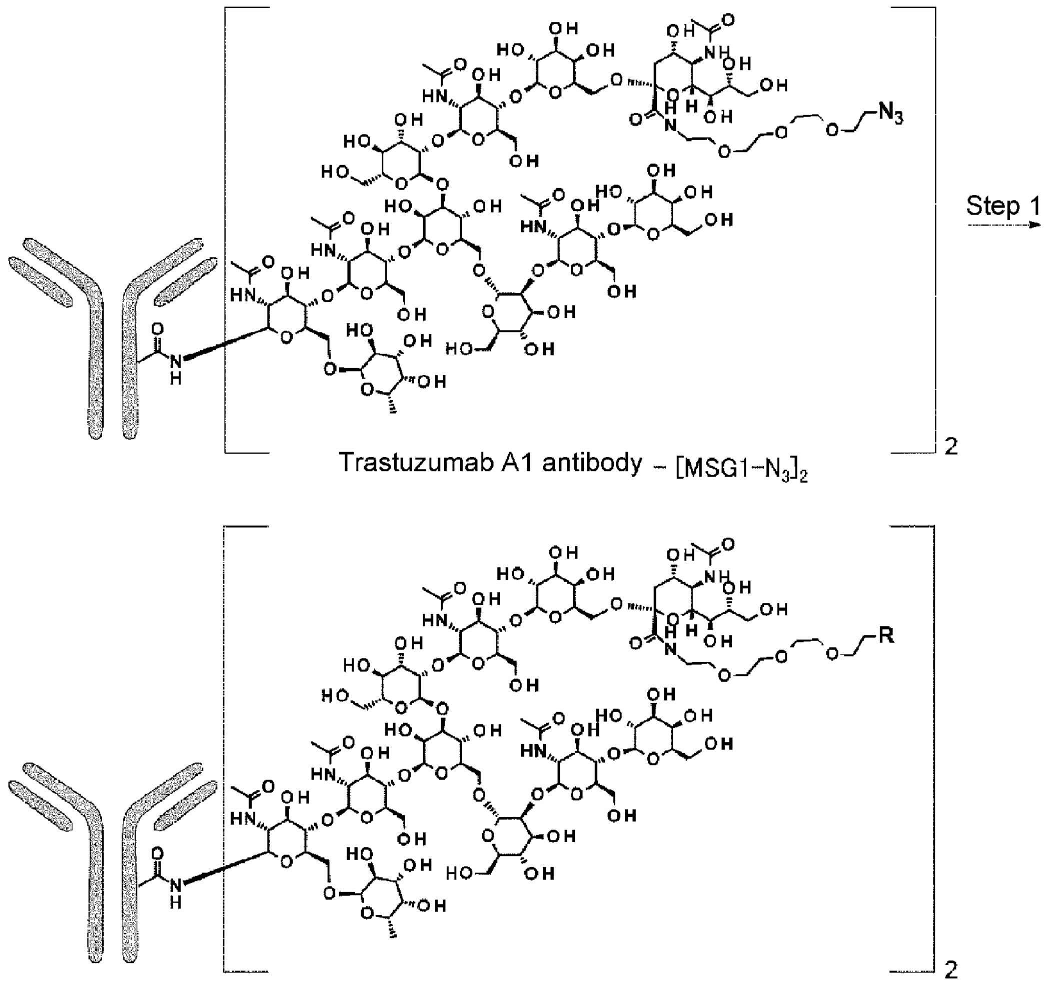


Trastuzumab A1 antibody – $[MSG1\text{-}N_3]_2$

[Formula 75]

R =

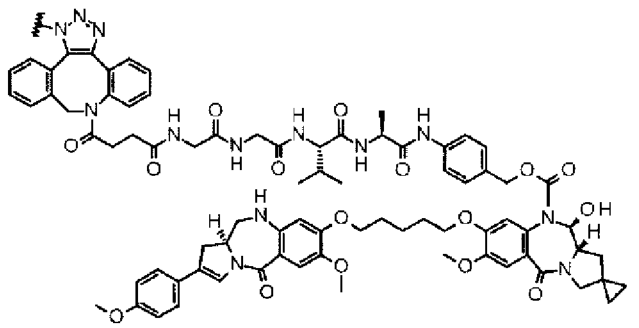

or

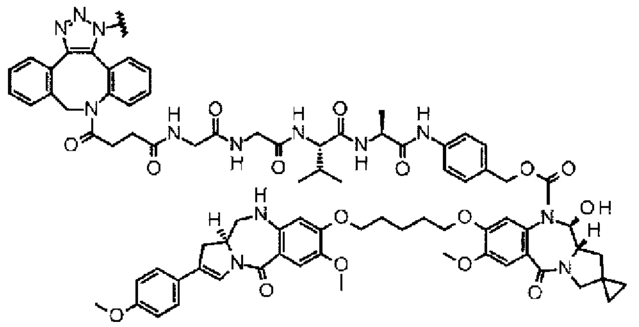

(In the compound obtained in Step 1 of Example 9, the triazole ring has geometric isomers, and the compound has a drug-linker as a mixture of the two structures shown above as R).

Step 1: Conjugation of antibody and drug-linker

To a phosphate buffered saline (pH 6.0) solution of the antibody (10.0 mg/mL, 0.40 mL) obtained in Step 2 of Example 6, 1,2-propanediol (0.767 mL) and a 10 mM dimethyl sulfoxide solution of compound (3-14) obtained in Step 13 of Example 2-3 (0.033 mL; 12 equivalents per antibody molecule) were added at room temperature, and the resultant was reacted using a tube rotator (MTR-103, AS ONE Corporation) at room temperature for 48 hours.

Purification operation: The solution was purified by using common operation D described later to afford 7.00 mL of a solution of the desired compound.
Characterization: The following characteristic values were obtained by using common operations E and F.
Antibody concentration: 0.48 mg/mL, antibody yield: 3.39 mg (85%), average number of conjugated drug molecules per antibody molecule (n): 1.7

Example 10: ADC2

As shown in the following reaction formula, ADC2 was synthesized by conjugating the antibody obtained in Step 2 of Example 7 and drug-linker 1 (3-14) obtained in Step 13 of Example 2-3. In the formula, R represents the drug-linker used in Examples.

[Formula 76]

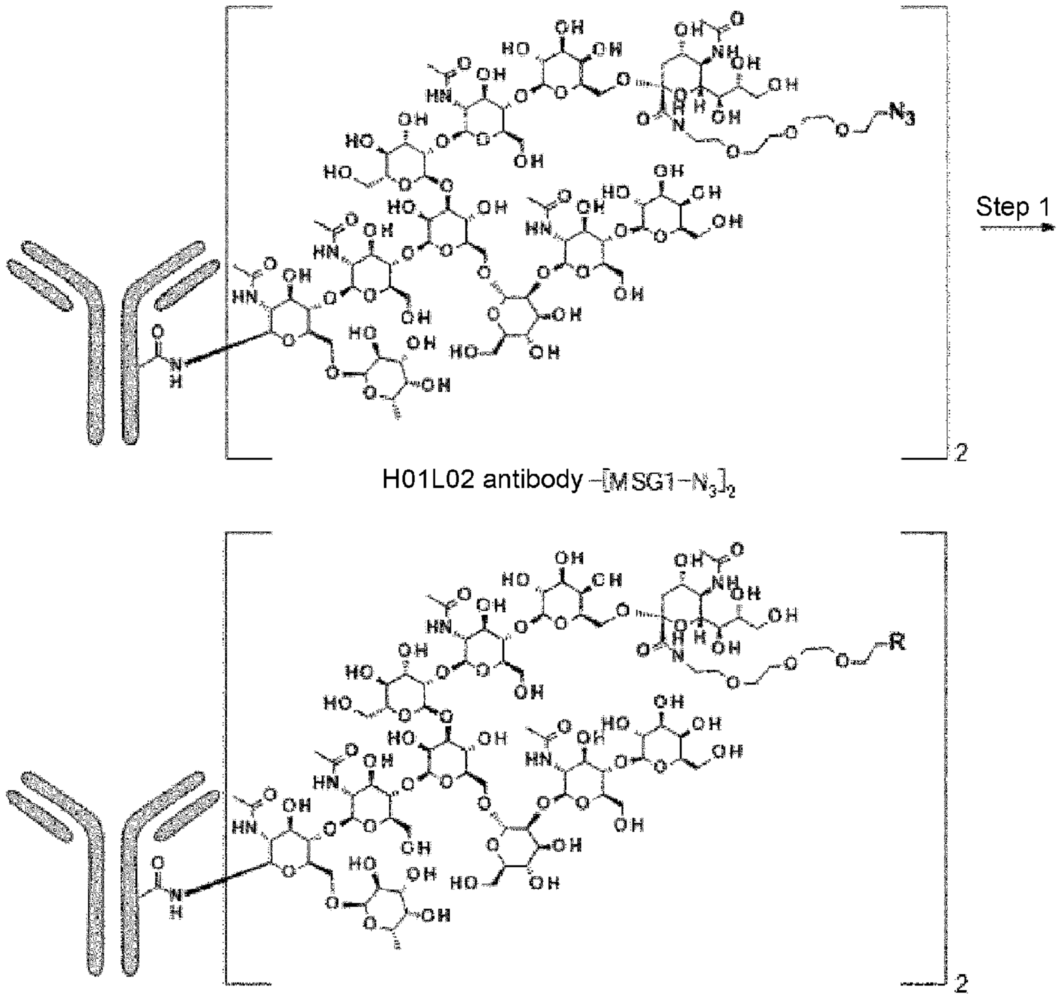


H01L02 antibody -[MSG1-$N_3$]$_2$

[Formula 77]

R = 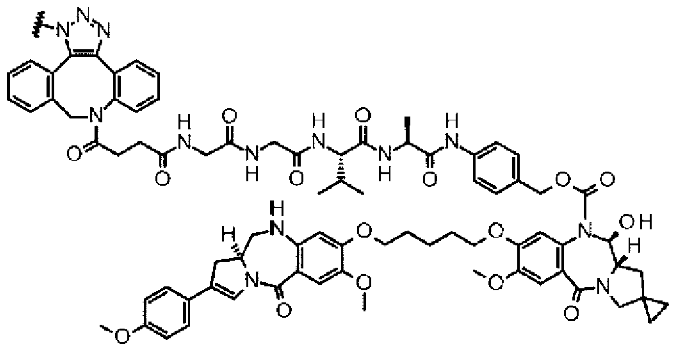

or

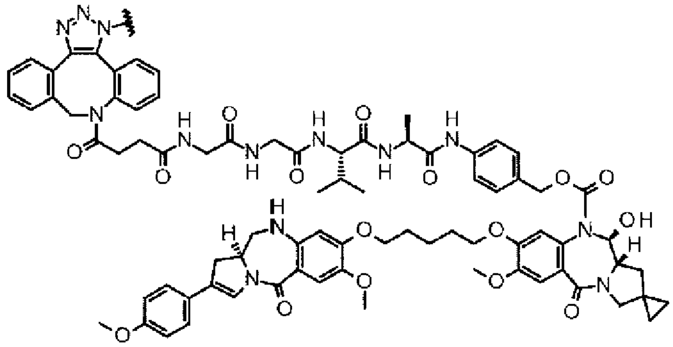

(In the compound obtained in Step 1 of Example 10, the triazole ring has geometric isomers, and the compound has a drug-linker as a mixture of the two structures shown above as R).

Step 1: Conjugation of antibody and drug-linker

To a phosphate buffered saline (pH 6.0) solution of the antibody (10.0 mg/mL, 400 μL) obtained in Step 2 of Example 7, 1,2-propanediol (200 μL) and a mixed solution of a 10 mM N,N-dimethylformamide solution of compound (3-14) obtained in Step 13 of Example 2-3 (33.1 μL; 12 equivalents per antibody molecule) and 1,2-propanediol (167 μL) were added at room temperature, and the resultant was reacted using a tube rotator (MTR-103, AS ONE Corporation) at room temperature for 48 hours.

Purification operation: The solution was purified by using common operation D to afford 2.5 mL of a solution of the desired compound.
Characterization: The following characteristic values were obtained by using common operations E and F.
Antibody concentration: 1.08 mg/mL, antibody yield: 2.71 mg (68%), average number of conjugated drug molecules per antibody molecule (n): 1.9

Example 11: ADC3

As shown in the following reaction formula, ADC3 was synthesized by conjugating the antibody obtained in Step 2 of Example 8 and drug-linker 1 (3-14) obtained in Step 13 of Example 3. In the formula, R represents the drug-linker used in Examples.

[Formula 78]

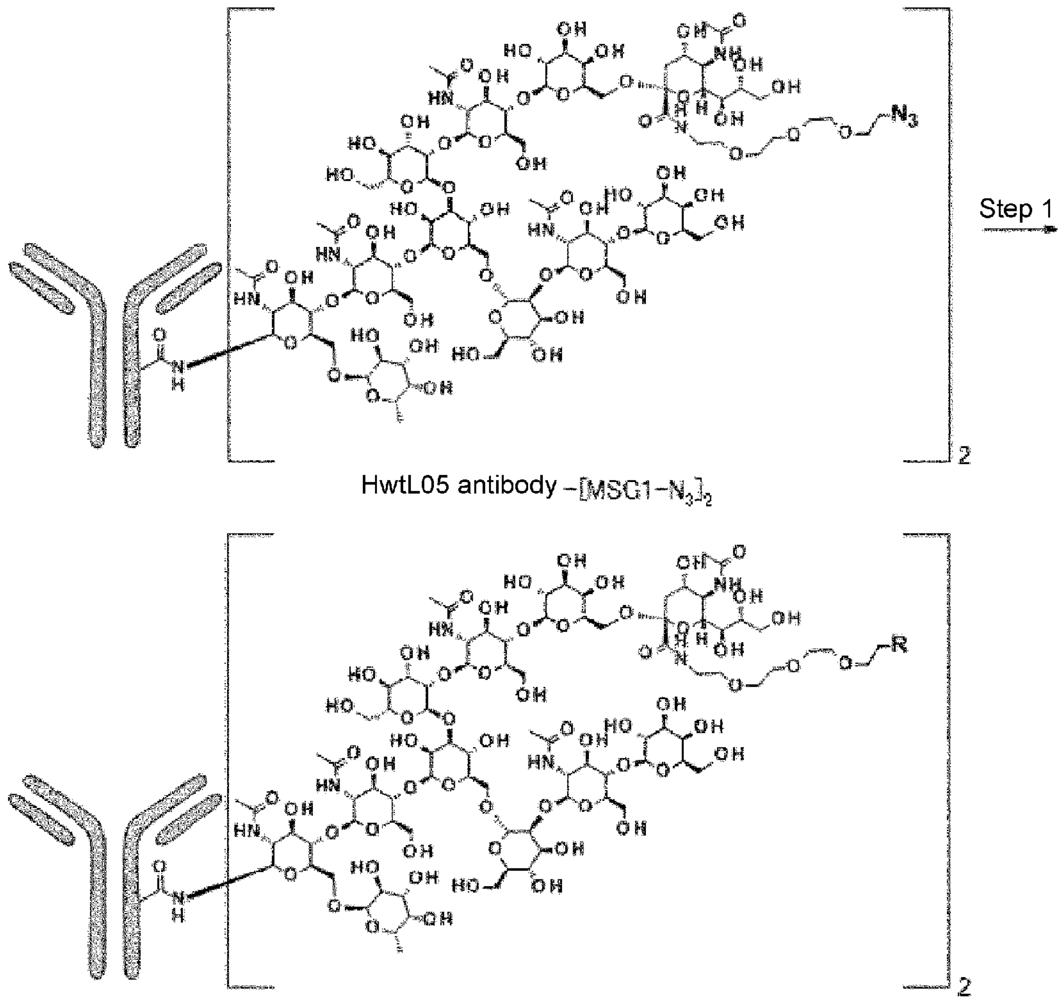


HwtL05 antibody $-[MSG1-N_3]_2$

[Formula 79]

R =

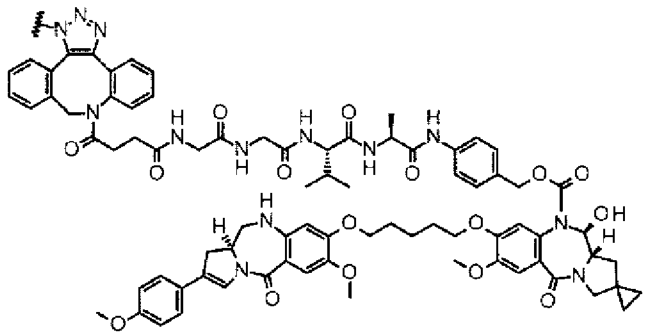

or

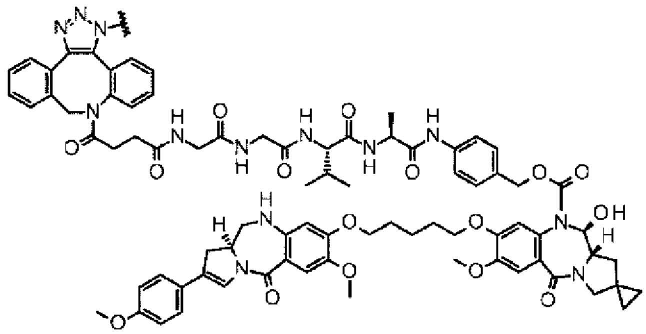

(In the compound obtained in Step 1 of Example 11, the triazole ring has geometric isomers, and the compound has a drug-linker as a mixture of the two structures shown above as R).

Step 1: Conjugation of antibody and drug-linker

To a phosphate buffered saline (pH 6.0) solution of the antibody (10.04 mg/mL, 0.400 mL) obtained in Step 2 of Example 8, 1,2-propanediol (0.367 mL) and a 10 mM dimethyl sulfoxide solution of compound (3-14) obtained in Step 13 of Example 2-3 (0.033 mL; 12 equivalents per antibody molecule) were added at room temperature, and the resultant was reacted using a tube rotator (MTR-103, AS ONE Corporation) at room temperature for 48 hours.

Purification operation: The solution was purified by using common operation D to afford 2.50 mL of a solution of the desired compound.
Characterization: The following characteristic values were obtained by using common operations E and F.
Antibody concentration: 0.89 mg/mL, antibody yield: 2.22 mg (55%), average number of conjugated drug molecules per antibody molecule (n): 1.8

Example 12: ADC4 and ADC5

ADC4 was synthesized by conjugating Trastuzumab A2 antibody-[MSG1-$N_3$]$_2$ obtained in Step 2 of Example 6 and drug-linker 1 (3-14) obtained in Example 2-3. In the formula, R represents the drug-linker used in Examples.

[Formula 80]

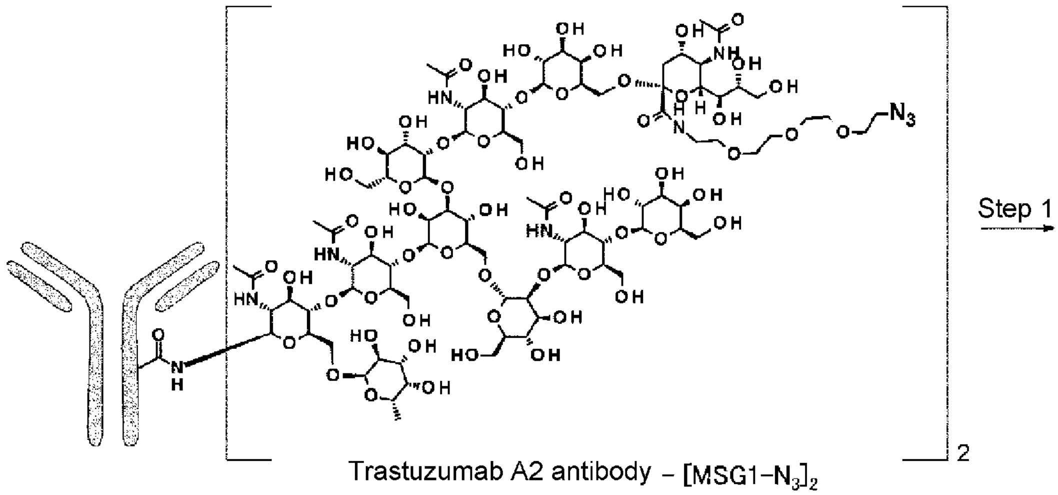


Trastuzumab A2 antibody - $[MSG1\text{-}N_3]_2$

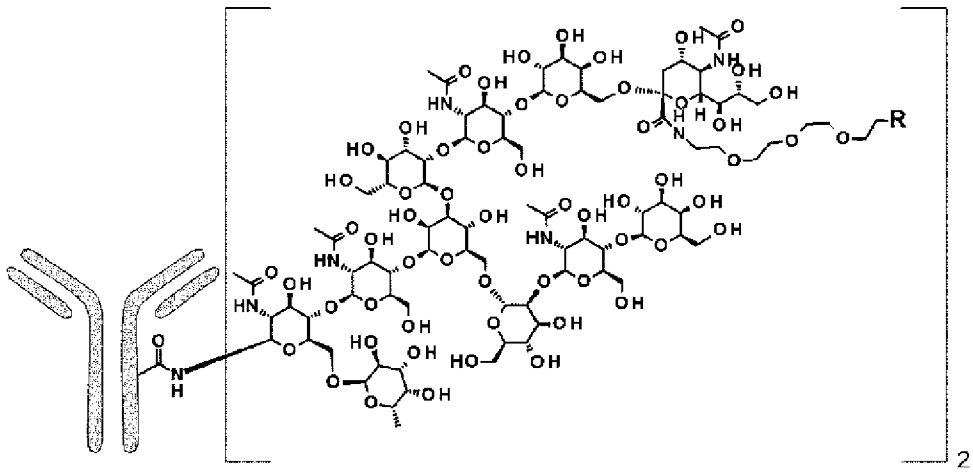

[Formula 81]

R =

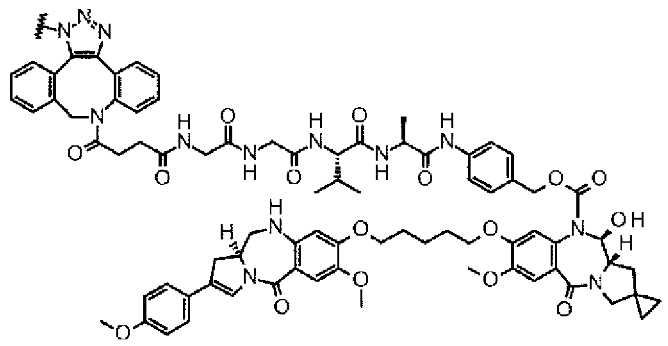

or

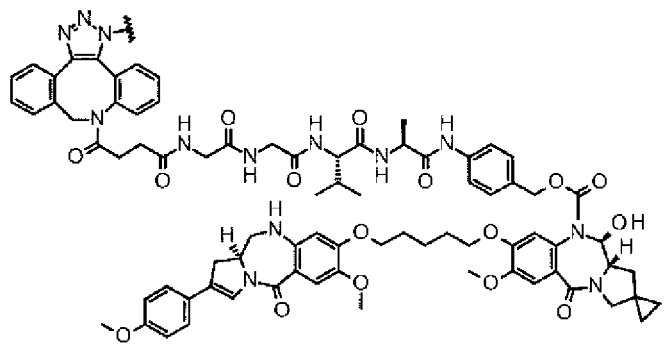

(In the compound obtained in Step 1 of Example 12, the triazole ring has geometric isomers, and the compound has a drug-linker as a mixture of the two structures shown above as R).

Step 1-1: Conjugation of antibody and drug-linker (ADC4)

To a phosphate buffered saline (pH 6.0) solution of Trastuzumab A2 antibody-[MSG1-$N_3$]$_2$ (heavy chain amino acid sequence: SEQ ID NO: 31, light chain amino acid sequence: SEQ ID NO: 32) (10.0 mg/mL, 0.50 mL) obtained in Step 2 of Example 6, 1,2-propanediol (0.486 mL) and a 10 mM dimethyl sulfoxide solution of compound (3-14) obtained in Step 13 of Example 2-3 (0.014 mL; 4 equivalents per antibody molecule) were added at room temperature, and the resultant was reacted using a tube rotator (MTR-103, AS ONE Corporation) at room temperature for 40 hours. Purification operation: The solution was purified by using common operation D described later to afford 2.50 mL of a solution of the desired compound. Characterization: The following characteristic values were obtained by using common operations E and F.
Antibody concentration: 1.12 mg/mL, antibody yield: 2.80 mg (56%), average number of conjugated drug molecules per antibody molecule (n): 1.8

Step 1-2: Conjugation of antibody and drug-linker (ADC5)

To a phosphate buffered saline (pH 6.0) solution of Trastuzumab A2 antibody-[MSG1-$N_3$]$_2$ (heavy chain amino acid sequence: SEQ ID NO: 31, light chain amino acid sequence: SEQ ID NO: 32) (10.0 mg/mL, 0.50 mL) obtained in Step 2 of Example 6, 1,2-propanediol (0.486 mL) and a 10 mM dimethyl sulfoxide solution of compound (3-14) obtained in Step 13 of Example 2-3 (0.014 mL; 12 equivalents per antibody molecule) were added at room temperature, and the resultant was reacted using a tube rotator (MTR-103, AS ONE Corporation) at room temperature for 40 hours. Purification operation: The solution was purified by using common operation D described later to afford 2.50 mL of a solution of the desired compound. Characterization: The following characteristic values were obtained by using common operations E and F.
Antibody concentration: 1.13 mg/mL, antibody yield: 2.82 mg (56%), average number of conjugated drug molecules per antibody molecule (n): 1.8

Example 13: Cell growth inhibition test for antibody-drug conjugate (1)

KPL-4 cells (Dr. Junichi Kurebayashi, Kawasaki Medical School, British Journal of Cancer, (1999)79(5/6). 707-717), a human breast cancer cell line of HER2 antigen-positive cells, were prepared with RPMI1640 Medium (Thermo Fisher Scientific; hereinafter, referred to as RPMI medium) containing 10% fetal bovine serum (Hyclone) to reach 6.25 $\times 10^3$ cells/mL, and 80 μL portions of them were added to a 96-well cell culture microplate. After addition of the cells, the cells were cultured at 37°C and 5% $CO_2$ overnight.

On the next day, 20 μL portions of the anti-HER2 antibody-drug conjugate ADC1, ADC2, or ADC3 diluted with RPMI medium to 100 nM, 20 nM, 4 nM, 0.8 nM, 0.16 nM, 0.032 nM, 6.4 pM, 1.3 pM, and 0.26 pM were added to the microplate. To each well without any antibody-drug conjugate, 20 μL of RPMI medium was added. KPL-4 was cultured at 37°C and 5% $CO_2$ for 6 days. After culturing, the microplate was taken out of the incubator, and left to stand at room temperature for 30 minutes. CellTiter-Glo Luminescent Cell Viability Assay (Promega Corporation) in an amount equivalent to that of the culture solution was added, and stirred using a plate mixer. The microplate was left to stand at room temperature for 10 minutes, and thereafter the amount of emission was measured by using a plate reader (PerkinElmer).

Cell survival rates were calculated by using the following formula.

$$\text{Cell survival rate (\%)} = a \div b \times 100$$

a: Mean value of amounts of emission from wells with test substance
b: Mean value of amounts of emission from wells with medium

$IC_{50}$ values were calculated by using the following formula.

$$IC_{50}\ (\text{nM}) = \text{antilog}((50\text{-}d) \times (LOG_{10}(b)\text{-}LOG_{10}(a)) \div (d\text{-}c) + LOG_{10}(b))$$

a: Concentration of test substance, a
b: Concentration of test substance, b
c: Cell survival rate when test substance of concentration a was added
d: Cell survival rate when test substance of concentration b was added

a and b satisfy a > b at points sandwiching a cell survival rate of 50%.

The antibody-drug conjugates ADC1 and ADC2 each exhibited an anticellular effect of $0.001 < IC_{50} < 0.01$ (nM) and ADC3 exhibited an anticellular effect of $0.01 < IC_{50} < 0.1$ (nM) on the KPL-4 cells.

Example 14: Cell growth inhibition test for antibody-drug conjugate (2)

JIMT-1 cells (Deutsche Sammlung von Mikroorganismen und Zellkulturen GmbH; DSMZ ACC 589), a human breast cancer cell line of HER2 antigen-positive cells, were prepared with DMEM Medium (Thermo Fisher Scientific; hereinafter, referred to a DMEM medium) containing 10% fetal bovine serum (Hyclone) to reach $1.3 \times 10^4$ cells/mL, and 80 μL portions of them were added to a 96-well cell culture microplate. After addition of the cells, the cells were cultured at 37°C and 5% $CO_2$ overnight.

On the next day, 20 μL portions of the anti-HER2 antibody-drug conjugate ADC1, ADC2, or ADC3 diluted with DMEM medium to 100 nM, 20 nM, 4 nM, 0.8 nM, 0.16 nM, 0.032 nM, 6.4 pM, 1.3 pM, and 0.26 pM were added to the microplate. To each well without any antibody-drug conjugate, 20 μL of DMEM medium was added. JIMT-1 was cultured at 37°C and 5% $CO_2$ for 6 days. After culturing, the microplate was taken out of the incubator, and left to stand at room temperature for 30 minutes. CellTiter-Glo Luminescent Cell Viability Assay (Promega Corporation) in an amount equivalent to that of the culture solution was added, and stirred using a plate mixer. The microplate was left to stand at room temperature for 10 minutes, and thereafter the amount of emission was measured by using a plate reader (PerkinElmer). Cell survival rates were calculated by using the same formula as in Example 13.

The antibody-drug conjugates ADC1 and ADC2 each exhibited an anticellular effect of $0.1 < IC_{50} < 1$ (nM) and ADC3 exhibited an anticellular effect of $1 < IC_{50} < 10$ (nM) on the JIMT-1 cells.

Example 15: Cell growth inhibition test for antibody-drug conjugate (3)

KPL-4 cells (Dr. Junichi Kurebayashi, Kawasaki Medical School, British Journal of Cancer, (1999)79(5/6). 707-717), a human breast cancer cell line of HER2 antigen-positive cells, were prepared with RPMI1640 Medium (Thermo

Fisher Scientific; hereinafter, referred to as RPMI medium) containing 10% fetal bovine serum (Hyclone) to reach 6.25 $\times 10^3$ cells/mL, and 80 μL portions of them were added to a 96-well cell culture microplate. After addition of the cells, the cells were cultured at 37°C and 5% $CO_2$ overnight.

On the next day, 20 μL portions of the anti-HER2 antibody-drug conjugate ADC4 diluted with RPMI medium to 100 nM, 20 nM, 4 nM, 0.8 nM, 0.16 nM, 0.032 nM, 6.4 pM, 1.3 pM, and 0.26 pM were added to the microplate. To each well without any antibody-drug conjugate, 20 μL of RPMI medium was added. KPL-4 was cultured at 37°C and 5% $CO_2$ for 6 days. After culturing, the microplate was taken out of the incubator, and left to stand at room temperature for 30 minutes. CellTiter-Glo Luminescent Cell Viability Assay (Promega Corporation) in an amount equivalent to that of the culture solution was added, and stirred using a plate mixer. The microplate was left to stand at room temperature for 10 minutes, and thereafter the amount of emission was measured by using a plate reader (PerkinElmer). Cell survival rates were calculated by using the same formula as in Example 13.

The antibody-drug conjugate ADC4 exhibited an anticellular effect of $0.001 < IC_{50} < 0.01$ (nM) on the KPL-4 cells.

Example 16: Antitumor test for antibody-drug conjugate (1)

Mouse: Four- to five-week-old female BALB/c nude mice (Charles River Laboratories Japan, Inc.) were habituated under SPF conditions for 4 to 7 days before being used for experiment. To the mice, sterilized pellets (FR-2, Funabashi Farms Co., Ltd.) were fed and sterilized tap water (prepared by adding 5 to 15 ppm sodium hypochlorite solution) was provided.

Assay and calculation formula: In all of the studies, the major axis and minor axis of a tumor were measured twice or three times a week by using an electronic digital caliper (CD-15CX, Mitutoyo Corp.), and the tumor volume ($mm^3$) was calculated. The calculation formula is as shown below.

$$\text{Tumor volume (mm}^3\text{)} = \text{Major axis (mm)} \times [\text{Minor axis (mm)}]^2 \times 1/2$$

Each of the antibody-drug conjugates and antibodies was diluted with 10 mM acetate buffer, 5% sorbitol, pH 5.5 (NACALAI TESQUE, INC.; ABS buffer), and a liquid volume of 10 mL/kg was intravenously administered into the tail vein. As a control group (Vehicle group), ABS buffer was administered in the same manner.

KPL-4 cells (Dr. Junichi Kurebayashi, Kawasaki Medical School, British Journal of Cancer, (1999) 79(5/6). 707-717) were suspended in Dulbecco's phosphate buffered saline (Sigma-Aldrich Co. LLC), and $1.5 \times 10^7$ cells were subcutaneously transplanted to the right flank of each female nude mouse (Day 0), and the mice were randomly grouped on Day 14. The anti-HER2 antibody-drug conjugate ADC1 or ADC2 was intravenously administered into the tail vein on Day 14 at a dose of 0.33 mg/kg. As a control group (Vehicle group), ABS buffer was administered in the same manner.

Figure 18 shows the results. The anti-HER2 antibody-drug conjugates ADC1 and ADC2 exhibited tumor growth-suppressing effect at a dose of 0.33 mg/kg. Intensity of tumor growth-suppressing effect was in the order of ADC2 and ADC1. No weight loss caused by administration of 0.33 mg/kg of any of the anti-HER2 antibody-drug conjugates ADC1 and ADC2 was found for the mice.

Example 17: Antitumor test for antibody-drug conjugate (2)

JIMT-1 cells (DSMZ ACC 589) were suspended in physiological saline (Otsuka Pharmaceutical Factory, Inc.), and $5 \times 10^6$ cells were subcutaneously transplanted to the right flank of each female nude mouse (Day 0), and the mice were randomly grouped on Day 11. The anti-HER2 antibody-drug conjugate ADC1 or ADC2 was administered into the tail vein on Day 11 at a dose of 0.4 mg/kg. As a control group (Vehicle group), ABS buffer was administered in the same manner.

Figure 19 shows the results. The anti-HER2 antibody-drug conjugates ADC1 and ADC2 were each found to have strong antitumor effect causing regression of tumor at a dose of 0.4 mg/kg. No weight loss caused by administration of any of the anti-HER2 antibody-drug conjugate ADC1 or ADC2 was found for the mice at any dose.

Example 18: Antitumor test for antibody-drug conjugate (3)

CFPAC-1 cells (American Type Culture Collection; ATCC CRL-1918) were suspended in physiological saline (Otsuka Pharmaceutical Factory, Inc.), and $5 \times 10^6$ cells were subcutaneously transplanted to the right flank of each female nude mouse (Day 0), and the mice were randomly grouped on Day 10. The anti-HER2 antibody-drug conjugate ADC1 or ADC2 was intravenously administered into the tail vein on Day 10 at a dose of 0.4 mg/kg. As a control group (Vehicle group), ABS buffer was administered in the same manner.

Figure 20 shows the results. Strong antitumor effect causing regression of tumor was found for the mice to which the anti-HER2 antibody-drug conjugate ADC1 had been administered. Tumor growth-suppressing effect was found for the mice to which the anti-HER2 antibody-drug conjugate ADC2 had been administered. No weight loss caused by administration of any of anti-HER2 antibody-drug conjugates ADC1 and ADC2 was found for the mice.

Example 19: Antitumor test for antibody-drug conjugate (4)

KPL-4 cells (Dr. Junichi Kurebayashi, Kawasaki Medical School, British Journal of Cancer, (1999) 79(5/6). 707-717) were suspended in physiological saline (Otsuka Pharmaceutical Factory, Inc.), and $1.5 \times 10^7$ cells were subcutaneously transplanted to the right flank of each female nude mouse (Day 0), and the mice were randomly grouped on Day 14. The antibody-drug conjugate ADC5 was intravenously administered into the tail vein on Day 14 at a dose of 0.4 mg/kg. As a control group (Vehicle group), ABS buffer was administered in the same manner.

Figure 23 shows the results. The antibody-drug conjugate ADC5 was found to have strong antitumor effect causing regression of tumor at a dose of 0.4 mg/kg. No weight loss caused by administration of the antibody-drug conjugate ADC5 was found for the mice at a dose of 0.4 mg/kg.

Industrial Applicability

Use of the anti-HER2 antibody-drug conjugate, anti-HER2 antibody and/or PBD derivative, and so on of the present invention enables treatment or prevention of various cancers.

Free Text of Sequence Listing

SEQ ID NO: 1 - Amino acid sequence of CDRH1 of heavy chain of Trastuzumab A1 and A2 and HwtL05 antibody heavy chain
SEQ ID NO: 2 - Amino acid sequence of CDRH2 of heavy chain of Trastuzumab A1 and A2 and HwtL05 antibody heavy chain
SEQ ID NO: 3 - Amino acid sequence of CDRH3 of heavy chain of Trastuzumab A1 and A2 and HwtL05 antibody heavy chain
SEQ ID NO: 4 - Amino acid sequence of CDRH3 of H01L02 antibody heavy chain SEQ ID NO: 5 - Amino acid sequence of CDRL1 of light chain of Trastuzumab A1 and A2
SEQ ID NO: 6 - Amino acid sequence including CDRL2 of light chain of Trastuzumab A1 and A2
SEQ ID NO: 7 - Amino acid sequence of CDRL3 of light chain of Trastuzumab A1 and A2
SEQ ID NO: 8 - Amino acid sequence of CDRL3 of H01L02 antibody and HwtL05 antibody light chain
SEQ ID NO: 9 - Nucleotide sequence of DNA fragment including DNA sequence encoding human light chain signal sequence and human κ chain constant region
SEQ ID NO: 10 - Nucleotide sequence of DNA fragment including DNA sequence encoding amino acids of human heavy chain signal sequence and human IgG1LALA constant region
SEQ ID NO: 11 - Heavy chain amino acid sequence of Trastuzumab A1
SEQ ID NO: 12 - Nucleotide sequence encoding heavy chain of Trastuzumab A1
SEQ ID NO: 13 - Heavy chain variable region amino acid sequence of Trastuzumab A1, A2, and Hwt
SEQ ID NO: 14 - Nucleotide sequence encoding Trastuzumab A1, A2, and Hwt
SEQ ID NO: 15 - Amino acid sequence of H01
SEQ ID NO: 16 - Nucleotide sequence encoding H01
SEQ ID NO: 17 - Heavy chain variable region amino acid sequence of H01
SEQ ID NO: 18 - Nucleotide sequence encoding heavy chain variable region of H01
SEQ ID NO: 19 - Amino acid sequence of light chain of Trastuzumab A1
SEQ ID NO: 20 - Nucleotide sequence encoding light chain of Trastuzumab A1
SEQ ID NO: 21 - Light chain variable region amino acid sequence of Trastuzumab A1 and A2
SEQ ID NO: 22 - Nucleotide sequence encoding light chain variable region of Trastuzumab A1 and A2
SEQ ID NO: 23 - Amino acid sequence of L02
SEQ ID NO: 24 - Polynucleotide sequence encoding L02
SEQ ID NO: 25 - Variable region amino acid sequence of L02
SEQ ID NO: 26 - Nucleotide sequence encoding variable region of L02
SEQ ID NO: 27 - Amino acid sequence of L05
SEQ ID NO: 28 - Polynucleotide sequence encoding L05

SEQ ID NO: 29 - Variable region amino acid sequence of L05
SEQ ID NO: 30 - Nucleotide sequence encoding variable region of L05
SEQ ID NO: 31 - Heavy chain amino acid sequence of Trastuzumab A2
SEQ ID NO: 32 - Amino acid sequence of light chain of Trastuzumab A2
SEQ ID NO: 33 - Heavy chain amino acid sequence of Trastuzumab
SEQ ID NO: 34 - Amino acid sequence of light chain of Trastuzumab

## Claims

1. An antibody-drug conjugate represented by the following formula:

[Formula 1]

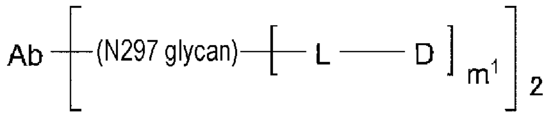

wherein

$m^1$ represents an integer of 1 or 2;
D is any one selected from the following group:

[Formula 2]

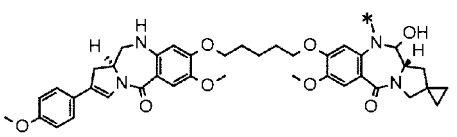
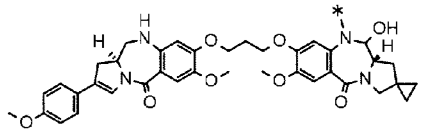
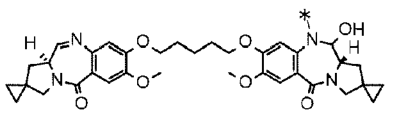
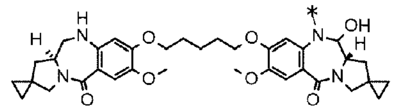

wherein

each asterisk * represents bonding to L;
L is a linker linking the glycan bonding to Asn297 of Ab (N297 glycan) and D; the N297 glycan is optionally remodeled; and
Ab represents an antibody or a functional fragment of the antibody, wherein the antibody specifically binds to HER2 and comprises a heavy chain comprising CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 3 or an amino acid sequence having one to several amino acid substitutions in the amino acid sequence represented by SEQ ID NO: 3, and a light chain comprising CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 7 or an amino acid sequence having one to several amino acid substitutions in the amino acid sequence represented by SEQ ID NO: 7.

2. The antibody-drug conjugate according to claim 1, wherein

L is represented by $-Lb-La-Lp-NH-B-CH_2-O(C=O)-*$, the asterisk * representing bonding to D;
B is a 1,4-phenyl group, a 2,5-pyridyl group, a 3,6-pyridyl group, a 2,5-pyrimidyl group, or a 2,5-thienyl group;
Lp represents any one selected from the following group:

-GGVA-, -GG-(D-)VA-, -VA-, -GGFG-, -GGPI-, -GGVCit-, -GGVK-, and - GGPL-;
La represents any one selected from the following group:

$-C(=O)-CH_2CH_2-C(=O)-$, $-C(=O)-(CH_2CH_2)_2-C(=O)-$,
$-C(=O)-CH_2CH_2-C(=O)-NH-(CH_2CH_2)_2-C(=O)-$,
$-C(=O)-CH_2CH_2-C(=O)-NH-(CH_2CH_2O)_2-CH_2-C(=O)-$,
$-C(=O)-CH_2CH_2-NH-C(=O)-(CH_2CH_2O)_4-CH_2CH_2-C(=O)-$, $-CH_2-OC(=O)-$, and $-OC(=O)-$; and

Lb is represented by the following formula:

[Formula 3]

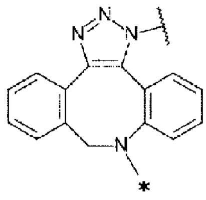

or

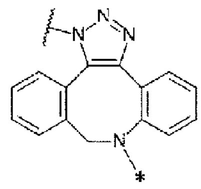

[Formula 4]

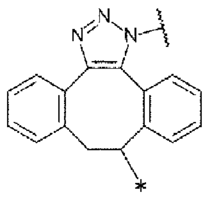

or

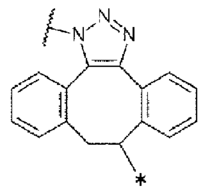

, or

[Formula 5]

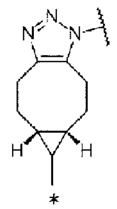

or

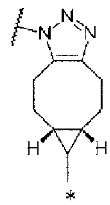

wherein, in each structural formula for Lb shown above,
each asterisk * represents bonding to La, and each wavy line represents bonding to N297 glycan or remodeled N297 glycan.

**3.** The antibody-drug conjugate according to claim 1 or 2, wherein
L represents any one selected from the following group:

$-Z^1-C(=O)-CH_2CH_2-C(=O)-GGVA-NH-B-CH_2-OC(=O)-$,
$-Z^1-C(=O)-CH_2CH_2-C(=O)-GG-(D-)VA-NH-B-CH_2-OC(=O)-$,
$-Z^1-C(=O)-CH_2CH_2-C(=O)-VA-NH-B-CH_2-OC(=O)-$,
$-Z^1-C(=O)-(CH_2CH_2)_2-C(=O)-VA-NH-B-CH_2-OC(=O)-$,
$-Z^1-C(=O)-CH_2CH_2-C(=O)-GGPI-NH-B-CH_2-OC(=O)-$,
$-Z^1-C(=O)-CH_2CH_2-C(=O)-GGFG-NH-B-CH_2-OC(=O)-$,
$-Z^1-C(=O)-CH_2CH_2-C(=O)-GGVCit-NH-B-CH_2-OC(=O)-$,
$-Z^1-C(=O)-CH_2CH_2-C(=O)-GGVK-NH-B-CH_2-OC(=O)-$,
$-Z^1-C(=O)-CH_2CH_2-C(=O)-GGPL-NH-B-CH_2-OC(=O)-$,
$-Z^1-C(=O)-CH_2CH_2-C(=O)-NH-(CH_2CH_2)_2-C(=O)-VA-NH-B-CH_2-OC(=O)-$,
$-Z^1-C(=O)-CH_2CH_2-C(=O)-NH-(CH_2CH_2O)_2-CH_2-C(=O)-VA-NH-B-CH_2-OC(=O)-$,
$-Z^1-C(=O)-CH_2CH_2-NH-C(=O)-(CH_2CH_2O)_4-CH_2CH_2-C(=O)-VA-NH-B-CH_2-OC(=O)-$,
$-Z^2-OC(=O)-GGVA-NH-B-CH_2-OC(=O)-$, and
$-Z^3-CH_2-OC(=O)-GGVA-NH-B-CH_2-OC(=O)-$, wherein
B represents a 1,4-phenyl group,
$Z^1$ represents the following structural formula:

[Formula 6]

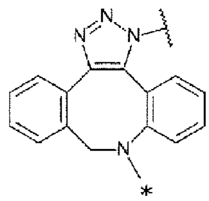

or

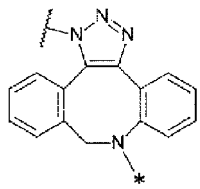

$Z^2$ represents the following structural formula:

[Formula 7]

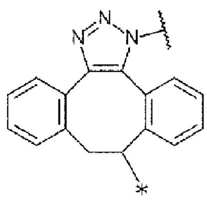

or

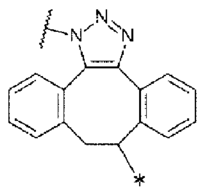

$Z^3$ represents the following structural formula:

[Formula 8]

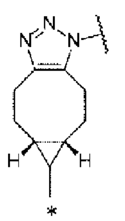

or

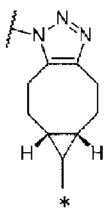

wherein, in each structural formula for $Z^1$, $Z^2$, and $Z^3$,
each asterisk * represents bonding to neighboring C(=O), OC(=O), or $CH_2$, and each wavy line represents bonding to N297 glycan or remodeled N297 glycan.

**4.** The antibody-drug conjugate according to claim 3, wherein
L represents any one selected from the following group:

$-Z^1-C(=O)-CH_2CH_2-C(=O)-GGVA-NH-B-CH_2-OC(=O)-$,
$-Z^1-C(=O)-CH_2CH_2-C(=O)-VA-NH-B-CH_2-OC(=O)-$,
$-Z^1-C(=O)-(CH_2CH_2)_2-C(=O)-VA-NH-B-CH_2-OC(=O)-$,
$-Z^1-C(=O)-CH_2CH_2-C(=O)-GGVCit-NH-B-CH_2-OC(=O)-$,
$-Z^1-C(=O)-CH_2CH_2-C(=O)-NH-(CH_2CH_2)_2-C(=O)-VA-NH-B-CH_2-OC(=O)-$,
$-Z^1-C(=O)-CH_2CH_2-C(=O)-NH-(CH_2CH_2O)_2-CH_2-C(=O)-VA-NH-B-CH_2-OC(=O)-$, and
$-Z^1-C(=O)-CH_2CH_2-NH-C(=O)-(CH_2CH_2O)_4-CH_2CH_2-C(=O)-VA-NH-B-CH_2-OC(=O)-$, wherein
B is a 1,4-phenyl group,
$Z^1$ represents the following structural formula:

[Formula 9]

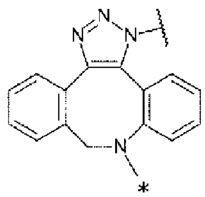

or

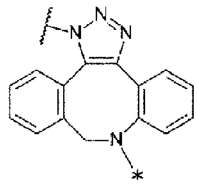

wherein, in the structural formula for $Z^1$, each asterisk * represents bonding to C(=O) neighboring to $Z^1$, and each wavy line represents bonding to N297 glycan or remodeled N297 glycan.

**5.** The antibody-drug conjugate according to any one of claims 1 to 4, wherein D is any selected from the following group:

[Formula 10]

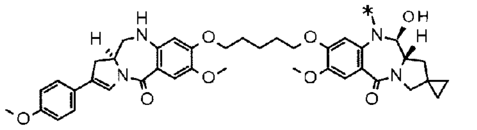

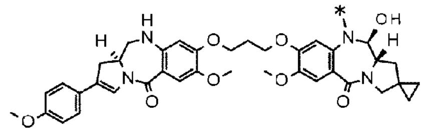

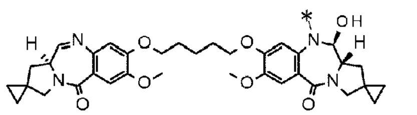

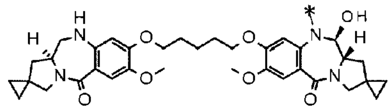

wherein
each asterisk * represents bonding to L.

**6.** The antibody-drug conjugate according to any one of claims 1 to 5, wherein the antibody comprises a heavy chain comprising CDRH1, CDRH2, and CDRH3 and a light chain comprising CDRL1, CDRL2, and CDRL3 as described in any of the following (a) to (c):

(a) CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 4, and CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 8;
(b) CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 3, and CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 8; and
(c) CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 3, and CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 7.

**7.** The antibody-drug conjugate according to any one of claims 1 to 6, wherein the antibody comprises a heavy chain variable region consisting of an amino acid sequence selected from the group consisting of the following (a) to (d) and a light chain variable region consisting of an amino acid sequence selected from the group consisting of the following (e) to (i):

(a) an amino acid sequence represented by SEQ ID NO: 13;
(b) an amino acid sequence represented by SEQ ID NO: 17;

(c) an amino acid sequence with a homology of at least 95% or higher to a sequence of a framework region excluding CDR sequences in any one of the sequences (a) and (b);
(d) an amino acid sequence having one to several amino acid deletions, substitutions, or additions in a sequence of a framework region excluding CDR sequences in any one of the sequences (a) and (b);
(e) an amino acid sequence represented by SEQ ID NO: 21;
(f) an amino acid sequence represented by SEQ ID NO: 25;
(g) an amino acid sequence represented by SEQ ID NO: 29;
(h) an amino acid sequence with a homology of at least 95% or higher to a sequence of a framework region excluding CDR sequences in any of the sequences (e) to (g); and
(i) an amino acid sequence having one to several amino acid deletions, substitutions, or additions in a sequence of a framework region excluding CDR sequences in any of the sequences (e) to (g).

**8.** The antibody-drug conjugate according to claim 7, wherein the antibody comprises a heavy chain variable region and a light chain variable region as described in any of the following (a) to (c):

(a) a heavy chain variable region consisting of an amino acid sequence represented by SEQ ID NO: 17 and a light chain variable region consisting of an amino acid sequence represented by SEQ ID NO: 25;
(b) a heavy chain variable region consisting of an amino acid sequence represented by SEQ ID NO: 13 and a light chain variable region consisting of an amino acid sequence represented by SEQ ID NO: 29; and
(c) a heavy chain variable region consisting of an amino acid sequence represented by SEQ ID NO: 13 and a light chain variable region consisting of an amino acid sequence represented by SEQ ID NO: 21.

**9.** The antibody-drug conjugate according to any one of claims 1 to 8, wherein the antibody is a chimeric antibody.

**10.** The antibody-drug conjugate according to any one of claims 1 to 8, wherein the antibody is a humanized antibody.

**11.** The antibody-drug conjugate according to claim 9 or 10, wherein the antibody comprises a heavy chain constant region of human IgG1, human IgG2, or human IgG4.

**12.** The antibody-drug conjugate according to claim 11, wherein the heavy chain constant region of the antibody is a heavy chain constant region of human IgG1, and leucine at the 234- and 235-positions specified by EU Index numbering in the heavy chain constant region is substituted with alanine.

**13.** The antibody-drug conjugate according to any one of claims 10 to 12, wherein the antibody comprises a heavy chain and a light chain as described in any one of the following (a) and (b):

(a) a heavy chain consisting of an amino acid sequence consisting of amino acid residues 20 to 469 of SEQ ID NO: 15 and a light chain consisting of an amino acid sequence consisting of amino acid residues 21 to 234 of SEQ ID NO: 23 (H01L02); and
(b) a heavy chain consisting of an amino acid sequence consisting of amino acid residues 20 to 469 of SEQ ID NO: 11 and a light chain consisting of an amino acid sequence consisting of amino acid residues 21 to 234 of SEQ ID NO: 27 (HwtL05).

**14.** The antibody-drug conjugate according to any one of claims 10 to 12, wherein the antibody comprises a heavy chain and a light chain as described in any one of the following (a) and (b):

(a) a heavy chain consisting of an amino acid sequence consisting of amino acid residues 20 to 469 of SEQ ID NO: 11 and a light chain consisting of an amino acid sequence consisting of amino acid residues 21 to 234 of SEQ ID NO: 19; and
(b) a heavy chain consisting of an amino acid sequence consisting of amino acid residues 20 to 469 of SEQ ID NO: 31 and a light chain consisting of an amino acid sequence consisting of amino acid residues 21 to 234 of SEQ ID NO: 32.

**15.** The antibody-drug conjugate according to any one of claims 1 to 14, wherein the antibody comprises one or two or more modifications selected from the group consisting of N-linked glycosylation, O-linked glycosylation, N-terminal processing, C-terminal processing, deamidation, isomerization of aspartic acid, oxidation of methionine, addition of a methionine residue at an N terminus, amidation of a proline residue, and deletion of one or two amino acid residues at the carboxyl terminus of a heavy chain.

**16.** The antibody-drug conjugate according to claim 15, wherein one or several amino acid residues are deleted at the carboxyl terminus of a heavy chain of the antibody.

**17.** The antibody-drug conjugate according to claim 16, wherein one amino acid residue is deleted at the carboxyl terminus of each of the two heavy chains of the antibody.

**18.** The antibody-drug conjugate according to any one of claims 1 to 5, wherein the antibody competes with the antibody according to any one of claims 6 to 17 for binding to HER2, or binds to a site of HER2 recognizable to the antibody according to any one of claims 6 to 17.

**19.** The antibody-drug conjugate according to any one of claims 1 to 18, wherein the N297 glycan is a remodeled glycan.

**20.** The antibody-drug conjugate according to any one of claims 1 to 19, wherein the N297 glycan is any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 11]

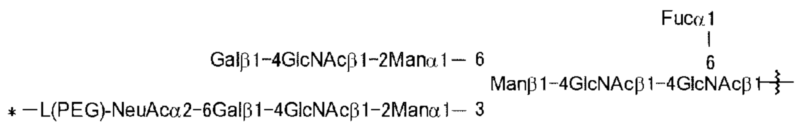

[N297-(Fuc)MSG1]

[Formula 12]

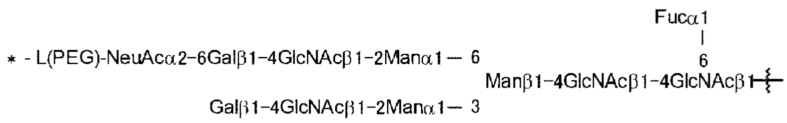

[N297-(Fuc)MSG2]

[Formula 13]

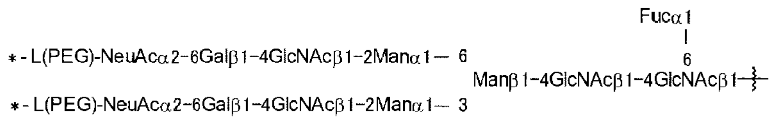

[N297-(Fuc)SG]

wherein

each wavy line represents bonding to Asn297 of the antibody,
L(PEG) in the N297 glycan represents $-NH-CH_2CH_2-(O-CH_2CH_2)n^5-*$, wherein $n^5$ represents an integer of 2 to 5, the amino group at the left end is bound via an amide bond to carboxylic acid at the 2-position of a sialic acid at the non-reducing terminal in each or either one of the 1-3 and 1-6 branched chains of β-Man in the N297 glycan, and the asterisk * represents bonding to a nitrogen atom at the 1- or 3-position of the triazole ring of $Z^1$ in L.

**21.** The antibody-drug conjugate according to claim 20, wherein $n^5$ is 3.

**22.** An antibody-drug conjugate represented by the following formula:

[Formula 14]

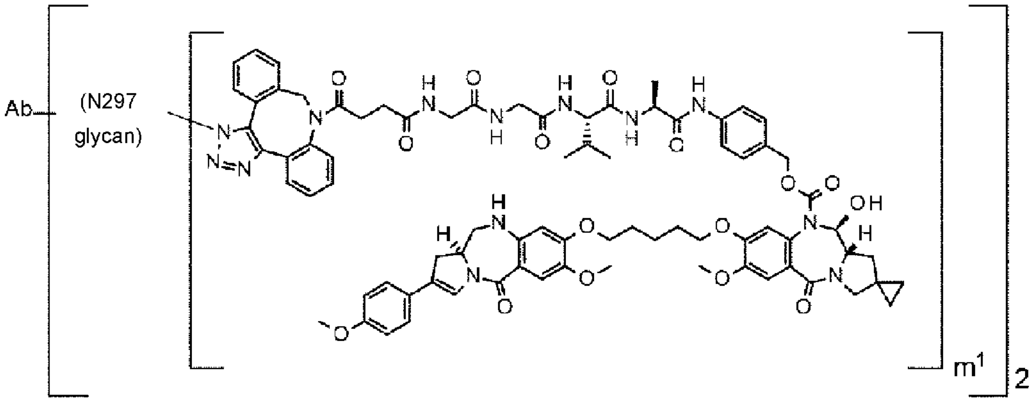

or

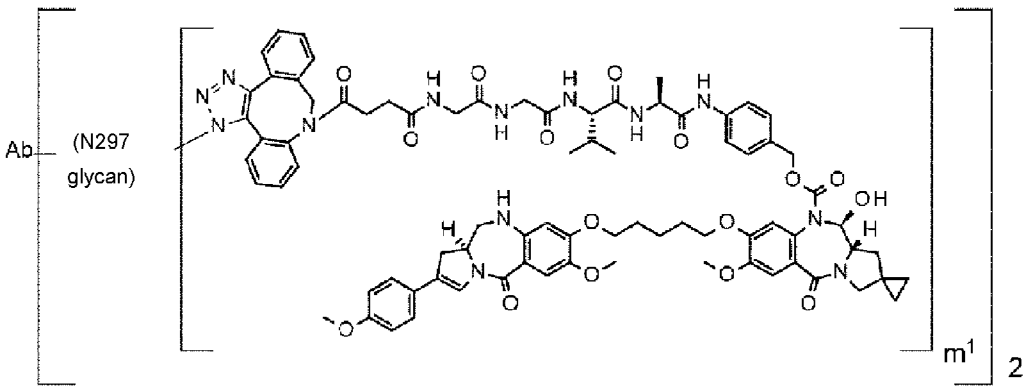

wherein, in each structural formula shown above,

$m^1$ represents an integer of 1 or 2;

Ab represents an antibody or a functional fragment of the antibody, wherein the antibody specifically binds to HER2 and comprises a heavy chain comprising CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 3 or an amino acid sequence having one to several amino acid substitutions in the amino acid sequence represented by SEQ ID NO: 3, and a light chain comprising CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 7 or an amino acid sequence having one to several amino acid substitutions in the amino acid sequence represented by SEQ ID NO: 7,

the N297 glycan is any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 15]

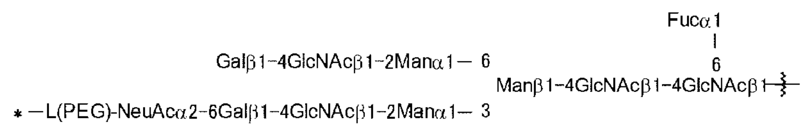

[N297-(Fuc)MSG1]

[Formula 16]

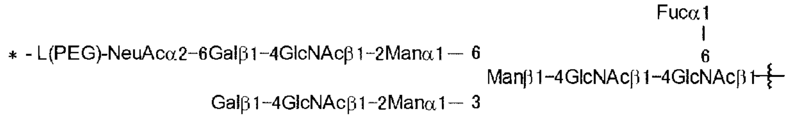

[N297-(Fuc)MSG2]

[Formula 17]

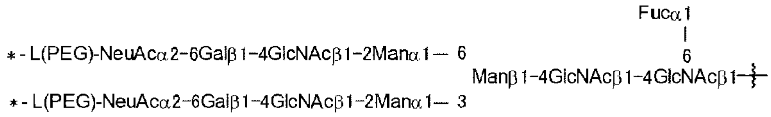

[N297-(Fuc)SG]

wherein

each wavy line represents bonding to Asn297 of the antibody,
L(PEG) in the N297 glycan represents -NH-$CH_2CH_2$-(O-$CH_2CH_2$)$_3$-*,
wherein the amino group at the left end is bound via an amide bond to carboxylic acid at the 2-position of a sialic acid at the non-reducing terminal of each or either one of the 1-3 and 1-6 branched chains of β-Man in the N297 glycan, and the asterisk * at the right end represents bonding to a nitrogen atom at the 1- or 3-position of the triazole ring in the corresponding structural formula.

**23.** An antibody-drug conjugate represented by the following formula:

[Formula 18]

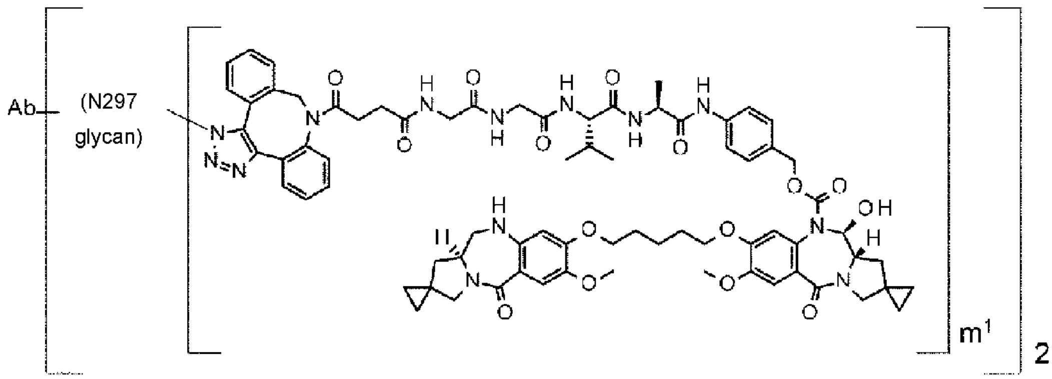

or

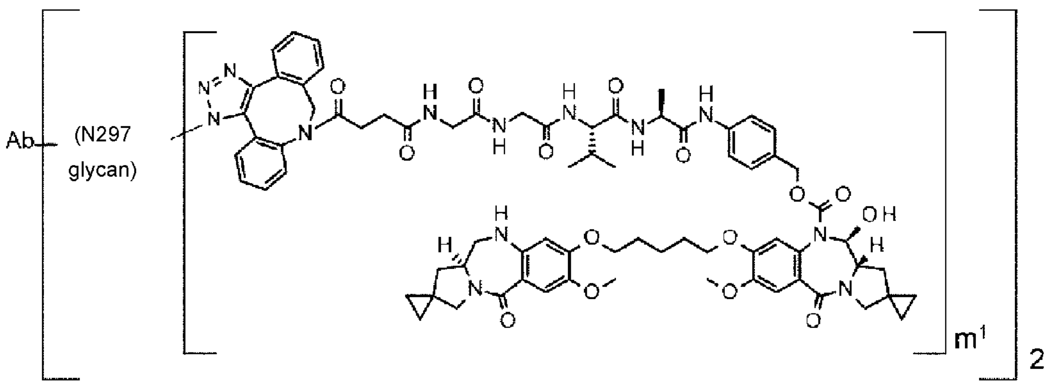

wherein, in each structural formula shown above,

$m^1$ represents an integer of 1 or 2;
Ab represents an antibody or a functional fragment of the antibody, wherein the antibody specifically binds to HER2 and comprises a heavy chain comprising CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 3 or an amino acid sequence having one to several amino acid substitutions in the amino acid sequence represented by SEQ ID NO: 3, and a light chain comprising CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 7 or an amino acid sequence having one to several amino acid substitutions in the amino acid sequence represented by SEQ ID NO: 7,
the N297 glycan is any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 19]

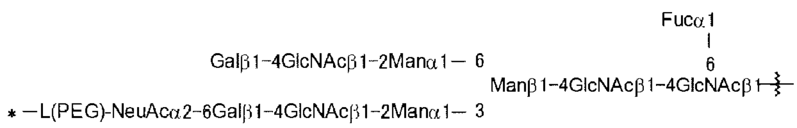

[N297-(Fuc)MSG1]

[Formula 20]

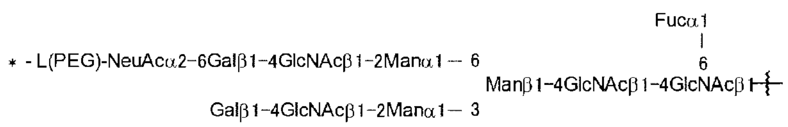

[N297-(Fuc)MSG2]

[Formula 21]

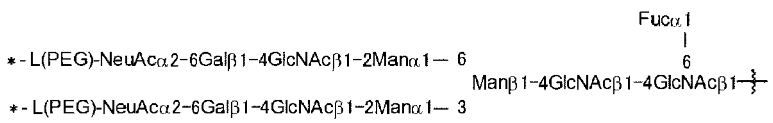

[N297-(Fuc)SG]

wherein

each wavy line represents bonding to Asn297 of the antibody,
L(PEG) in the N297 glycan represents $-NH-CH_2CH_2-(O-CH_2CH_2)_3-*$,
wherein the amino group at the left end is bound via an amide bond to carboxylic acid at the 2-position of a sialic acid at the non-reducing terminal of each or either one of the 1-3 and 1-6 branched chains of β-Man in the N297 glycan, and the asterisk * at the right end represents bonding to a nitrogen atom at the 1- or 3-position of the triazole ring in the corresponding structural formula.

**24.** An antibody-drug conjugate represented by the following formula:

[Formula 22]

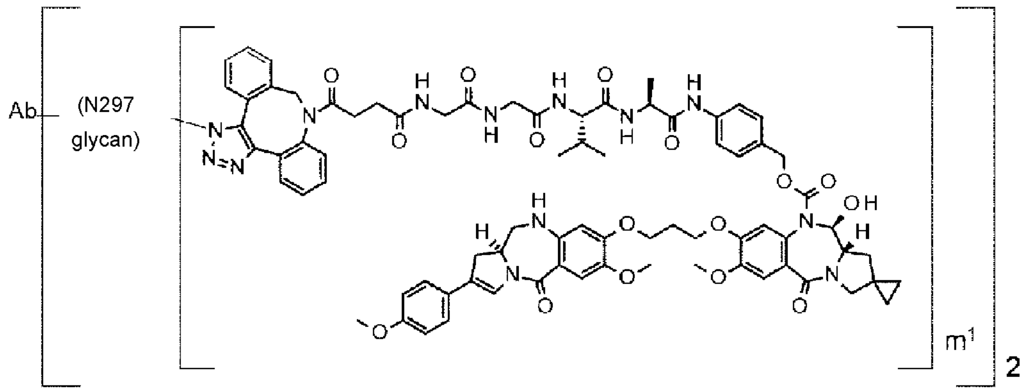

or

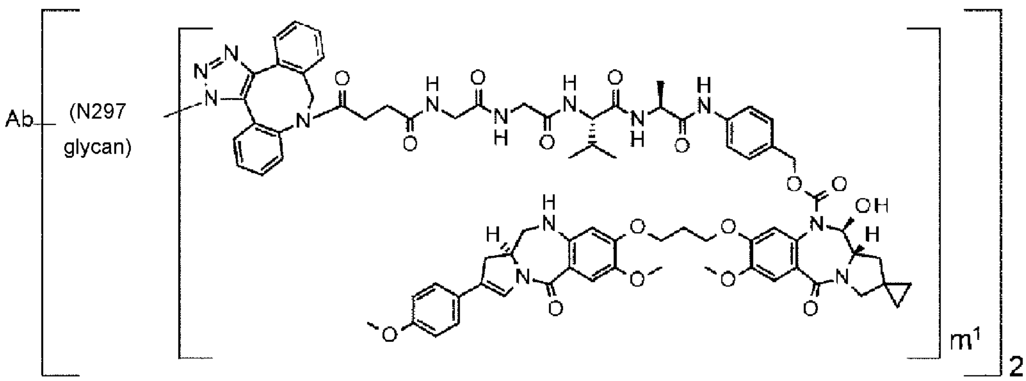

wherein, in each structural formula shown above,

$m^1$ represents an integer of 1 or 2;

Ab represents an antibody or a functional fragment of the antibody, wherein the antibody specifically binds to HER2 and comprises a heavy chain comprising CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 3 or an amino acid sequence having one to several amino acid substitutions in the amino acid sequence represented by SEQ ID NO: 3, and a light chain comprising CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 7 or an amino acid sequence having one to several amino acid substitutions in the amino acid sequence represented by SEQ ID NO: 7,

the N297 glycan is any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 23]

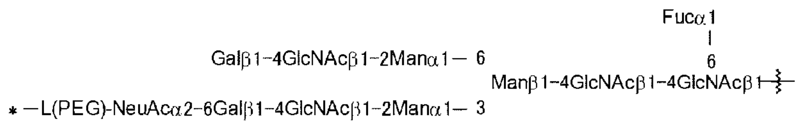

[N297-(Fuc)MSG1]

[Formula 24]

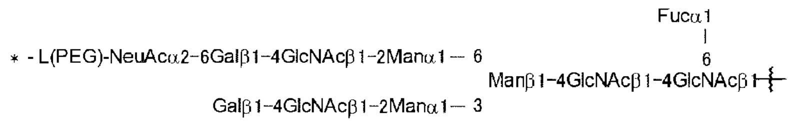

[N297-(Fuc)MSG2]

[Formula 25]

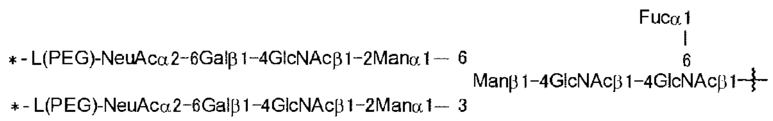

[N297-(Fuc)SG]

wherein

each wavy line represents bonding to Asn297 of the antibody,
L(PEG) in the N297 glycan represents $-NH-CH_2CH_2-(O-CH_2CH_2)_3-*$,
wherein the amino group at the left end is bound via an amide bond to carboxylic acid at the 2-position of a sialic acid at the non-reducing terminal of each or either one of the 1-3 and 1-6 branched chains of β-Man in the N297 glycan, and the asterisk * at the right end represents bonding to a nitrogen atom at the 1- or 3-position of the triazole ring in the corresponding structural formula.

**25.** An antibody-drug conjugate represented by the following formula:

[Formula 26]

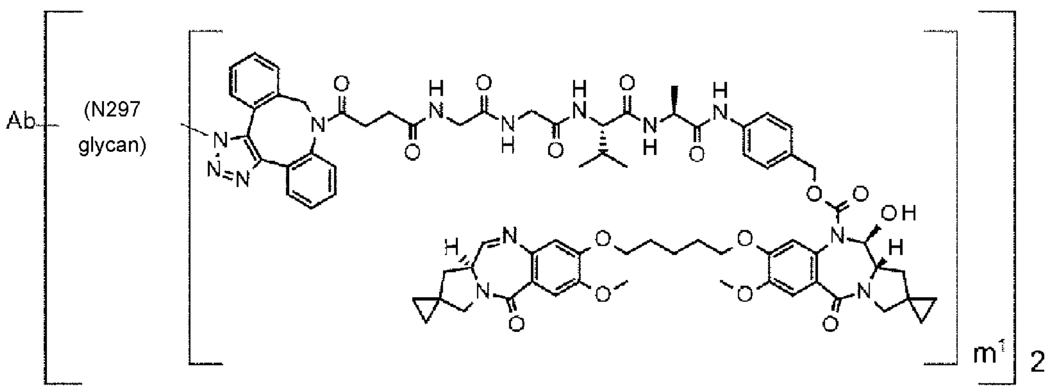

or

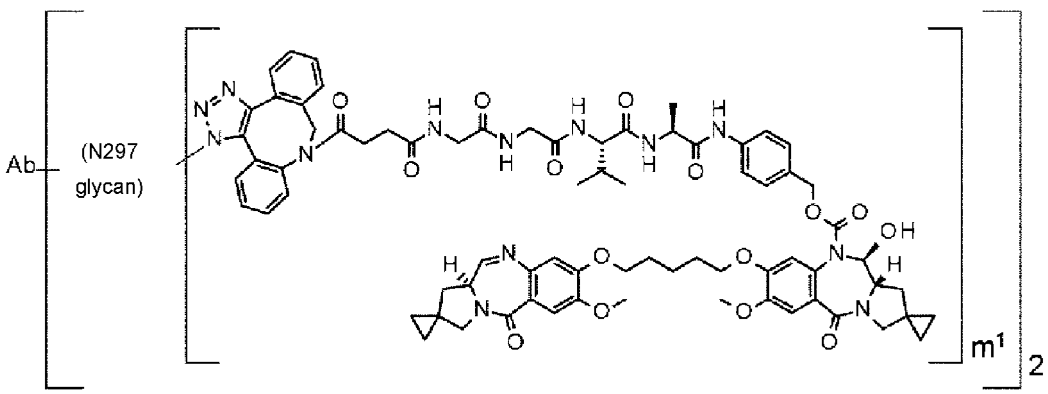

wherein, in each structural formula shown above,

$m^1$ represents an integer of 1 or 2;

Ab represents an antibody or a functional fragment of the antibody, wherein the antibody specifically binds to HER2 and comprises a heavy chain comprising CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 3 or an amino acid sequence having one to several amino acid substitutions in the amino acid sequence represented by SEQ ID NO: 3, and a light chain comprising CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 7 or an amino acid sequence having one to several amino acid substitutions in the amino acid sequence represented by SEQ ID NO: 7,

the N297 glycan is any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 27]

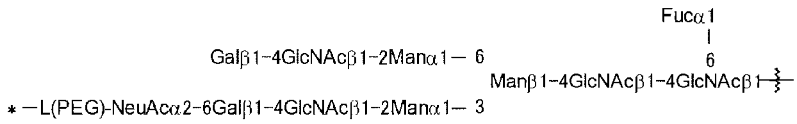

[N297-(Fuc)MSG1]

[Formula 28]

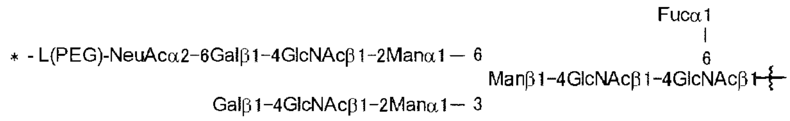

[N297-(Fuc)MSG2]

[Formula 29]

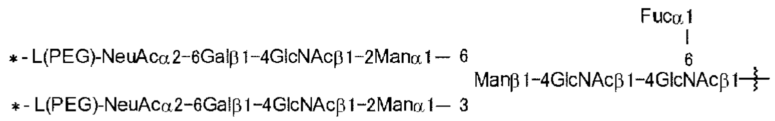

[N297-(Fuc)SG]

wherein

each wavy line represents bonding to Asn297 of the antibody,

L(PEG) in the N297 glycan represents $-NH-CH_2CH_2-(O-CH_2CH_2)_3-*$,

wherein the amino group at the left end is bound via an amide bond to carboxylic acid at the 2-position of a sialic acid at the non-reducing terminal of each or either one of the 1-3 and 1-6 branched chains of β-Man in the N297 glycan, and the asterisk * at the right end represents bonding to a nitrogen atom at the 1- or 3-position of the triazole ring in the corresponding structural formula.

**26.** The antibody-drug conjugate according to any one of claims 22 to 25, wherein the antibody comprises a heavy chain comprising CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 4, and a light chain comprising CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 8.

**27.** The antibody-drug conjugate according to any one of claims 22 to 25, wherein the antibody comprises a heavy chain comprising CDRH1 consisting of an amino acid sequence represented by SEQ ID NO: 1, CDRH2 consisting of an

amino acid sequence represented by SEQ ID NO: 2, and CDRH3 consisting of an amino acid sequence represented by SEQ ID NO: 3, and a light chain comprising CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 8.

**28.** The antibody-drug conjugate according to any one of claims 22 to 25, wherein the antibody comprises a heavy chain comprising CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 3, and a light chain comprising CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 7.

**29.** The antibody-drug conjugate according to any one of claims 22 to 26, wherein the antibody comprises a heavy chain variable region consisting of an amino acid sequence represented by SEQ ID NO: 17 and a light chain variable region consisting of an amino acid sequence represented by SEQ ID NO: 25.

**30.** The antibody-drug conjugate according to any one of claims 22 to 25 and 27, wherein the antibody comprises a heavy chain variable region consisting of an amino acid sequence represented by SEQ ID NO: 13 and a light chain variable region consisting of an amino acid sequence represented by SEQ ID NO: 29.

**31.** The antibody-drug conjugate according to any one of claims 22 to 25 and 28, wherein the antibody comprises a heavy chain variable region consisting of an amino acid sequence represented by SEQ ID NO: 13 and a light chain variable region consisting of an amino acid sequence represented by SEQ ID NO: 21.

**32.** The antibody-drug conjugate according to any one of claims 22 to 26 and 29, wherein the antibody comprises a heavy chain consisting of an amino acid sequence consisting of amino acid residues 20 to 469 of SEQ ID NO: 15 and a light chain consisting of an amino acid sequence consisting of amino acid residues 21 to 234 of SEQ ID NO: 23.

**33.** The antibody-drug conjugate according to any one of claims 22 to 25, 27, and 30, wherein the antibody comprises a heavy chain consisting of an amino acid sequence consisting of amino acid residues 20 to 469 of SEQ ID NO: 11 and a light chain consisting of an amino acid sequence consisting of amino acid residues 21 to 234 of SEQ ID NO: 27.

**34.** The antibody-drug conjugate according to any one of claims 22 to 25, 28, and 31, wherein the antibody comprises a heavy chain consisting of an amino acid sequence consisting of amino acid residues 20 to 469 of SEQ ID NO: 11 and a light chain consisting of an amino acid sequence consisting of amino acid residues 21 to 234 of SEQ ID NO: 19.

**35.** The antibody-drug conjugate according to any one of claims 22 to 25, 28, and 31, wherein the antibody comprises a heavy chain consisting of an amino acid sequence consisting of amino acid residues 20 to 469 of SEQ ID NO: 31 and a light chain consisting of an amino acid sequence consisting of amino acid residues 21 to 234 of SEQ ID NO: 32.

**36.** The antibody-drug conjugate according to any one of claims 22 to 35, wherein the antibody comprises one or two or more modifications selected from the group consisting of N-linked glycosylation, O-linked glycosylation, N-terminal processing, C-terminal processing, deamidation, isomerization of aspartic acid, oxidation of methionine, addition of a methionine residue at an N terminus, amidation of a proline residue, and deletion of one or two amino acid residues at the carboxyl terminus of a heavy chain.

**37.** An antibody or a functional fragment of the antibody, wherein the antibody specifically binds to HER2 and comprises a heavy chain comprising CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 3 or an amino acid sequence having one to several amino acid substitutions in the amino acid sequence represented by SEQ ID NO: 3, and a light chain comprising CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 7 or an amino acid sequence having one to several amino acid substitutions in the amino acid sequence represented by SEQ ID NO: 7.

**38.** The antibody according to claim 37 or a functional fragment of the antibody, the antibody comprising a heavy chain comprising CDRH1, CDRH2, and CDRH3 and a light chain comprising CDRL1, CDRL2, and CDRL3 as described

in any one of the following (a) and (b):

(a) CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 4, and CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 8; and
(b) CDRH1 consisting of an amino acid sequence represented by SEQ ID NO: 1, CDRH2 consisting of an amino acid sequence represented by SEQ ID NO: 2, and CDRH3 consisting of an amino acid sequence represented by SEQ ID NO: 3, and CDRL1 consisting of an amino acid sequence represented by SEQ ID NO: 5, CDRL2 consisting of an amino acid sequence consisting of amino acid residues 1 to 3 of SEQ ID NO: 6, and CDRL3 consisting of an amino acid sequence represented by SEQ ID NO: 8.

**39.** The antibody according to claim 37 or 38 or a functional fragment of the antibody, the antibody comprising a heavy chain variable region consisting of an amino acid sequence selected from the group consisting of the following (a) to (e) and a light chain variable region consisting of an amino acid sequence selected from the group consisting of the following (f) to (k):

(a) an amino acid sequence represented by SEQ ID NO: 13;
(b) an amino acid sequence represented by SEQ ID NO: 17;
(c) an amino acid sequence with a homology of at least 95% or higher to a sequence of a framework region excluding CDR sequences in any one of the sequences (a) and (b);
(d) an amino acid sequence having one to several amino acid deletions, substitutions, or additions in a sequence of a framework region excluding CDR sequences in any one of the sequences (a) and (b);
(e) an amino acid sequence represented by SEQ ID NO: 25;
(f) an amino acid sequence represented by SEQ ID NO: 29;
(g) an amino acid sequence with a homology of at least 95% or higher to a sequence of a framework region excluding CDR sequences in any of the sequences (e) and (f); and
(h) an amino acid sequence having one to several amino acid deletions, substitutions, or additions in a sequence of a framework region excluding CDR sequences in any of the sequences (e) and (f).

**40.** The antibody according to claim 39 or a functional fragment of the antibody, the antibody comprising a heavy chain variable region and a light chain variable region as described in any one of the following (a) and (b):

(a) a heavy chain variable region consisting of an amino acid sequence represented by SEQ ID NO: 17 and a light chain variable region consisting of an amino acid sequence represented by SEQ ID NO: 25; and
(b) a heavy chain variable region consisting of an amino acid sequence represented by SEQ ID NO: 13 and a light chain variable region consisting of an amino acid sequence represented by SEQ ID NO: 29.

**41.** The antibody according to any one of claims 37 to 40 or a functional fragment of the antibody, the antibody being a chimeric antibody.

**42.** The antibody according to any one of claims 37 to 40 or a functional fragment of the antibody, the antibody being a humanized antibody.

**43.** The antibody according to claim 41 or 42 or a functional fragment of the antibody, the antibody comprising a heavy chain constant region of human IgG1, human IgG2, or human IgG4.

**44.** The antibody according to claim 43 or a functional fragment of the antibody, wherein the heavy chain constant region is a heavy chain constant region of human IgG1, and leucine at the 234- and 235-positions specified by EU Index numbering in the heavy chain constant region is substituted with alanine.

**45.** The antibody according to any one of claims 42 to 44 or a functional fragment of the antibody, the antibody comprising a heavy chain and a light chain as described in the following (a) or (b):

(a) a heavy chain consisting of an amino acid sequence consisting of amino acid residues 20 to 469 of SEQ ID NO: 15 and a light chain consisting of an amino acid sequence consisting of amino acid residues 21 to 234 of SEQ ID NO: 23 (H01L02); and

(b) a heavy chain consisting of an amino acid sequence consisting of amino acid residues 20 to 469 of SEQ ID NO: 11 and a light chain consisting of an amino acid sequence consisting of amino acid residues 21 to 234 of SEQ ID NO: 27 (HwtL05).

**46.** An antibody or a functional fragment of the antibody, wherein the antibody competes with the antibody according to any one of claims 37 to 45 for binding to HER2, or binds to a site of HER2 recognizable to the antibody according to any one of claims 37 to 45.

**47.** A polynucleotide encoding the antibody according to any one of claims 37 to 46 or a functional fragment of the antibody.

**48.** An expression vector comprising the polynucleotide according to claim 47.

**49.** A host cell transformed with the expression vector according to claim 48.

**50.** The host cell according to claim 49, wherein the host cell is a eukaryotic cell.

**51.** The host cell according to claim 49 or 50, wherein the host cell is an animal cell.

**52.** A method for producing the antibody according to any one of claims 37 to 46 or a functional fragment of the antibody, the method comprising the steps of: culturing the host cell according to any one of claims 49 to 51; and collecting a targeted antibody from the culture obtained in the step of culturing.

**53.** An antibody obtained by using the method according to claim 52, or a functional fragment of the antibody.

**54.** The antibody according to any one of claims 37 to 46 and 53 or a functional fragment of the antibody, the antibody comprising one or two or more modifications selected from the group consisting of N-linked glycosylation, O-linked glycosylation, N-terminal processing, C-terminal processing, deamidation, isomerization of aspartic acid, oxidation of methionine, addition of a methionine residue at an N terminus, amidation of a proline residue, and deletion of one or two amino acid residues at the carboxyl terminus of a heavy chain.

**55.** The antibody according to claim 54 or a functional fragment of the antibody, wherein one or several amino acid residues are deleted at the carboxyl terminus of a heavy chain.

**56.** The antibody according to claim 55 or a functional fragment of the antibody, wherein one amino acid residue is deleted at the carboxyl terminus of each of the two heavy chains.

**57.** The antibody according to any one of claims 53 to 56 or a functional fragment of the antibody, wherein a proline residue at the carboxyl terminus of a heavy chain is further amidated.

**58.** A method for producing a glycan-remodeled antibody, the method comprising the steps of:

i) culturing the host cell according to any one of claims 49 to 51 and collecting a targeted antibody from the culture obtained;
ii) treating the antibody obtained in step i) with hydrolase to produce a (Fucα1,6)GlcNAc-antibody; and
iii) reacting the (Fucα1,6)GlcNAc-antibody with a glycan donor molecule in the presence of transglycosidase, the glycan donor molecule obtained by introducing a PEG linker having an azide group to the carbonyl group of carboxylic acid at the 2-position of a sialic acid in MSG (9) or SG (10) and oxazolinating the reducing terminal.

**59.** The method according to claim 58, further comprising the step of purifying the (Fucα1,6)GlcNAc-antibody through purification of a reaction solution in step ii) with a hydroxyapatite column.

**60.** A glycan-remodeled antibody obtained by using the method according to claim 58 or 59.

**61.** A method for producing the antibody-drug conjugate according to any one of claims 1 to 36, the method comprising a step of reacting the glycan-remodeled antibody according to claim 60 and a drug-linker.

**62.** An antibody-drug conjugate obtained by using the method according to claim 61.

**63.** The antibody-drug conjugate according to any one of claims 1 to 36, wherein the antibody is the antibody according to any one of claims 53 to 57 and 60.

**64.** The antibody-drug conjugate according to any one of claims 1 to 36, 62, and 63, wherein the N297 glycan is N297-(Fuc)MSG1.

**65.** The antibody-drug conjugate according to any one of claims 1 to 36 and 62 to 64, wherein $m^1$ is an integer of 1.

**66.** The antibody-drug conjugate according to any one of claims 1 to 36 and 62 to 65, wherein the average number of conjugated drug molecules per antibody molecule in the antibody-drug conjugate is 1 to 3 or 3 to 5.

**67.** A pharmaceutical composition comprising the antibody-drug conjugate according to any one of claims 1 to 36 and 62 to 66, or the antibody according to any one of claims 37 to 47, 53 to 57, and 60 or a functional fragment of the antibody.

**68.** The pharmaceutical composition according to claim 67, being an antitumor drug.

**69.** The pharmaceutical composition according to claim 68, wherein the tumor is expressing HER2.

**70.** A method for treating a tumor, wherein the antibody-drug conjugate according to any one of claims 1 to 36 and 62 to 66, or the antibody according to any one of claims 37 to 47, 53 to 57, and 60 or a functional fragment of the antibody is administered to an individual.

**71.** The method for treating a tumor according to claim 70, wherein the tumor is expressing HER2.

**72.** A method for treating a tumor, wherein a pharmaceutical composition comprising the antibody-drug conjugate according to any one of claims 1 to 36 and 62 to 66, or the antibody according to any one of claims 37 to 47, 53 to 57, and 60 or a functional fragment of the antibody, and at least one antitumor drug are administered to an individual simultaneously, separately, or consecutively.

**73.** The antibody according to any one of claims 37 to 47, 53 to 57, and 60 or a functional fragment of the antibody, wherein the antibody is conjugated to an additional compound.

[Figure 1]

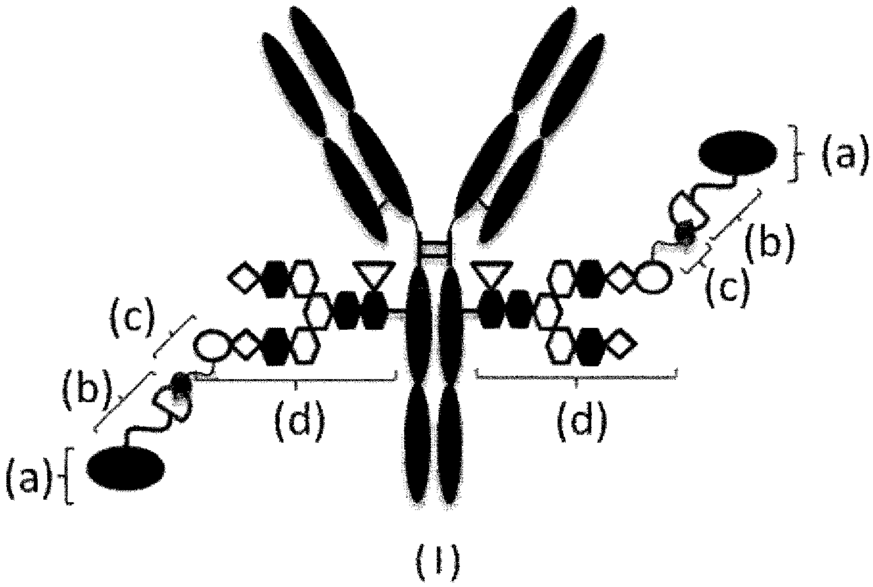


[Figure 2]

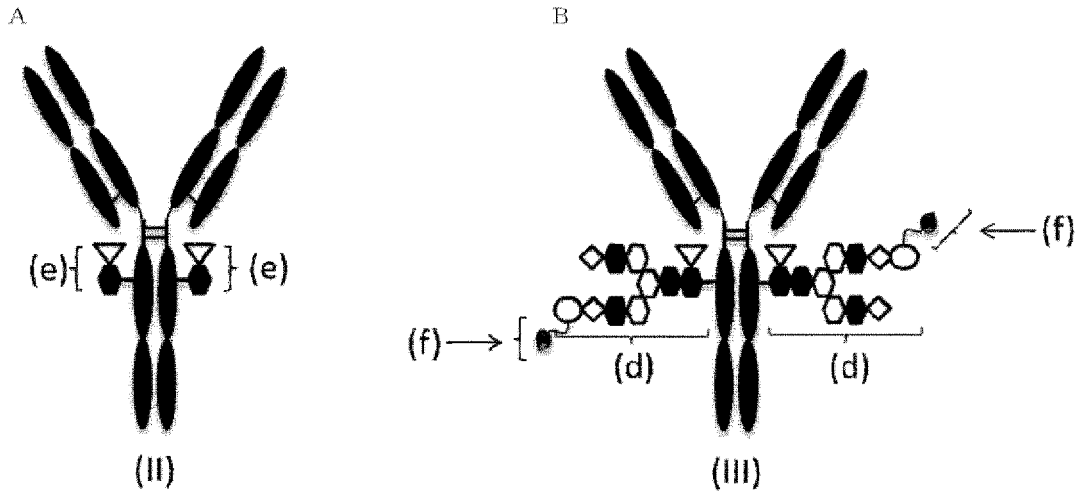

[Figure 3]

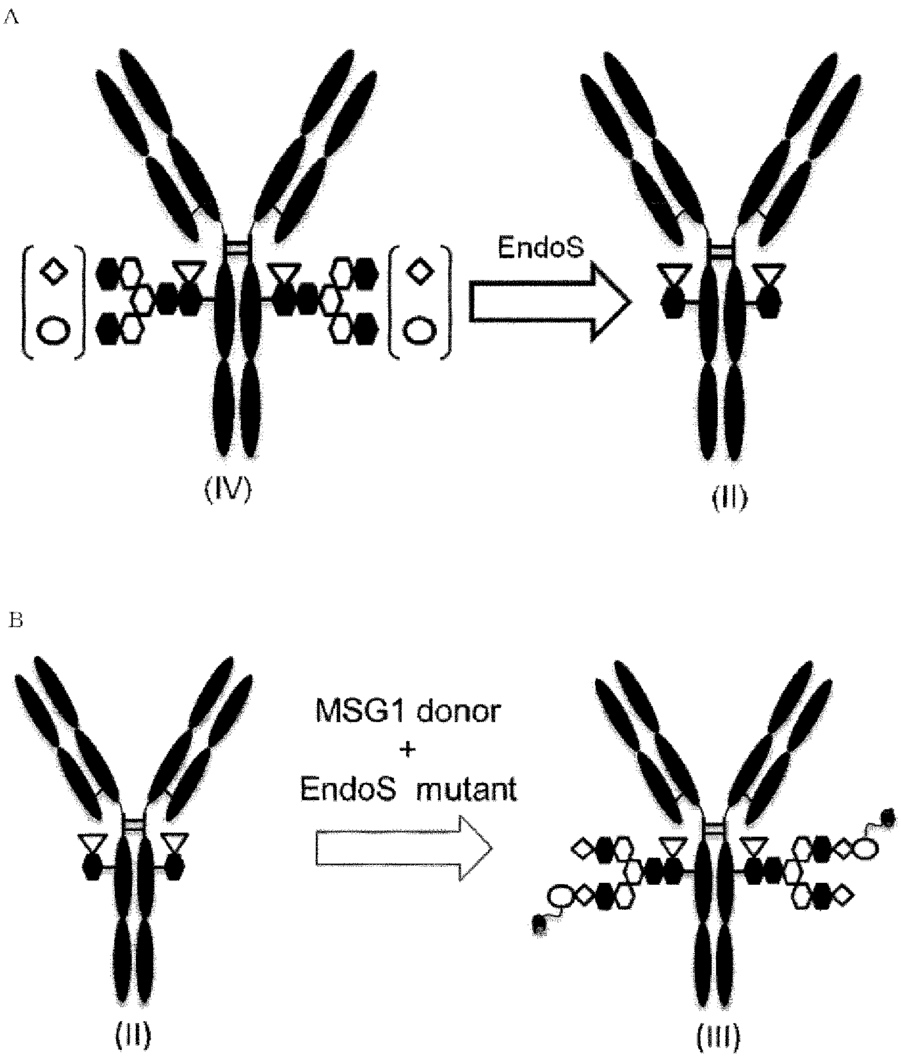


[Figure 4]

**Amino acid sequence of CDRH1 of heavy chain of Trastuzumab A1, A2, and HwtL05 antibody (SEQ ID NO: 1)**

GFNIKDTY

**Amino acid sequence of CDRH2 of heavy chain of Trastuzumab A1, A2, and HwtL05 antibody (SEQ ID NO: 2)**

IYPTNGYT

**Amino acid sequence of CDRH3 of heavy chain of Trastuzumab A1, A2, and HwtL05 antibody (SEQ ID NO: 3)**

SRWGGDGFYAMDY

[Figure 5]

**Amino acid sequence of CDRH1 of H01L02 antibody heavy chain (SEQ ID NO: 1)**

GFNIKDTY

**Amino acid sequence of CDRH2 of H01L02 antibody heavy chain (SEQ ID NO: 2)**

IYPTNGYT

**Amino acid sequence of CDRH3 of H01L02 antibody heavy chain (SEQ ID NO: 4)**

SRWGGDGFFAMDY

[Figure 6]

**Amino acid sequence of CDRL1 of light chain of Trastuzumab A1 and A2 (SEQ ID NO: 5)**

QDVNTA

**Amino acid sequence of CDRL2 of light chain of Trastuzumab A1 and A2 (amino acid sequence consisting of amino acid residues 1 to 3 of SEQ ID NO: 6)**

SASFLYS

**Amino acid sequence of CDRL3 of light chain of Trastuzumab A1 and A2 (SEQ ID NO: 7)**

QQHYTTPPT

[Figure 7]

**Amino acid sequence of CDRL1 of H01L02 antibody light chain and HwtL05 antibody light chain (SEQ ID NO: 5)**

QDVNTA

**Amino acid sequence of CDRL2 of H01L02 antibody light chain and HwtL05 antibody light chain (amino acid sequence consisting of amino acid residues 1 to 3 of SEQ ID NO: 6)**

SASFLYS

**Amino acid sequence of CDRL3 of H01L02 antibody light chain and HwtL05 antibody light chain (SEQ ID NO: 8)**

QQHATTPPT

[Figure 8]

**Amino acid sequence of heavy chain of Trastuzumab A1 (SEQ ID NO: 11)**

MKHLWFFLLLVAAPRWVLSEVQLVESGGGLVQPGGSLRLSCAASGFNIKDTYIHWVRQAPGKGLEWVARIYP

TNGYTRYADSVKGRFTISADTSKNTAYLQMNSLRAEDTAVYYCSRWGGDGFYAMDYWGQGTLVTVSSASTKG

PSVFPLAPSSKSTSGGTAALGCLVKDYFPEPVTVSWNSGALTSGVHTFPAVLQSSGLYSLSSVVTVPSSSLG

TQTYICNVNHKPSNTKVDKRVEPKSCDKTHTCPPCPAPEAAGGPSVFLFPPKPKDTLMISRTPEVTCVVVDV

SHEDPEVKFNWYVDGVEVHNAKTKPREEQYNSTYRVVSVLTVLHQDWLNGKEYKCKVSNKALPAPIEKTISK

AKGQPREPQVYTLPPSREEMTKNQVSLTCLVKGFYPSDIAVEWESNGQPENNYKTTPPVLDSDGSFFLYSKL

TVDKSRWQQGNVFSCSVMHEALHNHYTQKSLSLSPGK

[Figure 9]

**Amino acid sequence of heavy chain variable region of Trastuzumab A1, A2, and HwtL05 antibody(SEQ ID NO: 13)**

EVQLVESGGGLVQPGGSLRLSCAAS<u>GFNIKDTY</u>IHWVRQAPGKGLEWVAR<u>IYPTNGYT</u>RYADSVKGRFTISA

DTSKNTAYLQMNSLRAEDTAVYYC<u>SRWGGDGFYAMDY</u>WGQGTLVTVSS

[Figure 10]

**Amino acid sequence of heavy chain of H01L02 antibody (SEQ ID NO: 15)**

MKHLWFFLLLVAAPRWVLSEVQLVESGGGLVQPGGSLRLSCAASGFNIKDTYIHWVRQAPGKGLEWVARIYP

TNGYTRYADSVKGRFTISADTSKNTAYLQMNSLRAEDTAVYYCSRWGGDGFFAMDYWGQGTLVTVSSASTKG

PSVFPLAPSSKSTSGGTAALGCLVKDYFPEPVTVSWNSGALTSGVHTFPAVLQSSGLYSLSSVVTVPSSSLG

TQTYICNVNHKPSNTKVDKRVEPKSCDKTHTCPPCPAPEAAGGPSVFLFPPKPKDTLMISRTPEVTCVVVDV

SHEDPEVKFNWYVDGVEVHNAKTKPREEQYNSTYRVVSVLTVLHQDWLNGKEYKCKVSNKALPAPIEKTISK

AKGQPREPQVYTLPPSREEMTKNQVSLTCLVKGFYPSDIAVEWESNGQPENNYKTTPPVLDSDGSFFLYSKL

TVDKSRWQQGNVFSCSVMHEALHNHYTQKSLSLSPGK

[Figure 11]

**Amino acid sequence of heavy chain variable region of H01L02 antibody (SEQ ID NO: 17)**

EVQLVESGGGLVQPGGSLRLSCAAS<u>GFNIKDTY</u>IHWVRQAPGKGLEWVAR<u>IYPTNGYT</u>RYADSVKGRFTISA

DTSKNTAYLQMNSLRAEDTAVYYC<u>SRWGGDGFFAMDY</u>WGQGTLVTVSS

[Figure 12]

**Amino acid sequence of light chain of Trastuzumab A1 (SEQ ID NO: 19)**

MVLQTQVFISLLLWISGAYGDIQMTQSPSSLSASVGDRVTITCRASQDVNTAVAWYQQKPGKAPKLLIYSAS

FLYSGVPSRFSGSRSGTDFTLTISSLQPEDFATYYCQQHYTTPPTFGQGTKVEIKRTVAAPSVFIFPPSDEQ

LKSGTASVVCLLNNFYPREAKVQWKVDNALQSGNSQESVTEQDSKDSTYSLSSTLTLSKADYEKHKVYACEV

THQGLSSPVTKSFNRGEC

[Figure 13]

**Amino acid sequence of light chain variable region of Trastuzumab A1 and A2 (SEQ ID NO: 21)**

DIQMTQSPSSLSASVGDRVTITCRASQDVNTAVAWYQQKPGKAPKLLIYSASFLYSGVPSRFSGSRSGTDFT

LTISSLQPEDFATYYCQQHYTTPPTFGQGTKVEIK

[Figure 14]

**Amino acid sequence of light chain of H01L02 antibody (SEQ ID NO: 23)**

MVLQTQVFISLLLWISGAYGDIQMTQSPSSLSASVGDRVTITCRASQDVNTAVAWYQQKPGKAPKLLIYSAS

FLYSGVPSRFSGSRSGTDFTLTISSLQPEDFATYYCQQHATTPPTFGQGTKVEIKRTVAAPSVFIFPPSDEQ

LKSGTASVVCLLNNFYPREAKVQWKVDNALQSGNSQESVTEQDSKDSTYSLSSTLTLSKADYEKHKVYACEV

THQGLSSPVTKSFNRGEC

[Figure 15]

**Amino acid sequence of light chain variable region of H01L02 antibody (SEQ ID NO: 25)**

DIQMTQSPSSLSASVGDRVTITCRASQDVNTAVAWYQQKPGKAPKLLIYSASFLYSGVPSRFSGSRSGTDFT

LTISSLQPEDFATYYCQQHATTPPTFGQGTKVEIK

[Figure 16]

**Amino acid sequence of light chain of HwtL05 antibody (SEQ ID NO: 27)**

MVLQTQVFISLLLWISGAYGDIQMTQSPSSLSASVGDRVTITCRASQDVNTAVAWYQQKPGKAPKALIYSAS

FLYSGVPSRFSGSRSGTDFTLTISSLQPEDFATYYCQQHATTPPTFGQGTKVEIKRTVAAPSVFIFPPSDEQ

LKSGTASVVCLLNNFYPREAKVQWKVDNALQSGNSQESVTEQDSKDSTYSLSSTLTLSKADYEKHKVYACEV

THQGLSSPVTKSFNRGEC

[Figure 17]

**Amino acid sequence of light chain variable region of HwtL05 antibody (SEQ ID NO: 29)**

DIQMTQSPSSLSASVGDRVTITCRASQDVNTAVAWYQQKPGKAPKALIYSASFLYSGVPSRFSGSRSGTDFT

LTISSLQPEDFATYYCQQHATTPPTFGQGTKVEIK

[Figure 18]

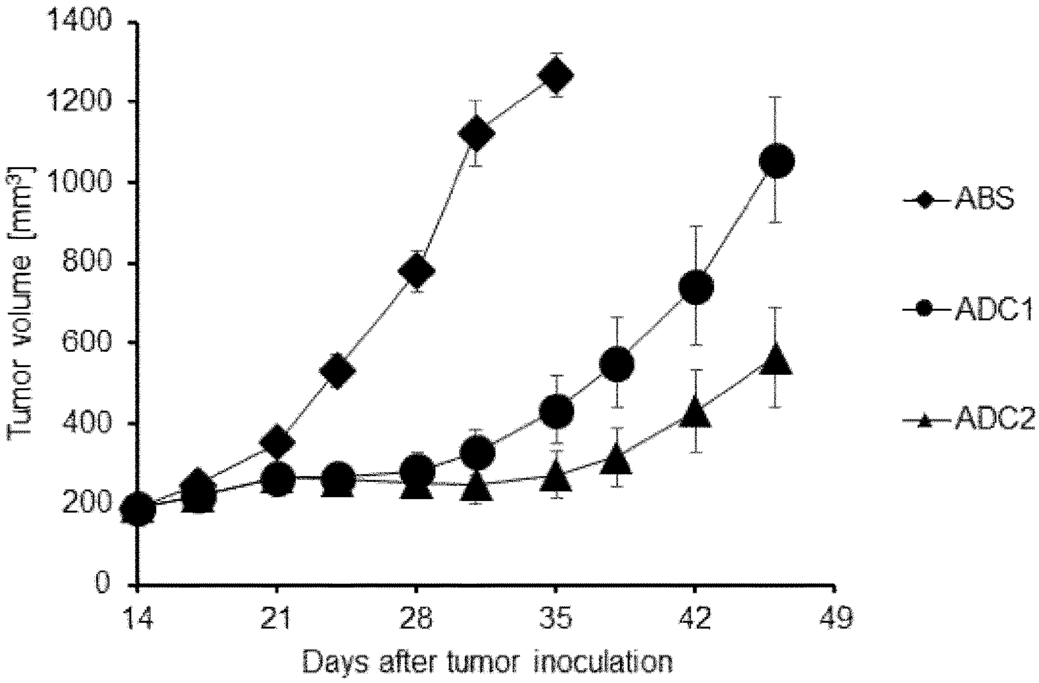
1400
1200
1000
800
600
400
200
0
Tumor volume [mm3]
14
21
28
35
42
49
Days after tumor inoculation
ABS
ADC1
ADC2

[Figure 19]

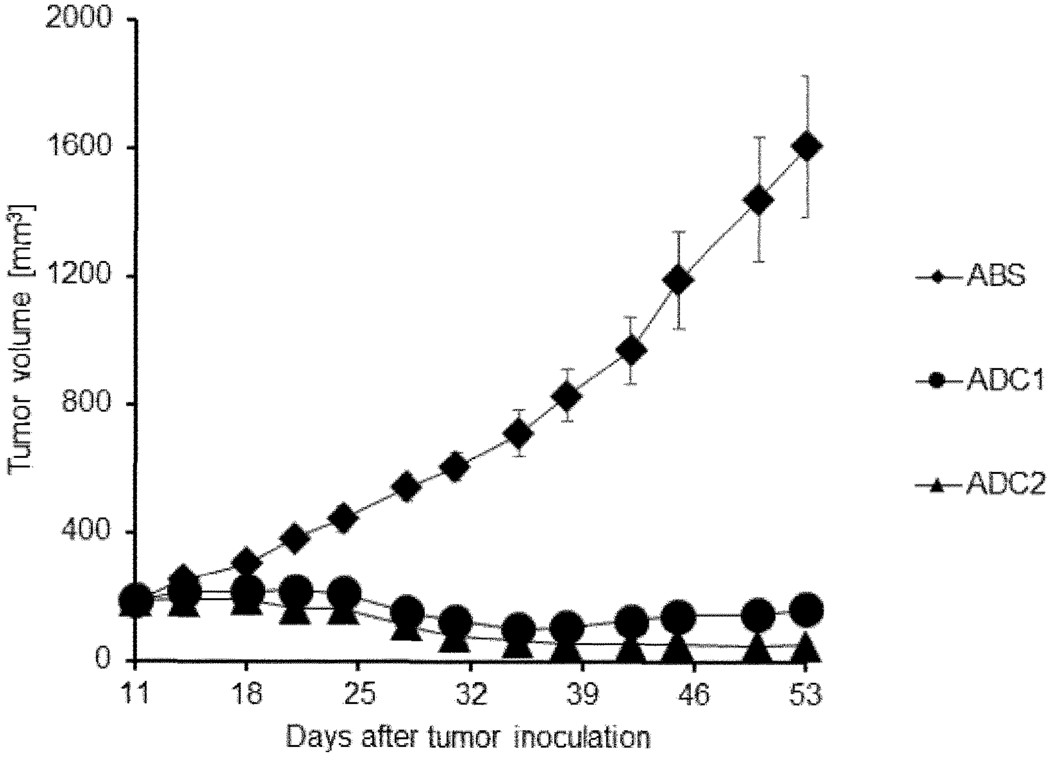


[Figure 20]

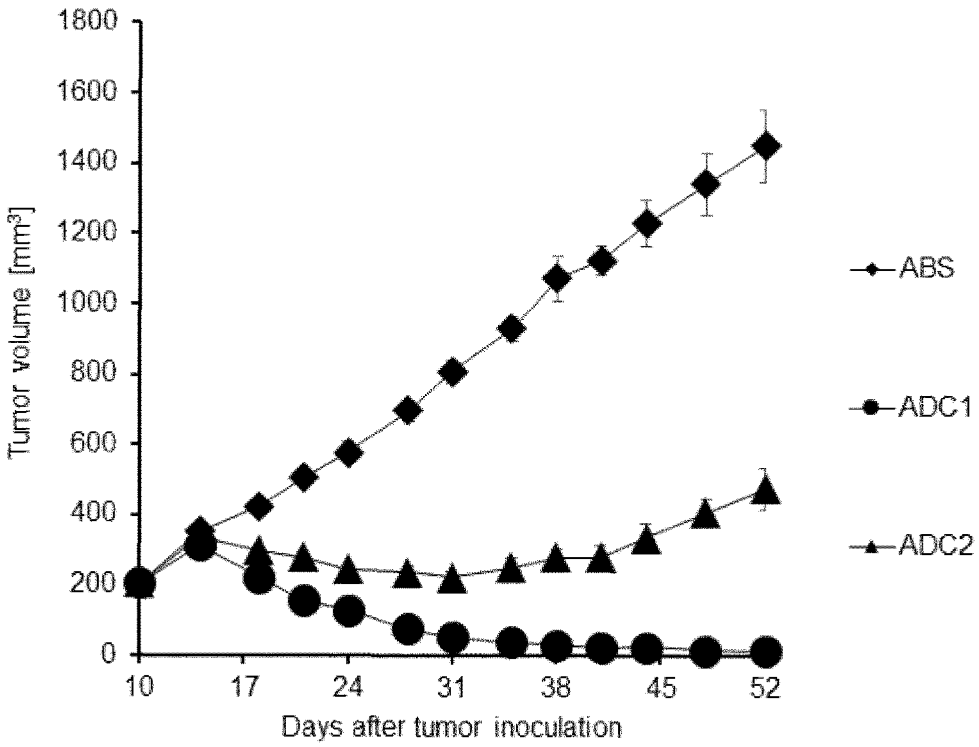

[Figure 21]

**Amino acid sequence of heavy chain of Trastuzumab A2 (SEQ ID NO: 31)**

MKHLWFFLLLVAAPRWVLSEVQLVESGGGLVQPGGSLRLSCAASGFNIKDTYIHWVRQAPGKGLEWVARIYP

TNGYTRYADSVKGRFTISADTSKNTAYLQMNSLRAEDTAVYYCSRWGGDGFYAMDYWGQGTLVTVSSASTKG

PSVFPLAPSSKSTSGGTAALGCLVKDYFPEPVTVSWNSGALTSGVHTFPAVLQSSGLYSLSSVVTVPSSSLG

TQTYICNVNHKPSNTKVDKKVEPKSCDKTHTCPPCPAPEAAGGPSVFLFPPKPKDTLMISRTPEVTCVVVD

VSHEDPEVKFNWYVDGVEVHNAKTKPREEQYNSTYRVVSVLTVLHQDWLNGKEYKCKVSNKALPAPIEKTIS

KAKGQPREPQVYTLPPSREEMTKNQVSLTCLVKGFYPSDIAVEWESNGQPENNYKTTPPVLDSDGSFFLYSK

LTVDKSRWQQGNVFSCSVMHEALHNHYTQKSLSLSPGK

**Signal sequence (1 - 19), Heavy chain variable region (20 - 120), Constant region (121 - 469)**

[Figure 22]

**Amino acid sequence of light chain of Trastuzumab A2 (SEQ ID NO: 32)**

MVLQTQVFISLLLWISGAYGDIQMTQSPSSLSASVGDRVTITCRASQDVNTAVAWYQQKPGKAPKLLIYSAS

FLYSGVPSRFSGSRSGTDFTLTISSLQPEDFATYYCQQHYTTPPTFGQGTKVEIKRTVAAPSVFIFPPSDEQ

LKSGTASVVCLLNNFYPREAKVQWKVDNALQSGNSQESVTEQDSKDSTYSLSSTLTLSKADYEKHKVYACEV

THQGLSSPVTKSFNRGEC

**Signal sequence (1 - 20), Heavy chain variable region (21 - 127), Constant region (128 - 234)**

[Figure 23]

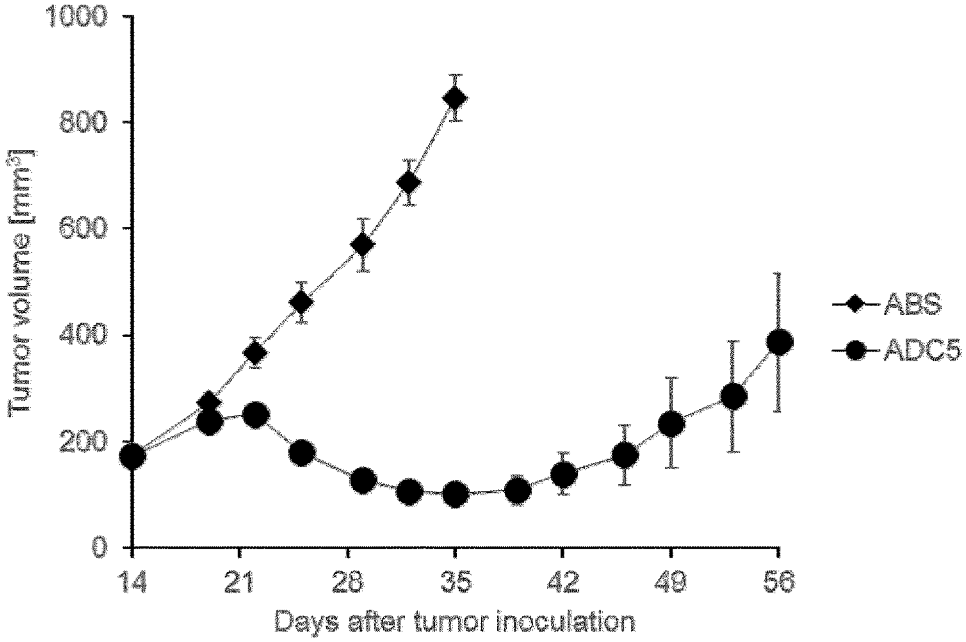

# INTERNATIONAL SEARCH REPORT

International application No.
PCT/JP2020/012885

A. CLASSIFICATION OF SUBJECT MATTER

A61K 39/395(2006.01)i; A61K 45/00(2006.01)i; A61P 35/00(2006.01)i; C07K 1/22(2006.01)i; C07K 7/06(2006.01)i; C07K 7/08(2006.01)i; C07K 16/28(2006.01)i; C07K 16/46(22006.01)i; C12N 5/10(2006.01)i; A61K 47/68(2017.01)i; C12N 15/13(2006.01)i; C12N 15/63(2006.01)i; A61K 31/4192(2006.01)i; A61K 31/551(2006.01)i; C12P 21/08(2006.01)i

FI: A61K47/68; A61K45/00; A61P35/00; A61K39/395 L; A61K39/395 T; C12N15/13; C12N15/63 Z; C12N5/10; C07K1/22; C12P21/08; C07K16/46; C07K16/28; C07K7/06; C07K7/08; A61K31/4192; A61K31/551 ZNA

According to International Patent Classification (IPC) or to both national classification and IPC

B. FIELDS SEARCHED

Minimum documentation searched (classification system followed by classification symbols)

A61K39/395; A61K45/00; A61P35/00; C07K1/22; C07K7/06; C07K7/08; C07K16/28; C07K16/46; C12N5/10; A61K47/68; C12N15/13; C12N15/63; A61K31/4192; A61K31/551; C12P21/08

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2012/075581 A1 (YM BIOSCIENCES INC.) 14.06.2012 (2012-06-14) tables 2-5, examples, SEQ ID NO: 1-6, 20, examples, page 8, paragraphs [0002]-[0003] | 37-38, 46-57<br>1-73 |
| Y | WO 2011/084496 A1 (ABBOTT BIOTHERAPEUTICS CORP.) 14.07.2011 (2011-07-14) table 1, page 32, SEQ ID NO: 692, page 59, SEQ ID NO: 1009, page 63, SEQ ID NO: 1112 | 1-73 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 June 2020 (04.06.2020) | 23 June 2020 (23.06.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No. PCT/JP2020/012885

C (Continuation). DOCUMENTS CONSIDERED TO BE RELEVANT

| Category* | Citation of document, with indication, where appropriate, of the relevant passages | Relevant to claim No. |
|---|---|---|
| Y | TSUCHIKAMA, Kyoji et al., "Antibody-drug conjugates: recent advances in conjugation and linker chemistries", Protein&Cell, 2018, vol. 9(1), pp. 33-46, ISSN: 1674-800X in particular, fig. 2, 6 | 1-73 |
| Y | JP 2015-534996 A (SYNAFFIX B.V.) 07.12.2015 (2015-12-07) claims, paragraph [0184] | 1-73 |
| Y | US 2009/0149449 A1 (THE UNITED STATES OF AMERICA, AS REPRESENTED BY THE SECRETARY, DEPT. OF HEALTH AND HUMAN SERVICE, et al.) 11.06.2009 (2009-06-11) claim 1, paragraph [0034] | 1-36, 61-72 |
| Y | JP 2017-526614 A (X-RX, INC.) 14.09.2017 (2017-09-14) paragraph [0132] | 1-36, 61-72 |
| Y | WO 2017/137556 A1 (ADC THERAPEUTICS SA) 17.08.2017 (2017-08-17) claims, pp. 32, 33 | 1-36, 61-72 |
| P, X | WO 2019/065964 A1 (DAIICHI SANKYO COMPANY, LIMITED) 04.04.2019 (2019-04-04) examples 85, 92, 115, 120, claims 60-61 | 1-36 |

Form PCT/ISA/210 (continuation of second sheet) (January 2015)

**INTERNATIONAL SEARCH REPORT**
Information on patent family members

International application No.
PCT/JP2020/012885

| Patent Documents referred in the Report | Publication Date | Patent Family | Publication Date |
|---|---|---|---|
| WO 2012/075581 A1 | 14 Jun. 2012 | US 2013/0266564 A1 | |
| WO 2011/084496 A1 | 14 Jul. 2011 | EP 2513148 A | |
| JP 2015-534996 A | 07 Dec. 2015 | US 2015/0320882 A1 claims, paragraph [0232]<br>WO 2014/065661 A1<br>EP 2911699 A1<br>CN 105142672 A | |
| US 2009/0149449 A1 | 11 Jun. 2009 | WO 2005/040170 A2<br>EP 1675857 A2 | |
| JP 2017-526614 A | 14 Sep. 2017 | US 2017/0037007 A1 paragraph [0205]<br>WO 2015/175171 A1<br>EP 3134079 A1<br>CN 107205972 A | |
| WO 2017/137556 A1 | 17 Aug. 2017 | GB 201602363 D | |
| WO 2019/065964 A1 | 04 Apr. 2019 | TW 201922788 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013173496 A
- WO 2014130879 A
- WO 2017004330 A
- WO 2017004025 A
- WO 2017020972 A
- WO 2016036804 A
- WO 2015095124 A
- WO 2015052322 A
- WO 2015052534 A
- WO 2016115191 A
- WO 2015052321 A
- WO 2015031693 A
- WO 2011130613 A
- WO 2005040170 A
- WO 2017137556 A
- WO 9007861 A
- WO 2012075581 A
- WO 2011084496 A
- US 20180501692 A
- US 5821337 A
- WO 2013154206 A
- WO 9201047 A
- WO 9220791 A
- WO 9306213 A
- WO 9311236 A
- WO 9319172 A
- WO 9501438 A
- WO 9515388 A
- WO 199954342 A
- WO 200061739 A
- WO 200231140 A
- WO 2007133855 A
- WO 2013120066 A
- US 2016361436 A
- WO 20110278681 A
- WO 2017010559 A
- WO 2003038043 A
- WO 2012087596 A
- US 20150283262 A
- WO 2011130598 A
- WO 2016149546 A


### Non-patent literature cited in the description


- *Angewandte Chemie Internationl Edition,* 2016, vol. 55, 2-29
- *Chemical Reviews,* 2010, vol. 111, 2815-2864
- Antibiotics III. Springer Verlag, 3-11
- *Accounts of Chemical Research,* 1986, vol. 19, 230
- *Journal of the American Chemical Society,* 1992, vol. 114, 4939
- *Journal of Organic Chemistry,* 1996, vol. 61, 8141
- *Science,* 1985, vol. 230 (4730), 1132-1139
- *EMBO J.,* 1997, vol. 16, 1647-1655
- *EMBO J.,* 1996, vol. 15, 254-264
- *J Biom Chem.,* 1994, vol. 269, 14661-14665
- *Science,* 1987, vol. 237, 178-182
- *Proc Natl Acad Sci U S A.,* 1987, vol. 84, 7159-7163
- *Eur. J Surg Oncol.,* 1997, vol. 23, 30-35
- *Oncogene,* 2008, vol. 27 (47), 6120-6130
- *Oncol Rep.,* 2006, vol. 15 (1), 65-71
- *Science,* 1987, vol. 235, 177-182
- *Ann Oncol,* 2008, vol. 19, 1523-1529
- *Mol Cell Biol,* 1986, vol. 6, 955-958
- *Science,* 1989, vol. 244, 707-712
- *Diagn Mol Pathol,* 2001, vol. 10, 139-152
- *Cell Death and Differentiation,* 2008, vol. 15, 751-761
- *Molecular Biology of the Cell,* December 2004, vol. 15, 5268-5282
- *Bio Techniques,* January 2000, vol. 28, 162-165
- *Proc. Natl. Acad. Sci. U.S.A.,* 1984, vol. 81, 6851-6855
- *Nature,* 1986, vol. 321, 522-525
- **ALTSCHUL, STEPHEN F. ; THOMAS L.MADDEN ; ALEJANDRO A. SCHAAFFER ; JINGHUI ZHANG ; ZHENG ZHANG ; WEBB MILLER ; DAVID J.LIPMAN.** Gapped BLAST and PSI-BLAST: a new generation of protein database search programs. *Nucleic Acids Res.,* 1997, vol. 25, 3389-3402
- *Nature Genetics,* 1997, vol. 16, 133-143
- *Nucl. Acids Res.,* 1998, vol. 26, 3447-3448
- Animal Cell Technology: Basic and Applied Aspects. Kluwer Academic Publishers, 1999, vol. 10, 69-73
- *Proc. Natl. Acad. Sci. USA,* 2000, vol. 97, 722-727

- *Investigative Ophthalmology & Visual Science.,* 2002, vol. 43 (7), 2301-2308
- *Briefings in Functional Genomics and Proteomics,* 2002, vol. 1 (2), 189-203
- *Ophthalmology,* 2002, vol. 109 (3), 427-431
- *Nature Biotechnology,* 2005, vol. 23 (9), 1105-1116
- *Annu. Rev. Immunol,* 1994, vol. 12, 433-455
- *Pharmaceutical Development and Technology,* 2007, vol. 12, 265-273
- *Cell,* 1981, vol. 23, 175-182
- *Proc. Natl. Acad. Sci. U.S.A.,* 1980, vol. 77, 4126-4220
- *Journal of Chromatography A,* 1995, vol. 705, 129-134
- *Analytical Biochemistry,* 2007, vol. 360, 75-83
- *Proc. Natl. Acad. Sci. U.S.A.,* 15 May 1969, vol. 63 (1), 78-85
- Strategies for Protein Purification and Characterization: A Laboratory Course Manual. Cold Spring Harbor Laboratory Press, 1996
- Antibodies: A Laboratory Manual. Cold Spring Harbor Laboratory, 1988
- *ACS Chemical Biology,* 2012, vol. 7, 110
- *ACS Medicinal Chemistry Letters,* 2016, vol. 7, 1005
- *Bioconjugate Chemistry,* 2015, vol. 26, 2233
- *Angew. Chem. Int. Ed.,* 2016, vol. 55, 2361-2367
- *Biotechnol. Prog.,* 2012, vol. 28, 608-622
- *Anal. Chem.,* 2013, vol. 85, 715-736
- **EDELMAN et al.** *Proc. Natl. Acad. Sci. U.S.A.,* 15 May 1969, vol. 63 (1), 78-85
- *J. Org. Chem.,* 2009, vol. 74 (5), 2210-2212
- *JACS.,* 2012, vol. 134, 12308-12318
- *JACS.,* 2004, vol. 126, 15046-15047
- *Protein Science,* 1995, vol. 4, 2411-2423
- *Tetrahedron,* 1995, vol. 51, 5617
- *Tetrahedron Letters,* 2012, vol. 53, 3847
- **DR. JUNICHI KUREBAYASHI.** British Journal of Cancer. Kawasaki Medical School, 1999, vol. 79, 707-717